# EUROPEAN PATENT APPLICATION

(11) **EP 4 467 561 A1**
(43) Date of publication of application: **27.11.2024**
(21) Application number: 23740350.6
(22) Date of filing: 16.01.2023
(51) Int. Cl.: C07K 7/08, A61K 38/10, A61K 38/12, A61K 47/60, A61K 47/61, A61K 47/68, A61P 9/10, A61P 43/00, C07K 14/765, C07K 16/00, C07K 19/00, C12N 15/13

(54) **TSP1 INHIBITOR**

(30) Priority: 17.01.2022 JP 2022005132
(71) Applicant: Daiichi Sankyo Company, Limited, Tokyo 103-8426 (JP); PeptiDream Inc., Kawasaki-shi, Kanagawa 210-0821 (JP)
(72) Inventor: NOGAMI, Kagayaki, Tokyo 103-8426 (JP); YAMAGUCHI, Takahiro, Tokyo 103-8426 (JP); FURUKAWA, Akihiro, Tokyo 103-8426 (JP); SAITO, Hironao, Tokyo 103-8426 (JP); ISHIGAI, Yutaka, Tokyo 103-8426 (JP)
(74) Representative: Vossius & Partner Patentanwälte Rechtsanwälte mbB
(86) International application number: PCT/JP2023/000979
(87) International publication number: WO 2023/136342

(57) **Abstract**

The present invention provides a cyclic peptide represented by formula (I) 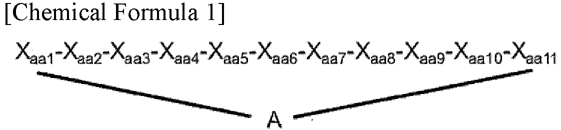 [wherein A is selected from the ring-forming groups A₁ to A₅; Xₐₐ₁ is a residue of an aromatic amino acid; Xₐₐ₂ is a residue of an aromatic amino acid, a basic amino acid, or an aliphatic amino acid; Xₐₐ₃ and Xₐₐ₈ each have a structure independently selected from a residue of an amino acid represented by formula (III) or (III') in which thiol groups of Xₐₐ₃ and Xₐₐ₈ form a bond; Xₐₐ₄ is a residue of a neutral amino acid; Xₐₐ₈ is a residue of a basic amino acid; Xₐₐ₆ is a residue of a neutral amino acid or an acidic amino acid; Xₐₐ₇ is a residue of an aromatic amino acid; Xₐₐ₉ is a residue of an aromatic amino acid, an aliphatic amino acid, or a basic amino acid; Xₐₐ₁₀ is a residue of an aromatic amino acid; and Xₐₐ₁₁ is a residue of an aliphatic amino acid], or a pharmaceutically acceptable salt thereof.

## Description

### TECHNICAL FIELD

The present invention relates to a cyclic peptide or pharmaceutically acceptable salt thereof, which can improve blood flow by inhibiting a function of thrombospondin 1 (TSP1) and is useful for the treatment or prophylaxis of diseases such as critical limb ischemia; a conjugate of the cyclic peptide having an improved pharmacokinetic-pharmacodynamic (PK-PD) profile; a medicament containing them as active components; and a method for producing the conjugate etc.

### BACKGROUND ART

Peripheral arterial disease (PAD) is a disease caused by atherosclerosis in blood vessels of the legs that reduces the flow of blood, which can lead to various symptoms such as pain. Severe PAD is referred to as critical limb ischemia (CLI), which accounts for 1 to 3% PAD patients. CLI is a condition in which pain at rest occurs due to the obstruction of blood flow to lower limbs and ulcer or necrosis occurs in the periphery of lower limbs. CLI is classified as stages III to IV and stages 4 to 6, respectively, according to the Fontaine and Rutherford classifications (NPL 1).

CLI has been considered problematic due to poor prognosis and high mortality, and studies have been made on a therapy in CLI patients, in which the progress of ulcer or necrosis of lower limbs is suppressed by remodeling the vascular system through revascularization in ischemic tissues and delivering oxygen and nutrients to the ischemic tissues (NPL 2). Various attempts have heretofore been made to apply a gene therapy using pro-angiogenic growth factors for CLI, but unlike in animal studies, no such therapy has been demonstrated to show efficacy in clinical trials. In light of such clinical trial results, another focus has been placed on angiostatic factors, proposing a hypothesis on the importance of the balance between pro-angiogenic and angiostatic factors (NPLs 3 and 4).

Chronic limb threatening ischemia (CLTI), which has been proposed in recent years, is a general term for diseases with risks of limb amputation, such as lower limb ischemia, tissue defects, neuropathy, and infections, which require therapeutic interventions. CLTI is a more comprehensive concept that includes not only ischemia but also other conditions, for example, diabetic foot. The severity of CLTI is assessed using the WIfI classification on a grading scale of 0 to 3 for the severity of "Wound; W", "Ischemia; I", and "foot Infection; fI", respectively (NPL 22).

Thrombospondin 1 (TSP1), a glycoprotein reported to act in an inhibitory manner on angiogenesis, is reported to be released from activated platelets and to be synthesized and secreted by numerous cell types, including endothelial cells, smooth myocytes, renal mesangial cells, tubular cells, and podocytes (NPLs 5 to 7). The expression of TSP 1 increases in the blood of peripheral vascular disease patients and in the lower-limb skeletal muscles of CLI patients, and this finding suggests involvement of TSP1 in the progress of ulcer or necrosis in lower limbs of CLI patients (NPLs 8 and 9). Since TSP1 limits blood flow recovery after hindlimb ischemia-reperfusion in rats, and TSP1-deficient mice show enhanced angiogenesis after hindlimb ischemia, it is considered that TSP1 has an inhibitory effect on the blood flow recovery after hindlimb ischemia (NPLs 10 and 11). Further, since TSP1-deficient mice show increased blood flow in wounded areas and increased reduction in wound size, it can be expected that inhibiting TSP1 function has a healing effect on recognized wounds in CLI patients (NPL 12).

TSP1 has not only angiostatic activity but also other pharmacological activities, and is considered to be involved in many diseases. TSP1, secreted from platelets, enhances the levels of PAI-1 in the fibrinolytic system and stimulates thrombogenesis (NPL 13). Obese, insulin-resistant patients show increased TSP1 levels, suggesting that TSP1 acts on macrophages to promote obesity-induced inflammatory responses and exacerbate insulin resistance (NPLs 14 and 15). TSP1 promotes renal podocyte apoptosis and induces podocyte dysfunction (NPL 16). TSP1 binds to signal-regulatory protein-α on non-phagocytic cells, activates NADPH oxidase, limits vasodilatation, and induces renal ischemia reperfusion injury (NPL 17). These results suggest the possibility that TSP 1 inhibition may ameliorate myocardial infarction, and inflammatory responses and insulin resistance in obese patients, as well as renal disorders including ischemia-reperfusion injury. Further, platelet-derived microparticles released by TSP1 induce vaso-occlusive crisis in patients with sickle cell disease (NPLs 18 and 19). Furthermore, TSP1 is reported to promote the migration and adhesion of monocytes, contributing to enhanced vascular inflammatory responses during aortic aneurysm development (NPL 20). It is reported that TSP1 may be involved in both the suppression and promotion of cancers through acting on the growth, adhesion and invasion of cancer cells (NPL 21).

Meanwhile, there are some antibodies (PTL 1) and polypeptides (PTL 2) known as antagonists for the binding of TSP1 to the CD47 receptor. The present applicant has also developed macrocyclic polypeptides that can bind to TSP1 to block the adhesion of cells such as vascular endothelial cells to TSP1.

### CITATION LIST

### PATENT LITERATURE

PTL 1: WO 2008/060785
PTL 2: US 2014/296477
PTL 3: WO 2018/052002

### NON PATENT LITERATURE

NPL 1: Kinlay S. Circ Cardiovasc Interv. 2016; 9: e001946.
NPL 2: Davies MG. Methodist Debakey Cardiovasc J. 2012; 8: 20-27.
NPL 3: Hellsten Y, Hoier B. Biochem. Soc. Trans. 2014; 42: 1616-1622.
NPL 4: Olfert IM. Microcirculation 2016; 23: 146-156.
NPL 5: Jimenez B, Volpert OV, Crawford SE, et al. Nat Med. 2000; 6: 41-48.
NPL 6: Henkin J, Volpert OV. Expert Opin Ther Targets. 2011; 15(12): 1369-1386.
NPL 7: Murphy-Ullrich JE, Iozzo RV. Matrix Biol. 2012; 31(3): 152-154.
NPL 8: Smadja DM, d'Audigier C, Bieche I, et al. Arterioscler Thromb Vasc Biol. 2011; 31: 551-559.
NPL 9: Favier J, Germain S, Emmerich J, et al. J Pathol 2005; 207: 358-366.
NPL 10: Isenberg JS, Romeo MJ, Abu-Asab M, et al. Circ Res. 2007; 100(5): 712-720.
NPL 11: Csanyi G, Yao M, Rodriguez AI, et al. Arterioscler Thromb Vasc Biol. 2012; 32: 2966-2973.
NPL 12: Soto-Pantoja DR, Shih HB, Maxhimer JB, et al. Matrix Biol. 2014; 37: 25-34.
NPL 13: Prakash P, Kulkami PP, Chauhan AK. Blood 2015; 125(2): 399-406.
NPL 14: Varma V, Yao-Borengasser A, Bodies AM, et al. Diabetes. 2008; 57(2): 432-439.
NPL 15: Li Y, Tong X, Rumala C, et al. PLoS One. 2011; 6(10): e26656.
NPL 16: Cui W, Maimaitiyiming H, Zhou Q, et al. Biochim Biophys Acta. 2015; 1852(7): 1323-1333.
NPL 17: Yao M, Rogers NM, Csanyi G, et al. J Am Soc Nephrol. 2014; 25(6): 1171-1186.
NPL 18: Hagag AA, Elmashad G, Abd El-Lateef AE. Mediterr J Hematol Infect Dis. 2014; 6(1): e2014044.
NPL 19: Camus SM, Gausseres B, Bonnin P, et al. Blood. 2012; 120(25): 5050-5058.
NPL 20: Liu Z, Morgan S, Ren J, et al. Circ Res. 2015; 117(2): 129-141.
NPL 21: Esemuede N, Lee T, Pierre-Paul D. et al., The role of thrombospondin-1 in human disease. J Surg Res. 2004;122(1):135-42.
NPL 22: Conte MS, Bradbury AW, et al.: Global vascular guidelines on the management of chronic limb-threatening ischemia. Eur J Vasc Endovasc Surg. 2019; 58: S1-S109 e133.

### SUMMARY OF INVENTION

### TECHNICAL PROBLEM

An object of the present invention is to provide novel compounds which can improve blood flow by inhibiting a function of TSP1 and are useful for the treatment or prophylaxis of diseases such as critical limb ischemia. Another object of the present invention is to provide compounds which can improve blood flow by inhibiting a function of TSP1 and have an improved PK-PD (pharmacokinetic-pharmacodynamic) profile, for example, compounds exhibiting excellent efficacy at a lower frequency of administration.

### SOLUTION TO PROBLEM

The present inventors have made intensive studies to achieve the aforementioned object, and as a result, found that cyclic peptides having particular amino acid sequences, which are made bicyclic by side chains of two amino acid residues among amino acid residues constituting the ring being coupled to each other (bicyclic peptides), bind to TSP 1 to block the adhesion of cells to TSP1, and thereby can improve blood flow by inhibiting a function of TSP1; and have further found that compounds which can improve blood flow by inhibiting a function of TSP1 are conjugated with Fc-containing molecules, and thereby can improve blood flow at a lower frequency of administration. The present inventors have conducted additional studies based on these findings, thus completing the present invention.

More specifically, the present invention provides the following.
[1] A cyclic peptide represented by formula (I) 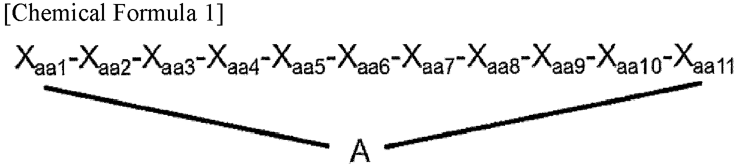 [wherein A is selected from a ring-forming groups of the formulas: 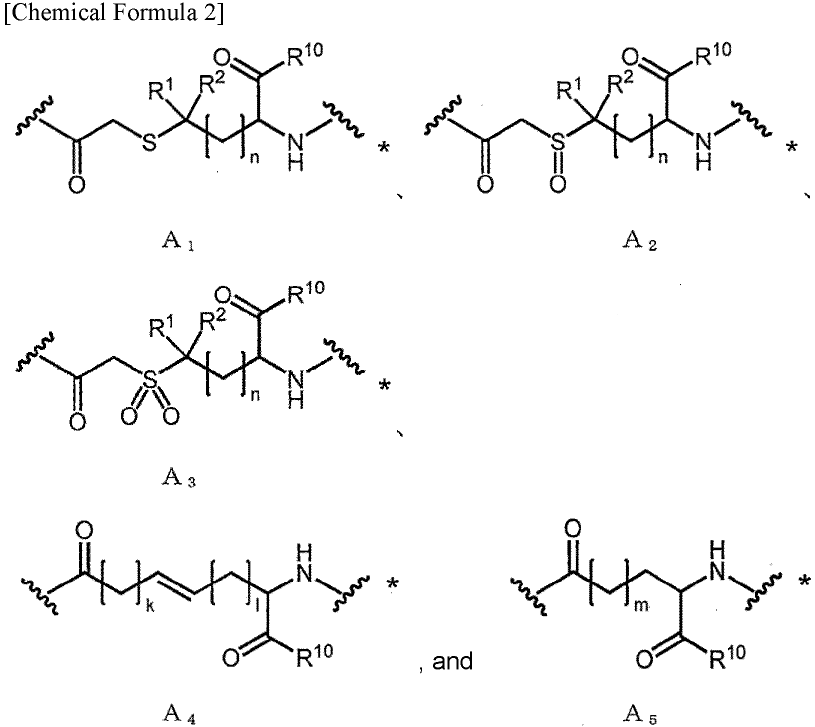 wherein 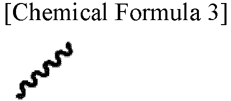
   represents a point of attachment to the N-terminal amino group of Xₐₐ₁, 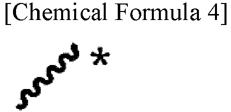
   represents a point of attachment to the C-terminal carbonyl group of Xₐₐ₁₁,
   R¹ and R² are each independently a hydrogen atom or C₁₋₃ alkyl,
   R¹⁰ is an amino group or a hydroxy group,
   n is an integer of 0 to 3 (e.g., 0, 1, 2, or 3),
   k and l are each independently an integer of 0 to 3 (e.g., 0, 1, 2, or 3),
   m is an integer of 1 to 7 *(e.g.,* 1, 2, 3, 4, 5, 6, or 7);
   Xₐₐ₁ is a residue of an aromatic amino acid;
   Xₐₐ₂ is a residue of an aromatic amino acid, a basic amino acid, or an aliphatic amino acid;
   Xₐₐ₃ and Xₐₐ₈ each have a structure independently selected from a residue of an amino acid represented by formula (III) 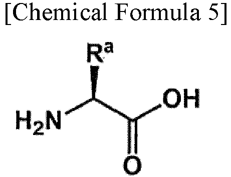
   [wherein R^{a} is a group represented by the formula -(CH₂)ₜSH (wherein t is an integer of 1 to 3 (e.g., 1, 2, or 3)] or formula (III') 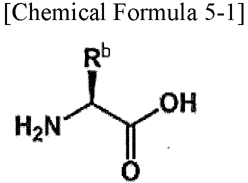
   [wherein R^{b} is a group represented by the formula -CHᵥ(CH₃)₂₋ᵥCH_{w}(CH₃)_{2-w}SH (wherein v is an integer of 0 to 2; when v is 0 or 1, w is 2; and when v is 2, w is 0 or 1) or - CHₓ(CH₃)₂₋ₓSH (wherein x is 0 or 1)], in which thiol groups of Xₐₐ₃ and Xₐₐ₈ form a bond being represented by -S-S-, -S-CH₂-S-, or -S-X-S- (wherein X is selected from 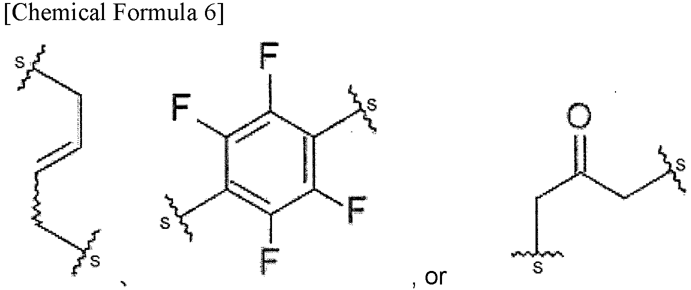 (wherein 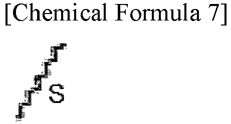 represents a point of attachment to S)), and at least one of Xₐₐ₃ and Xₐₐ₈ is a residue of the amino acid represented by formula (III);
   Xₐₐ₄ is a residue of a neutral amino acid;
   Xₐₐ₅ is a residue of a basic amino acid;
   Xₐₐ₆ is a residue of a neutral amino acid or an acidic amino acid;
   Xₐₐ₇ is a residue of an aromatic amino acid;
   Xₐₐ₉ is a residue of an aromatic amino acid, an aliphatic amino acid, or a basic amino acid;
   Xₐₐ₁₀ is a residue of an aromatic amino acid;
   Xₐₐ₁₁ is a residue of an aliphatic amino acid;
   wherein the aliphatic amino acid is an amino acid represented by formula (IIa) 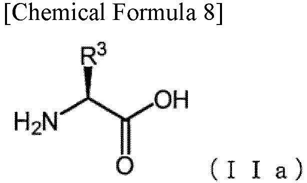
   (wherein R³ is C₁₋₆ alkyl, C₂₋₆ alkenyl or C₃₋₆ cycloalkyl);
   the aromatic amino acid is an amino acid represented by formula (IIb) 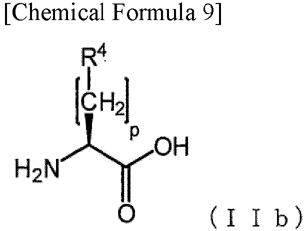
   (wherein R⁴ is an aromatic group selected from phenyl, thienyl, pyridyl, naphthyl, indolyl, benzofuranyl, and benzothienyl, wherein the aromatic group may be substituted with one or more substituents independently selected from the group consisting of C₁₋₃ alkyl, halogen atoms, hydroxy, and C₁₋₃ alkoxy; and p is an integer of 0 to 3 (e.g., 0, 1, 2, or 3));
   the basic amino acid is an amino acid represented by formula (IIc) 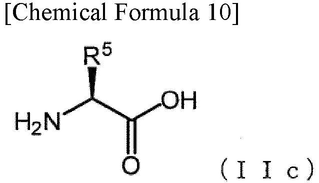
   [wherein R⁵ is a group represented by
      the formula -(CH₂)_{qa}NH₂ (wherein qa is an integer of 1 to 6 (e.g., 1, 2, 3, 4, 5, or 6)), the formula 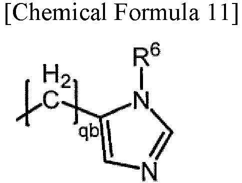
   (wherein R⁶ is a hydrogen atom or C₁₋₃ alkyl, and qb is an integer of 1 to 6 (e.g., 1, 2, 3, 4, 5, or 6)),
      the formula -(CH₂)_{qc}NHC(=NH)NH₂ (wherein qc is an integer of 1 to 6 (e.g., 1, 2, 3, 4, 5, or 6)), or
      the formula 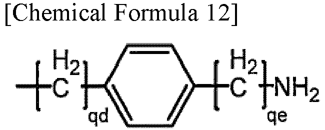
   (wherein qd and qe are each independently an integer of 1 to 3 (*e.g*., 1, 2, or 3))];
   the neutral amino acid is an amino acid represented by formula (IId) 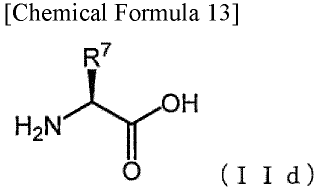
   [wherein R⁷ is a group represented by the formula -(CH₂)ᵣₐNHCONH₂ (wherein ra is an integer of 1 to 6 (*e.g*., 1, 2, 3, 4, 5, or 6)) or the formula -(CH₂)_{rb}SH (wherein rb is an integer of 1 to 3 (*e.g.,* 1, 2, or 3))], Gly, Met, Pro, 3HyP, Asn, Gln, Ser, ^{m}S, MS, or Thr;
   the acidic amino acid is an amino acid represented by formula (IIe) 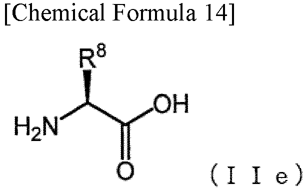
   [wherein R⁸ is a group represented by the formula -(CH₂)ₛCOOH (wherein s is an integer of 1 to 6 (e.g., 1, 2, 3, 4, 5, or 6))], or
      a pharmaceutically acceptable salt thereof.
[2] The cyclic peptide or pharmaceutically acceptable salt thereof as set forth in [1], wherein
   Xₐₐ₁ is a 2Nal residue;
   Xₐₐ₂ is a residue of Arg, AGB, PHG, or Tle;
   in the structure of Xₐₐ₃ and Xₐₐ₈, the residue of an amino acid represented by formula (III) or (III') is a residue of HCY or Cys;
   Xₐₐ₄ is a Gly residue;
   Xₐₐ₅ is a residue of Arg or AGB;
   Xₐₐ₆ is a residue of Asn or Asp;
   Xₐₐ₇ is a Trp residue;
   Xₐₐ₉ is a residue of Val, Arg, PHG, 4PAL, or AGB;
   Xₐₐ₁₀ is a Trp residue; and
   Xₐₐ₁₁ is a residue of Val, CPTG, or CHXG.
[3] The cyclic peptide or pharmaceutically acceptable salt thereof as set forth in [1] or [2], wherein A is a ring-forming group 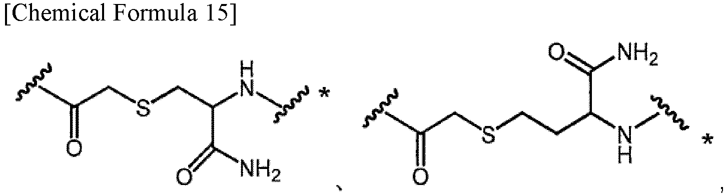 or 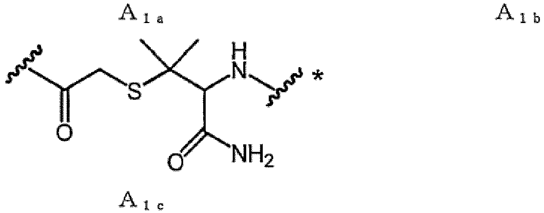
[4] The cyclic peptide or pharmaceutically acceptable salt thereof as set forth in [3], wherein A is a ring-forming group 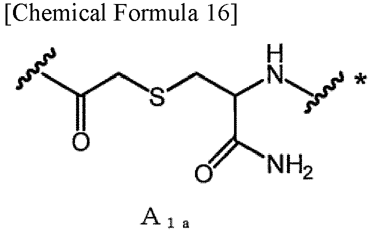
[5] The cyclic peptide or pharmaceutically acceptable salt thereof as set forth in any one of [1] to [4], wherein in the structure of Xₐₐ₃ and Xₐₐ₈, the thiol groups form the bond represented by -S-S- or -S-CH₂-S-.
[6] The cyclic peptide or pharmaceutically acceptable salt thereof as set forth in any one of [1] to [5], wherein the cyclic peptide or pharmaceutically acceptable salt thereof is selected from the group consisting of compounds represented by the formulas: 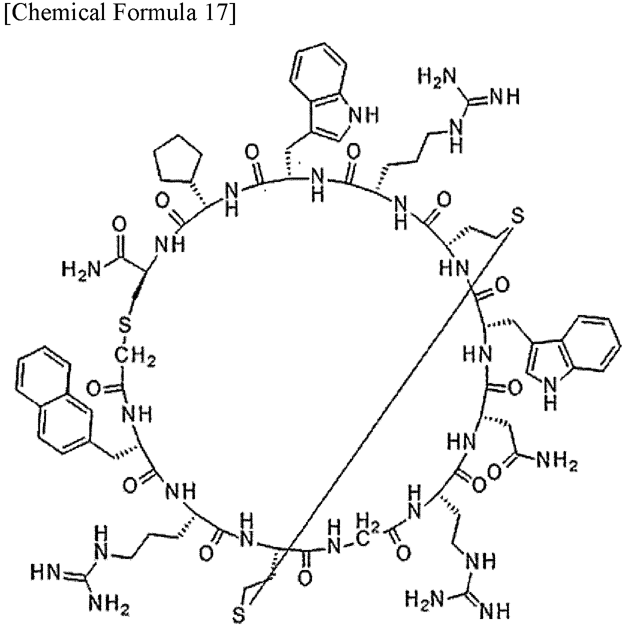 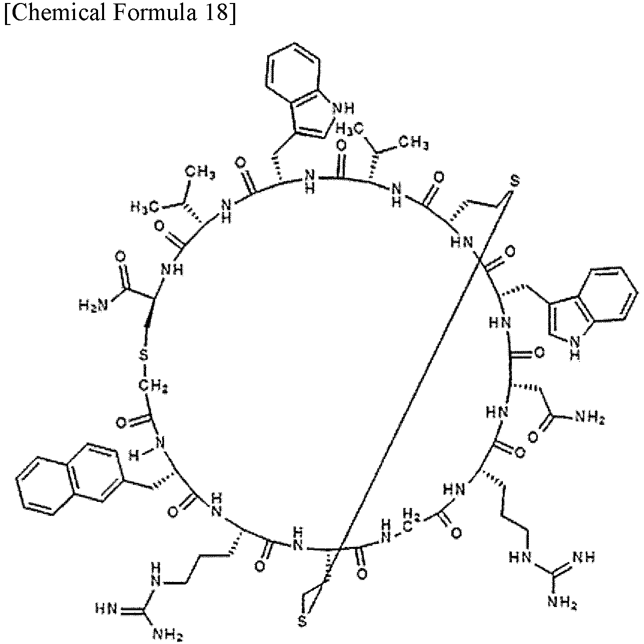 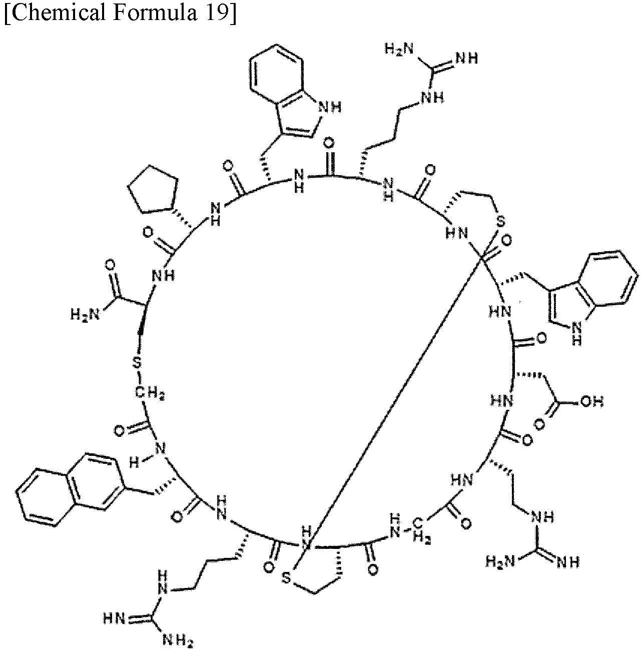 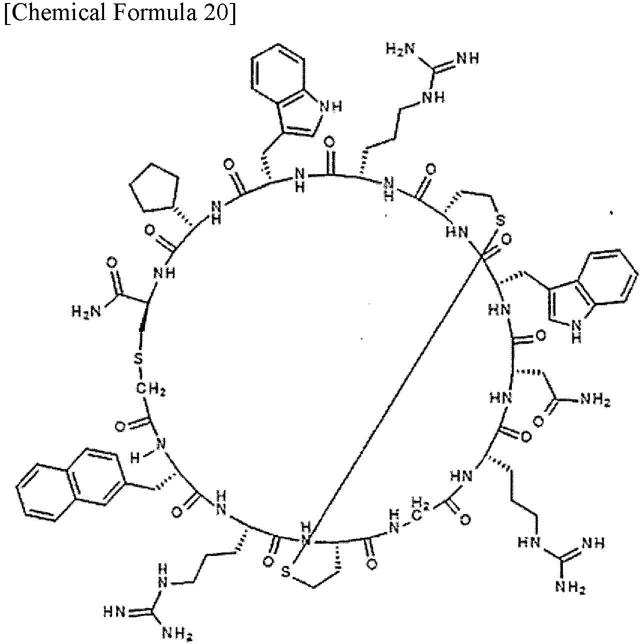 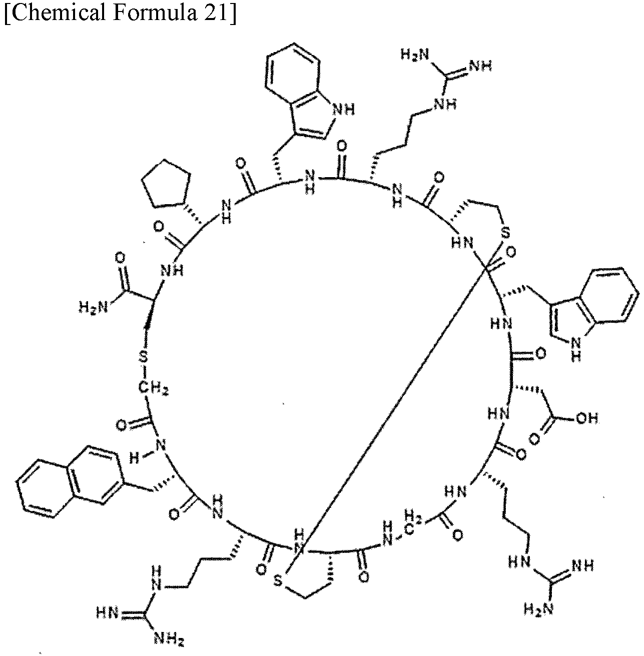 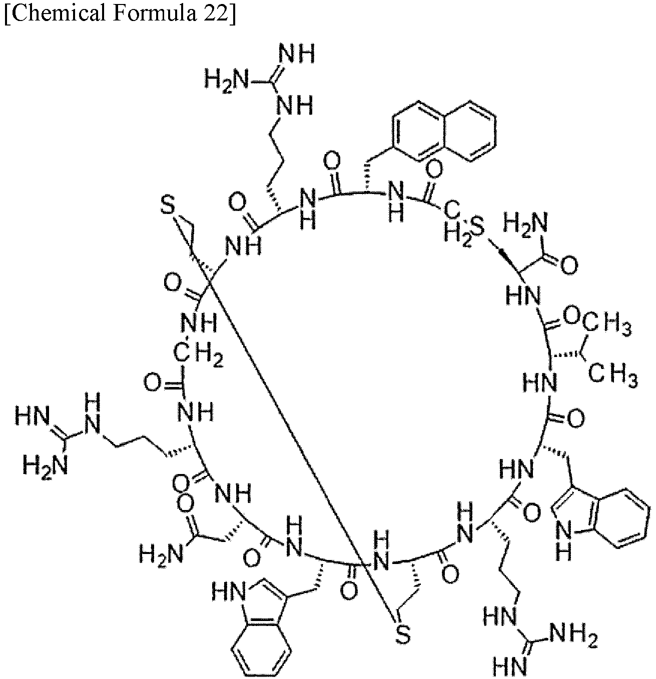 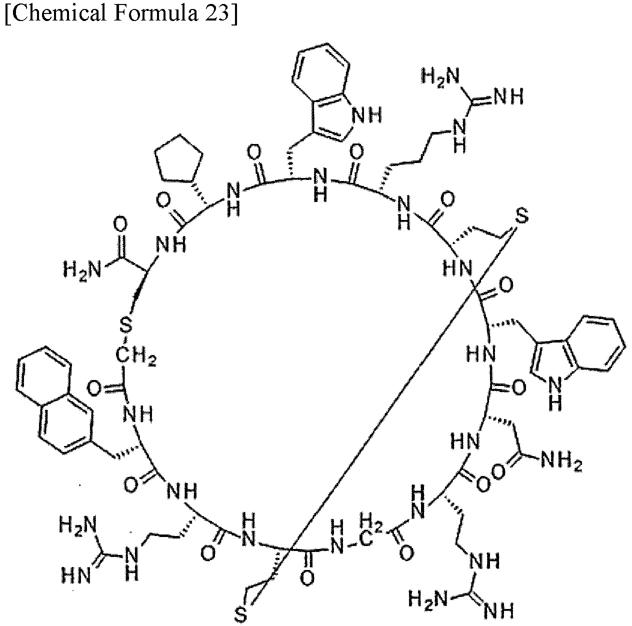 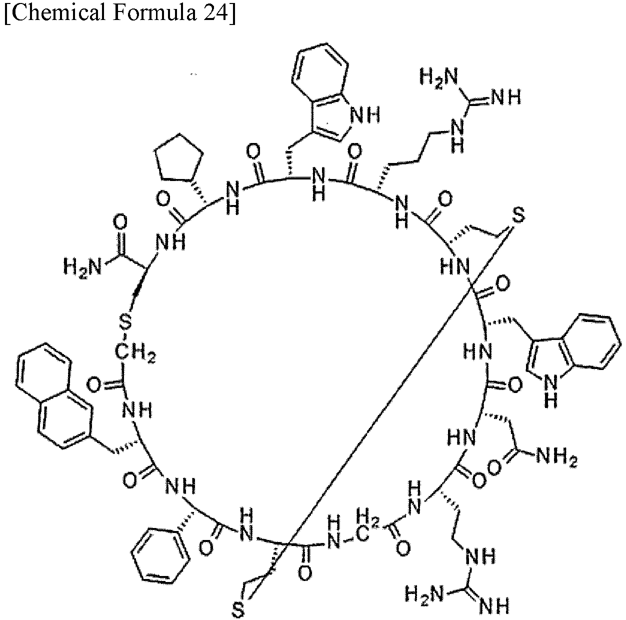 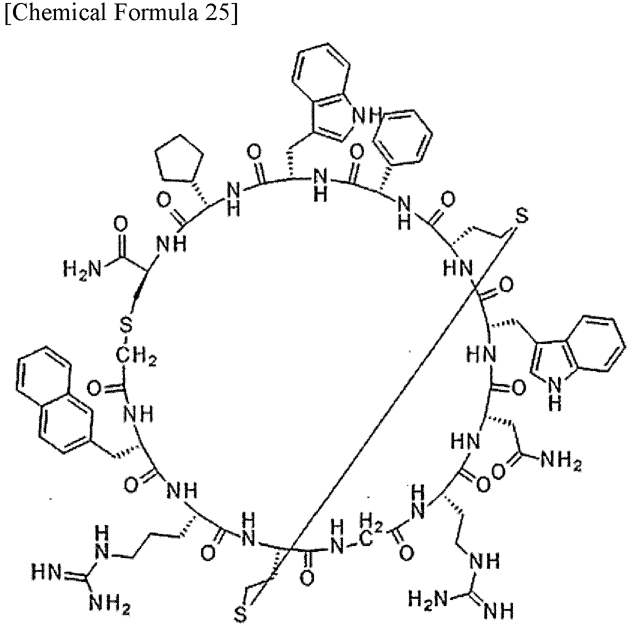 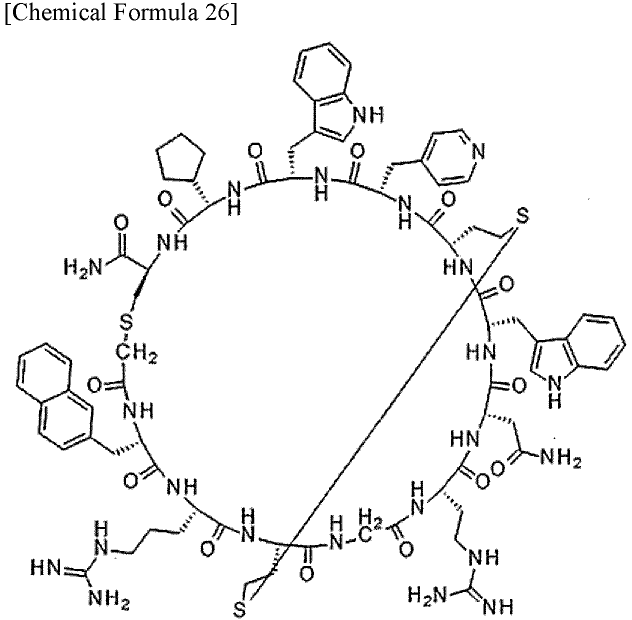 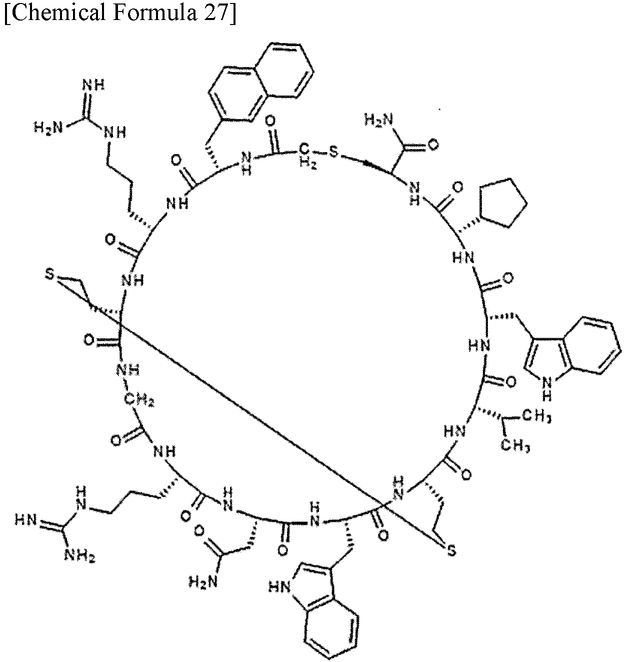 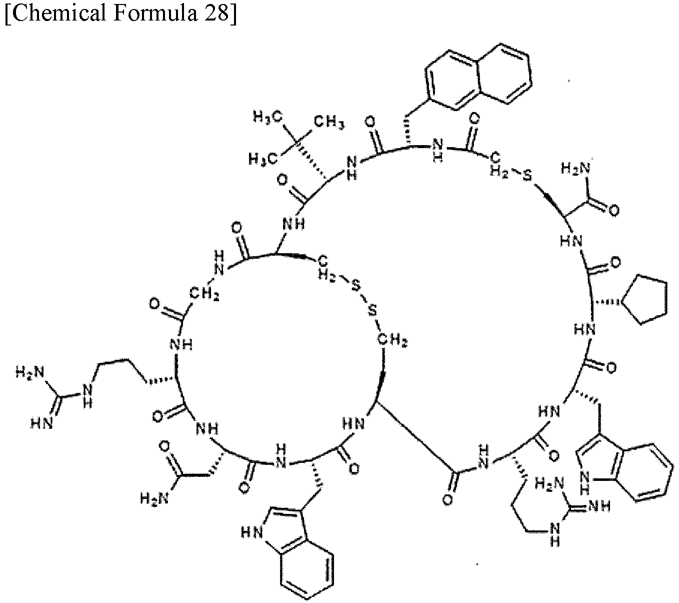 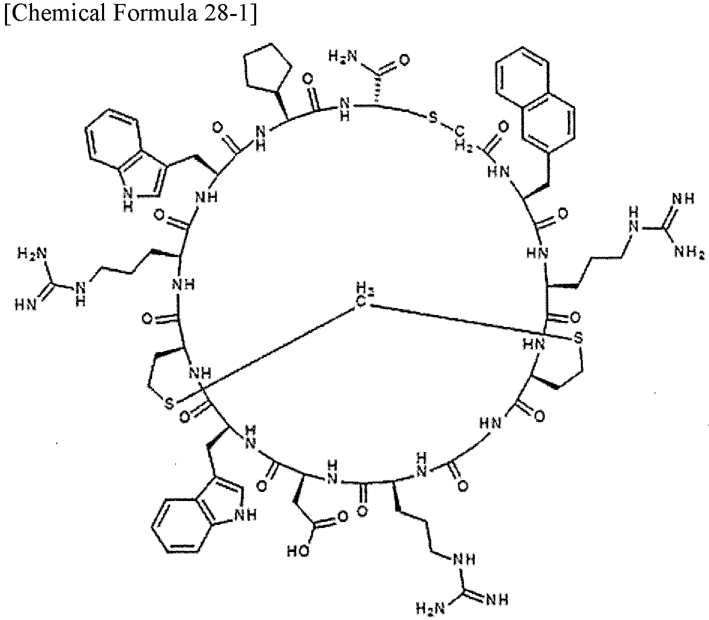
[7] A pharmaceutical composition comprising, as an active component, the cyclic peptide or pharmaceutically acceptable salt thereof as set forth in any one of [1] to [6].
[8] The pharmaceutical composition as set forth in [7], for use in the treatment or prophylaxis of a disease that can be treated or prevented by inhibiting a function of TSP1.
[9] The pharmaceutical composition as set forth in [8], for use in the treatment or prophylaxis of critical limb ischemia, peripheral arterial disease, or chronic limb threatening ischemia.
[10] A blood flow improving agent comprising, as an active component, the cyclic peptide or pharmaceutically acceptable salt thereof as set forth in any one of [1] to [6].
[11] A conjugate comprising:
   the cyclic peptide as set forth in any one of [1] to [6], or
   a cyclic peptide represented by formula (X) 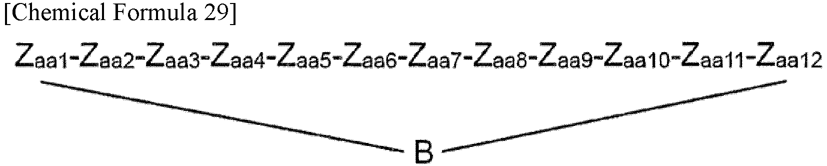
   [wherein B is selected from the ring-forming groups of the formulas: 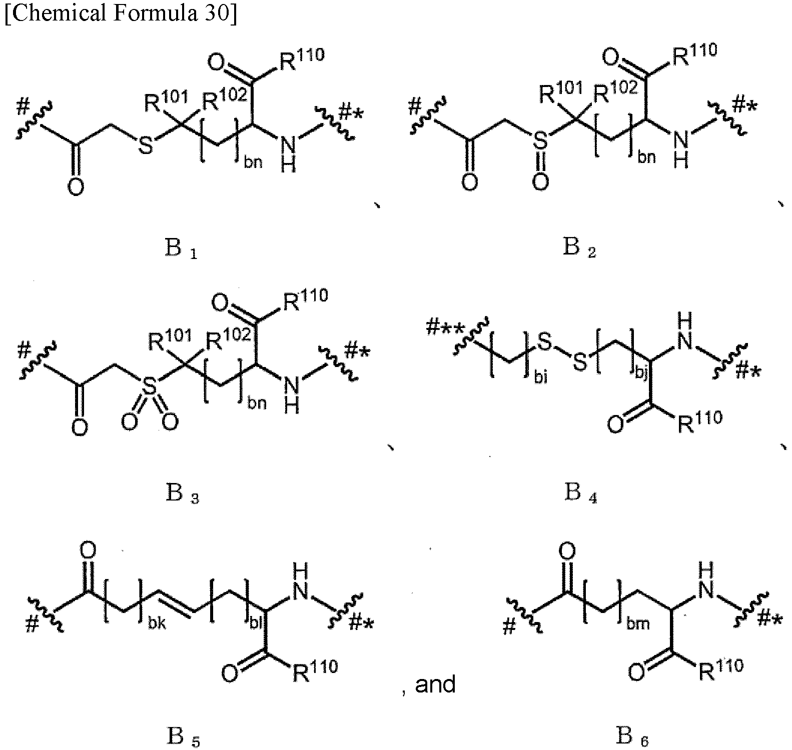
   wherein 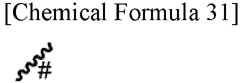
   represents a point of attachment to the N-terminal amino group of Zₐₐ₁, or in the absence of Zₐₐ₁, represents a point of attachment to the N-terminal amino group of Zₐₐ₂, 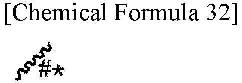
   represents a point of attachment to the C-terminal carbonyl group of Zₐₐ₁₂, 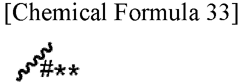
   represents a point of attachment to α carbon of Zₐₐ₁, or in the absence of Zₐₐ₁, represents a point of attachment to α carbon of Zₐₐ₂,
   R¹⁰¹ and R¹⁰² are each independently a hydrogen atom or C₁₋₃ alkyl,
   R¹¹⁰ is amino or hydroxy,
   bn is an integer of 0 to 3 (e.g., 0, 1, 2, or 3),
   bi and bj are each independently an integer of 1 to 3 (e.g., 1, 2, or 3),
   bk and bl are each independently an integer of 0 to 3 (e.g., 0, 1, 2, or 3),
   bm is an integer of 1 to 7 *(e.g.,* 1, 2, 3, 4, 5, 6, or 7);
   Zₐₐ₁ is a residue of an aliphatic amino acid, an aromatic amino acid, a basic amino acid, a neutral amino acid, or an acidic amino acid, or is absent;
   Zₐₐ₂ is a residue of an aromatic amino acid or a neutral amino acid;
   Zₐₐ₃ is a residue of an aliphatic amino acid, an aromatic amino acid, or a basic amino acid;
   Zₐₐ₄ is Ser, Thr, Ala, or ^{m}S;
   Zₐₐ₅ is Gly or Ser;
   Zₐₐ₆ is a residue of a basic amino acid or a neutral amino acid;
   Zₐₐ₇ is a residue of a neutral amino acid or an acidic amino acid;
   Zₐₐ₈ is a residue of an aromatic amino acid;
   Zₐₐ₉ is a residue of an aliphatic amino acid, a neutral amino acid, or an aromatic amino acid;
   Zₐₐ₁₀ is a residue of a basic amino acid, an aliphatic amino acid, an aromatic amino acid, or a neutral amino acid;
   Zₐₐ₁₁ is a residue of an aromatic amino acid; and
   Zₐₐ₁₂ is a residue of an aliphatic amino acid, an aromatic amino acid, or a basic amino acid,
   wherein the aliphatic amino acid, the aromatic amino acid, the basic amino acid, the neutral amino acid, and the acidic amino acid are as defined in [1]; and
   a carrier molecule bonded to the cyclic peptide, or
   a pharmaceutically acceptable salt thereof.
[12] The conjugate or pharmaceutically acceptable salt thereof as set forth in [11], wherein
   Zₐₐ₁ is a residue of Arg, Lys, His, Gly, Ala, Asn, Thr, Ser, Met, Leu, Ile, Val, Gln, Phe, Tyr, Trp, or Cys, or is absent;
   Zₐₐ₂is a residue of Phe, Tyr, Trp, 2Nal, 4CF, or DCF;
   Zₐₐ₃ is a residue of Ile, Leu, Nle, Tle, Trp, 2Nal, 4CF, or Arg;
   Zₐₐ₄ is a Ser residue;
   Zₐₐ₅ is a Gly residue;
   Zₐₐ₆ is a residue of Arg, Lys, His, Ser, Cit, or AGB;
   Zₐₐ₇ is a residue of Asn or Asp;
   Zₐₐ₈ is a residue of Trp or 2Nal;
   Zₐₐ₉ is a residue of Val, Nle, Ahp, or Met;
   Zₐₐ₁₀ is a residue of Arg, AGB, Lys, His, AMF, Phg, or Val;
   Zₐₐ₁₁ is a residue of Trp or 2Nal;
   Zₐₐ₁₂ is a residue of Val, Tle, CPTG, CHXG, or Phe; and
   B is a ring-forming group 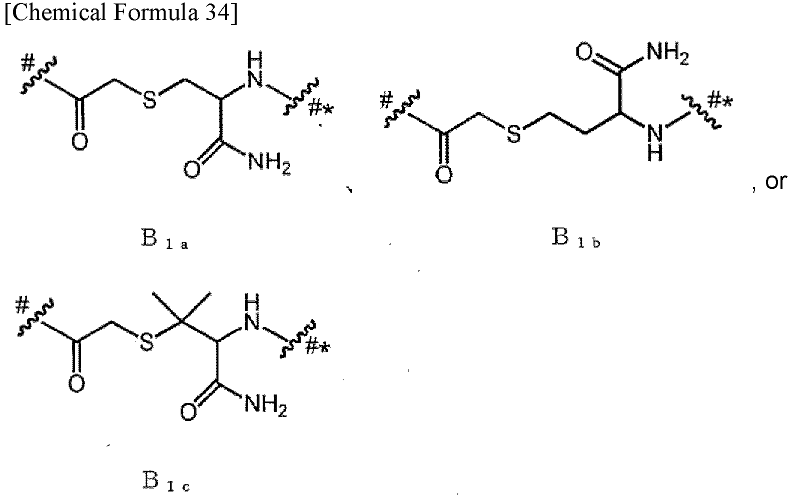
[13] The conjugate or pharmaceutically acceptable salt thereof as set forth in [12], wherein
   Zₐₐ₁ is absent;
   Zₐₐ₂ is a 2Nal residue;
   Zₐₐ₃ is a residue of Arg or Tle;
   Zₐₐ₄ is a Ser residue;
   Zₐₐ₅ is a Gly residue;
   Zₐₐ₆ is a residue of Arg or AGB;
   Zₐₐ₇ is a residue of Asn or Asp;
   Zₐₐ₈ is a Trp residue;
   Zₐₐ₉ is a residue of Val or Nle;
   Zₐₐ₁₀ is a residue of Arg, AGB, or Val;
   Zₐₐ₁₁ is a Trp residue;
   Zₐₐ₁₂ is a residue of Val, CPTG, or CHXG; and B is 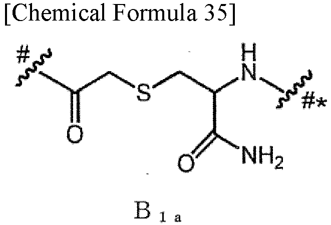
[14] The conjugate or pharmaceutically acceptable salt thereof as set forth in [13], wherein the cyclic peptide represented by formula (X) is selected from the group consisting of compounds represented by the formulas: 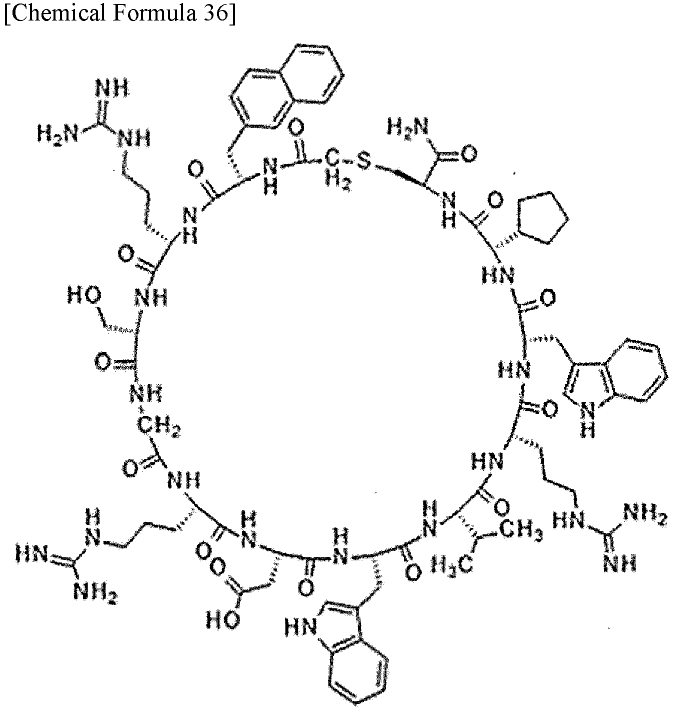 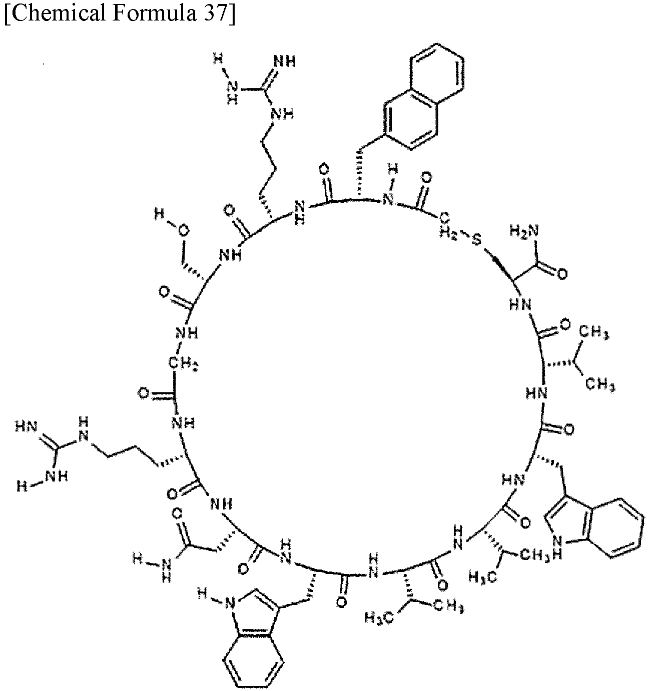 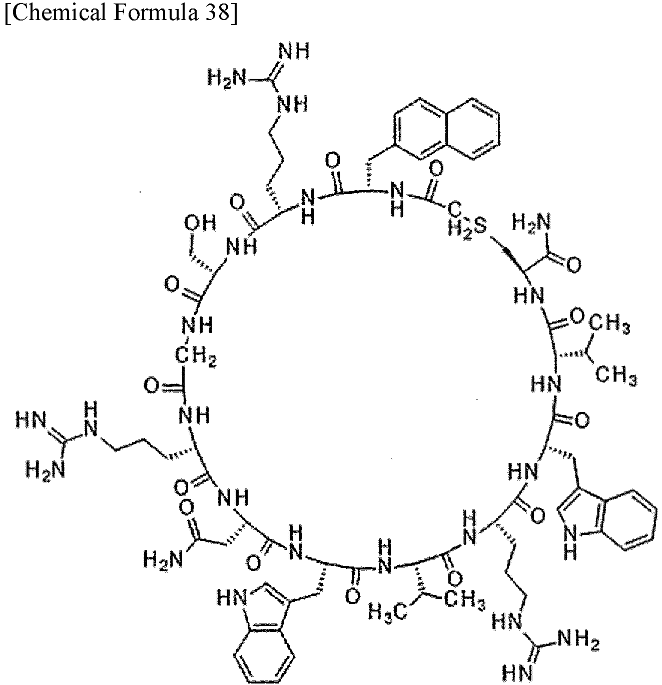 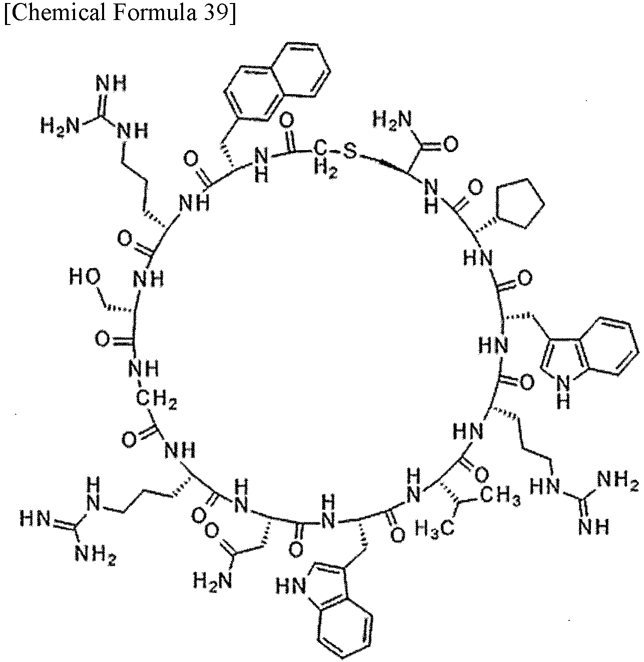 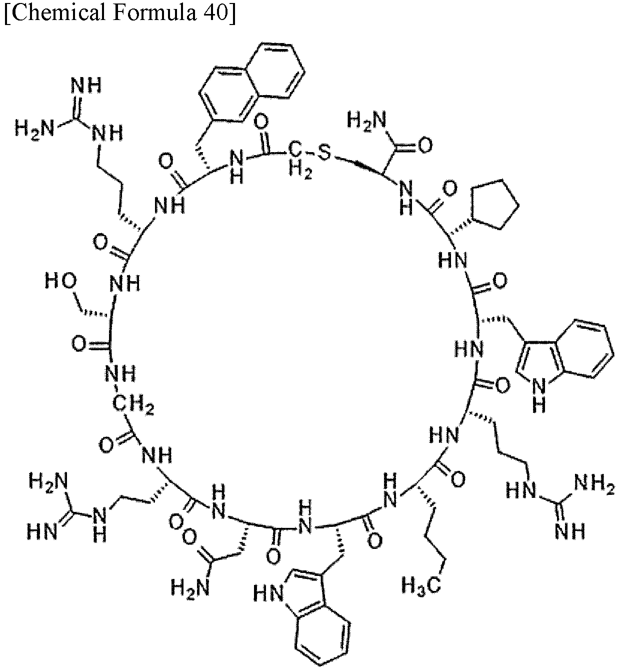 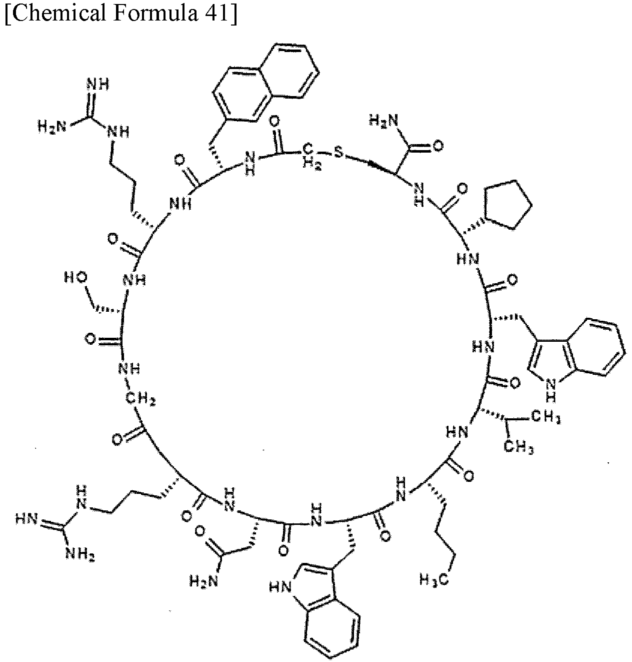 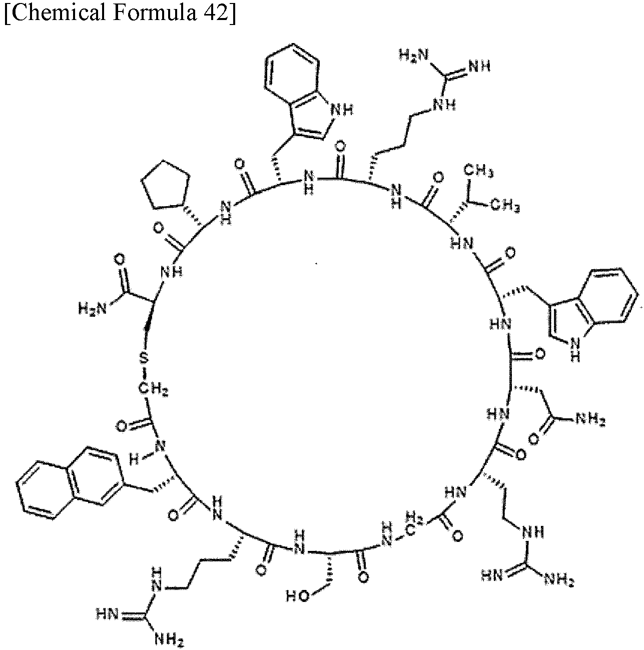 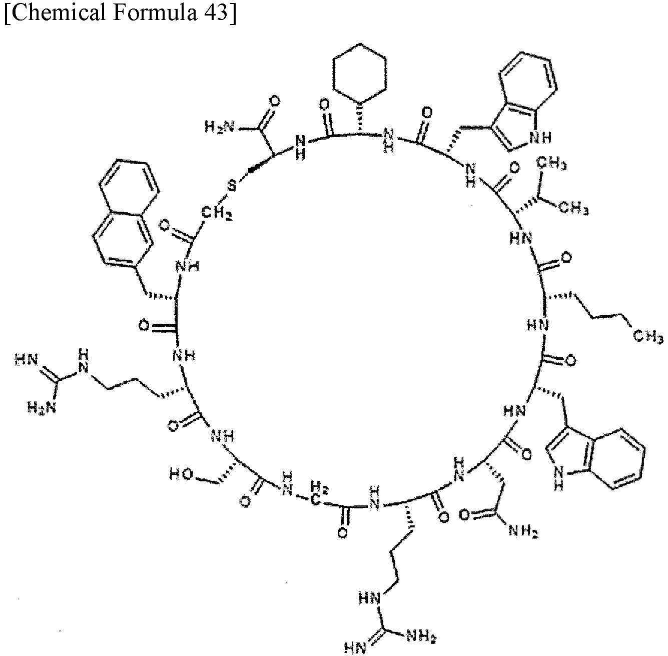 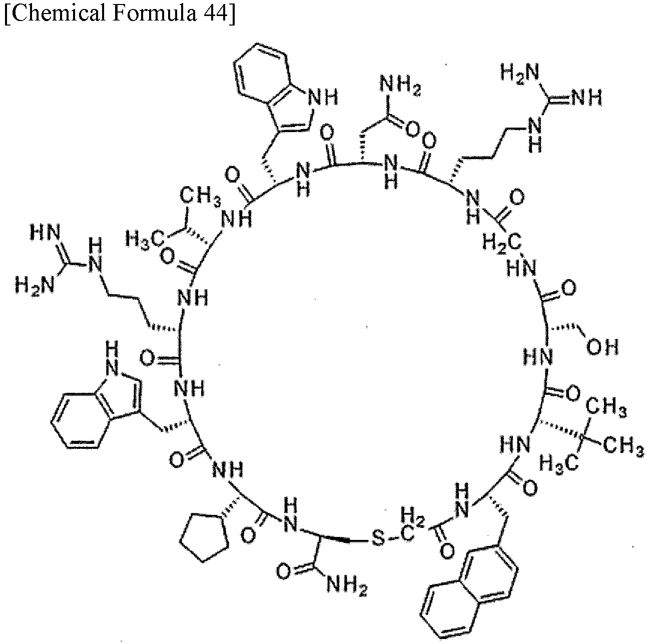 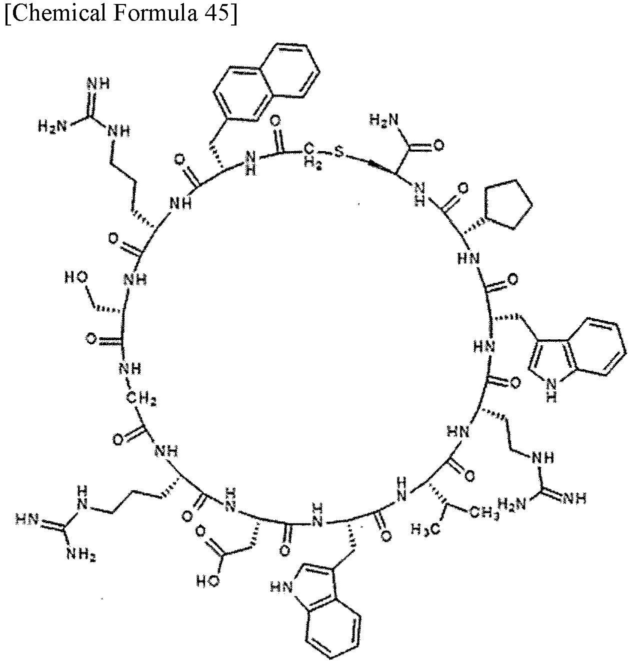 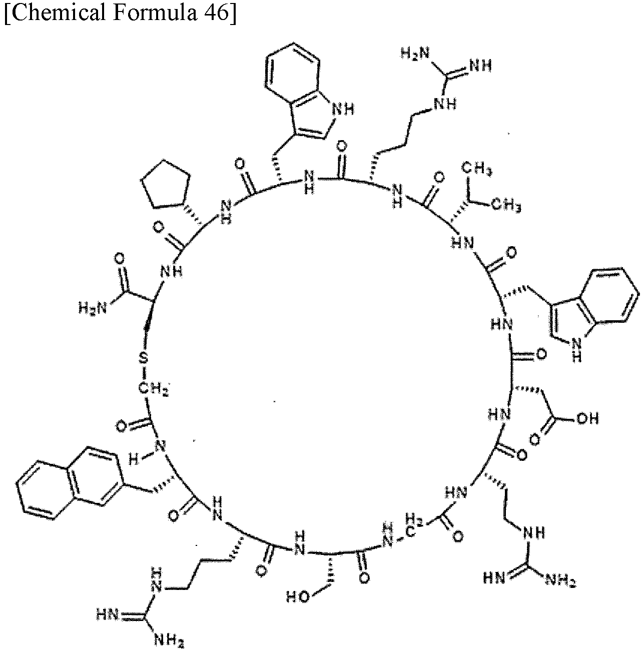 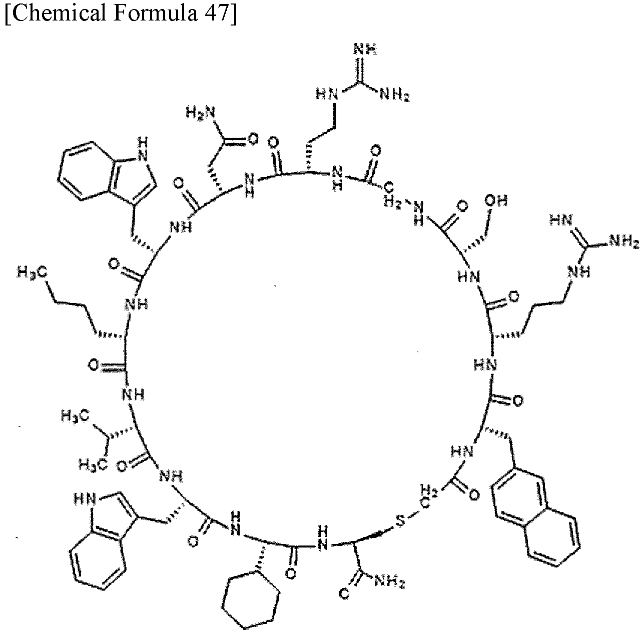 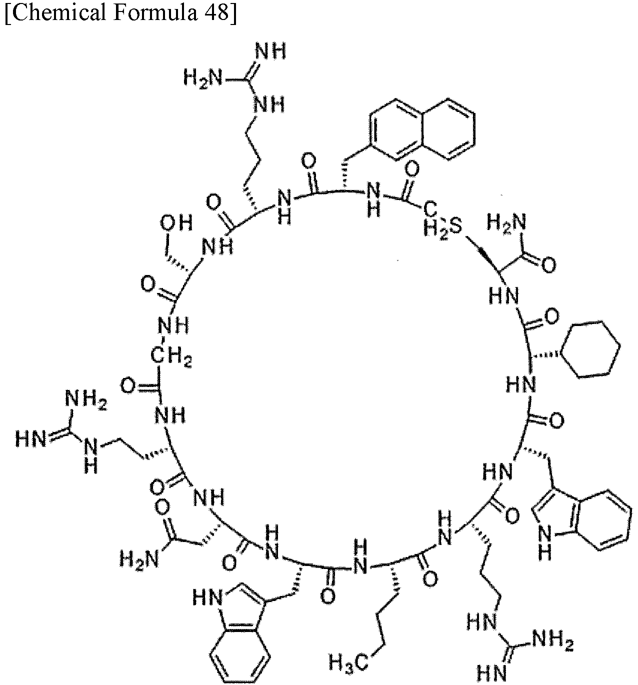 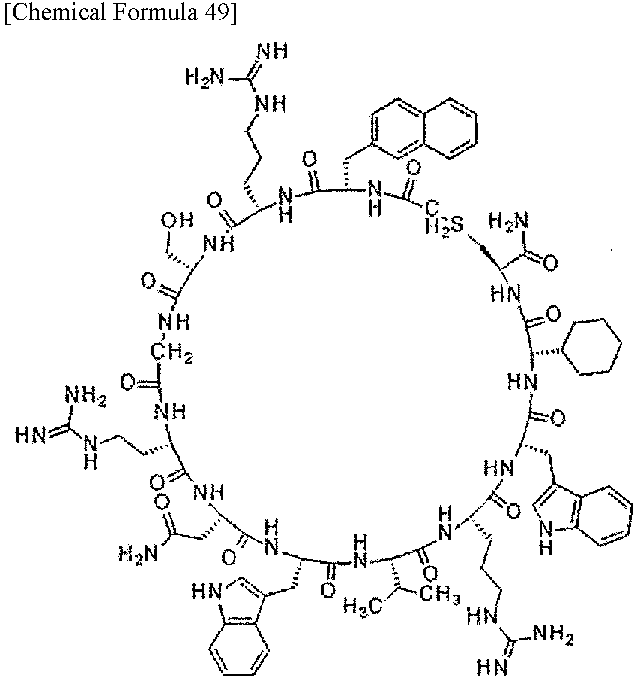 and 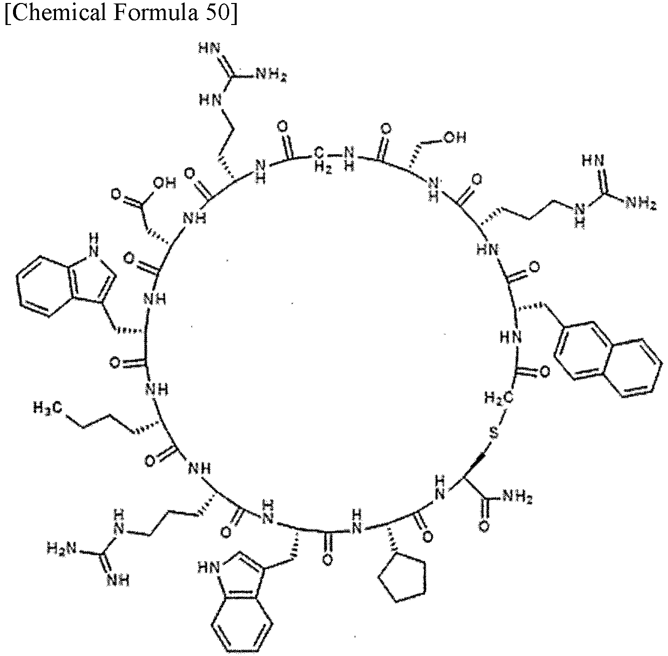
[15] The conjugate or pharmaceutically acceptable salt thereof as set forth in any one of [11] to [14], wherein the carrier molecule is human serum albumin or a fragment or variant thereof.
[16] The conjugate or pharmaceutically acceptable salt thereof as set forth in any one of [11] to [14], wherein the carrier molecule is an Fc-containing molecule.
[17] The conjugate or pharmaceutically acceptable salt thereof as set forth in [16], wherein the Fc-containing molecule is a full-length IgG, a full-length IgG heavy chain, a fragment of IgG comprising all or part of an Fc region, or a variant thereof.
[18] The conjugate or pharmaceutically acceptable salt thereof as set forth in [16] or [17], wherein the Fc-containing molecule comprises CH2 and CH3 domains of a constant region of an IgG heavy chain.
[19] The conjugate or pharmaceutically acceptable salt thereof as set forth in any one of [16] to [18], wherein the Fc-containing molecule comprises at least one amino acid residue of a cysteine residue corresponding to position 226, a cysteine residue corresponding to position 229, and an asparagine residue corresponding to position 297 according to the Eu index in a human IgG heavy chain.
[20] The conjugate or pharmaceutically acceptable salt thereof as set forth in any one of [16] to [19], wherein the Fc-containing molecule comprises an amino acid sequence corresponding to positions 221 to 230, 222 to 230, 223 to 230, 224 to 230, 225 to 230, or 226 to 230 according to the EU index in a human IgG heavy chain.
[21] The conjugate or pharmaceutically acceptable salt thereof as set forth in any one of [16] to [20], wherein the Fc-containing molecule comprises substitutions of a leucine residue corresponding to position 234 and a leucine residue corresponding to position 235 according to the Eu index in a human IgG heavy chain with alanine residues, or substitutions of a leucine residue corresponding to position 234, a leucine residue corresponding to position 235, and a proline residue corresponding to position 329 according to the Eu index in a human IgG heavy chain with alanine residues.
[22] The conjugate or pharmaceutically acceptable salt thereof as set forth in any one of [16] to [21], wherein the Fc-containing molecule is CH consisting of a constant region of a human IgG heavy chain, CLCH consisting of a constant region of a human IgG, or a molecule consisting of an Fc region of a human IgG or a fragment thereof, or a variant thereof.
[23] The conjugate or pharmaceutically acceptable salt thereof as set forth in [22], wherein the Fc-containing molecule is
   an antibody (mAb-A) consisting of a combination of a heavy chain consisting of an amino acid sequence at amino acid positions 20 to 474 of SEQ ID NO: 125 and a light chain consisting of an amino acid sequence at amino acid positions 21 to 234 of SEQ ID NO: 127;
   CH (CH-A) consisting of an amino acid sequence at amino acid positions 20 to 349 of SEQ ID NO: 129;
   CH (CH-B) consisting of an amino acid sequence at amino acid positions 20 to 349 of SEQ ID NO: 133;
   CLCH (CLCH-A) consisting of a combination of a heavy chain consisting of an amino acid sequence at amino acid positions 20 to 349 of SEQ ID NO: 129 and a light chain consisting of an amino acid sequence at amino acid positions 21 to 125 of SEQ ID NO: 131;
   CLCH (CLCH-B) consisting of a combination of a heavy chain consisting of an amino acid sequence at amino acid positions 20 to 349 of SEQ ID NO: 133 and a light chain consisting of an amino acid sequence at amino acid positions 21 to 125 of SEQ ID NO: 131;
   an Fc fragment (Fc-B) consisting of an amino acid sequence at amino acid positions 21 to 243 of SEQ ID NO: 135;
   an Fc fragment (Fc-A) consisting of an amino acid sequence at amino acid positions 21 to 247 of SEQ ID NO: 137; or
   an Fc fragment (Fc-C) consisting of an amino acid sequence at amino acid positions 21 to 247 of SEQ ID NO: 139;
      or
   a variant thereof.
[24] The conjugate or pharmaceutically acceptable salt thereof as set forth in any one of [16] to [23], wherein the Fc-containing molecule comprises one or two or more modifications selected from the group consisting of N-linked glycosylation, O-linked glycosylation, N-terminal processing, C-terminal processing, deamidation, isomerization of aspartic acid, oxidation of methionine, addition of a methionine residue to the N-terminus, amidation of a proline residue, and a deletion of one or two amino acid residues at the carboxyl terminus of a heavy chain.
[25] The conjugate or pharmaceutically acceptable salt thereof as set forth in [24], wherein one or two amino acid residues are deleted at the carboxyl terminus of a heavy chain of the Fc-containing molecule.
[26] The conjugate or pharmaceutically acceptable salt thereof as set forth in [25], wherein one amino acid residue is deleted at the carboxyl terminus of each of two heavy chains of the Fc-containing molecule.
[27] The conjugate or a pharmaceutically acceptable salt thereof as set forth in any one of [24] to [26], wherein the proline residue at the carboxyl terminus of a heavy chain of the Fc-containing molecule is further amidated.
[28] The conjugate or pharmaceutically acceptable salt thereof as set forth in any one of [11] to [27], wherein the cyclic peptide and the carrier molecule are bonded to each other via a linker structure.
[29] The conjugate or pharmaceutically acceptable salt thereof as set forth in [28], wherein the linker structure comprises at least one structure selected from the group consisting of a polyoxyalkylene chain, an amino acid residue, and an oligopeptide chain consisting of two or more amino acid residues.
[30] The conjugate or pharmaceutically acceptable salt thereof as set forth in [29], wherein the polyoxyalkylene chain contained in the linker structure is a polyethylene glycol (PEG) chain.
[31] The conjugate or pharmaceutically acceptable salt thereof as set forth in [30], wherein the total degree of polymerization of the PEG chain contained in backbone of the linker structure is 6 to 100.
[32] The conjugate or pharmaceutically acceptable salt thereof as set forth in [29], wherein the amino acid residue contained in the linker structure is a residue of Gly, N-methylglycine, Asp, D-Asp, Glu, D-Glu, Lys, D-Lys, β-alanine, Ser, D-Ser, a non-natural amino acid comprising a polyoxyalkylene chain in a side chain, or a non-natural amino acid comprising an SG chain in a side chain.
[33] The conjugate or pharmaceutically acceptable salt thereof as set forth in [29], wherein the oligopeptide chain contained in the linker structure consists of 2 to 20 (e.g., 1, 2, 3, 4, 5, 6, 7, 8, 9, 10, 11, 12, 13, 14, 15, 16, 17, 18, 19, or 20) amino acid residues.
[34] The conjugate or pharmaceutically acceptable salt thereof as set forth in [29], wherein the oligopeptide chain contained in the linker structure comprises one or more amino acid residues selected from the group consisting of Gly, N-methylglycine, Asp, D-Asp, Glu, D-Glu, Lys, D-Lys, β-alanine, Ser, D-Ser, a non-natural amino acid comprising a polyoxyalkylene chain in a side chain, and a non-natural amino acid comprising an SG chain in a side chain.
[35] The conjugate or pharmaceutically acceptable salt thereof as set forth in [29], wherein an N-terminal amino group of the amino acid residue or oligopeptide chain contained in the linker structure forms an amide bond with a carbonyl group to which R¹⁰ of the ring-forming group A or R¹¹⁰ of the ring-forming group B is bonded, where R¹⁰ or R¹¹⁰ is substituted with NH of the N-terminal amino group.
[36] The conjugate or pharmaceutically acceptable salt thereof as set forth in any one of [28] to [35], wherein the linker structure comprises a linking group C that binds to the carrier molecule.
[37] The conjugate or pharmaceutically acceptable salt thereof as set forth in [36], wherein the linking group C comprises a maleimide group-derived moiety in a thioether bond formed by reaction of a maleimide group with a thiol group or 1,2,3-triazole ring.
[38] The conjugate or pharmaceutically acceptable salt thereof as set forth in [37], wherein a maleimide group is bonded to a thiol group of a cysteine residue of the carrier molecule to form a thioether bond.
[39] The conjugate or pharmaceutically acceptable salt thereof as set forth in [37], wherein the carrier molecule is an Fc-containing molecule, wherein the 1,2,3-triazole ring contained in the linking group C is bonded to a glycan which is bonded to an asparagine residue corresponding to position 297 according to the Eu index of the Fc-containing molecule (N297-linked glycan).
[40] The conjugate or pharmaceutically acceptable salt thereof as set forth in any one of [36] to [39], wherein
   the linking group C is represented by any of the following formulas: 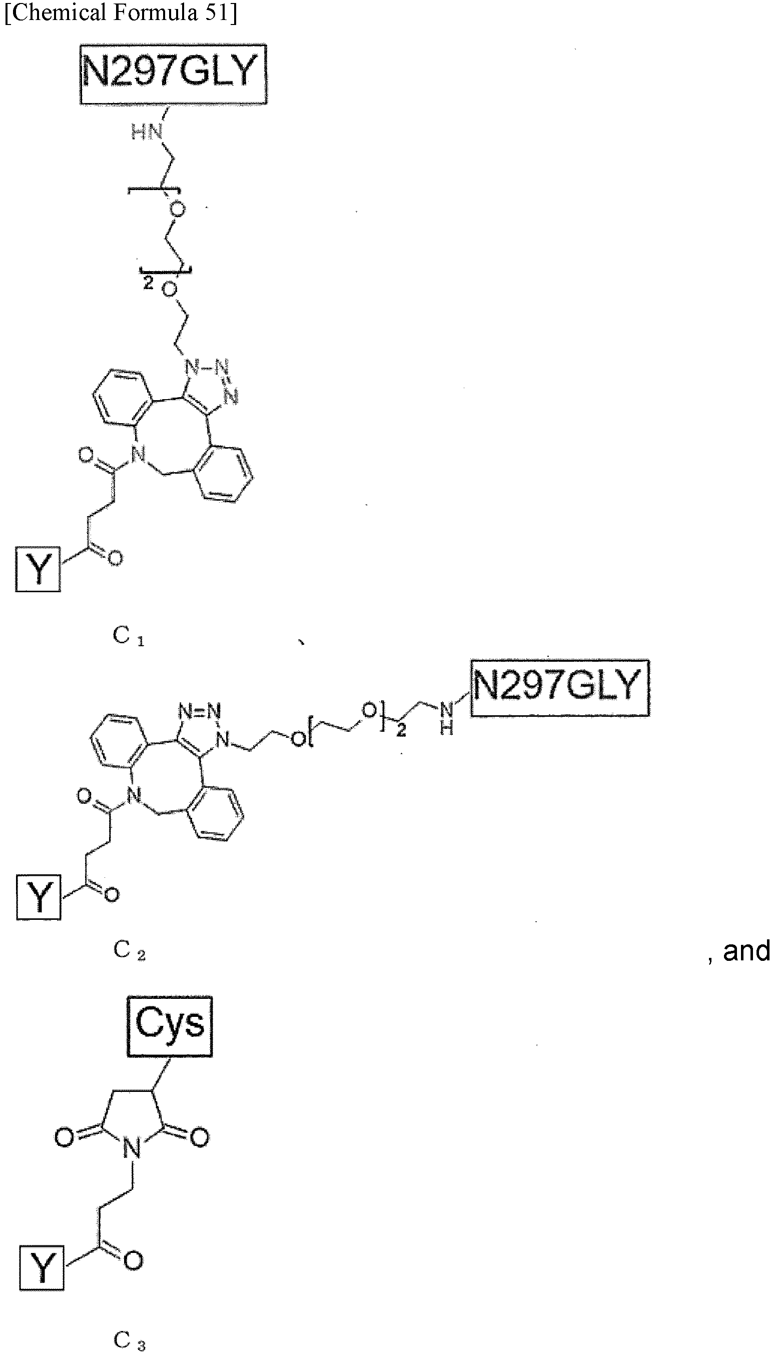
   (wherein
      N297GLY represents binding to the non-reducing terminus of N297-linked glycan of the Fc-containing molecule;
      Cys represents binding to the thiol group of a cysteine residue of the carrier molecule; and
      Y represents a point of attachment to an adjacent amino acid residue in the linker structure).
[41] The conjugate or pharmaceutically acceptable salt thereof as set forth in [39] or [40], wherein
   the N297-linked glycan of the Fc-containing molecule is at least one selected from glycans N297-(Fuc) SG, N297-(Fuc)MSG1, and N297-(Fuc)MSG2 having structures represented by the following formulas: 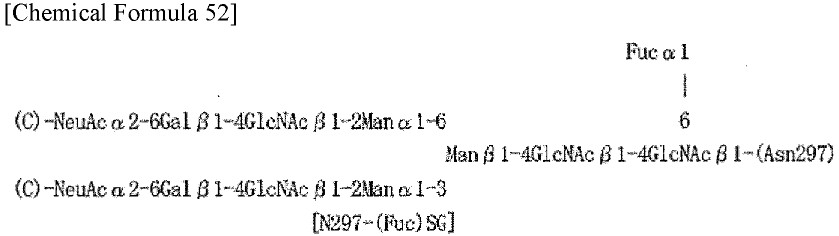 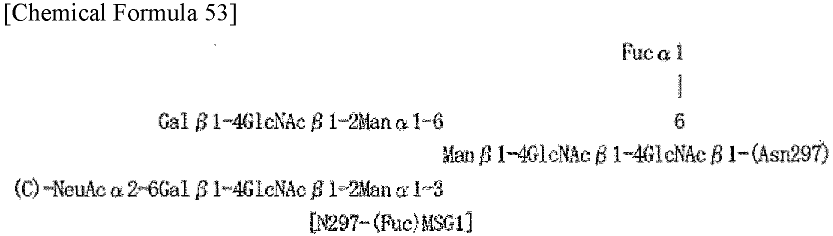 and 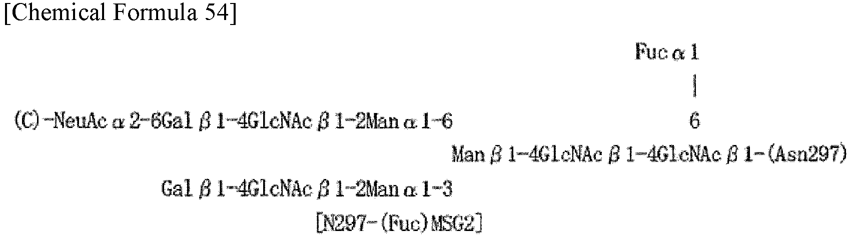 [wherein (C) represents a point of attachment to the linking group C and (Asn297) represents a point of attachment to the asparagine residue corresponding to position 297 according to the Eu index of the Fc-containing molecule].
[42] The conjugate or pharmaceutically acceptable salt thereof as set forth in [41], wherein the N297-linked glycan of the Fc-containing molecule is N297-(Fuc)SG.
[43] The conjugate or pharmaceutically acceptable salt thereof as set forth in any one of [36] to [42], wherein
   the linker structure is represented by formula (XX)

      -L1-L2-L3-C-

      [wherein
      L1 is an oligopeptide containing 3 to 6 (e.g., 3, 4, 5, or 6) neutral amino acid residues bonded in a linear manner;
      L2 is L2a or L2b, wherein
      L2a is a non-natural amino acid residue comprising a polyoxyalkylene chain in backbone, or an oligopeptide comprising a non-natural amino acid residue comprising a polyoxyalkylene chain in backbone, wherein the total degree of polymerization of the polyoxyalkylene chain contained in backbone of L2a is 6 to 100 (e.g., 5 to 100, preferably 6 to 50, more preferably 10 to 50, even more preferably 10 to 40 or 20 to 40), and
      L2b is an oligopeptide containing 10 to 20 (e.g., 10, 11, 12, 13, 14, 15, 16, 17, 18, 19, or 20) natural or non-natural α-amino acid residues bonded in a linear manner;
      L3 is an amino acid residue represented by the formula 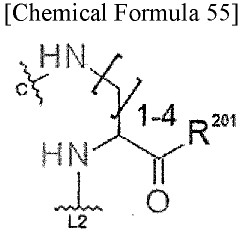
      (wherein R²⁰¹ is a hydroxy group or an amino group, 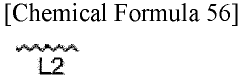
      represents a point of attachment to an adjacent amino acid residue in L2, and 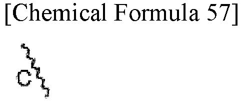
      represents a point of attachment to the linking group C);
      C is the linking group C;
      the N-terminal amino group of L1 forms an amide bond with a carbonyl group to which R¹⁰ of the ring-forming group A or R¹¹⁰ of the ring-forming group B is bonded, where R¹⁰ or R¹¹⁰ is substituted with NH of the N-terminal amino group;
      L1 and L2 are joined together by an amide bond; and
      L2 and L3 are joined together by an amide bond].
[44] The conjugate or pharmaceutically acceptable salt thereof as set forth in [43], wherein the neutral amino acid residue contained in L1 is a residue of one or more amino acids selected from the group consisting of Gly, Ser, D-Ser, N-methylglycine (NMeG), and β-alanine (bAla).
[45] The conjugate or pharmaceutically acceptable salt thereof as set forth in [43] or [44], wherein the polyoxyalkylene chain in backbone, which is contained in L2a, is a PEG chain.
[46] The conjugate or pharmaceutically acceptable salt thereof as set forth in [45], wherein the non-natural amino acid residue comprising a PEG chain in backbone, which is contained in L2a, is a residue of an amino acid represented by the formula 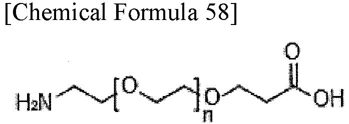 [wherein n represents an integer of 5 to 40 (e.g., 5, 11, 23, or 35)].
[47] The conjugate or pharmaceutically acceptable salt thereof as set forth in [46], wherein the total degree of polymerization of the PEG chain is 10 to 40 (e.g., 12, 18, 24, 30, or 36).
[48] The conjugate or pharmaceutically acceptable salt thereof as set forth in [43] or [44], wherein an α-amino acid of L2b is glycine or N-methylglycine.
[49] The conjugate or pharmaceutically acceptable salt thereof as set forth in [48], wherein the oligopeptide of L2b is oligo-N-methylglycine with 13 consecutive N-methylglycines.
[50] The conjugate or pharmaceutically acceptable salt thereof as set forth in any one of [43] to [49], wherein L2 comprises a non-natural amino acid residue comprising a PEG chain in a side chain or a non-natural amino acid residue comprising an SG chain in a side chain.
[51] The conjugate or pharmaceutically acceptable salt thereof as set forth in [50], wherein the non-natural amino acid comprising a PEG chain in a side chain is KPEG.
[52] The conjugate or pharmaceutically acceptable salt thereof as set forth in [50], wherein the non-natural amino acid comprising an SG chain in a side chain is KSG.
[53] The conjugate or pharmaceutically acceptable salt thereof as set forth in any one of [43] to [52], wherein a total of 1 to 3 (e.g., 1, 2, or 3) acidic amino acid residues is contained at one or more positions not adjacent to the ring-forming group A or the ring-forming group B in backbone of the oligopeptide of L1 and L2.
[54] The conjugate or pharmaceutically acceptable salt thereof as set forth in [53], wherein the acidic amino acid residue is an amino acid selected from the group consisting of Asp, D-Asp, Glu, and D-Glu.
[55] The conjugate or pharmaceutically acceptable salt thereof as set forth in any one of [43] to [54], wherein L1 is an oligopeptide comprising or consisting of the sequence Gly-Gly-Gly-Gly,
   Gly-Ser-Gly-Ser,
   NMeG-NMeG-NMeG-NMeG,
   bAla-bAla-bAla-bAla, or
   Gly-Gly-Glu-Gly-Gly.
[56] The conjugate or pharmaceutically acceptable salt thereof as set forth in [55], wherein L1 is an oligopeptide consisting of a sequence of two acidic amino acid residues coupled to the C-terminus of
   Gly-Gly-Gly-Gly,
   Gly-Ser-Gly-Ser,
   NMeG-NMeG-NMeG-NMeG, or
   bAla-bAla-bAla-bAla.
[57] The conjugate or pharmaceutically acceptable salt thereof as set forth in [56], wherein the acidic amino acid residue is an Asp residue.
[58] The conjugate or pharmaceutically acceptable salt thereof as set forth in any one of [43] to [55], wherein L2 is an oligopeptide consisting of the sequence
   Asp-Asp-KPEG-P12P-P12P,
   KSG-P12P-P12P,
   Asp-Asp-P12P,
   Asp-Asp-P12P-P12P, or
   Asp-Asp-P12P-KPEG-P12P.
[59] The conjugate or pharmaceutically acceptable salt thereof as set forth in any one of [43] to [58], wherein L3 is a Lys residue.
[60] The conjugate or pharmaceutically acceptable salt thereof as set forth in [43], wherein the linker structure is
   a structure in which L1 is an oligopeptide consisting of the sequence Gly-Gly-Gly-Gly or Gly-Ser-Gly-Ser, L2a is an oligopeptide consisting of the sequence Asp-Asp-KPEG-P12P-P12P, and L3 is a Lys residue;
   a structure in which L1 is an oligopeptide consisting of the sequence Gly-Gly-Gly-Gly, L2a is an oligopeptide consisting of the sequence KSG-P12P-P12P, and L3 is a Lys residue;
   a structure in which L1 is an oligopeptide consisting of the sequence Gly-Gly-Gly-Gly, L2a is an oligopeptide consisting of the sequence Asp-Asp-P12P, and L3 is a Lys residue;
   a structure in which L1 is an oligopeptide consisting of the sequence Gly-Ser-Gly-Ser, L2a is an oligopeptide consisting of the sequence Asp-Asp-P12P-P12P, and L3 is a Lys residue; or
   a structure in which L1 is an oligopeptide consisting of the sequence Gly-Gly-Gly-Gly, L2a is an oligopeptide consisting of the sequence Asp-Asp-P12P-KPEG-P12P, and L3 is a Lys residue.
[61] The conjugate or pharmaceutically acceptable salt thereof as set forth in [11], wherein the cyclic peptide and the linker structure are represented by any of the formulas: 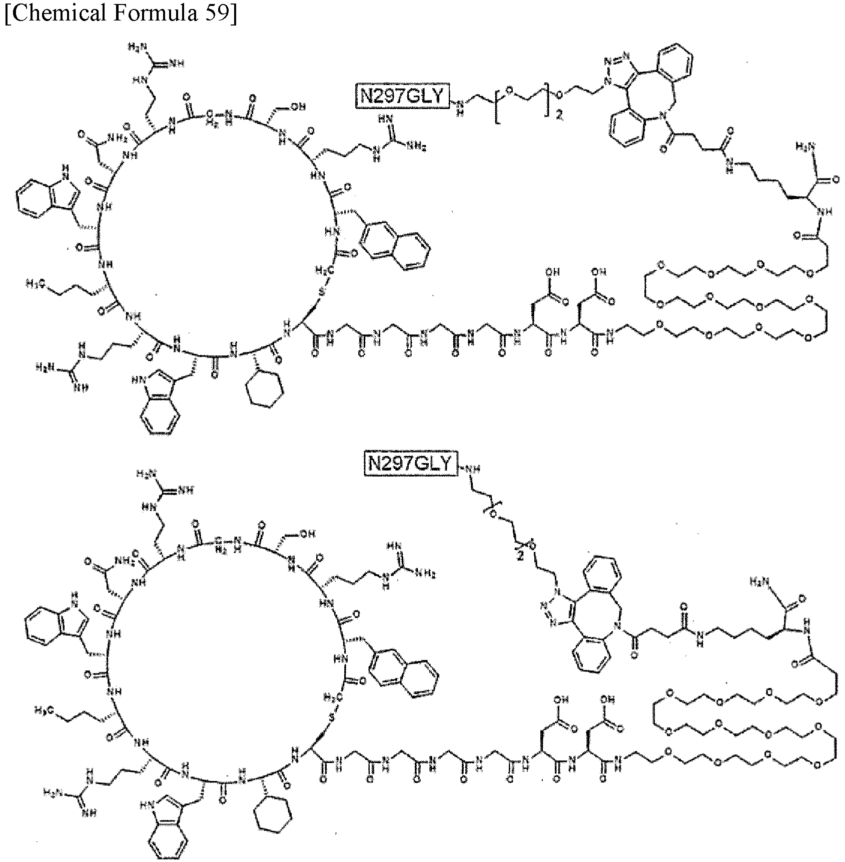 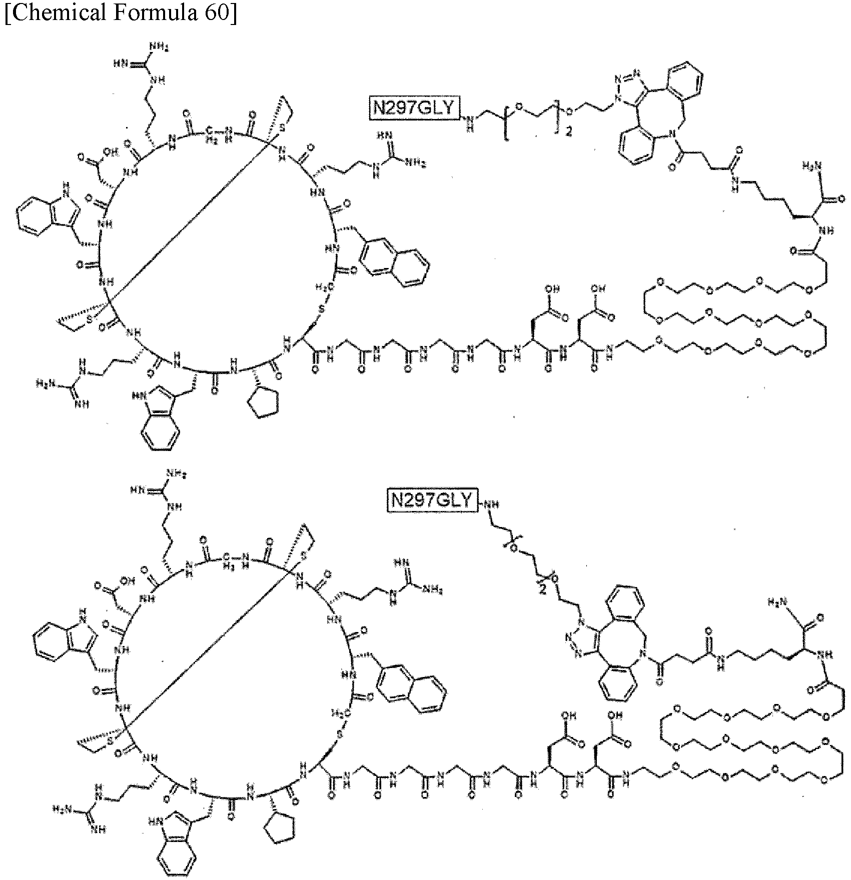 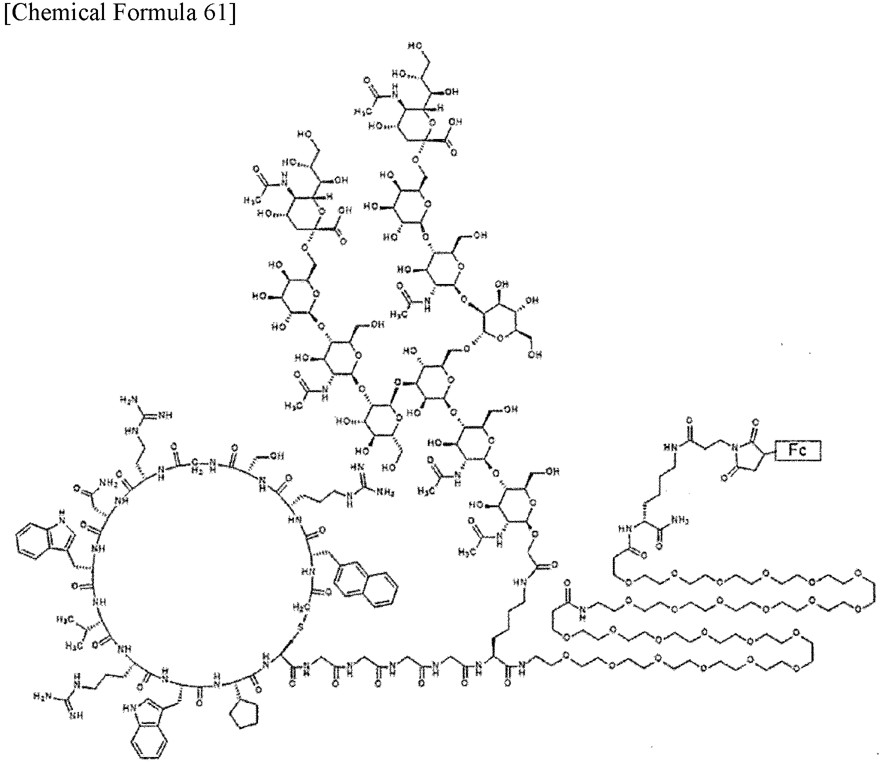 and 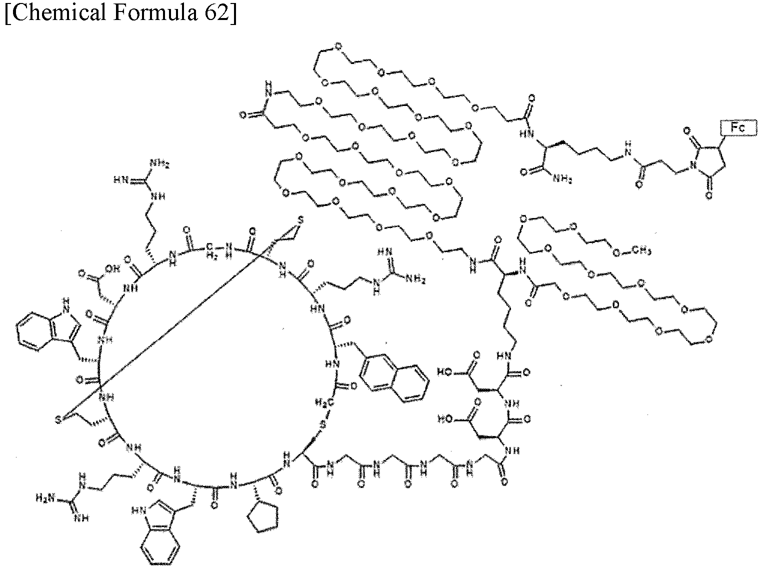 (wherein
   N297GLY represents binding of 1,2,3-triazole ring contained in the linking group C to the non-reducing terminus of N297-linked glycan of the Fc-containing molecule; and
   Fc represents binding of the maleimide group contained in the linking group C to the thiol group of a cysteine residue of the Fc-containing molecule).
[62] A pharmaceutical composition comprising, as an active component, the conjugate or pharmaceutically acceptable salt thereof as set forth in any one of [11] to [61].
[63] The pharmaceutical composition as set forth in [62], for use in the treatment or prophylaxis of a disease that can be treated or prevented by inhibiting a function of TSP1.
[64] The pharmaceutical composition as set forth in [63], for use in the treatment or prophylaxis of critical limb ischemia, peripheral arterial disease, or chronic limb threatening ischemia.
[65] A blood flow improving agent comprising, as an active component, the conjugate or pharmaceutically acceptable salt thereof as set forth in any one of [11] to [61].
[66] A method for producing the conjugate or pharmaceutically acceptable salt thereof as set forth in any one of [11] to [61], the method comprising the steps of:
   (1) synthesizing an oligopeptide X-A-L or Z-B-L represented by the following formula from the C-terminal side by a solid-phase synthesis method:
      Xₐₐ₁-Xₐₐ₂-Xₐₐ₃-Xₐₐ₄-Xₐₐ₅-Xₐₐ₆-Xₐₐ₇-Xₐₐ₈-Xₐₐ₉-Xₐₐ₁₀-Xₐₐ₁₁-A₀-L1-L2-L3 or
      Zₐₐ₁-Zₐₐ₂-Zₐₐ₃-Zₐₐ₄-Zₐₐ₅-Zₐₐ₆-Zₐₐ₇-Zₐₐ₈-Zₐₐ₉-Zₐₐ₁₀-Zₐₐ₁₁-Zₐₐ₁₂-B₀-L1-L2-L3 wherein
      Xₐₐ₁ to Xₐₐ₁₁ are as defined in [1] (provided that in the structure of Xₐₐ₃ and Xₐₐ₈, thiol groups remain as they are without forming a bond);
      L1 to L3 are as defined in [43] (provided that when L2 comprises a residue of a non-natural amino acid comprising a glycan in a side chain, the residue is replaced with a Lys residue);
      Zₐₐ₁ to Zₐₐ₁₂ are as defined in [11];
      A₀ is a residue represented by the formula 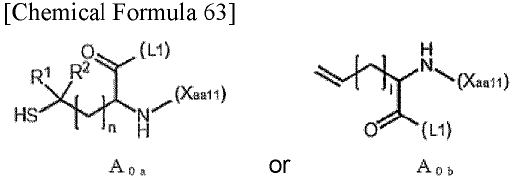 (wherein (Xₐₐ₁₁) represents a point of attachment to Xₐₐ₁₁, (L1) represents a point of attachment to the N-terminal amino acid residue in L1, and l and n are as defined in [1];
      B₀ is a residue represented by the formula 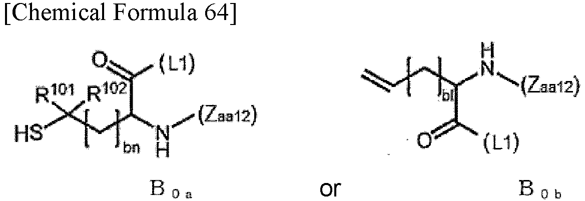 (wherein (Zₐₐ₁₂) represents a point of attachment to Zₐₐ₁₂, (L1) represents a point of attachment to the N-terminal amino acid residue in L1, and bl and bn are as defined in [11]),
   (2) cyclizing the oligopeptide synthesized in step (1) to afford a cyclic peptide by
      (a) joining the N-terminal amino group of Xₐₐ₁ to A₀ₐ to form any of the ring-forming groups A₁ to A₃ (which are coupled to L1 at the position of R¹⁰) in the oligopeptide X-A-L;
      (b) joining the N-terminal amino group of Xₐₐ₁ to A_{0b} to form the ring-forming group A₄ or As (which are coupled to L1 at the position of R¹⁰) in the oligopeptide X-A-L;
      (c) joining the N-terminal amino group of Zₐₐ₁ or Zₐₐ₂ to B₀ₐ to form any of the ring-forming groups B₁ to B₄ (which are coupled to L1 at the position of R¹¹⁰) in the oligopeptide Z-B-L; or
      (d) joining the N-terminal amino group of Zₐₐ₁ or Zₐₐ₂ to B_{0b} to form the ring-forming group B₅ or B₆ (which is coupled to L1 at the position of R¹¹⁰) in the oligopeptide Z-B-L,
   (3) when the oligopeptide X-A-L is synthesized in step (1), allowing the thiol groups of Xₐₐ₃ and Xₐₐ₈ of the oligopeptide X-A-L cyclized in step (2) to form a bond represented by -S-S-, - S-CH₂-S-, or -S-X-S- (wherein X is as defined in [1]) to afford a bicyclic peptide;
   (4) when L2 comprises a residue of a non-natural amino acid comprising a glycan in a side chain, which is replaced with a Lys residue in the oligopeptide X-A-L or Z-B-L, introducing a glycan into the Lys residue to thereby form a residue of a non-natural amino acid comprising a glycan in a side chain;
   (5) binding a reactive group capable of forming a maleimide group or a 1,2,3-triazole ring to L3; and
   (6) binding the cyclic peptide to the carrier molecule via the reactive group of step (5).
[67] A method for the treatment or prophylaxis of a disease or symptom, the method comprising administering to a subject a therapeutically or prophylactically effective amount of the cyclic peptide or pharmaceutically acceptable salt thereof as set forth in any one of [1] to [6].
[68] The method as set forth in [67], wherein the disease or symptom is a disease that can be treated or prevented by inhibiting a function of TSP1.
[69] The method as set forth in [68], wherein the disease or symptom is critical limb ischemia, peripheral arterial disease, or chronic limb threatening ischemia.
[70] A method for improving blood flow, the method comprising administering to a subject a therapeutically or prophylactically effective amount of the cyclic peptide or pharmaceutically acceptable salt thereof as set forth in any one of [1] to [6].
[71] The cyclic peptide or pharmaceutically acceptable salt thereof as set forth in any one of [1] to [6], for use in the treatment or prophylaxis of a disease or symptom.
[72] The cyclic peptide or pharmaceutically acceptable salt thereof as set forth in [71], wherein the disease or symptom is a disease that can be treated or prevented by inhibiting a function of TSP1.
[73] The cyclic peptide or pharmaceutically acceptable salt thereof as set forth in [72], wherein the disease or symptom is critical limb ischemia, peripheral arterial disease, or chronic limb threatening ischemia.
[74] The cyclic peptide or pharmaceutically acceptable salt thereof as set forth in any one of [1] to [6], for use in improving blood flow.
[75] Use of the cyclic peptide or pharmaceutically acceptable salt thereof as set forth in any one of [1] to [6], in the production of a pharmaceutical for the treatment or prophylaxis of a disease or symptom.
[76] The use as set forth in [75], wherein the disease or symptom is a disease that can be treated or prevented by inhibiting a function of TSP1.
[77] The use as set forth in [76], wherein the disease or symptom is critical limb ischemia, peripheral arterial disease, or chronic limb threatening ischemia.
[78] Use of the cyclic peptide or pharmaceutically acceptable salt thereof as set forth in any one of [1] to [6], in the production of a blood flow improving agent.
[79] A method for the treatment or prophylaxis of a disease or symptom, the method comprising administering to a subject a therapeutically or prophylactically effective amount of the conjugate or pharmaceutically acceptable salt thereof as set forth in any one of [11] to [61].
[80] The method as set forth in [79], wherein the disease or symptom is a disease that can be treated or prevented by inhibiting a function of TSP1.
[81] The method as set forth in [80], wherein the disease or symptom is critical limb ischemia, peripheral arterial disease, or chronic limb threatening ischemia.
[82] A method for improving blood flow, the method comprising administering to a subject a therapeutically or prophylactically effective amount of the conjugate or pharmaceutically acceptable salt thereof as set forth in any one of [11] to [61].
[83] The conjugate or pharmaceutically acceptable salt thereof as set forth in any one of [11] to [61], for use in the treatment or prophylaxis of a disease or symptom.
[84] The conjugate or pharmaceutically acceptable salt thereof as set forth in [83], wherein the disease or symptom is a disease that can be treated or prevented by inhibiting a function of TSP 1.
[85] The conjugate or pharmaceutically acceptable salt thereof as set forth in [84], wherein the disease or symptom is critical limb ischemia, peripheral arterial disease, or chronic limb threatening ischemia.
[86] The conjugate or pharmaceutically acceptable salt thereof as set forth in any one of [11] to [61], for use in improving blood flow.
[87] Use of the conjugate or pharmaceutically acceptable salt thereof as set forth in any one of [11] to [61], in the production of a pharmaceutical for the treatment or prophylaxis of a disease or symptom.
[88] The use as set forth in [87], wherein the disease or symptom is a disease that can be treated or prevented by inhibiting a function of TSP1.
[89] The use as set forth in [88], wherein the disease or symptom is critical limb ischemia, peripheral arterial disease, or chronic limb threatening ischemia.
[90] Use of the conjugate or pharmaceutically acceptable salt thereof as set forth in any one of [11] to [61], in the production of a blood flow improving agent.

### ADVANTAGEOUS EFFECTS OF INVENTION

The cyclic peptide or pharmaceutically acceptable salt thereof of the present invention is useful for the treatment or prophylaxis of diseases that can be treated or prevented by inhibiting a function of TSP1, such as critical limb ischemia. The conjugate or pharmaceutically acceptable salt thereof of the present invention can provide a means of treatment or prophylaxis of diseases or symptoms that can be treated or prevented by inhibiting a function of TSP1 at a lower frequency of administration.

### DESCRIPTION OF EMBODIMENTS

### (Definitions)

As referred to herein, natural amino acids mean unmodified amino acids commonly found in naturally occurring proteins, and more particularly mean alanine, arginine, asparagine, aspartic acid, cysteine, glutamic acid, glutamine, glycine, histidine, isoleucine, leucine, lysine, methionine, phenylalanine, proline, serine, threonine, tryptophane, tyrosine, and valine. Herein, the natural amino acids may be indicated by three-letter codes or one-letter codes (*i.e.,* alanine: Ala or A; arginine: Arg or R; asparagine: Asn or N; aspartic acid: Asp or D; cysteine: Cys or C; glutamic acid: Glu or E; glutamine: Gln or Q; glycine: Gly or G; histidine: His or H; isoleucine: Ile or I; leucine: Leu or L; lysine: Lys or K; methionine: Met or M; phenylalanine: Phe or F; proline: Pro or P; serine: Ser or S; threonine: Thr or T; tryptophane: Trp or W; tyrosine: Tyr or Y; valine: Val or V).

As referred to herein, non-natural amino acids mean other amino acids than the natural amino acids, and also include modified versions of natural amino acids. The abbreviations for the non-natural amino acids referred to herein are listed below. As referred to herein, lower case letters of the alphabet can be replaced by upper case letters in abbreviations hereinbelow. For example, 2Nal, Nle, Phg, Tle, and Hcy are synonymous with 2NAL, NLE, PHG, TLE, and HCY, respectively. 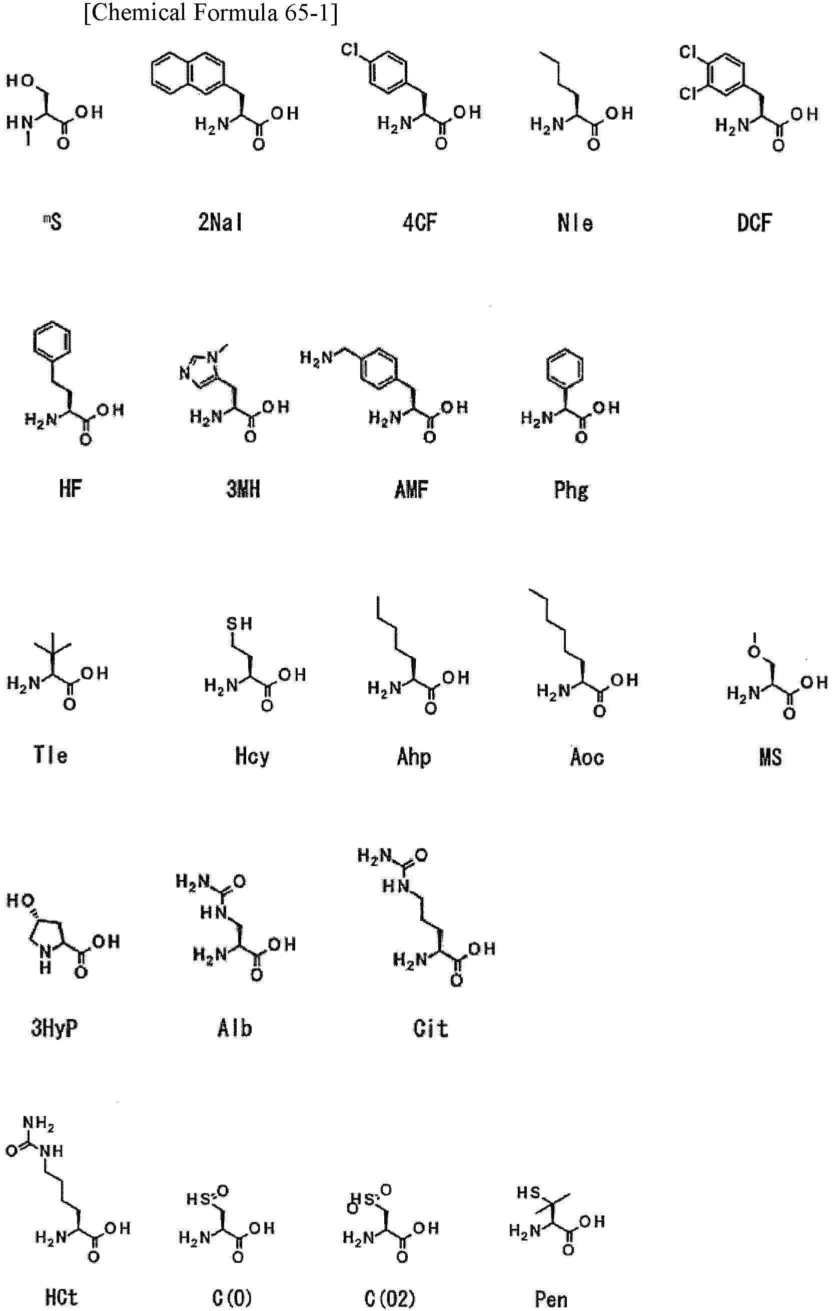 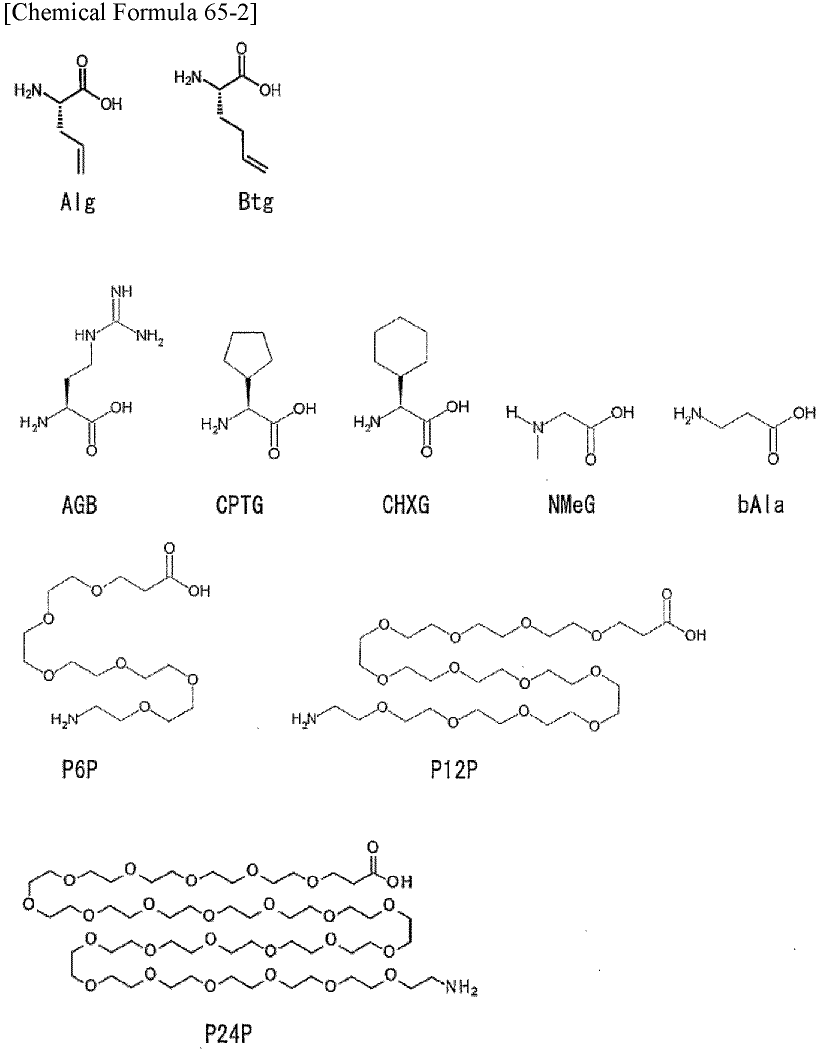 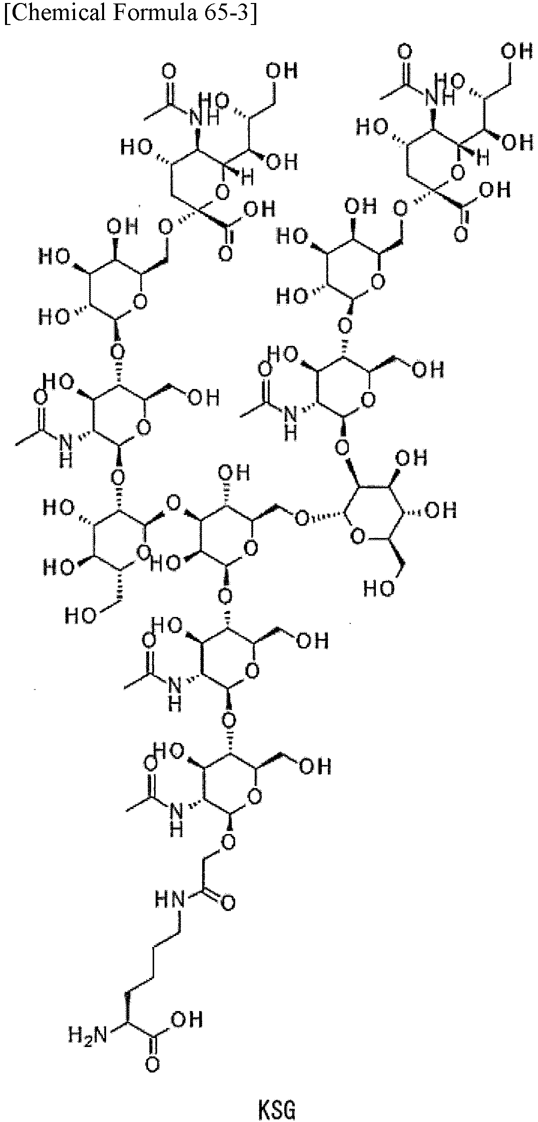 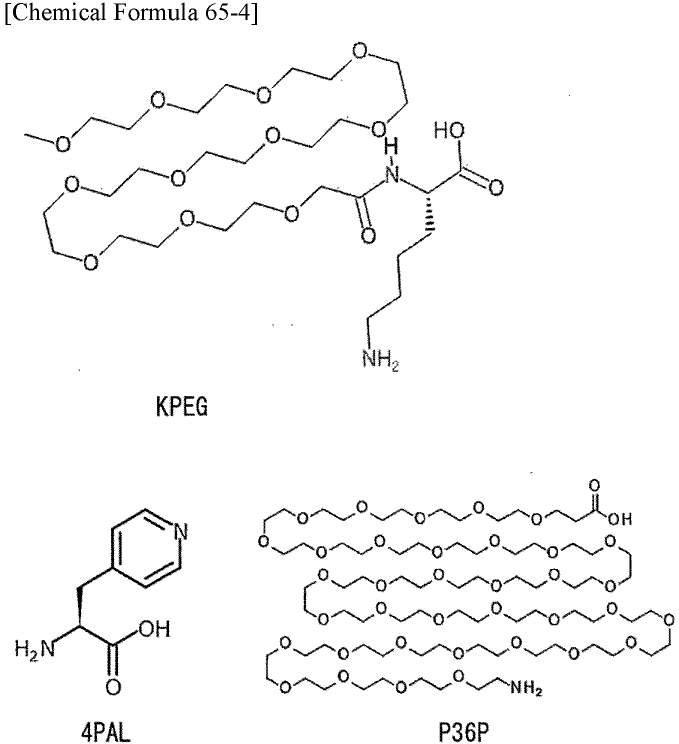
^{m}S: N-Methyl-L-serine
2Nal: 3-(2-Naphthyl)-L-alanine
4CF: 4-Chloro-L-phenylalanine
Nle: L-Norleucine
DCF: L-3,4-Dichlorophenylalanine
HF: L-Homophenylalanine
3MH: 3-Methyl-L-histidine
AMF: L-4-Aminomethylphenylalanine
Phg: L-Phenylglycine
Tle: L-Tert-Leucine
Hcy: L-Homocysteine
Ahp: (2S)-2-Aminoheptanoic acid
Aoc: (2S)-2-Amino-octanoic acid
MS: L-O-Methylserine
3Hyp: L-Hydroxyproline
Alb: L-2-Amino-3-ureidopropionic acid
Cit: L-Citrulline
HCt: L-Homocitrulline
C(O): (2R)-2-amino-3-hydrosulfinyl-propanoic acid
C(O2): (2R)-2-amino-3-hydrosulfonyl-propanoic acid
Pen: L-Penicillamine
Alg: L-Allylglycine
Btg: (2S)-2-Aminohex-5-enoic acid
AGB: (2S)-2-Amino-4-guanidino-butanoic acid
CPTG: L-Cyclopentylglycine
CHXG: L-Alpha-cyclohexylglycine
NMeG: Sarcosine
bAla: Beta-alanine
P6P: 3-[2-[2-[2-[2-[2-(2-Aminoethoxy)ethoxy]ethoxy]ethoxy]ethoxy]ethoxy]propanoic acid
P12P: 3-[2-[2-[2-[2-[2-[2-[2-[2-[2-[2-[2-(2-Aminoethoxy)ethoxy] ethoxy] ethoxy] ethoxy] ethoxy] ethoxy] ethoxy] ethoxy] ethoxy] ethoxy] et hoxy]propanoic acid
P24P: 3-[2-[2-[2-[2-[2-[2-[2-[2-[2-[2-[2-[2-[2-[2-[2-[2-[2-[2-[2-[2-[2-[2-[2-(2-Aminoethoxy)ethoxy] ethoxy] ethoxy] ethoxy] ethoxy] ethoxy] ethoxy] ethoxy] ethoxy] ethoxy] et hoxy] ethoxy] ethoxy] ethoxy] ethoxy] ethoxy] ethoxy] ethoxy] ethoxy] ethoxy] ethoxy] ethoxy]eth oxy]propanoic acid
KSG: (2R,4S,5R,6R)-5-acetamido-2-[[(2R,3R,4S,5R,6S)-6-[(2R,3S,4R,5R,6S)-5-acetamido-6-[(2S,3S,4S,5S,6R)-2-[[(2R,3R,4S,5S,6S)-6-[(2R,3S,4R,5R,6S)-5-acetamido-6-[(2R,3S,4R,5R,6R)-5-acetamido-6-[2-[[(5S)-5-amino-5-carboxy-pentyl]amino]-2-oxo-ethoxy]-4-hydroxy-2-(hydroxymethyl)tetrahydropyran-3-yl]oxy-4-hydroxy-2-(hydroxymethyl)tetrahydropyran-3-yl]oxy-4-[(2R,3S,4S,5S,6R)-3-[(2S,3R,4R,5S,6R)-3-acetamido-5-[(2S,3R,4S,5R,6R)-6-[[(2R,4S,5R,6R)-5-acetamido-2-carboxy-4-hydroxy-6-[(1R,2R)-1,2,3-trihydroxypropyl]tetrahydropyran-2-yl]oxymethyl]-3,4,5-trihydroxy-tetrahydropyran-2-yl]oxy-4-hydroxy-6-(hydroxymethyl)tetrahydropyran-2-yl]oxy-4,5-dihydroxy-6-(hydroxymethyl)tetrahydropyran-2-yl]oxy-3,5-dihydroxy-tetrahydropyran-2-yl]methoxy]-4,5-dihydroxy-6-(hydroxymethyl)tetrahydropyran-3-yl]oxy-4-hydroxy-2-(hydroxymethyl)tetrahydropyran-3-yl]oxy-3,4,5-trihydroxy-tetrahydropyran-2-yl]methoxy]-4-hydroxy-6-[(1R,2R)-1,2,3-trihydroxypropyl]tetrahydropyran-2-carboxylic acid
KPEG: (2S)-6-amino-2-[[2-[2-[2-[2-[2-[2-[2-[2-[2-[2-(2-methoxyethoxy)ethoxy]ethoxy]ethoxy]ethoxy]ethoxy]ethoxy]ethoxy]ethoxy]ethoxy]acetyl]a mino]hexanoic acid
4PAL: L-4-Pyridylalanine
P36P: 3-[2-[2-[2-[2-[2-[2-[2-[2-[2-[2-[2-[2-[2-[2-[2-[2-[2-[2-[2-[2-[2-[2-[2-[2-[2-[2-[2-[2-[2-[2-[2-[2-[2-[2-[2-(2-aminoethoxy)ethoxy]ethoxy]ethoxy]ethoxy]ethoxy]ethoxy]ethoxy]ethoxy]ethoxy]ethoxy]eth oxy] ethoxy] ethoxy] ethoxy]ethoxy] ethoxy] ethoxy] ethoxy] ethoxy] ethoxy] ethoxy] ethoxy]etho xy] ethoxy] ethoxy] ethoxy] ethoxy] ethoxy] ethoxy] ethoxy] ethoxy] ethoxy] ethoxy] ethoxy] ethox y]propanoic acid

As referred to herein, non-natural amino acids also include PEN analogous compounds represented by the following structural formulas.

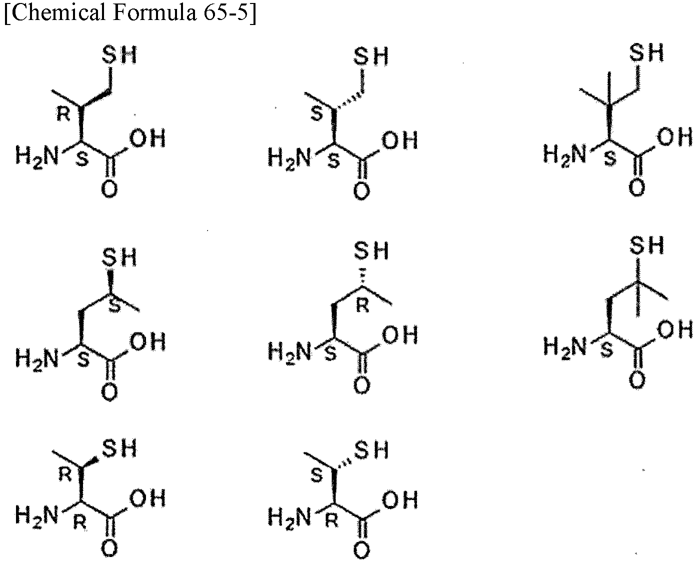

When the term "amino acid(s)" is used herein to describe a constitutional amino acid(s) of an oligopeptide and a polypeptide, this term refers to an amino acid residue(s). Unless otherwise specified, the amino acids as referred to herein are L-amino acids. When a specific amino acid is shown to be in the D-form, "D -" may be added to the abbreviation of the amino acid *(e.g.,* D-Asp, D-Glu, D-Lys, or D-Ser). As referred to herein, D-Asp is sometimes denoted by "d".

As referred to herein, the aliphatic amino acid is an amino acid represented by formula (IIa): 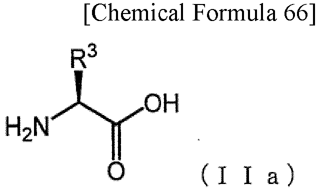 (wherein R³ is C₁₋₆ alkyl, C₂₋₆ alkenyl or C₃₋₆ cycloalkyl).

Examples of the natural aliphatic amino acid include Ala, Val, Leu, and Ile. Examples of the non-natural aliphatic amino acid include, but are not limited to, lNle, Tle, Ahp, Aoc, Alg, Btg, CPTG, and CHXG.

As referred to herein, the aromatic amino acid is an amino acid represented by formula (IIb): 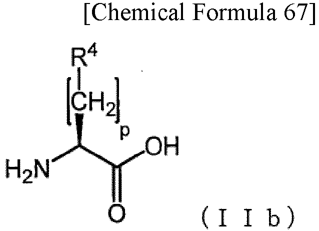 (wherein R⁴ is an aromatic group selected from phenyl, thienyl, pyridyl, naphthyl, indolyl, benzofuranyl, and benzothienyl, wherein the aromatic group may be substituted with one or more substituents independently selected from the group consisting of C₁₋₃ alkyl, halogen atoms, hydroxy, and C₁₋₃ alkoxy; and p is an integer of 0 to 3 (e.g., 0, 1, 2, or 3)).

Examples of the natural aromatic amino acid include Phe, Tyr, and Trp. Examples of the non-natural aromatic amino acid include, but are not limited to, 2Nal, 4CF, DCF, HF, Phg, and 4PAL.

As referred to herein, the basic amino acid is an amino acid represented by formula (IIc): 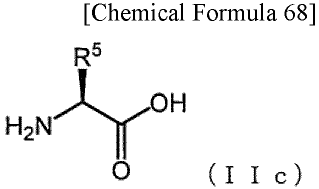
[wherein R⁵ is a group represented by
   the formula -(CH₂)_{qa}NH₂ (wherein qa is an integer of 1 to 6 (e.g., 1, 2, 3, 4, 5, or 6)),
   the formula 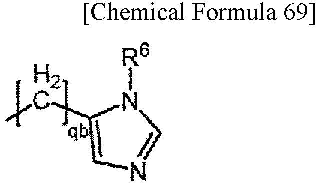
(wherein R⁶ is a hydrogen atom or C₁₋₃ alkyl, and qb is an integer of 1 to 6 (e.g., 1, 2, 3, 4, 5, or 6)),
   the formula -(CH₂)_{qc}NHC(=NH)NH₂ (wherein qc is an integer of 1 to 6 (e.g., 1, 2, 3, 4, 5, or 6)), or
   the formula 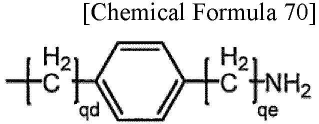
(wherein qd and qe are each independently an integer of 1 to 3 (e.g., 1, 2, or 3))].

Examples of natural basic amino acids include Lys, His, and Arg. Examples of non-natural basic amino acids include, but are not limited to, 3MH, AMF, and AGB.

As referred to herein, the neutral amino acid is an amino acid represented by formula (IId): 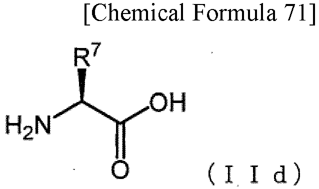 [wherein R⁷ is a group represented by the formula -(CH₂)ᵣₐNHCONH₂ (wherein ra is an integer of 1 to 6 (*e.g*., 1, 2, 3, 4, 5, or 6)) or the formula -(CH₂)_{rb}SH (wherein rb is an integer of 1 to 3 (*e.g.,* 1, 2, or 3))], Gly, Met, Pro, 3HyP, Asn, Gln, Ser, ^{m}S, MS, Thr, C(O), C(O2), Pen, or a Pen analog.

Examples of the natural neutral amino acid include Gly, Ser, Thr, Cys, Met, Pro, Asn, and Gln. Examples of the non-natural neutral amino acid include, but are not limited to, 3HyP, ^{m}S, MS, Alb, Cit, Hct, HCY, C(O), C(O2), Pen, and a Pen analog.

As referred to herein, the acidic amino acid is an amino acid represented by formula (IIe): 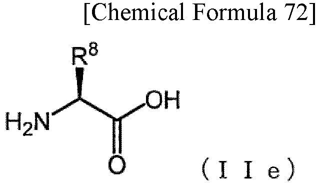 [wherein R⁸ is a group represented by the formula -(CH₂)ₛCOOH (wherein s is an integer of 1 to 6 (e.g., 1, 2, 3, 4, 5, or 6))].

Examples of the natural acidic amino acid include Asp and Glu.

As referred to herein, C₁₋₃ alkyl refers to a straight or branched chain alkyl having 1 to 3 carbon atoms, such as methyl, ethyl, 1-propyl, or 2-propyl.

As referred to herein, C₁₋₆ alkyl refers to a straight or branched chain alkyl having 1 to 6 carbon atoms, such as methyl, ethyl, 1-propyl, 2-propyl, 1-butyl, 2-butyl, 2-methyl-1-propyl, 2-methyl-2-propyl, 1-pentyl, 2-pentyl, 3-pentyl, 2-methyl-2-butyl, 3-methyl-2-butyl, 1-hexyl, 2-hexyl, 3-hexyl, 2-methyl-1-pentyl, 3-methyl-1-pentyl, 2-ethyl-1-butyl, 2,2-dimethyl-1-butyl, or 2,3-dimethyl-1-butyl.

As referred to herein, C₂₋₆ alkenyl refers to a straight or branched chain alkenyl having 2 to 6 carbon atoms and containing one or more double bonds, such as vinyl, 1 propenyl, 2-propenyl(allyl), 1-butenyl, 2-butenyl, 3-butenyl, 1-methyl-2-propenyl, 2-methyl-2-propenyl, 1-pentenyl, 2-pentenyl, 3-pentenyl, 4-pentenyl, 1-methyl-2-butenyl, 2 methyl-2-butenyl, 1-hexenyl, 2-hexenyl, 3-hexenyl, 4-hexenyl, 5-hexenyl, or 3-methyl-2-pentenyl, with vinyl, 2-propenyl(allyl), 3-butenyl, 4-pentenyl, or 5-hexenyl being preferred.

As referred to herein, C₃₋₆ cycloalkyl refers to a cyclic alkyl group having 3 to 6 carbon atoms, such as cyclopropyl group, cyclobutyl group, cyclopentyl group, or cyclohexyl group.

As referred to herein, the halogen atoms are F, Cl, Br, or I.

As referred to herein, C₁₋₃ alkoxy refers to a hydroxyl group substituted with one C₁₋₃ alkyl group as defined above, such as methoxy, ethoxy, 1-propoxy, or 2-propoxy.

As referred to herein, the term "glycan" means a structural unit composed of two or more monosaccharides bonded by a glycosidic bond. Specific monosaccharides and glycans are sometimes abbreviated, for example, as "GlcNAc-" and "SG-". When any of these abbreviations is used in a structural formula, the abbreviation is shown with an intention that an oxygen atom or nitrogen atom involved in a glycosidic bond at the reducing terminus to another structural unit is not included in the abbreviation indicating the glycan, unless specifically defined.

As referred to herein, a monosaccharide as a basic unit of a glycan is indicated for convenience so that in the ring structure, the position of a carbon atom bonded to an oxygen atom constituting the ring and directly bonded to a hydroxy group (or oxygen atom involved in a glycosidic bond) is defined as the 1-position (2-position only for sialic acids), unless otherwise specified.

The monosaccharide contained in the glycan is not particularly limited as long as it has the basic structure of a sugar, and various monosaccharides such as 6-membered and 5-membered sugars can be used. The monosaccharide may be a naturally occurring sugar or an artificially synthesized sugar, and a naturally occurring sugar is preferred. Examples of the monosaccharide include glucose (Glu), fructose (Fru), mannose (Man), galactose (Gal), glucosamine (Glc), N-acetylglucosamine (GlcNAc), glucuronic acid (GlucA), neuraminic acid (Neu), sialic acid/N-acetylneuraminic acid (Sia/NeuNAc/NeuSAc), galactosamine, N-acetylgalactosamine (GalNAc), xylose (Xyl), iduronic acid (IdoA), fucose (Fuc), aldotriose, glyceraldehyde, aldotetrose, erythrose, threose, aldopentose, ribose, lyxose, arabinose, aldohexose, allose, talose, gulose, altrose, idose, ketotriose, dihydroxyacetone, ketotetrose, erythrulose, ketopentose, xylulose, ribulose, ketohexose, psicose, sorbose, and tagatose.

When the glycan as referred to herein is indicated as a sign (*e.g*., GLY, SG, MSG, GlcNAc), the sign is intended, unless otherwise defined, to include carbon atoms ranging to the reducing terminus and exclude N or O involved in an N- or O-glycosidic bond.

As referred to herein, sialyl glycan (hereinafter referred to as "SG") refers to an N-linked complex glycan known to exhibit no antigenicity in the human body, the glycan being represented by the following structural formula and sequence: 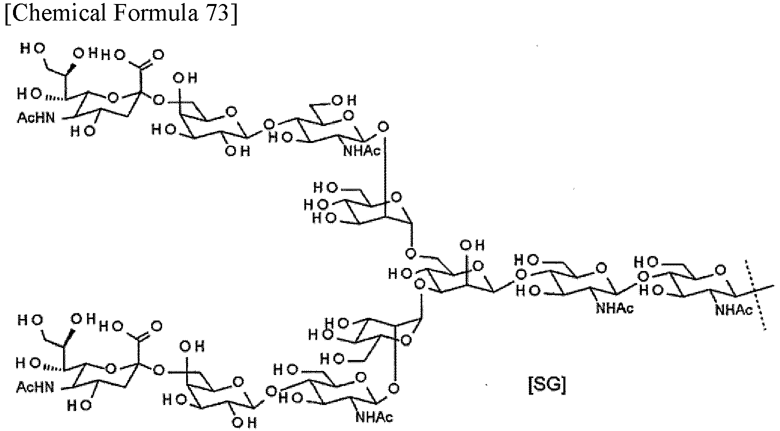 and 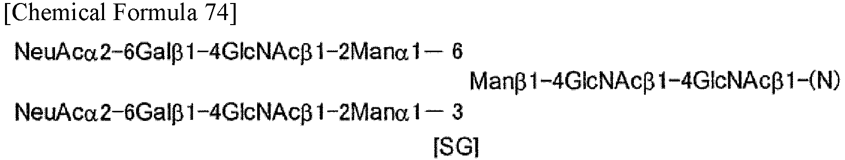 (wherein "-(N)" represents binding to a side chain of Asn through an N-glycosidic bond).

As referred to herein, the TSP1 inhibitory activity refers to the activity to inhibit at least one effect of TSP1, including angiostatic effect. The TSP1 inhibitory activity is measured by a cell adhesion inhibition assay using human TSP 1 and vascular endothelial cells as described hereinbelow in the Examples section. In this assay, when the 50% inhibitory concentration (IC₅₀) of a test substance is 500 nM or less, the test substance is determined to have TSP1 inhibitory activity.

### I. Cyclic Peptide

The present invention provides novel cyclic peptides having TSP 1 inhibitory activity or pharmaceutically acceptable salts thereof.

In one mode, the compound of the present invention is a cyclic peptide represented by formula (I) 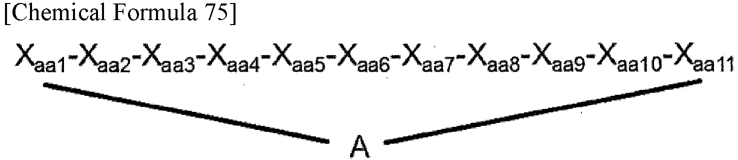 (hereinafter referred to as "the cyclic peptide I of the present(this) invention"). The cyclic peptide I of this invention has formed therein a cyclic structure in which the amino acids Xₐₐ₁ to Xₐₐ₁₁ are joined together by amide bonds to form an oligopeptide chain, and the N-terminal amino acid (Xₐₐ₁) and the C-terminal amino acid (Xₐₐ₁₁) of the oligopeptide chain are joined together via a ring-forming group A.

In formula (I), A is selected from the following ring-forming groups A₁ to A₅ having an amino acid structure:

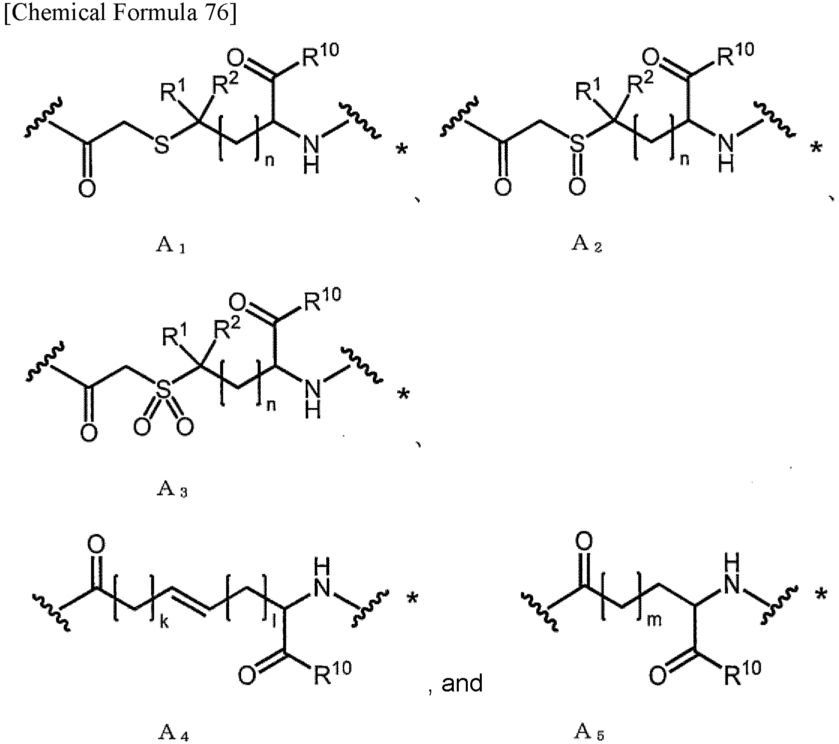

As found in either terminus of A, 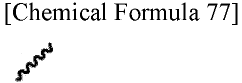 represents a point of attachment to the N-terminal amino group of Xₐₐ₁, and 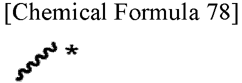 represents a point of attachment to the C-terminal carbonyl group of Xₐₐ₁₁. The asymmetric centers in the amino acid structures contained in the aforementioned ring-forming groups may have the R- or S-configuration.

In one mode, A is 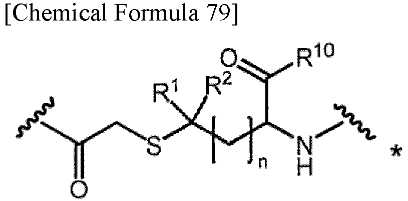 (hereinafter referred to as "A₁"). In the ring-forming group A₁, n is an integer of 0 to 3 *(e.g.,* 0, 1, 2, or 3), R¹ and R² are each independently a hydrogen atom or C₁₋₃ alkyl, and R¹⁰ is amino or hydroxy. C₁₋₃ alkyl is preferably methyl.

A preferred mode of A₁ is a ring-forming group 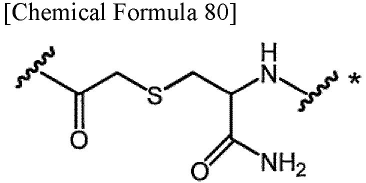 wherein R1 and R² are each a hydrogen atom, R¹⁰ is amino, and n is 0 (hereinafter referred to as "A₁ₐ"). A₁ₐ can be obtained by, for example, allowing the -SH group of Cys (provided that the carboxy group is converted to amide) which is amide-bonded to the C-terminus of Xₐₐ₁₁ to react with -COCH₂X (wherein X is a leaving group such as Cl) which is bonded to the N-terminal amino group of Xₐₐ₁. In such a ring-forming group, the Cys may be an L-amino acid or a D-amino acid. In particular, that version of the ring-forming group A₁ₐ, where the Cys (provided that the carboxy group is converted to amide) which is amide-bonded to the C-terminus of Xₐₐ₁₁ is an L-amino acid, is referred to as "A₁ₐ₁".

Another preferred mode of A₁ is a ring-forming group 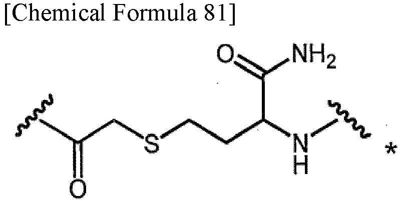 wherein R¹ and R² are each a hydrogen atom, R¹⁰ is amino, and n is 1 (hereinafter referred to as "A_{1b}"). A_{1b} can be obtained by, for example, allowing the -SH group of HCY (provided that the carboxy group is converted to amide) which is amide-bonded to the C-terminus of Xₐₐ₁₁ to react with -COCH₂X (wherein X is a leaving group such as Cl) which is bonded to the N-terminal amino group of Xₐₐ₁. In such a ring-forming group, the HCY may be an L-amino acid or a D-amino acid. In particular, that version of the ring-forming group A_{1b}, where the HCY (provided that the carboxy group is converted to amide) which is amide-bonded to the C-terminus of Xₐₐ₁₁ is an L-amino acid, is referred to as "A_{1b1}".

Another preferred mode of A₁ is a ring-forming group 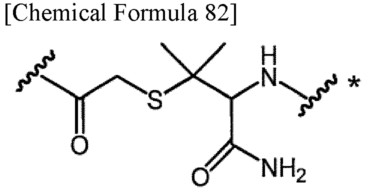 wherein R¹ and R² are each methyl, R¹⁰ is amino, and n is 0 (hereinafter referred to as "A_{1c}"). A_{1c} can be obtained by, for example, allowing the -SH group of Pen (provided that the carboxy group is converted to amide) which is amide-bonded to the C-terminus of Xₐₐ₁₁ to react with -COCH₂X (wherein X is a leaving group such as Cl) which is bonded to the N-terminal amino group of Xₐₐ₁. In such a ring-forming group, the Pen may be an L-amino acid or a D-amino acid. In particular, that version of the ring-forming group A_{1c}, where the Pen (provided that the carboxy group is converted to amide) which is amide-bonded to the C-terminus of Xₐₐ₁₁ is an L-amino acid, is referred to as "A_{1c1}".

In another mode, the ring-forming group A is 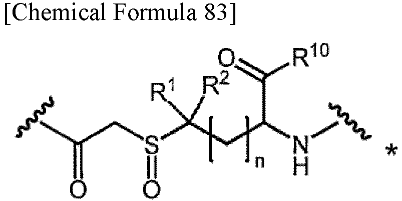 (hereinafter referred to as "A₂"). In the ring-forming group A₂, n is an integer of 0 to 3 (e.g., 0, 1, 2, or 3), R¹ and R² are each independently a hydrogen atom or C₁₋₃ alkyl, and R¹⁰ is amino or hydroxy. A₂ can be obtained by, for example, oxidizing the sulfide group of the ring-forming group A₁ as shown above to sulfoxide.

A preferred mode of A₂ is that version of said ring-forming group, where R¹ and R² are each a hydrogen atom, R¹⁰ is amino, and n is 0 (hereinafter referred to as "A₂ₐ"). A₂ₐ can be obtained by, for example, oxidizing the sulfide group of the ring-forming group A₁ₐ as shown above to sulfoxide. In particular, the ring-forming group obtained by oxidizing the sulfide group of A₁ₐ₁ to sulfoxide is referred to as "A₂ₐ₁".

In another mode, A is 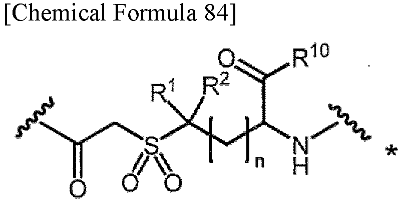 (hereinafter referred to as "A₃"). In the ring-forming group A₃, n is an integer of 0 to 3 (*e.g*., 0, 1, 2, or 3), R¹ and R² are each independently a hydrogen atom or C₁₋₃ alkyl, and R¹⁰ is amino or hydroxy. A₃ can be obtained by, for example, oxidizing the sulfide group of the ring-forming group A₁ as shown above to sulfone.

A preferred mode of A₃ is that version of said ring-forming group, where R¹ and R² are each a hydrogen atom, R¹⁰ is amino, and n is 0 (hereinafter referred to as "A₃ₐ"). A₃ₐ can be obtained by, for example, oxidizing the sulfide group of the ring-forming group A₁ₐ as shown above to sulfone. In particular, the ring-forming group obtained by oxidizing the sulfide group of A₁ₐ₁ to sulfone is referred to as "A₃ₐ₁".

In another mode, A is 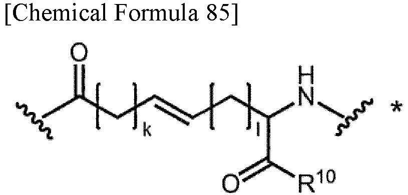 (hereinafter referred to as "A₄"). In the ring-forming group A₄, k and l are each independently an integer of 0 to 3 (e.g., 0, 1, 2, or 3), and R¹⁰ is amino or hydroxy. The ring-forming group A₄ can be obtained by, for example, olefin metathesis of the amino acid (provided that the carboxy group may be converted to amide) which has the group (CH₂)ₗCH=CH₂ on its side chain and is amide-bonded to the C-terminus of Xₐₐ₁₁, and the group -CO(CH₂)ₖCH=CH₂ which is bonded to the N-terminal amino group of Xₐₐ₁. In such a ring-forming group, the amino acid which is amide-bonded to the C-terminus of Xₐₐ₁₁ may be an L-amino acid or a D-amino acid.

In another mode, A is 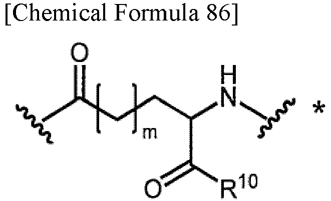 (hereinafter referred to as "As"). In the ring-forming group A₅, m is an integer of 1 to 7 *(e.g.,* 1, 2, 3, 4, 5, 6, or 7), and R¹⁰ is amino or hydroxy. As can be obtained by, for example, reducing the carbon-carbon double bond of A₄.

In a preferred mode, the ring-forming group A is A₁ₐ, A_{1b}, or A_{1c}, more preferably A₁ₐ.

In formula (I), Xₐₐ₁ is a residue of an aromatic amino acid. In a preferred mode, Xₐₐ₁ is a 2Nal residue.

In formula (I), Xₐₐ₂ is a residue of an aromatic amino acid, a basic amino acid, or an aliphatic amino acid. In a preferred mode, Xₐₐ₂ is a residue of Arg, AGB, PHG, or Tle.

In formula (I), Xₐₐ₃ and Xₐₐ₈ each have a structure independently selected from a residue of an amino acid represented by formula (III) 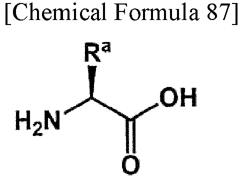
[wherein R^{a} is a group represented by the formula -(CH₂)ₜSH (wherein t is an integer of 1 to 3 *(e.g.,* 1, 2, or 3))] or formula (III') 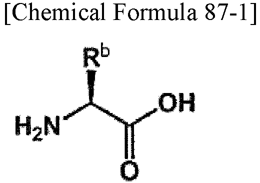
[wherein R^{b} is a group represented by the formula -CHᵥ(CH₃)₂₋ᵥCH_{w}(CH₃)_{2-w}SH (wherein v is an integer of 0 to 2; when v is 0 or 1, w is 2; and when v is 2, w is 0 or 1) or - CHₓ(CH₃)₂₋ₓSH (wherein x is 0 or 1)], in which thiol groups of Xₐₐ₃ and Xₐₐ₈ form a bond represented by -S-S-, -S-CH₂-S-, or -S-X-S- (wherein X is selected from 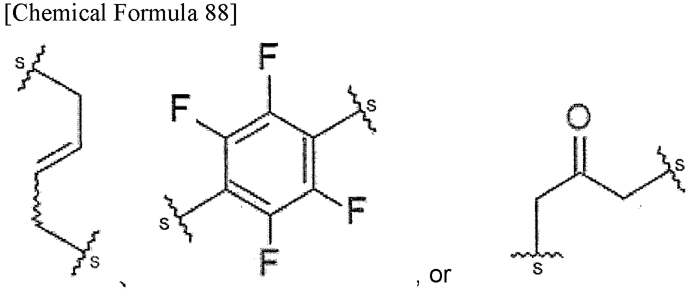
(wherein 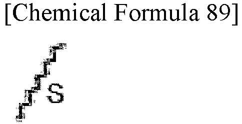
represents a point of attachment to S)), and at least one of Xₐₐ₃ and Xₐₐ₈ is a residue of the amino acid represented by formula (III). The structure is one in which the thiol groups of the side chains of two amino acid residues present at positions Xₐₐ₃ and Xₐₐ₈ of the cyclic peptide I of the present invention are crosslinked as described above. In one embodiment, Xₐₐ₃ and Xₐₐ₈ have a structure in which the Xₐₐ₃ residue is an HCY residue; the Xₐₐ₈ residue is an HCY residue, Cys residue, or Pen residue, preferably an HCY residue or Cys residue, more preferably an HCY residue; and the thiol groups of Xₐₐ₃ and Xₐₐ₈ are crosslinked as described above, preferably through a bond represented by -S-S- or -S-CH₂-S-, more preferably through a bond represented by -S-S- (*i.e.,* disulfide bond). In another embodiment, in the structure of Xₐₐ₃ and Xₐₐ₈, the Xₐₐ₃ residue is a Cys residue; the Xₐₐ₈ residue is an HCY residue, Cys residue, or Pen residue; and the thiol groups of Xₐₐ₃ and Xₐₐ₈ are crosslinked as described above, preferably through a bond represented by -S-S- or -S-CH₂-S-, more preferably through a bond represented by -S-S- (*i.e.,* disulfide bond). In another embodiment, in the structure of Xₐₐ₃ and Xₐₐ₈, the Xₐₐ₃ residue is a Pen residue; the Xₐₐ₈ residue is an HCY residue; and the thiol groups of Xₐₐ₃ and Xₐₐ₈ may be crosslinked as described above. In a preferred mode, in the structure of Xₐₐ₃ and Xₐₐ₈, the residue of an amino acid represented by formula (III) or (III') is an HCY residue or Cys residue. In a more preferred mode, in Xₐₐ₃ and Xₐₐ₈, the thiol groups of the side chains of two HCY residues present at positions Xₐₐ₃ and Xₐₐ₈ of the cyclic peptide I of the present invention are crosslinked as described above. It should be noted that the HCY residue is a residue of an amino acid represented by formula (III), where t = 2.

In a preferred mode, in the structure of Xₐₐ₃ and Xₐₐ₈, thiol groups form a bond represented by -S-S- or -S-CH₂-S-, more preferably -S-S-. In a more preferred mode, in the structure of Xₐₐ₃ and Xₐₐ₈, the residue of an amino acid represented by formula (III) or (III') is an HCY residue, and the thiol groups form a bond represented by -S-S-.

In formula (I), Xₐₐ₄ is a residue of a neutral amino acid. In a preferred mode, Xₐₐ₄ is a Gly residue.

In formula (I), Xₐₐ₅ is a residue of a basic amino acid. In a preferred mode, Xₐₐ₅ is a residue of Arg or AGB.

In formula (I), Xₐₐ₆ is a residue of a neutral amino acid or an acidic amino acid. In a preferred mode, Xₐₐ₆ is a residue of Asn or Asp.

In formula (I), Xₐₐ₇ is a residue of an aromatic amino acid. In a preferred mode, Xₐₐ₇ is a Trp residue.

In formula (I), Xₐₐ₉ is a residue of an aromatic amino acid, an aliphatic amino acid, or a basic amino acid. In a preferred mode, Xₐₐ₉ is a residue of Val, Arg, PHG, 4PAL, or AGB, more preferably a residue of Val, Arg, PHG, or 4PAL.

In formula (I), Xₐₐ₁₀ is a residue of an aromatic amino acid. In a preferred mode, Xₐₐ₁₀ is a Trp residue.

In formula (I), Xₐₐ₁₁ is a residue of an aliphatic amino acid. In a preferred mode, Xₐₐ₁₁ is a residue of Val, CPTG, or CHXG, more preferably a residue of Val or CPTG.

A preferred mode of the cyclic peptide I of the present invention is a cyclic peptide represented by formula (I), wherein
Xₐₐ₁ is a 2Nal residue;
Xₐₐ₂ is a residue of Arg, AGB, PHG, or Tle;
in the structure ofXₐₐ₃ and Xₐₐ₈, the residue of an amino acid represented by formula (III) or (III') is an HCY residue or Cys;
Xₐₐ₄ is a Gly residue;
Xₐₐ₅ is a residue of Arg or AGB;
Xₐₐ₆ is a residue of Asn or Asp;
Xₐₐ₇ is a Trp residue;
Xₐₐ₉ is a residue of Val, Arg, PHG, 4PAL, or AGB;
Xₐₐ₁₀ is a Trp residue; and
Xₐₐ₁₁ is a residue of Val, CPTG, or CHXG.

A yet more preferred mode of the cyclic peptide I of the present invention is a cyclic peptide selected from the group consisting of the compounds represented by the following formulas:

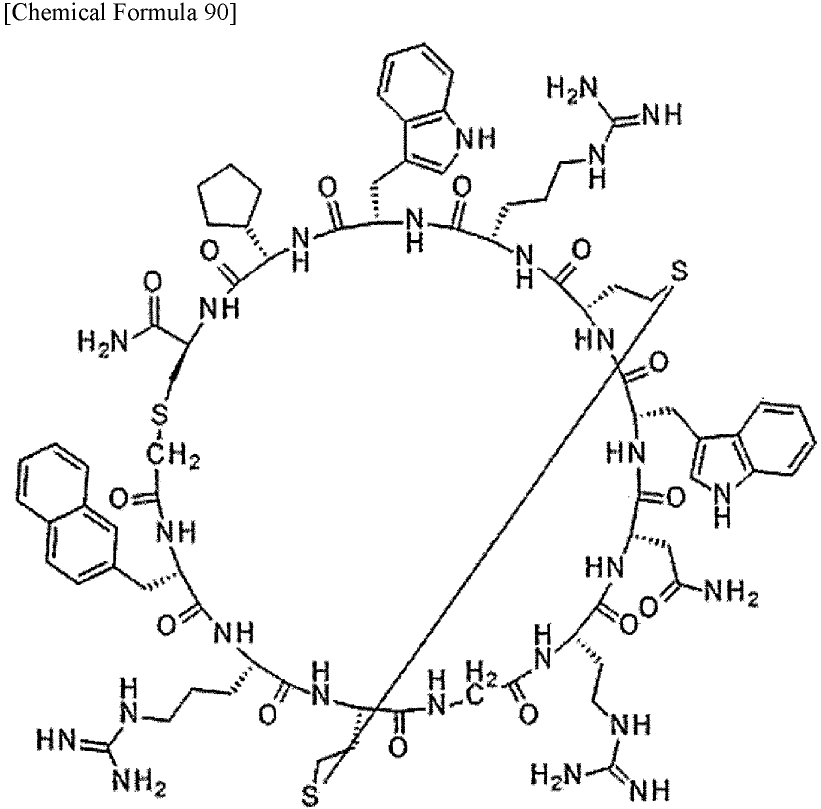

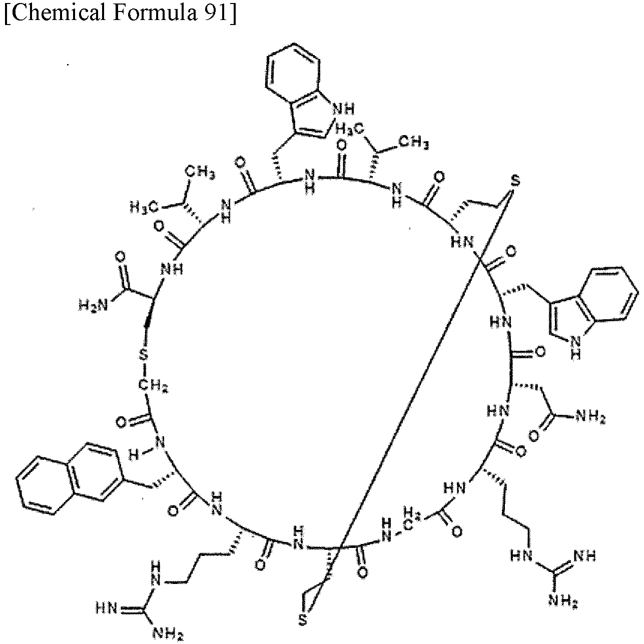

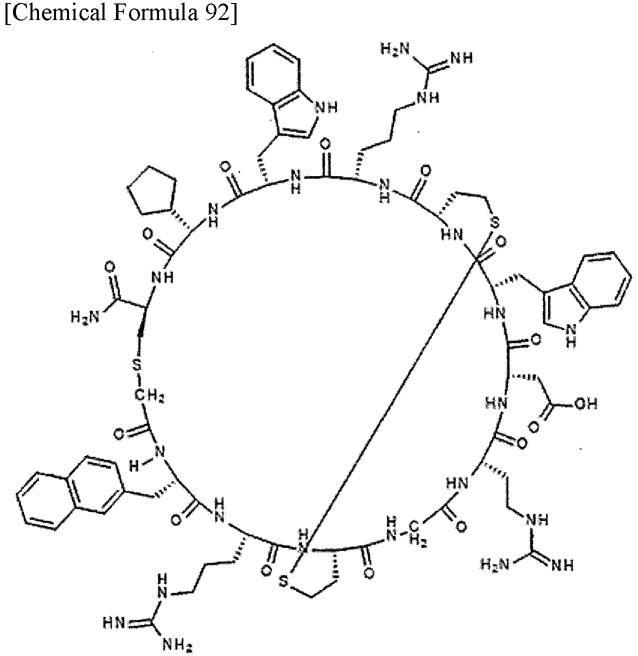

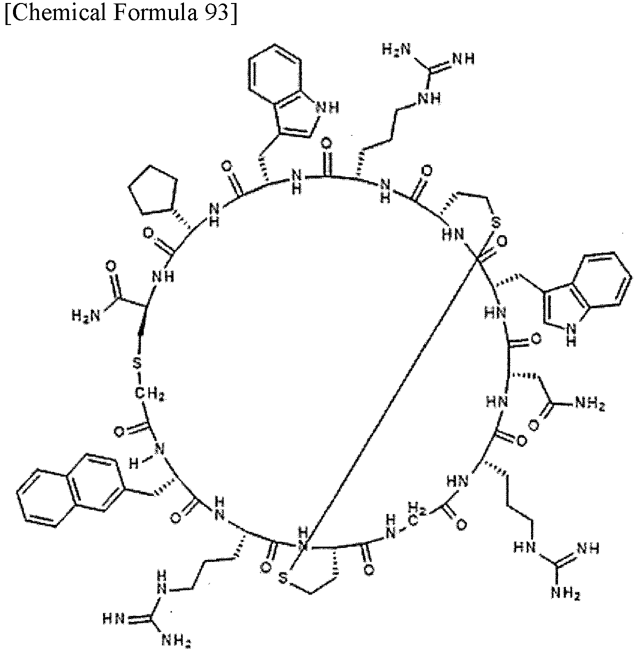

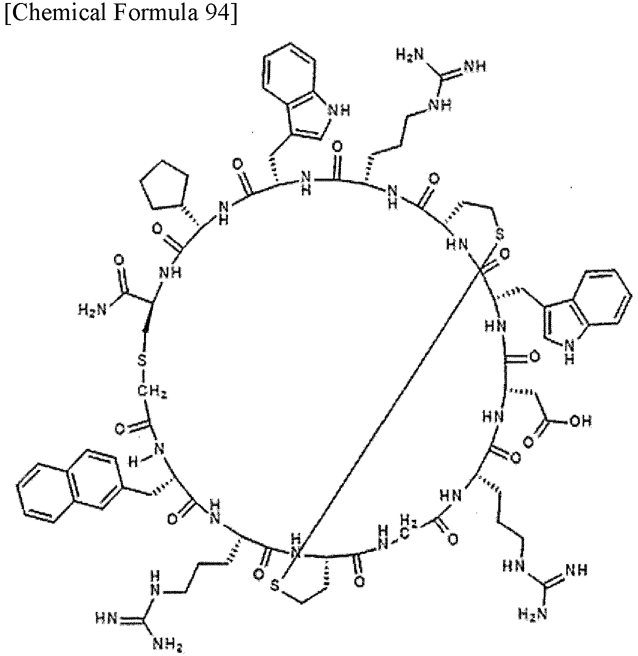

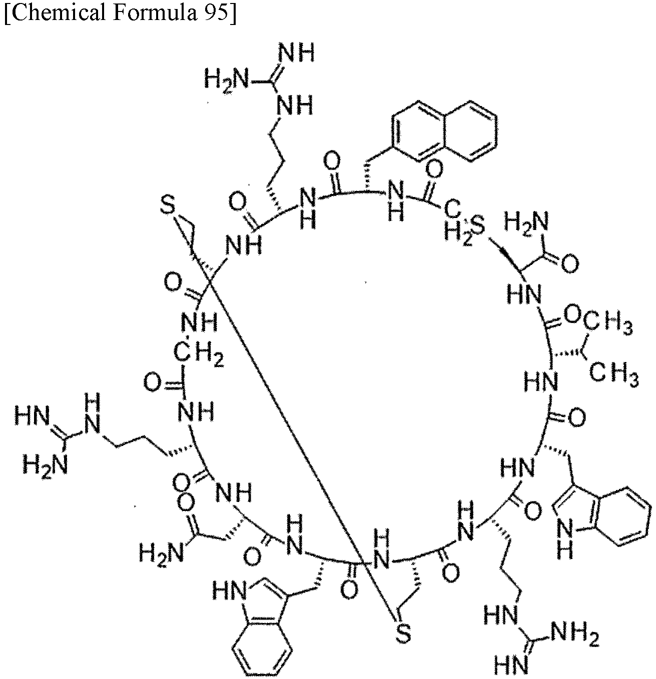

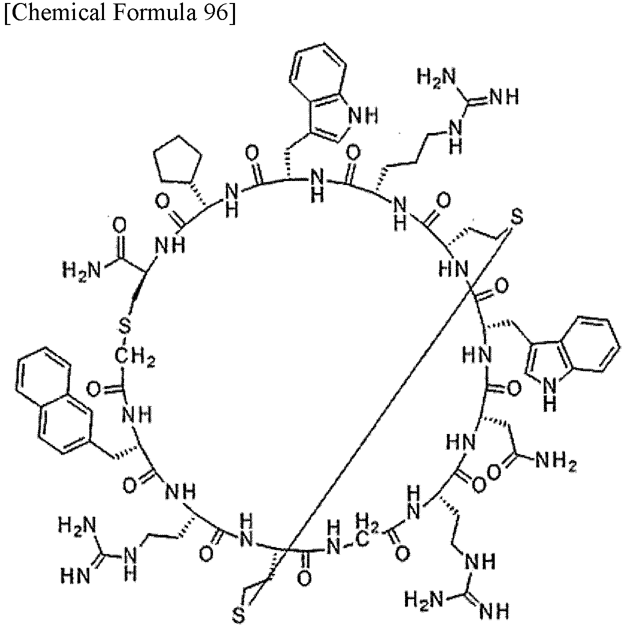

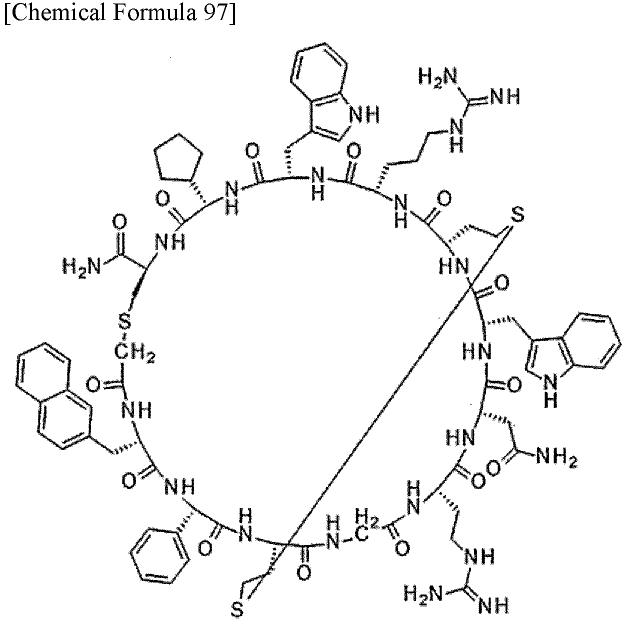

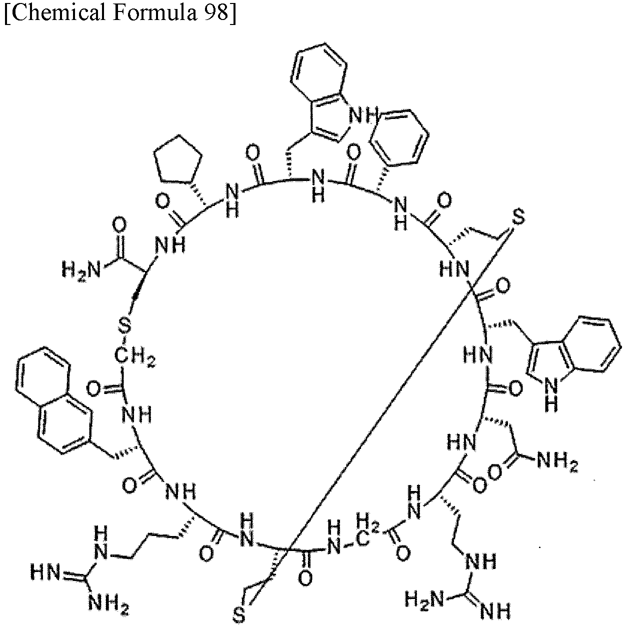

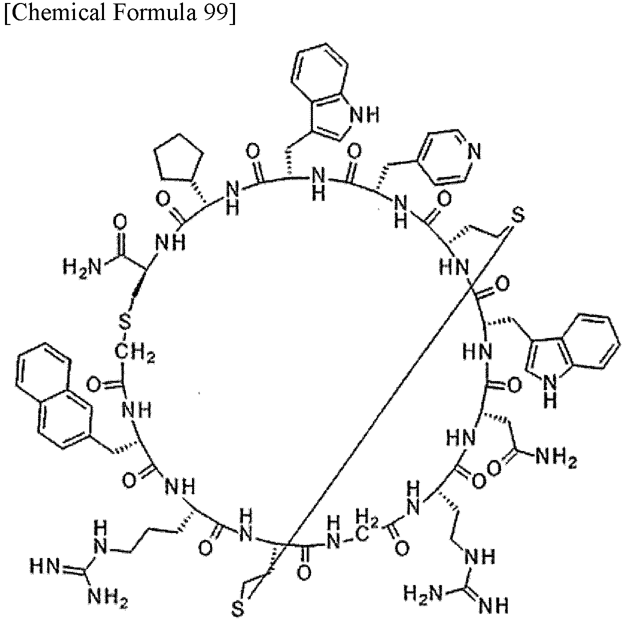

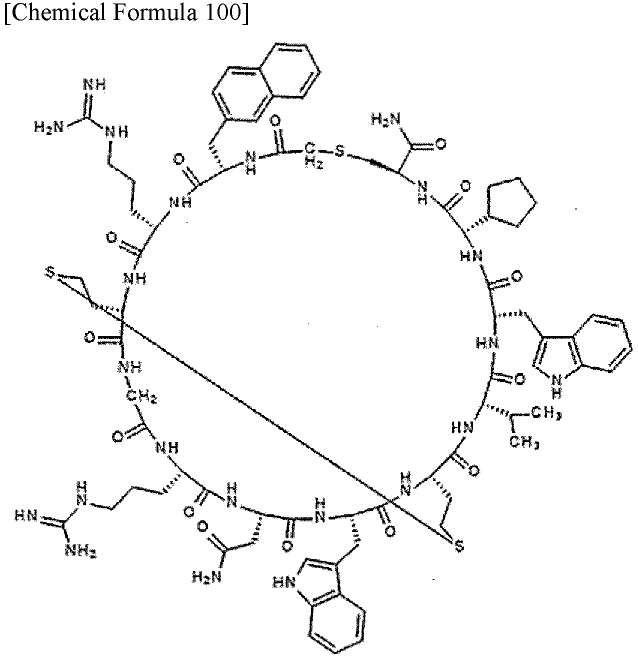

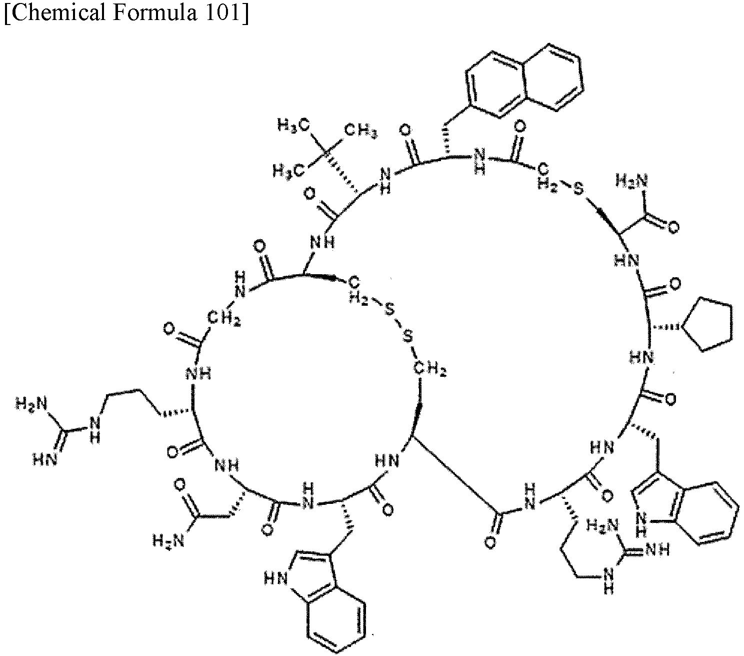

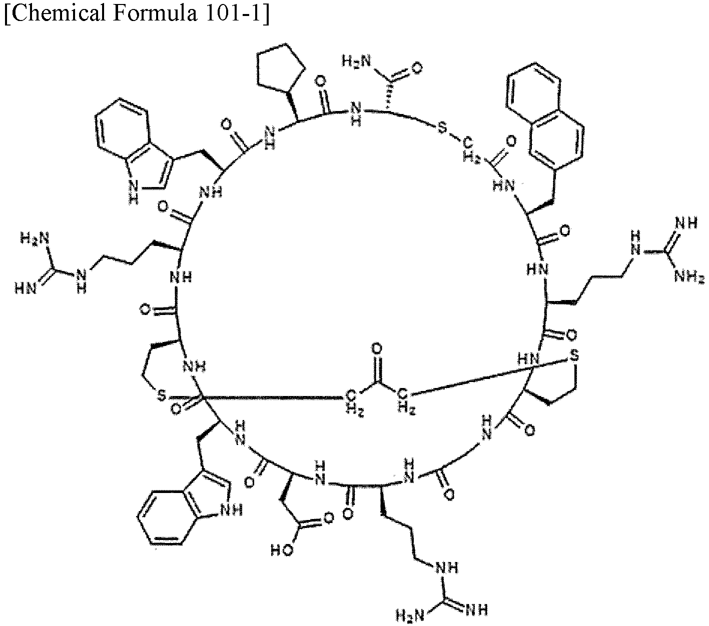

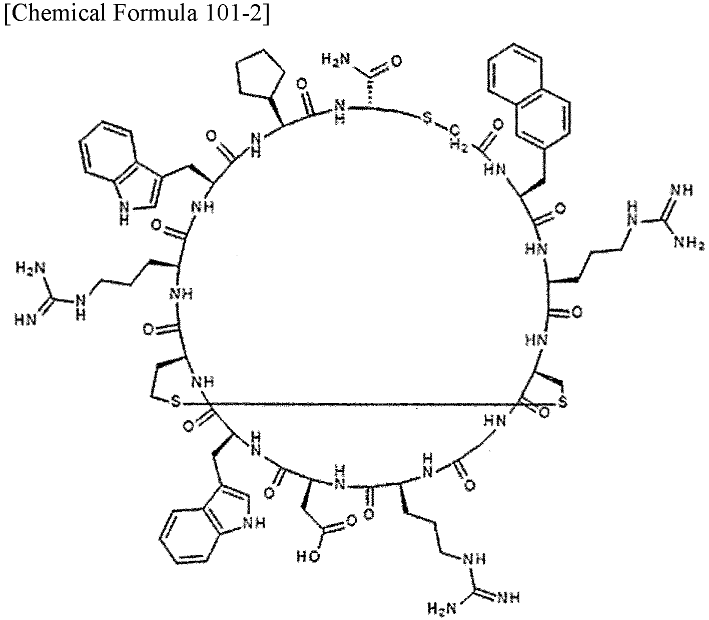

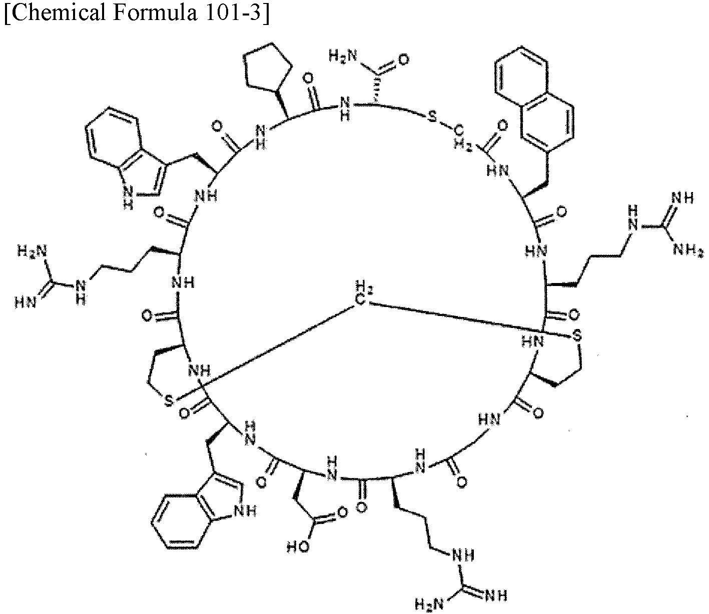

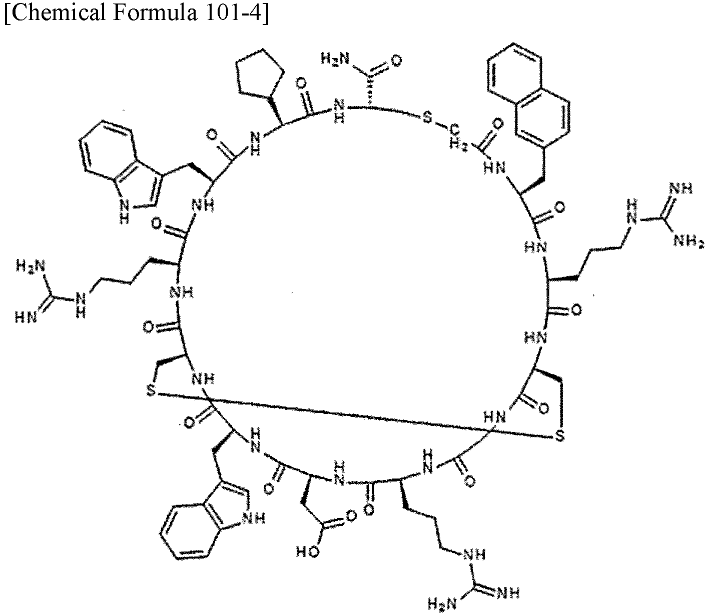

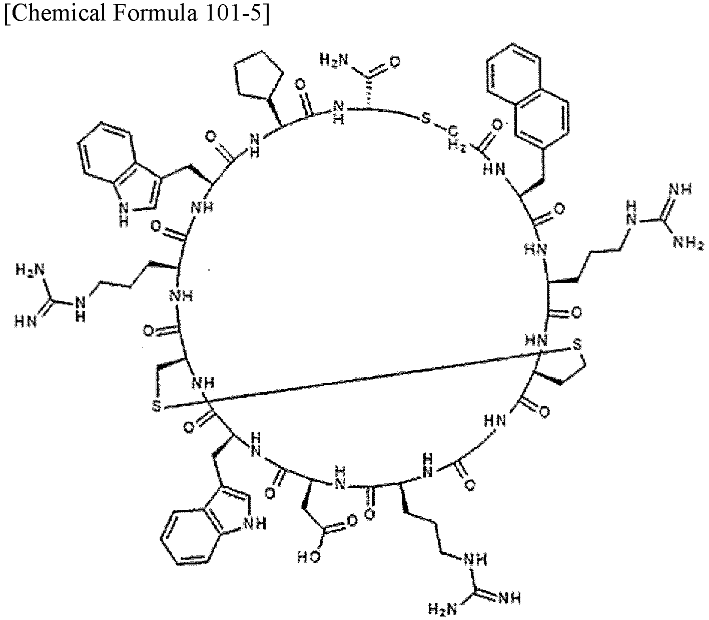

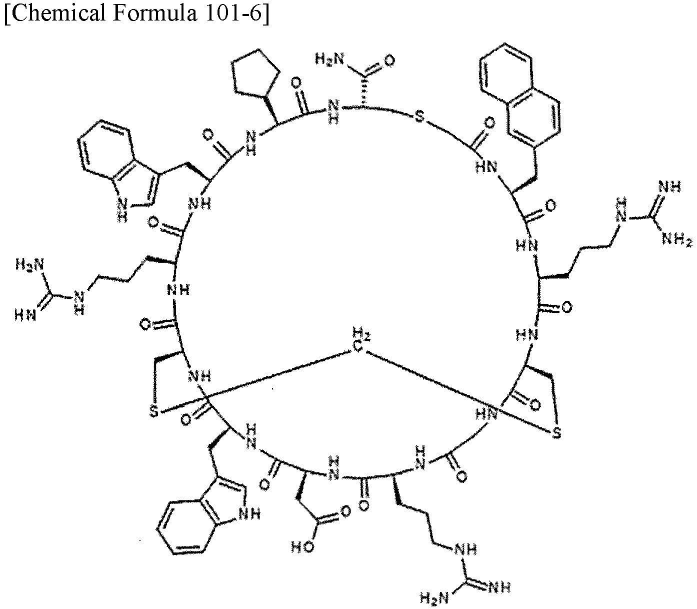

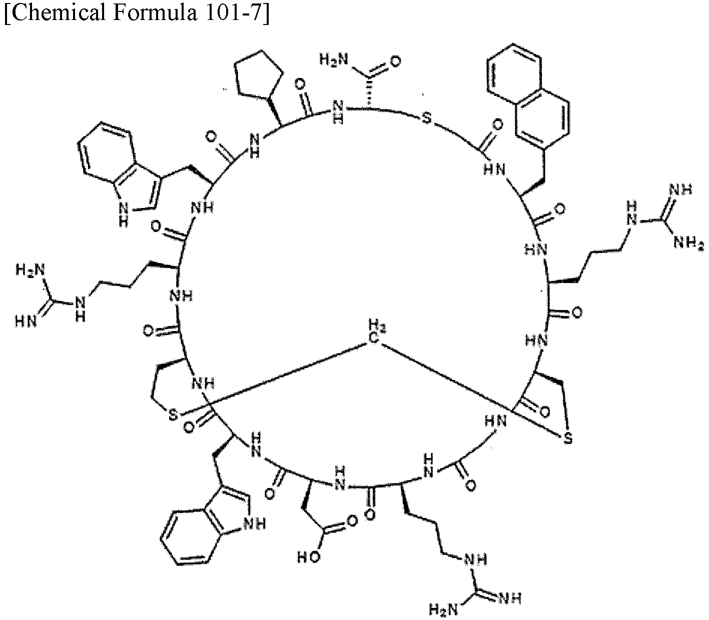

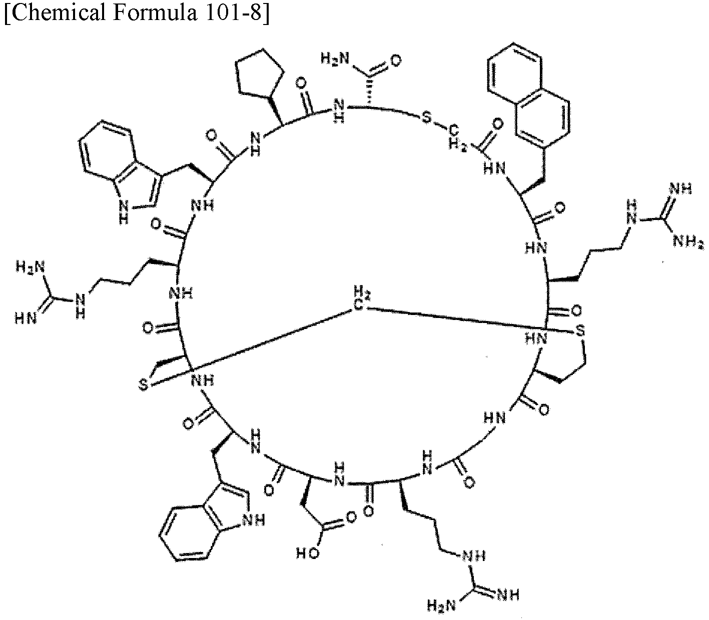

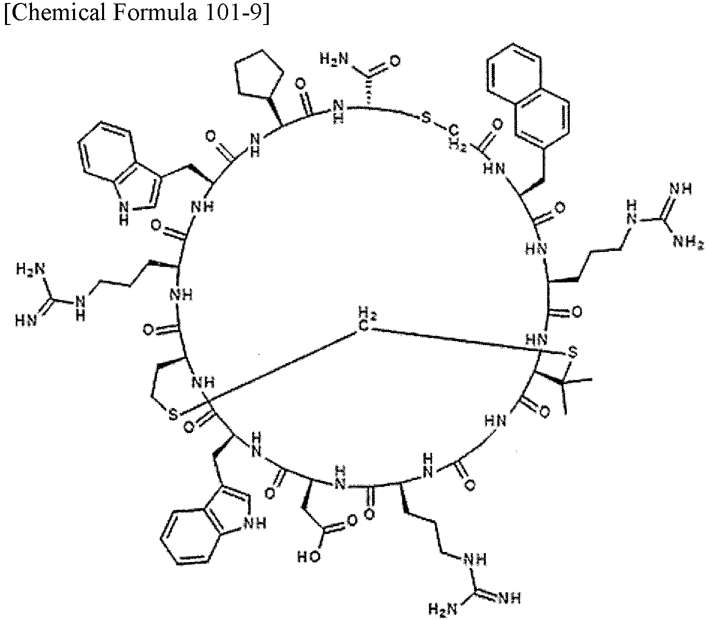

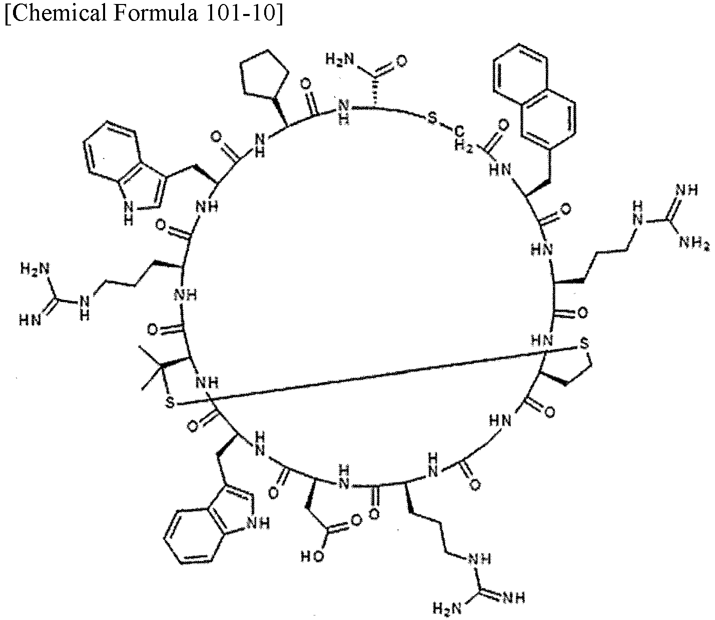

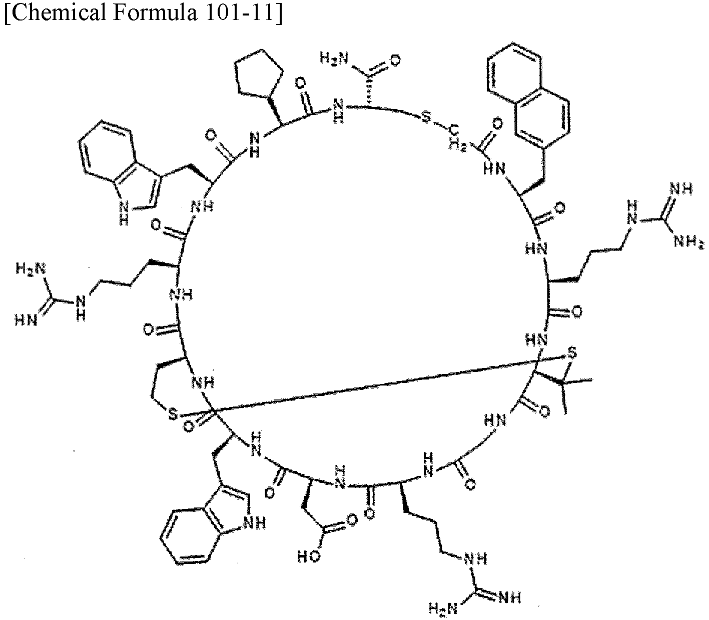

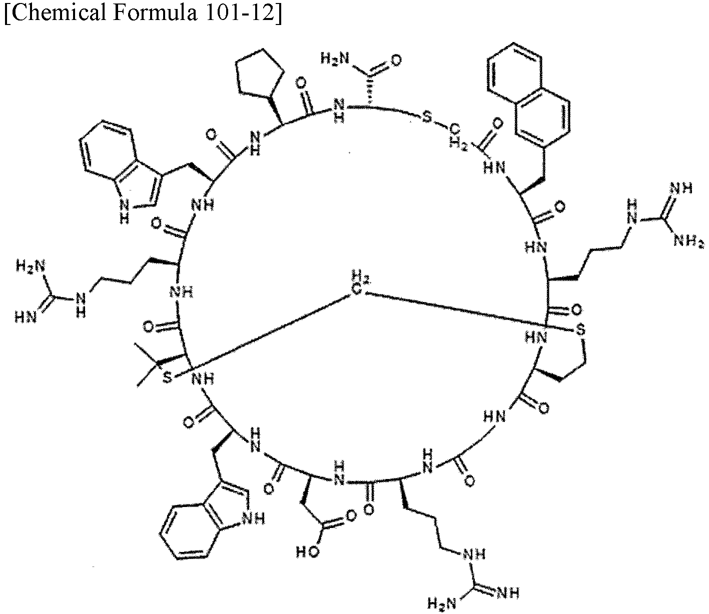

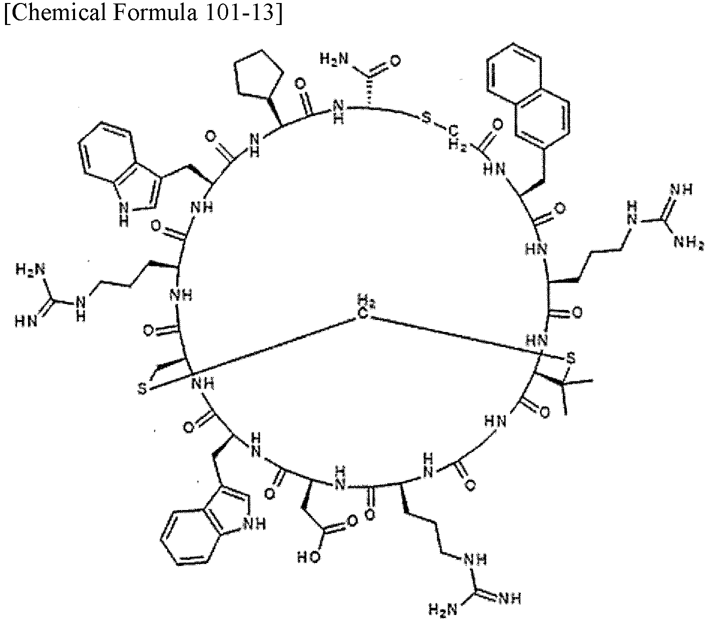

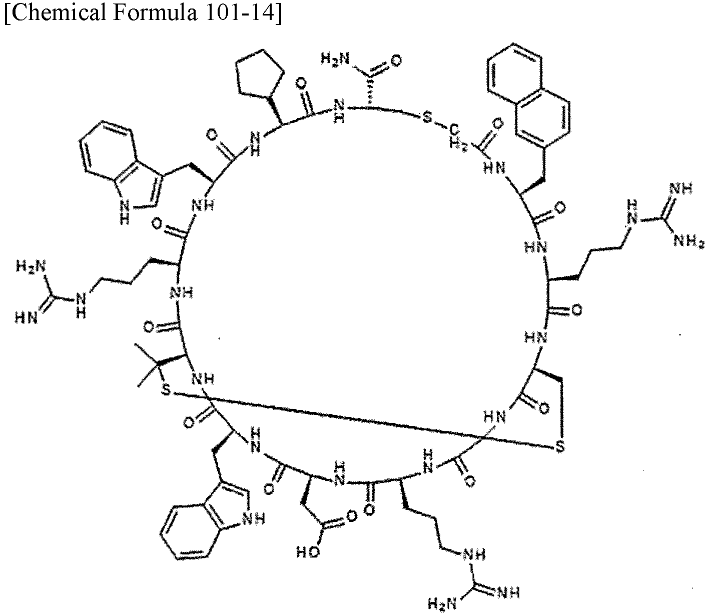

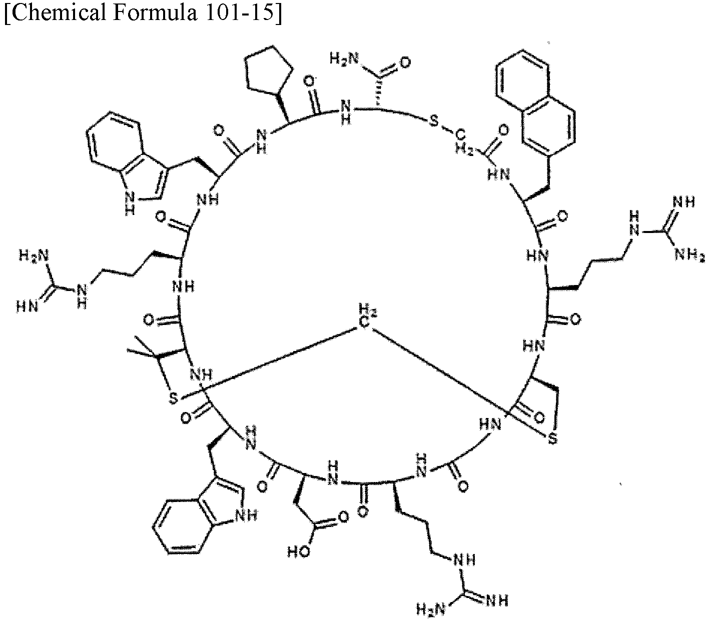

The cyclic peptides shown above are Peptide-1 to Peptide-12 and Peptide-125 to Peptide-139 of Examples as shown hereinbelow. In all of these cyclic peptides, the ring-forming group A is A₁ₐ₁.

When the cyclic peptide I of the present invention contains a basic group, the cyclic peptide I can combine with an acid to form a salt thereof, and such a salt is included in this invention. Examples of such a salt include inorganic acid salt, organic acid salt, amino acid salt, and sulfonate. Examples of the inorganic acid salt include hydrochloride, hydrobromate, sulfate, nitrate, and phosphate. Example of the organic acid salt include acetate, oxalate, malonate, fumarate, maleate, phthalate, and trifluoroacetate. Examples of the amino acid salt include glutamate, and aspartate. Examples of the sulfonate include methanesulfonate, benzenesulfonate, p-toluenesulfonate, 2,4-dimethylbenzenesulfonate, 2,4,6-trimethylbenzenesulfonate, 4-ethylbenzenesulfonate, and naphthalenesulfonate. A preferred mode of the pharmaceutically acceptable salt of the cyclic peptide I of this invention is an acetate, a hydrochloride, or a trifluoroacetate, more preferably an acetate.

When the cyclic peptide I of the present invention contains an acidic group, the cyclic peptide I can combine with a base to form a salt thereof, and such a salt is also included in this invention. Examples of such a salt include metal salt, inorganic amine salt, organic amine salt, and amino acid salt. Examples of the metal salt include: alkali metal salts such as sodium salt, potassium salt, and lithium salt; alkaline-earth metal salts such as calcium salt and magnesium salt; as well as aluminum salt, iron salt, zinc salt, copper salt, nickel salt, and cobalt salt. Examples of the inorganic amine salt include ammonium salt. Examples of the organic amine salt include morpholine salt, glucosamine salt, ethylenediamine salt, guanidine salt, diethylamine salt, triethylamine salt, dicyclohexylamine salt, diethanolamine salt, piperazine salt, and tetramethylammonium salt. Examples of the amino acid salt include lysine salt, and arginine salt.

The cyclic peptide I or pharmaceutically acceptable salt thereof of the present invention may be used in a crystalline form. Such a crystal may be formed exclusively of the cyclic peptide I or pharmaceutically acceptable salt thereof of this invention, or the cyclic peptide I or pharmaceutically acceptable salt thereof may be formed as a cocrystal or a solvate (*e.g.,* hydrate). One type of the cyclic peptide I or the pharmaceutically acceptable salt thereof of this invention may be used alone, or two or more types thereof may be used in combination as appropriate.

The cyclic peptide I or pharmaceutically acceptable salt thereof of the present invention can form an isotopic compound in which one or more constitutional atom is substituted with an isotopic atom in a non-natural proportion. The isotopic atom may be radioactive or non-radioactive, and examples thereof include deuterium (²H; D), tritium (³H; T), carbon-14 (¹⁴C), and iodine-125 (¹²⁵I). Compounds labeled with a radioactive isotopic atom can be used as therapeutic or prophylactic agents for various diseases, research reagents (e.g., assay reagent), diagnostic agents (e.g., image diagnostic agent), or the like. This invention also includes such a radioactive or non-radioactive isotopic compound.

The cyclic peptide I or pharmaceutically acceptable salt thereof of the present invention can be prepared by a method known in the art, such as chemical synthesis or cell-free translation system. For example, chemical synthesis of the cyclic peptide of this invention can be carried out according to a common solid-phase synthesis method using a 9-fluorenylmethoxycarbonyl group (Fmoc group) as an amino group-protecting group. Solid-phase synthesis can be performed using a commercially available automatic synthesizer (e.g., Syro II (produced by Biotage Japan), Liberty Blue (produced by CEM)).

For example, the cyclic peptide I of the present invention containing the ring-forming group A₁ can be obtained by following the procedure described below.

With the use of an automatic peptide synthesizer, amino acids protected with an amino group-protecting group (*e.g.,* Fmoc group) are coupled to a solid support (*e.g.,* Rink Amide Resin AM (produced by Novaviochem), 2-chlorotrityl chloride resin (produced by Novaviochem)) in a stepwise manner starting with the C-terminal amino acid (the C-terminal amino acid is a thiol-containing amino acid). Deprotection of amino groups is performed by a method known to skilled artisans (e.g., using 20% piperidine/1-methyl-2-pyrrolidinone to remove Fmoc groups). After the N-terminal amino acid is coupled and the amino group of this amino acid is deprotected, a group for forming a ring-forming group (*e.g.,* chloromethylcarbonyl group) is introduced (*e.g*., to allow chloroacetic acid to react with the N-terminal amino group) to form a cyclic peptide. Then, the resulting peptide is cleaved from the peptide resin by a conventional procedure (*e.g.,* using trifluoroacetic acid/ethanedithiol/triisopropylsilane/water to cleave from Rink Amide Resin AM). After a crude peptide is recovered by ether precipitation, the peptide of interest is purified by a conventional procedure (e.g., reverse-phase high-performance liquid chromatography).

The resulting cyclic peptides can be subjected to a conventional method (*e.g*., iodine oxidation method) to form a bond such as a disulfide bond between Xₐₐ₃ and Xₐₐ₈, thereby obtaining a bicyclic peptide.

The resulting peptide of interest can be converted to a desired salt by a conventional procedure.

The cyclic peptide I or pharmaceutically acceptable salt thereof of the present invention containing the ring-forming group A₂ or A₃ can be obtained by, for example, oxidizing the cyclic peptide I or pharmaceutically acceptable salt thereof of this invention containing the ring-forming group A₁ with an oxidizing agent (*e.g.*, m-chloroperoxybenzoic acid, hydrogen peroxide, or dimethyldioxirane).

For example, the cyclic peptide I of the present invention containing the ring-forming group A₄ or As can be obtained by following the procedure described below.

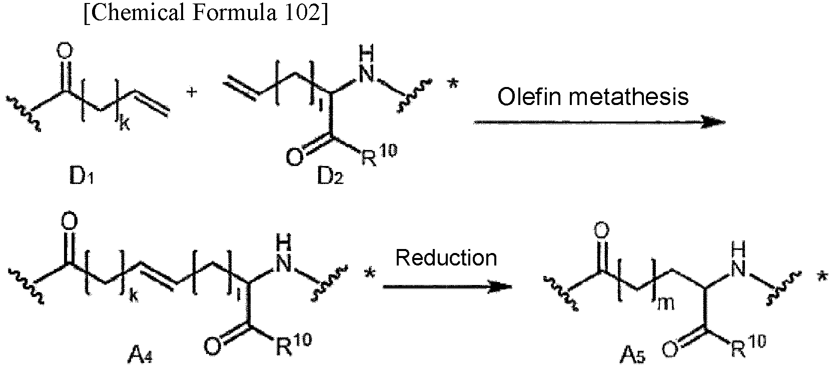

An oligopeptide containing the terminal groups D₁ and D₂ can be prepared using an automatic peptide synthesizer by taking the steps of coupling amino acids protected with an amino group-protecting group (e.g., Fmoc group) to a solid support in a stepwise manner starting with the C-terminal amino acid (the C-terminal amino acid is an olefin-containing amino acid), deprotecting the amino group of the N-terminal amino acid, and introducing a group for forming a ring-forming group (*e.g.*, 2-propenyl group).

After the oligopeptide containing the terminal groups D₁ and D2 is prepared, said oligopeptide is subjected to olefin metathesis reaction in the presence of an appropriate catalyst (*e.g*., 2nd generation Grubbs' catalyst), whereby the cyclic peptide containing the ring-forming group A₄ can be prepared. When the cyclic peptide containing the ring-forming group A₄ is subjected to reduction reaction under appropriate conditions (*e.g.,* in the presence of hydrogen and a Wilkinson's catalyst), the cyclic peptide containing the ring-forming group A₅ can be obtained.

The cyclic peptide I or pharmaceutically acceptable salt thereof of the present invention has TSP1 inhibitory activity and has an IC₅₀ of 500 nM or less, preferably 200 nM or less, as measured by a cell adhesion inhibition assay using human TSP1 and vascular endothelial cells as described hereinbelow in the Examples section. Therefore, the cyclic peptide I or pharmaceutically acceptable salt thereof of the present invention has an ability to block the adhesion of vascular endothelial cells to TSP 1, and are thus useful as blood flow improving agents.

In addition, the cyclic peptide I of the present invention has enhanced metabolic stability as compared with the monocyclic peptide due to its bicyclic nature. Therefore, the cyclic peptide I or pharmaceutically acceptable salt thereof of the present invention is useful for providing a means of treatment or prophylaxis of diseases or symptoms that can be treated or prevented by inhibiting a function of TSP 1 at lower frequency of administration than before.

II. Pharmaceutical composition comprising, as active component, cyclic peptide I or pharmaceutically acceptable salt thereof of the present invention

The present invention further provides a pharmaceutical composition comprising, as an active component, the cyclic peptide I or pharmaceutically acceptable salt thereof of the present invention (hereinafter referred to as "the pharmaceutical composition I of the present(this) invention"). The pharmaceutical composition I of this invention has excellent TSP1 inhibitory activity, and is useful for the treatment or prophylaxis of diseases or symptoms that can be treated or prevented by inhibiting a function of TSP1 (*e.g*., various diseases, including diseases that may be caused by angiogenesis inhibition, diseases that may be caused by increased thrombogenesis, inflammatory diseases, diseases that may be caused by deterioration of renal cellular function, diseases that may be caused by suppression of vasorelaxation, ischemic diseases, and cancerous diseases; and various symptoms, including vaso-occlusive crisis associated with sickle cell disease, angiogenesis inhibition, tissue necrosis, insulin resistance, and common wounds). Examples of the diseases that may be caused by angiogenesis inhibition include critical limb ischemia, and peripheral vascular disorder. Examples of the diseases that may be caused by increased thrombogenesis include myocardial infarction, and peripheral vascular disorder (PAD). Examples of the inflammatory diseases may include obesity-induced inflammation, and inflammation during aortic aneurysm development. Examples of the diseases that may be caused by deterioration of renal cellular function include diabetic nephropathy, IgA nephropathy, chronic renal failure, and acute renal failure. Examples of the diseases that may be caused by suppression of vasorelaxation include kidney injury, and ischemia-reperfusion injury. Examples of the ischemic diseases include myocardial infarction, and angina. Examples of the cancerous diseases include squamous cancer, breast cancer, and pancreatic cancer. TSP1 is reported to promote the progress of squamous cancer, breast cancer, and pancreatic cancer (refer to e.g., NPL 21, p.39 left col. line 14 from bottom to right col. line 3, and Table 4).

The pharmaceutical composition I of the present invention has excellent TSP 1 inhibitory activity and may have a blood flow improving effect, and is thus useful for the treatment or prophylaxis of diseases that can be treated or prevented by improving blood flow. Examples of such diseases include chronic limb threatening ischemia, critical limb ischemia, peripheral vascular disorder, myocardial infarction, angina, kidney injury, and ischemia-reperfusion injury. Examples of the chronic limb threatening ischemia include diabetic foot, and examples of the diabetic foot include diabetic foot ulcer.

In a preferred mode, the pharmaceutical composition I of this invention is useful for the treatment or prophylaxis of critical limb ischemia, peripheral arterial disease, or chronic limb threatening ischemia, and is, for example, useful for the acceleration of wound healing in critical limb ischemia patients and for the improvement of prognosis after endovascular treatment for critical limb ischemia.

It is known that the expression of TSP1 increases in the lower-limb skeletal muscles of critical limb ischemia patients. Although not wishing to be bound by any theory, it is believed that the pharmaceutical composition I of the present invention blocks the adhesion of vascular endothelial cells to TSP1, and thereby can promote angiogenesis or vasodilation inhibited by TSP1. By virtue of this function, the pharmaceutical composition I of this invention can accelerate wound healing in critical limb ischemia patients and improve prognosis (*e.g*., prevent restenosis) when combined with endovascular treatment with a catheter or the like.

As referred to herein, the treatment or prophylaxis of diseases or symptoms include prevention of the onset of diseases or suppression or inhibition of the exacerbation or progress of such diseases, alleviation of one or more symptoms found in individuals suffering from diseases or suppression of the exacerbation or progress of such symptoms, and treatment or prophylaxis of secondary diseases.

The pharmaceutical composition I of the present invention can be formulated into a pharmaceutical preparation by mixing the cyclic peptide I or pharmaceutically acceptable salt thereof of this invention with any appropriate pharmaceutically acceptable additives. For example, the pharmaceutical composition I of this invention can be administered as oral preparations such as tablets, capsules and granules, or as parenteral preparations such as injectables and percutaneous absorption agents.

Such pharmaceutical preparations are prepared by a well-known method using different additives, including excipient, binder, disintegrant, lubricant, emulsifier, stabilizer, diluent, injectable solvent, solubilizer, suspending agent, isotonizing agent, buffer, analgesic, antiseptic, and antioxidant.

Examples of the excipient include organic excipients or inorganic excipients. Examples of the organic excipients include sugar derivatives such as lactose and saccharose; starch derivatives such as cornstarch and potatostarch; cellulose derivatives such as crystalline cellulose; and gum Arabic. Examples of the inorganic excipients include sulfates such as calcium sulfate.

Examples of the binder include such excipients as mentioned above; gelatin; polyvinylpyrrolidone; and polyethylene glycol.

Examples of the disintegrant include such excipients as mentioned above; chemically modified starch or cellulose derivatives such as sodium croscarmellose and sodium carboxymethyl starch; and crosslinked polyvinylpyrrolidone.

Examples of the lubricant include talc; stearic acid; colloidal silica; waxes such as beeswax and spermaceti wax; sulfates such as sodium sulfate; lauryl sulfates such as sodium lauryl sulfate; and such starch derivatives as mentioned above as excipients.

Examples of the emulsifier include colloidal clays such as bentonite and veegum; anionic detergents such as sodium lauryl sulfate; cationic detergents such as benzalkonium chloride; and nonionic detergents such as polyoxyethylene alkyl ether.

Examples of the stabilizer include p-hydroxybenzoic esters such as methylparaben and propylparaben; alcohols such as chlorobutanol; and phenolic compounds such as phenol and cresol.

Examples of the diluent include water, ethanol, and propylene glycol.

Examples of the injectable solvent include water, ethanol, and glycerin.

Examples of the solubilizer include polyethylene glycol, propylene glycol, D mannitol, benzyl benzoate, ethanol, trisaminomethane, cholesterol, triethanolamine, sodium carbonate, and sodium citrate.

Examples of the suspending agent include various detergents such as stearyl triethanolamine, sodium lauryl sulfate, lauryl aminopropionic acid, lecithin, benzalkonium chloride, benzethonium chloride, and glyceryl monostearate; and hydrophilic polymers such as polyvinyl alcohol, polyvinylpyrrolidone, carboxymethylcellulose sodium, methylcellulose, hydroxymethylcellulose, hydroxyethylcellulose, and hydroxypropylcellulose.

Examples of the isotonizing agent include sodium chloride, glycerin, and D mannitol.

Examples of the buffer include phosphate, acetate, carbonate, citrate and other buffer solutions.

Examples of the analgesic include benzyl alcohol.

Examples of the antiseptic include p-hydroxybenzoic esters, chlorobutanol, benzyl alcohol, phenethyl alcohol, dehydroacetic acid, and sorbic acid.

Examples of the antioxidant include sulfites and ascorbic acid.

The subject to be administered the pharmaceutical composition I of the present invention is, for example, a mammalian animal, preferably a human.

The administration route of the pharmaceutical composition I of the present invention can be either of oral and parenteral administrations, and a suitable administration route can be selected depending on the disease to be treated. Further, the administration route of said inventive pharmaceutical composition can be either of systemic and topical administrations. Examples of parenteral administrations include intravenous administration, intraarterial administration, intramuscular administration, intracutaneous administration, subcutaneous administration, intraperitoneal administration, percutaneous administration, intraosseous administration, and intraarticular administration. A preferred mode of the administration route of said inventive pharmaceutical composition I is intravenous administration, subcutaneous administration, or percutaneous administration.

The cyclic peptide I or pharmaceutically acceptable salt thereof of the present invention is administered to a subject in a therapeutically or prophylactically effective amount. As used herein, the term "therapeutically or prophylactically effective amount" means an amount required for an active agent to exhibit a therapeutic or prophylactic effect in consideration of particular disease, dosage form, and administration route, and is determined, as appropriate, depending on the species of a subject, type of a disease, symptom, sex, age, pre-existing condition, and other elements.

The dose of the cyclic peptide I or pharmaceutically acceptable salt thereof of the present invention is determined, as appropriate, depending on the species of a subject, type of a disease, symptom, sex, age, pre-existing condition, and other elements. Human adults can take medication with said inventive cyclic peptide I or pharmaceutically acceptable salt thereof in a dose of generally 0.1 to 1000 mg/kg, preferably 0.1 to 10 mg/kg, once every 1 to 7 days, or two or three or more times daily.

The pharmaceutical composition I of the present invention may be combined with at least one known therapeutic agent or therapy. For example, during critical limb ischemia treatment, the pharmaceutical composition I of this invention can be administered before, after or simultaneously with endovascular treatment with a catheter or the like.

The cyclic peptide I or pharmaceutically acceptable salt thereof of this invention can be delivered by means of a drug-eluting stent (DES). The drug-eluting stent refers to a stent that has an active drug carried on its surface so that it can gradually elute the active drug after being placed into a blood vessel.

Furthermore, the present invention is directed to a method for the treatment or prophylaxis of a disease or symptom, the method comprising administering a therapeutically or prophylactically effective amount of the cyclic peptide I or pharmaceutically acceptable salt thereof of this invention to a subject in need thereof.

As referred to in the present invention, the "subject" is, for example, a mammalian animal, preferably a human.

As referred to in the present invention, the term "administration" includes oral and parenteral administrations, and can be either of systemic and topical administrations. Examples of parenteral administrations include intravenous administration, intraarterial administration, intramuscular administration, intracutaneous administration, subcutaneous administration, intraperitoneal administration, percutaneous administration, intraosseous administration, and intraarticular administration. A preferred mode of the "administration" as referred to in this invention is intravenous administration, subcutaneous administration, or percutaneous administration.

Examples of the "disease(s) or symptom(s)" as referred to in the present invention include diseases or symptoms that can be treated or prevented by inhibiting a function of TSP1. According to this invention, the treatment or prophylaxis of critical limb ischemia, peripheral arterial disease, or chronic limb threatening ischemia can be achieved, for example.

### III. Conjugate

The present invention further provides a conjugate or pharmaceutically acceptable salt thereof in which the cyclic peptide is bonded to the carrier molecule.

### 1. Carrier Molecule

In the conjugate of the present invention, the carrier molecule functions as a carrier that improves pharmacokinetics by binding to the cyclic peptide. The carrier molecule of the present invention is not limited to a particular molecule as long as it functions as a carrier for the cyclic peptide and the conjugate of this invention can exert a TSP-1 inhibitory effect (*i.e.,* has TSP-1 inhibitory activity). The carrier molecule can be, for example, a variety of natural or synthetic polymers, and suitably natural or non-natural proteins can be adopted. From the viewpoint of pharmacokinetics, the carrier molecule of this invention has binding affinity with a neonatal Fc receptor (FcRn). The binding affinity with FcRn allows utilization of the FcRn-mediated recycling mechanism and the extension of the blood half-life of the conjugate of the present invention. Therefore, the carrier molecule of the present invention is a molecule capable of binding to cyclic peptides and has binding affinity with FcRn. When a protein is adopted as a carrier molecule having such binding affinity with FcRn, the carrier molecule may be, for example, human serum albumin or a fragment or variant thereof, an albumin binding protein, and an Fc-containing molecule as shown hereinbelow. The carrier molecule can be produced by a known technique such as chemical synthesis, cell-free translation system, and protein synthesis system using animal cells.

The binding affinity of a carrier molecule to FcRn may be measured by a conventional method using surface plasmon resonance (SPR) technology or the like. The measurement can be performed using a commercially available instrument, for example, Biacore (produced by GE Healthcare). The carrier molecule in the present invention preferably has binding affinity with FcRn that is comparable to that of molecules known to bind FcRn, such as human serum albumin and human IgG.

Human serum albumin is the most abundant protein in the plasma and is composed of three domains (domains I, II and III) having a high homology, each of which is segmented into two subdomains (A and B). In particular, domain III of human serum albumin is known to be important for binding of human serum albumin with FcRn (JP 2019-513724 and Structure, 2013, 21, 1966-1978). Since human serum albumin has binding affinity with FcRn and has a long half-life in blood of about 20 days, the albumin is preferred as a carrier molecule to improve pharmacokinetics, and albumin-based technologies have been developed. A method for binding human serum albumin or a fragment or variant thereof to a cyclic peptide is not particularly limited as long as it functions as a carrier molecule for the cyclic peptide and the conjugate of the present invention can exert a TSP-1 inhibitory effect, and a known method can be used. Examples of such a method include a method of binding via a cysteine residue contained in human serum albumin (Org. Biomol. Chem., 2019. 17, 7870-7873), a method of binding via a glutamine residue contained in human serum albumin by transglutaminase, and binding via a lysine residue contained in human serum albumin. When the fragment or variant of human serum albumin is used as the carrier molecule of the present invention, any fragment or variant may be used as long as it retains the binding affinity with FcRn and binding site to the cyclic peptide, but preferably contains domain III of human serum albumin and the binding site to the cyclic peptide. The carrier molecule of the present invention is more preferably full-length human serum albumin.

The Fc-containing molecule of the present invention is coupled to a cyclic peptide to form a conjugate, which functions as a carrier for sustaining the cyclic peptide in blood for a long time. The Fc-containing molecule of the present invention refers to a molecule containing at least all or part of an Fc region derived from IgG. Examples of the Fc-containing molecule of the present invention include full-length IgG, a full-length IgG heavy chain, an IgG fragment containing all or part of the Fc region, and a variant thereof in which the amino acid sequence has been partially deleted or modified. In addition, the Fc-containing molecule of the present invention may be a heavy chain alone or may have a light chain corresponding to the structure of the heavy chain. IgG is a heterodimeric protein composed of two heavy chains and two light chains held together via disulfide bonds and noncovalent bonds. The heavy and light chains each have a variable region and a constant region and recognize antigen in the variable region. Also, the constant region of the heavy chain further contains a CH1 domain, a CH2 domain, and a CH3 domain, in which the CH1 and CH2 domains are connected via a hinge site. IgG has a domain structure, and is composed of a Fab region (antigen-binding fragment) containing a variable region and having an antigen-binding ability, and an Fc region (fragment crystallizable) that binds to an Fc receptor. The Fc region refers to a region from the N-terminal to the C-terminal of the hinge site, which is a part of the IgG heavy chain and is cleaved by papain, and refers to a region consisting of a part of the hinge site derived from the constant region of the IgG heavy chain, a CH2 domain, and a CH3 domain. Examples of the amino acid sequence corresponding to the Fc region in human IgG1 include the sequences at amino acid positions 1 to 232, 2 to 232, 3 to 232, 4 to 232, 5 to 232, 6 to 232, 7 to 232, 8 to 232, 9 to 232, 10 to 232, or 11 to 232 of SEQ ID NO: 148 (corresponding to positions 216 to 447, 217 to 447, 218 to 447, 219 to 447, 220 to 447, 221 to 447, 222 to 447, 223 to 447, 224 to 447, 225 to 447, and 226 to 447, respectively, in the Eu index (Eu INDEX) specified in Edelman et al., (Proc. Natl. Acad. USA, (1969) Vol. 63, pp. 78-85). The Fc region also has binding affinity with FcRn and is involved in the recycling mechanism of IgG through binding to FcRn. This binding to FcRn involves amino acid residues in the CH2 and CH3 domains. Therefore, the Fc-containing molecule of the present invention preferably has CH2 and CH3 domains to retain the binding affinity with FcRn. The term "heavy chain" for the Fc-containing molecule of the present invention may include a full-length heavy chain, a heavy chain of an IgG fragment containing all or part of the Fc region, and a variant thereof in which the amino acid sequence has been partially deleted or modified. Also, the term "light chain" for the Fc-containing molecule of the present invention may include a full-length light chain and a variant in which the amino acid sequence has been partially deleted or modified.

A method for binding the Fc-containing molecule to a cyclic peptide is not particularly limited as long as it functions as a carrier for the cyclic peptide and the conjugate of the present invention can exert a TSP-1 inhibitory effect, and a known method can be used. Examples of such a method include a method of binding via a glycan contained in an Fc-containing molecule, a method of binding via a cysteine residue contained in an Fc-containing molecule, a method of mutating an amino acid residue at a specific position to a cysteine residue and binding via the cysteine residue, a method of binding via a glutamine residue contained in an Fc-containing molecule by transglutaminase, and a method of mutating an amino acid residue at a specific position to a glutamine residue and binding via the glutamine residue by transglutaminase. In a preferred mode, the Fc-containing molecule of the present invention is linked to a cyclic peptide via a glycan attached to the side chain of an asparagine residue (referred to as "Asn297" (Asn at position 297 in the Eu index)) that undergoes modification by a well-conserved N-linked glycan in the Fc region of the IgG heavy chain (referred to as "N297-linked glycan") or via well-conserved thiol groups of Cys226 (Cys at position 226 in the Eu index) and/or Cys229 (Cys at position 229 in the Eu index) at the hinge site of the Fc region of the IgG heavy chain. Therefore, the Fc-containing molecule of the present invention preferably has at least an amino acid residue in the Fc region of human IgG corresponding to Asn297 or an amino acid residue at the hinge site of human IgG corresponding to Cys226 or Cys229.

When the Fc-containing molecule of the present invention is used as the carrier molecule of the present invention, the sequence is not particularly limited as long as it retains the binding affinity with FcRn and the binding site to the cyclic peptide. The Fc-containing molecule preferably contains amino acid sequences corresponding to the CH2 and CH3 domains, and amino acid residues in the Fc region of human IgG corresponding to Asn297 or amino acid residues at the hinge site of human IgG corresponding to Cys226 and/or Cys229. Examples of such an Fc-containing molecule include full-length human IgG, a full-length human IgG heavy chain, a human IgG fragment containing all or part of the Fc region, and a variant thereof in which the amino acid sequence has been partially deleted or modified. In addition, the Fc-containing molecule of the present invention may be a heavy chain alone or may have a light chain corresponding to the structure of the heavy chain.

The Fc-containing molecule of the present invention also includes a modified Fc-containing molecule which has been biologically modified. Biological modifications include post-translational modification (e.g., N-linked or O-linked glycosylation, N- or C-terminal processing, deamidation, aspartate isomerization, and methionine oxidation) and addition of a methionine residue at the N-terminus by expression using a prokaryotic host cell.

In the case of antibodies produced in animal cells, it is known that a lysine residue at the carboxyl terminus of the heavy chain is deleted (Journal of Chromatography A, 705:129-134 (1995)) and that two amino acid residues, glycine and lysine, at the carboxyl terminus of the heavy chain are also deleted and a proline residue newly positioned at the carboxyl terminus is amidated (Analytical Biochemistry, 360: 75-83 (2007)). However, the deletion and modification of these heavy chain sequences do not affect the antigen-binding ability and effector function (complement activation, antibody-dependent cellular cytotoxicity effect, etc.) of the antibody. Thus, the Fc-containing molecule of the present invention also include an Fc-containing molecule with such modification and a functional fragment of the Fc-containing molecule, a deletion mutant in which one or two amino acid residues are deleted at the carboxyl terminus of a heavy chain, and a deletion mutant in which an amino acid residue at the carboxyl terminus is amidated (*e.g*., a heavy chain in which a proline residue at the carboxyl terminus is amidated). As long as it functions as a carrier for the cyclic peptide and the conjugate of the present invention can exert a TSP-1 inhibitory effect, however, the deletion mutant at the carboxyl terminus of the heavy chain of the Fc-containing molecule according to the present invention is not limited to the above types. When the Fc-containing molecule according to the present invention includes two heavy chains, they may be any one of the heavy chains selected from the group consisting of heavy chains without a deletion at the carboxyl terminus and the above-mentioned deleted mutant, or a combination of any two. The quantitative ratio of each deletion mutant can be affected by the type and culture conditions of animal cells that produce the Fc-containing molecule of the present invention, but the Fc-containing molecule of this invention is preferably one in which one amino acid residue at the carboxyl terminus is deleted in each of the two heavy chains.

In one mode, the Fc-containing molecule of the present invention may include one or two or more modifications selected from the group consisting of N-linked glycosylation, O-linked glycosylation, N-terminal processing, C-terminal processing, deamidation, isomerization of aspartic acid, oxidation of methionine, addition of a methionine residue to the N-terminus, amidation of a proline residue, and a deletion of one or two amino acid residues at the carboxyl terminus of a heavy chain.

The subclass of IgG serving as an origin of the Fc-containing molecule of the present invention is not particularly limited, and any subclass may be selected. The subclass is preferably IgG1, IgG2, IgG3 or IgG4, more preferably IgG1, even more preferably human IgG1. The amino acid sequences of IgG constant regions are well conserved, and each amino acid is identified by the EU Index provided by Edelman et al. (Proc. Natl. Acad. USA,

(1969) Vol. 63, pp. 78-85). For example, Asn297 to which an N-linked glycan is attached in the Fc region corresponds to position 297 in the Eu index, and Cys226 and Cys229 to which a linker structure is linked in the hinge site correspond to positions 226 and 229 in the Eu index, respectively. In addition, the Fc region, hinge site, and heavy chain constant region derived from human IgG that can be adopted in the Fc-containing molecule of the present invention correspond to regions of the amino acid sequences at positions 216 to 447, 216 to 230, and 118 to 447 in the Eu index, respectively. Examples of specific amino acid sequences corresponding to the Fc region, hinge region, light chain constant region, and heavy chain constant region derived from human IgG1 that can be adopted in the Fc-containing molecule of the present invention include the amino acid sequence at amino acid positions 118 to 349 of SEQ ID NO: 129 (amino acid sequence of human IgG1 heavy chain constant region; nucleotide sequence encoding the amino acid sequence is shown in SEQ ID NO: 128) (provided that the N-terminus can be shortened to amino acid position 128), the amino acid sequence at amino acid positions 118 to 132 of SEQ ID NO: 129, the amino acid sequence at amino acid positions 21 to 125 of SEQ ID NO: 131 (amino acid sequence of human IgG1 light chain constant region; nucleotide sequence encoding the amino acid sequence is shown in SEQ ID NO: 130) (signal peptides at amino acid positions 1 to 20), and the amino acid sequence at amino acid positions 20 to 349 of SEQ ID NO: 129 (signal peptides at amino acid positions 1 to 19), respectively. The amino acid is unambiguously identified by display according to the EU index even if the actual amino acid position varies due to molecular fragmentation or regional deficiency. Regarding the constant regions of human IgG1, human IgG2, human IgG3, or human IgG4 that can be adopted in the Fc-containing molecule of the present invention, a plurality of allotypic sequences have been reported in Sequences of proteins of immunological interest, NIH Publication No. 91-3242, all of which can be adopted in the Fc-containing molecule of the present invention. For example, in the sequence of human IgG1, the amino acid sequences at positions 356 and 358 indicated by the Eu index include D and E, and L and M, respectively, both of which can be adopted in the Fc-containing molecule of the present invention.

In the case of adopting full-length IgG as the Fc-containing molecule of this invention, it is sufficient if the full-length IgG functions as a carrier for the cyclic peptide and the conjugate of the present invention can exert a TSP-1 inhibitory effect. In one mode, IgGs that do not recognize substances normally present in the human body as antigens in the variable region can be adopted. Since IgG directed to a nonhuman animal protein as an antigen might exhibit cross reactivity with a human molecules in the presence of a corresponding or related human molecule, it is preferred to select a monoclonal antibody against an antigen free from corresponding or related human molecules. Even IgG that might recognize a component in the human body as an antigen can be adopted if the recognition of the antigen does not adversely affect the TSP-1 inhibitory effect exerted by the conjugate of the present invention. On the other hand, even if the recognition of the antigen adversely affects the TSP-1 inhibitory effect exerted by the conjugate of the present invention, those molecules can be adopted as Fc-containing molecules of this invention when the antigen-recognition ability is attenuated or deleted by introducing a mutation into the variable region by a genetic engineering technique. The full-length IgG for use as the Fc-containing molecule of the present invention is preferably IgG directed to a nonmammalian organism-derived molecule as an antigen, more preferably IgG directed to a microbe-derived molecule as an antigen. Examples of the IgG directed to the microbe-derived molecule as an antigen include lipopolysaccharide (LPS) as an antigen. Such a monoclonal antibody is described in, for example, WO 2015/046505. Specific examples thereof include mAb-A consisting of a combination of a heavy chain consisting of an amino acid sequence at amino acid positions 20 to 474 of SEQ ID NO: 125 (signal peptides at amino acid positions 1 to 19; nucleotide sequence encoding the heavy chain is shown in SEQ ID NO: 124), and a light chain consisting of an amino acid sequence at amino acid positions 21 to 234 of SEQ ID NO: 127 (signal peptides at amino acid positions 1 to 20; nucleotide sequence encoding the light chain is shown in SEQ ID NO: 126).

In the case of adopting an antibody fragment including all or part of the Fc region as the Fc-containing molecule of this invention, it is sufficient if the antibody fragment functions as a carrier for the cyclic peptide, and the conjugate of the present invention can exert a TSP-1 inhibitory effect. For example, a CH consisting of a constant region obtained by deleting a variable region from the heavy chain of IgG, a molecule consisting of the Fc region of IgG, or a molecule consisting of a part of the Fc region of IgG can be adopted.

Specific examples of the amino acid sequence of CH consisting of the constant region obtained by deleting the variable region from a human IgG1 heavy chain include the amino acid sequence at amino acid positions 20 to 349 of SEQ ID NO: 129 (signal peptides at amino acid positions 1 to 19; nucleotide sequence encoding the CH is shown in SEQ ID NO: 128). When CH is selected as the Fc-containing molecule of this invention, CH may be adopted alone or as CLCH in combination with CL consisting of the constant region of the light chain. Specific examples of the amino acid sequence of the CL of the IgG1 light chain include amino acid positions 21 to 125 of SEQ ID NO: 131 (amino acids at amino acid positions 1 to 20 constitute a signal peptide; nucleotide sequence encoding the CL is shown in SEQ ID NO: 130), and examples of the CLCH include CLCH formed in combination of the CH represented by SEQ ID NO: 129 and the CL represented by SEQ ID NO: 131.

Specific examples of the amino acid sequence of a molecule consisting of a part of the Fc region of human IgG1 include the amino acid sequence at amino acid positions 21 to 243 of SEQ ID NO: 135 (signal peptides at amino acid positions 1 to 20; nucleotide sequence encoding amino acid sequence corresponding to the Fc region is shown in SEQ ID NO: 134).

In the case of adopting full-length IgG, a full-length IgG heavy chain, or an IgG fragment containing all or part of the Fc region, and a variant thereof, in which the amino acid sequence has been partially deleted or modified, as the Fc-containing molecule of this invention, any molecule may be used as long as it retains the binding affinity with FcRn and the binding site to a cyclic peptide. When the Fc-containing molecule of this invention is derived from an antibody molecule having all or part of the variable region while retaining an antigen recognition ability for a component in the human body, and when the recognition of the antigen adversely affects the TSP-1 inhibitory effect exerted by the conjugate of the present invention, the antigen recognition ability is preferably attenuated or deleted by introducing a mutation into the variable region by a genetic engineering technique. In other words, the Fc-containing molecule of the present invention in one mode may be a variant in which the variable region of IgG has been deleted or modified so that antigen recognition ability is attenuated or deleted. Examples of such a variant include CH consisting only of the constant region obtained by deleting the variable region from the human IgG1 heavy chain, CLCH obtained by combining CL and CH consisting only of the constant region of the light chain, and an IgG fragment containing all or part of the Fc region. Examples of the variant having a partially modified amino acid sequence adopted as the Fc-containing molecule of the present invention include molecules having an amino acid sequence with high homology (*e.g.,* 80% or more, 90% or more, 95% or more, or 99% or more) to the amino acid sequence before modification, or an amino acid sequence in which one to several (*e.g.,* 2, 3, 4, 5, 6, 7, 8, 9, or 10) amino acid residues are substituted, deleted, inserted, or added while retaining the binding affinity with FcRn and the binding site to a cyclic peptide.

In the case of adopting the IgG fragment containing all or part of the Fc region or a variant thereof in which the amino acid sequence has been partially deleted or modified as the Fc-containing molecule of this invention, any molecule may be used as long as it retains the binding affinity with FcRn and the binding site to a cyclic peptide. To retain the binding affinity with FcRn, IgG fragment or variant thereof preferably has CH2 and CH3 domains, and further contain all or a part of the hinge site from the viewpoint of increasing the structural freedom of the Fc-containing molecule. Thus, a fragment consisting of the CH2 domain, CH3 domain, and part of the hinge site of IgG can be adopted as such an Fc-containing molecule, for example. When the hinge site is not adopted as the binding site between the Fc-containing molecule and the cyclic peptide, the length of the sequence corresponding to the hinge site can be appropriately adjusted and included in the Fc-containing molecule, but CPPC (amino acid sequence corresponding to positions 226 to 229 in the Eu index; for example, amino acid positions 128 to 131 of SEQ ID NO: 129) is preferably included. Examples of the hinge site contained in the Fc-containing molecule of this invention include the amino acid sequences of hinge sites corresponding to positions 221 to 230, 222 to 230, 223 to 230, 224 to 230, 225 to 230, and 226 to 230 in the Eu index. In human IgG1, these sequences are each DKTHTCPPCP, KTHTCPPCP, THTCPPCP, HTCPPCP, TCPPCP, and CPPCP (sequences at amino acid positions 123 to 132, 122 to 132, 123 to 132, 124 to 132, 125 to 132, 126 to 132, 127 to 132, and 128 to 132 of SEQ ID NO: 129, respectively). Examples of antibody fragments containing such a hinge site that are derived from human IgG1 include an Fc-containing molecule consisting of an amino acid sequence in which 1 to 10 consecutive amino acids from the N-terminus may be deleted in the amino acid sequence of Glu at position 118 to Lys at position 349 of SEQ ID NO: 129 or a variant in which the amino acid sequence has been modified, and preferably an Fc-containing molecule consisting of Asp at position 123 to Lys at position 349, Lys at position 124 to Lys at position 349, Thr at position 125 to Lys at position 349, His at position 126 to Lys at position 349, Thr at position 127 to Lys at position 349, or Cys at position 128 to Lys at position 349 of SEQ ID NO: 129. Examples of antibody fragments derived from human IgG1 include an amino acid sequence at amino acid positions 22 to 243 of SEQ ID NO: 135; an amino acid sequence at amino acid positions 26 to 247 of SEQ ID NO: 137 (Leu residues (Leu234) corresponding to position 234 and (Leu235) corresponding to position 235 in the Eu index are substituted with Ala); an amino acid sequence at amino acid positions 26 to 247 of SEQ ID NO: 139 (Leu residues (Leu234) corresponding to position 234 and (Leu235) corresponding to position 235 in the Eu index are substituted with Ala, and a Pro residue (Pro329) corresponding to position 329 is substituted with Ala); or Fc-containing molecules comprising amino acid sequences with T, HT, THT, KTHT, or DKTHT added to the N-terminus thereof, or variants in which amino acid sequences have been modified. Preferably, the Fc-containing molecule of the present invention may be a polypeptide (also referred to herein as "Fc-B") consisting of an amino acid sequence at amino acid positions 21 to 243 of SEQ ID NO: 135; a polypeptide consisting of an amino acid sequence at amino acid positions 25 to 247 of SEQ ID NO: 137; a polypeptide (also referred to herein as "Fc-A") consisting of an amino acid sequence at amino acid positions 21 to 247 of SEQ ID NO: 137; a polypeptide consisting of an amino acid sequence at amino acid positions 25 to 247 of SEQ ID NO: 139; a polypeptide (also referred to herein as "Fc-C") consisting of an amino acid sequence at amino acid positions 21 to 247 of SEQ ID NO: 139; or a variant thereof. Asn297 in these Fc fragments is Asn at position 93 in SEQ ID NO: 135, Asn at position 97 in SEQ ID NO: 137, and Asn at position 97 in SEQ ID NO: 139. Cys226 in these Fc fragments is Cys at position 22 in SEQ ID NO: 135, Cys at position 26 in SEQ ID NO: 137, and Cys at position 26 in SEQ ID NO: 139. Cys229 in these Fc fragments is Cys at position 25 in SEQ ID NO: 135, Cys at position 29 in SEQ ID NO: 137, and Cys at position 29 in SEQ ID NO: 139.

In the case of designing an antibody fragment or variant as the Fc-containing molecule of the present invention, a variant having the substitution, deletion, insertion, and/or addition of 1 to several (preferably 20 or less, more preferably 15 or less, even more preferably 10 or less, further preferably 7, 6, 5, 4, 3, 2 or 1, per engineering site) amino acids at 1 to several sites (preferably 5 or less sites, more preferably 3, 2, or 1 site(s)) may be adopted without impairing functions as the carrier protein, as long as Cys226, Cys229, or Asn297 that contributes to binding to cyclic peptides, and neighboring amino acids thereof are maintained. As for the engineering site in an amino acid sequence, for example, the substitution, deletion insertion, and/or addition of amino acids may be performed at the N-terminus and the C-terminus. In particular, N-terminal amino acids may influence the production of the Fc-containing molecule by a bioengineering approach and can be engineered into an amino acid sequence suitable for the desired production system. Examples of such amino acid sequence variants include variants in which one or two amino acid residues have been deleted at the carboxyl terminus of a human IgG heavy chain (e.g., a heavy chain in which the lysine residue corresponding to position 447 according to the Eu index at the carboxyl terminus has been deleted, or a heavy chain in which the lysine residue and the glycine residue corresponding to position 446 according to the Eu index have been deleted). Amino acids Leu234 and Leu235 or Pro329 in the EU index are known as sites that influence the exhibition of effector activity by T-cell activation through the binding of the antibody to an Fc receptor. In some cases, this effector activity can be eliminated so as to reduce the risk of adverse reaction by substituting Leu234 and Leu235 with Ala (the resulting mutant is referred to as a "LALA form") or further substituting Pro329 with Ala (the resulting mutant is referred to as an "LALA-PA form") (US 5885573A for LALA form). Such engineering may be carried out, if necessary. CLCH-B (antibody fragment consisting of a heavy chain constant region consisting of the amino acid sequence of SEQ ID NO: 133 and a corresponding light chain constant region consisting of the amino acid sequence of SEQ ID NO: 131) is the LALA form of CLCH-A (antibody fragment consisting of a heavy chain constant region consisting of the amino acid sequence of SEQ ID NO: 129 and a corresponding light chain constant region consisting of the amino acid sequence of SEQ ID NO: 131), and Fc-A is the LALA form of Fc-B. The LALA form has been confirmed to function properly as a carrier molecule in a conjugate with peptides (WO 2018/003983). Thus, in one mode, the Fc-containing molecule of the present invention may include substitutions of a leucine residue corresponding to position 234 and a leucine residue corresponding to position 235 according to the Eu index in the human IgG heavy chain with alanine residues or substitutions of a leucine residue corresponding to position 234, a leucine residue corresponding to position 235, and a proline residue corresponding to position 329 according to the Eu index in the human IgG heavy chain with alanine residues.

The Fc-containing molecule of this invention may also be appended with chemical modifications such as the attachment of chemical moieties to the amino acid backbone, N-linked or O-linked carbohydrate chains. Alternatively, a signal sequence, a peptidase recognition sequence, or a Tag sequence such as GST may be added thereto for the purpose of improving the expression or purification efficiency of the molecule of interest.

In one mode, the Fc-containing molecule of the present invention may be an antibody composed of a combination of a heavy chain consisting of an amino acid sequence selected from the group consisting of:
(a) an amino acid sequence at amino acid positions 20 to 474 of SEQ ID NO: 125;
(b) an amino acid sequence having at least 95% or more (*e.g*., 96% or more, 97% or more, 98% or more, or 99% or more) homology to the sequence of (a);
(c) an amino acid sequence having a deletion, substitution or addition of 1 to 10 (*e.g*., 1, 2, 3, 4, 5, 6, 7, 8, 9, or 10) amino acids in the sequence of (a); and
(d) an amino acid sequence in which 1 to 3 (*e.g*., 1, 2, or 3) C-terminal amino acid residues are deleted in the sequence of (a), and
a light chain consisting of an amino acid sequence selected from the group consisting of:
(e) an amino acid sequence at amino acid positions 21 to 234 of SEQ ID NO: 127;
(f) an amino acid sequence having at least 95% or more (*e.g.*, 96% or more, 97% or more, 98% or more, or 99% or more) homology to the sequence of (e);
(g) an amino acid sequence having a deletion, substitution or addition of 1 to 10 (*e.g.*, 1, 2, 3, 4, 5, 6, 7, 8, 9, or 10) amino acids in the sequence of (e); and
(h) an amino acid sequence in which 1 to 3 (*e.g*., 1, 2, or 3) C-terminal amino acid residues are deleted in the sequence of (e).

In one mode, the Fc-containing molecule of the present invention may be an antibody composed of a combination of a heavy chain consisting of an amino acid sequence selected from the group consisting of:
(a) an amino acid sequence at amino acid positions 20 to 349 of SEQ ID NO: 129;
(b) an amino acid sequence at amino acid positions 20 to 349 of SEQ ID NO: 133;
(c) an amino acid sequence having at least 95% or more (*e.g.*, 96% or more, 97% or more, 98% or more, or 99% or more) homology to the sequence of (a) or (b);
(d) an amino acid sequence having a deletion, substitution or addition of 1 to 10 (*e.g*., 1, 2, 3, 4, 5, 6, 7, 8, 9, or 10) amino acids in the sequence of (a) or (b); and
(e) an amino acid sequence in which 1 to 3 (*e.g*., 1, 2, or 3) C-terminal amino acid residues are deleted in the sequence of (a) or (b), and
a light chain consisting of an amino acid sequence selected from the group consisting of:
(f) an amino acid sequence at amino acid positions 21 to 234 of SEQ ID NO: 131;
(g) an amino acid sequence having at least 95% or more (*e.g*., 96% or more, 97% or more, 98% or more, or 99% or more) homology to the sequence of (f);
(h) an amino acid sequence having a deletion, substitution or addition of 1 to 10 (*e.g.*, 1, 2, 3, 4, 5, 6, 7, 8, 9, or 10) amino acids in the sequence of (f); and
(i) an amino acid sequence in which 1 to 3 (*e.g*., 1, 2, or 3) C-terminal amino acid residues are deleted in the sequence of (f).

In one mode, the Fc-containing molecule of the present invention may be CH consisting of an amino acid sequence selected from the group consisting of:
(a) an amino acid sequence at amino acid positions 20 to 349 of SEQ ID NO: 129;
(b) an amino acid sequence at amino acid positions 20 to 349 of SEQ ID NO: 133;
(c) an amino acid sequence having at least 95% or more (*e.g.*, 96% or more, 97% or more, 98% or more, or 99% or more) homology to the sequence of (a) or (b);
(d) an amino acid sequence having a deletion, substitution or addition of 1 to 10 (*e.g*., 1, 2, 3, 4, 5, 6, 7, 8, 9, or 10) amino acids in the sequence of (a) or (b); and
(e) an amino acid sequence in which 1 to 3 (*e.g.*, 1, 2, or 3) C-terminal amino acid residues are deleted in the sequence of (a) or (b).

In one mode, the Fc-containing molecule of the present invention may be an Fc fragment consisting of an amino acid sequence selected from the group consisting of:
(a) an amino acid sequence at amino acid positions 21 to 243 of SEQ ID NO: 135;
(b) an amino acid sequence at amino acid positions 21 to 247 of SEQ ID NO: 137;
(c) an amino acid sequence at amino acid positions 21 to 247 of SEQ ID NO: 139;
(d) an amino acid sequence having at least 95% or more (*e.g*., 96% or more, 97% or more, 98% or more, or 99% or more) homology to the sequences of (a) to (c);
(e) an amino acid sequence having a deletion, substitution or addition of 1 to 10 (e.g., 1, 2, 3, 4, 5, 6, 7, 8, 9, or 10) amino acids in the sequences of (a) to (c); and
(f) an amino acid sequence in which 1 to 3 (*e.g.,* 1, 2, or 3) C-terminal amino acid residues are deleted in the sequences of (a) to (c).

### (1) Fc-containing molecule binding to cyclic peptide via N297-linked glycan

In one mode, the conjugate of the present invention may be a compound bonded to a cyclic peptide via N297-linked glycan of the Fc-containing molecule. When the Fc-containing molecule used in the present invention is produced using animal cells, heterogeneous glycans may be attached to Asn297 of the Fc-containing molecule by posttranslational modification in the course of production. In a preferred mode, the Fc-containing molecule may have a N297-linked glycan which is remodeled into a SG-type glycan having any of structures given below (SG-type N297-linked glycan). 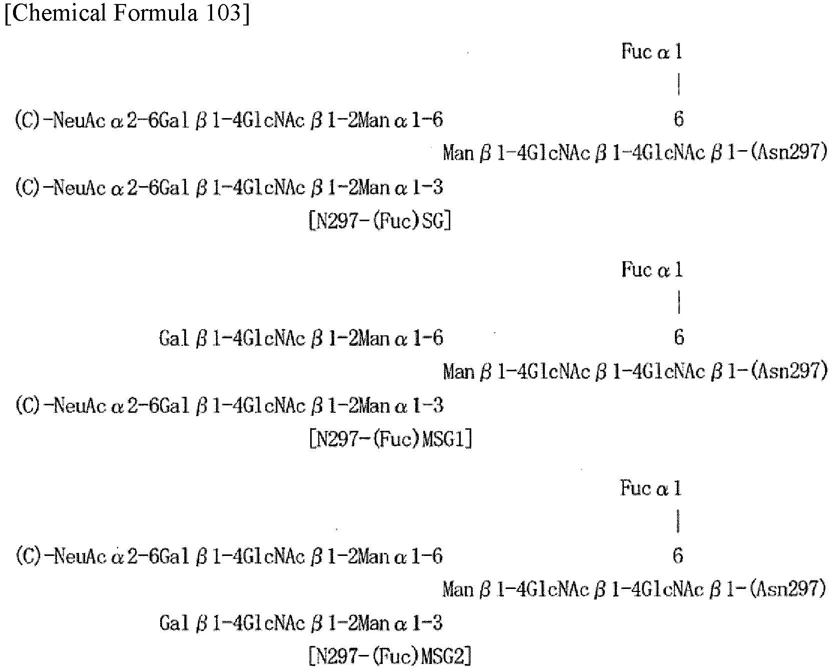 (wherein (C) represents binding of the linking group C to a carbonyl group bonded to the 2-position of a sialic acid, and (Asn297) represents binding to the side chain of Asn297 of the Fc-containing molecule through an N-glycosidic bond).

In a preferred mode, the N297-linked glycan of the Fc-containing molecule can be at least one selected from N297-(Fuc)SG, N297-(Fuc)MSG1, and N297-(Fuc)MSG2. More preferably, the N297-linked glycan of the Fc-containing molecule includes N297-(Fuc)SG. Even more preferably, the N297-linked glycan of the Fc-containing molecule can be N297-(Fuc)SG.

Usually, glycans are attached to Asn297 in both the monomers constituting a dimer during production from animal cells to produce a normal form having two N297-linked glycans per dimeric Fc-containing molecule. Depending on production conditions or the structure of the Fc-containing molecule, however, a glycan deletion mutant in which a N297-linked glycan is attached to only one of the monomers (molecule having one N297-linked glycan per dimeric Fc-containing molecule) may be produced at a given rate. Even such a Fc-containing molecule containing a glycan deletion mutant can be used in the present invention.

When the N297-linked glycan in the conjugate of the present invention is N297-(Fuc)MSG1 or N297-(Fuc)MSG2, the conjugate is a molecule having two linker structures and two cyclic peptides bonded thereto (divalent conjugate) because the Fc-containing molecule is a dimer usually having the N297-linked glycan in both the monomers (when the Fc-containing molecule is a glycan deletion mutant, N297-(Fuc)MSG1 or N297-(Fuc)MSG2 is attached to only one of the monomers to form a monovalent conjugate). On the other hand, when the N297-linked glycan is N297-(Fuc)SG, the conjugate is a molecule having four linker structures and four cyclic peptides bonded thereto (tetravalent conjugate) because the Fc-containing molecule is a dimer (when the Fc-containing molecule is a glycan deletion mutant, N297-(Fuc)SG is attached to only one of the monomers to form a divalent conjugate). The conjugate of the present invention may be a mixture of normal forms and glycan deletion mutants (in the present invention, such a mixture is indicated as a conjugate in a normal form for the sake of convenience).

The conjugate of this invention may be a mixture of molecules having plural types of N297-linked glycans or a molecule having N297-linked glycan having any one structure. In a preferred mode, the conjugate of this invention is a molecule having SG-type N297-linked glycan having any one structure, more preferably a tetravalent conjugate having N297-(Fuc)SG as the N297-linked glycan with four cyclic peptides bonded thereto (or a divalent conjugate for a glycan deletion mutant).

In addition, when one molecule of the conjugate of the present invention contains a plurality of cyclic peptides, they may be a single (one type of) cyclic peptide or a combination of a plurality of cyclic peptides. The conjugate of this invention preferably contains a single cyclic peptide, more preferably a single bicyclic peptide.

In addition, sialic acids of the SG-type N297-linked glycan may remain unreacted in the synthesis reaction of the conjugate. In the conjugate of this invention, as long as at least one cyclic peptide is bonded to one Fc-containing molecule, the glycan may contain unreacted sialic acid. For example, the sialic acid of N297-(Fuc)MSG1 or N297-(Fuc)MSG2 in one Fc-containing molecule constituting the dimer may remain unreacted, resulting in a conjugate with one cyclic peptide bonded thereto (monovalent conjugate). The conjugate of this invention may be such a monovalent conjugate or a mixture of a monovalent conjugate and a divalent conjugate. For example, one or both sialic acids of N297-(Fuc)SG (disialo-form) in one of the Fc-containing molecules constituting the dimer may remain unreacted, resulting in a conjugate with one to three cyclic peptides bonded thereto (monovalent to trivalent conjugate). The conjugate of this invention may be any of monovalent to tetravalent conjugates or a mixture thereof.

In the SG-type N297-linked glycan structure, fucosylated GlcNAc(Fucα1,6)GlcNAc at the reducing terminus is derived from the Fc-containing molecule produced in an animal cell and thereby remodels a glycan on the non-reducing terminal side into a glycan structure similar to that of SG.

Such a Fc-containing molecule having the SG-type N297-linked glycan can be produced according to a method described in, for example, WO 2013/120066 and WO 2018/003983. When the Fc-containing molecule is produced as a gene recombinant protein using animal cells according to a known method, the N297-linked glycan has a fucosylated N-linked glycan structure as a basic structure. In this case, the Fc-containing molecule is obtained as a mixture of antibodies or fragments thereof having glycans of various structures with diverse modifications in the structure at the non-reducing terminus or constituent sugars. Such an Fc-containing molecule produced in an animal cell is treated with hydrolase such as EndoS so that the glycosidic bond between GlcNAcβ1 and 4GlcNAc of a chitobiose structure at the reducing terminus can be hydrolyzed to obtain an Fc-containing molecule having a single glycan structure with only (Fucα1,6)GlcNAc as the N297-linked glycan (referred to as a "(Fucα1,6)GlcNAc-Fc-containing molecule").

For example, EndoS or a mutant enzyme thereof that maintains hydrolytic activity can be used as such an enzyme for use in the hydrolysis reaction of the N297-linked glycan. The (Fucα1,6)GlcNAc-Fc-containing molecule obtained by the hydrolysis reaction can be reacted as a glycan acceptor molecule with a SG-type glycan donor molecule by using a glycosyltransferase such as an EndoS D233Q mutant to obtain a Fc-containing molecule having a SG-type N297-linked glycan having the structure described above.

When the compound of interest is a conjugate having a tetravalent cyclic peptide, a glycan donor molecule having SG(10) (glycan lacking one GlcNAc at the reducing terminus of the glycan moiety of SG) as a glycan is used in a transglycosylation reaction thereof. Such a SG(10) glycan used may be obtained by hydrolysis or the like from, for example, sialyl glycopeptide (hereinafter, referred to as "SGP") contained in chicken egg yolk or may be a SG(10) glycan alone such as a commercially available disialooctasaccharide (Tokyo Chemical Industry Co., Ltd.).

When the compound of interest is a conjugate having a divalent cyclic peptide, a glycan donor molecule having MSG1(9) (glycan structure lacking sialic acid at the non-reducing terminus in the 1-6 branched chain of the glycan of SG(10) β-Man and having sialic acid only in the 1-3 branched chain of the glycan) or MSG2(9) (glycan structure lacking sialic acid at the non-reducing terminus in the 1-3 branched chain of the glycan of SG(10) β-Man and having sialic acid only in the 1-6 branched chain of the glycan) can be used as a glycan. Such a glycan may be adopted by separation according to a method described in WO 2018/003983 using commercially available monosialo-Asn free (1S2G/1G2S-10NC-Asn, GlyTech, Inc.) as a starting material or may be adopted as a mixture without separation.

GlcNAc at the reducing terminus of the SG-type glycan contained in the donor molecule is preferably used after being activated, for example, in the form of oxazoline by treatment with 2-chloro-1,3-dimethyl-1H-benzimidazol-3-ium-chloride, but does not have to be activated in the case of using two types of enzymes, as shown hereinbelow, at the same time.

The glycan donor molecule may have a functional group (*e.g*., azide group) at the non-reducing terminus to form a bond between a linker structure and the Fc-containing molecule. For example, the SG-type glycan contained in the glycan donor molecule may have a functional group (*e.g.,* azide group) in a carboxylic acid contained in the sialic acid at the non-reducing terminus thereof to form a bond between a linker structure and the Fc-containing molecule.

Various enzymes can be adopted as such an enzyme for use in the transglycosylation reaction as long as the enzyme has activity of transferring a complex glycan to the N297-linked glycan. EndoS D233Q is preferred which is a variant of EndoS that suppresses hydrolysis reaction by the substitution of Asp at position 233 with Gln. The transglycosylation reaction using EndoS D233Q is described in WO 2013/120066. Alternatively, an engineered enzyme such as a further mutant of EndoS D233Q, *i.e.,* EndoS D233Q/Q303L, EndoS D233Q/E350A, EndoS D233Q/E350Q, EndoS D233Q/E350D, EndoS D233Q/E350N, or EndoS D233Q/D405A may be used. A transglycosylation reaction using such a variant of EndoS D233Q is described in WO 2017/010559.

The purification operation of the Fc-containing molecule or intermediate thereof after the glycan remodeling (sugar hydrolysis and transglycosylation reaction) of the Fc-containing molecule is aimed at separating the Fc-containing molecule from low-molecular weight compounds and the enzymes used in the reaction. Such purification usually employs gel filtration chromatography, ion exchange chromatography, affinity chromatography, or the like. Purification using a hydroxyapatite column may be further performed, and from the viewpoint of improving transglycosylation reaction efficiency, a hydroxyapatite column is particularly preferably used in the purification operation of the intermediate after sugar hydrolysis (see WO 2018/003983).

Usual transglycosylation reactions require activating the reducing terminus of the glycan donor, and such an active donor is time consuming and costly. By using two enzymes at the same time, SGP, (SG-)Asn (structure in which SG is joined to the side chain of Asn), or the like having an unactivated reducing terminus can be directly transglycosylated as a glycan donor to the N297-linked glycan of the Fc-containing molecule (see WO 2018/003983), whereby a naturally obtainable or commercially available glycopeptide or the like can be used in the transglycosylation reaction directly, thus enabling efficient glycan remodeling.

For the two types of Endo enzymes used, it is important to combine properly enzyme A (EndoM-like enzyme) for a wide range of complex glycans as substrates and enzyme B (EndoS-like enzyme) for the N297-linked glycan of the Fc-containing molecule as a substrate.

Examples of the enzyme A include EndoM, EndoOm, EndoCC, and mutants of EndoM, EndoOm, and EndoCC with reduced hydrolytic activity. The enzyme A is preferably EndoM N175Q, EndoCC N180H, or EndoOm N194Q.

Examples of the enzyme B include EndoS, EndoS2 (EndoS49), and mutants of EndoS and EndoS2 (EndoS49) with reduced hydrolytic activity. Preferred examples of the enzyme B include EndoS D233Q, EndoS D233Q/Q303L, EndoS D233Q/E350A, EndoS D233Q/E350Q, EndoS D233Q/E350D, EndoS D233Q/E350N, and EndoS D233Q/D405A.

The structure of the glycan donor is not particularly limited as long as the glycan donor has a glycan structure that is recognized by the enzyme A which is adopted. Various molecules, such as naturally obtained molecules and molecules synthesizable in combination with chemical reaction or enzymatic reaction, can be used. Any substituent other than R=H may be used as substituent R at the anomer site. In the case of an N-linked glycan structure (see the formulas given below; substituent R of the anomer site is R at the reducing terminus of the following structural formula), examples thereof include amide structures and azide.

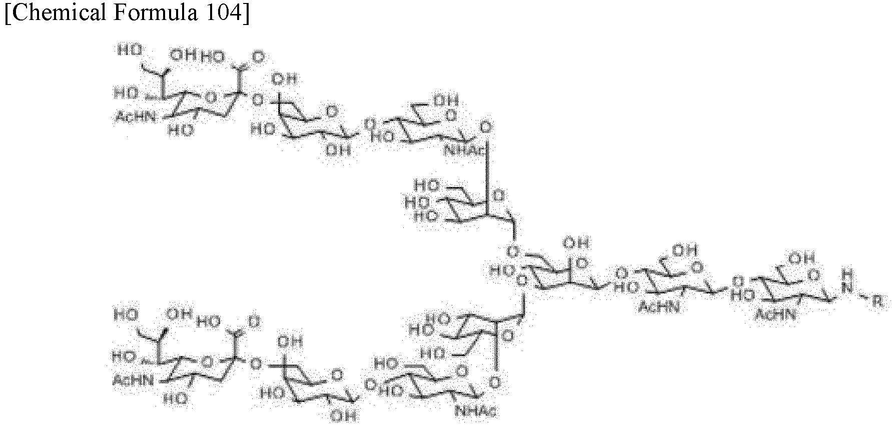

In the case of an O-linked glycan structure (see the formulas given below; substituent R of the anomer site is R at the reducing terminus of the following structural formula), examples thereof include ethylene glycol structures, glycolic acid structures, and a benzyl group, an allyl group, and a p-nitrophenyl group used as protective groups for the anomer hydroxy group.

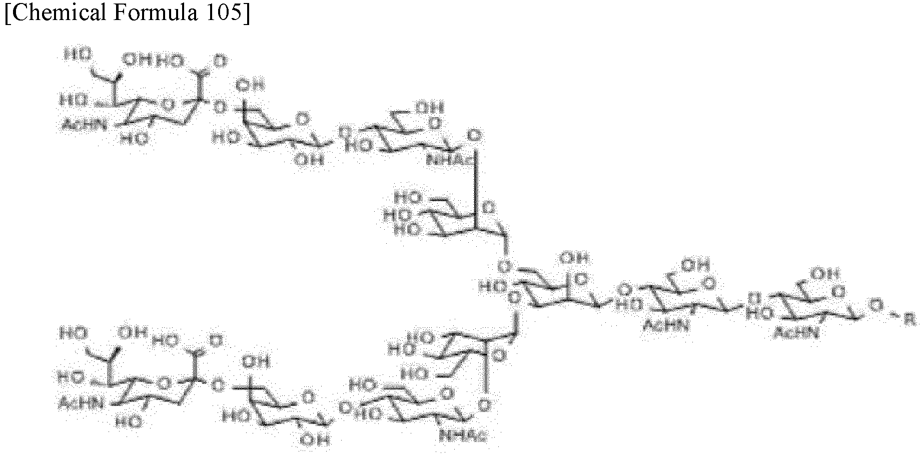

The structure at the non-reducing terminus of the glycan donor is not particularly limited as long as the structure is recognized by enzyme A. Various structures can be used such as natural glycan structures, non-reducing terminal glycan deletion mutants of natural glycan structures, structures where an arbitrary hydroxy group is modified with phosphoric acid, and structures chemically bonded to a linker structure. Preferred examples of the glycan donor include SGP, (SG-)Asn, and ([N₃-PEG(3)]₂-SG)-Asn-PEG(3)-N₃.

The acceptor molecule is not particularly limited as long as the acceptor molecule is an Fc-containing molecule having the N297-linked glycan. Various types such as mAb, CLCH, and Fc fragments can be appropriately selected and used. The reaction temperature in the transglycosylation reaction can be appropriately selected according to the optimum temperatures of the enzymes to be used and is usually 15 to 50°C, preferably 25 to 40°C. In a method of using two enzymes, the transfer reaction may not properly proceed if one of the enzymes is deactivated at the optimum temperature of the other enzyme. Therefore, it is preferred to select a combination of enzymes similar in conditions such as the optimum reaction temperature.

### (2) Fc-containing molecule binding to cyclic peptide via thiol group

In one mode, the conjugate of the present invention may be a compound bonded to a cyclic peptide via a thiol group of the Fc-containing molecule. The Fc-containing molecule having a thiol group can be obtained by a method known to skilled artisans (Hermanson, G.T, Bioconjugate Techniques, pp.56-136, pp.456-493 Academic Press (1996)). Examples include, but are not limited to; Traut's reagent is reacted with the amino group; N-succinimidyl S-acetylthioalkanoates are reacted with the amino group of the Fc-containing molecule followed by reaction with hydroxylamine; after reacting with N-succinimidyl 3-(pyridyldithio)propionate, the Fc-containing molecule is reacted with a reducing agent; the Fc-containing molecule is reacted with a reducing agent such as dithiothreitol, 2-mercaptoethanol, and tris(2-carboxyethyl)phosphine hydrochloride (TCEP) to reduce the disulfide bond at a hinge part in the Fc-containing molecule to form a thiol group. In a preferred mode, the thiol group of the Fc-containing molecule can be a thiol group produced by reduction of the disulfide bond at a hinge part in the Fc-containing molecule.

In one example, using 0.3 to 3 molar equivalents of TCEP as a reducing agent per disulfide at hinge part in the Fc-containing molecule and reacting with the Fc-containing molecule in a buffer solution containing a chelating agent, the Fc-containing molecule with partially or completely reduced disulfide at hinge part in the Fc-containing molecule can be obtained. Examples of the chelating agent include ethylenediaminetetraacetic acid (EDTA) and diethylenetriaminepentaacetic acid (DTPA). It can be used at concentration of 1 mM to 20 mM. Examples of the buffer solution which may be used include a solution of sodium phosphate, sodium borate, or sodium acetate. As a specific example, the Fc-containing molecule can be reacted with TCEP at 4°C to 37°C for 1 to 4 hours to obtain an Fc-containing molecule having partially or completely reduced thiol group. The resulting Fc-containing molecules can be appropriately purified by various chromatography, ultrafiltration, and the like. In particular, from the viewpoint of preventing reduction of the disulfide bond in the cyclic peptide, it is preferable to purify the Fc-containing molecule by ultrafiltration or the like to remove the reducing agent.

A reaction that adds a thiol group to the cyclic peptide-linker moiety (*e.g*., Michael addition reaction with a maleimide group) can be performed to attach the cyclic peptide-linker moiety by a thioether bond.

### 2. Cyclic peptide

The cyclic peptide in the conjugate of the present invention is the cyclic peptide I of this invention as set forth in I above or a cyclic peptide represented by the following formula (X): 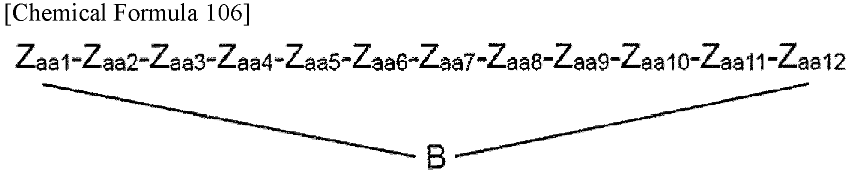 (hereinafter referred to as "the cyclic peptide II of the present(this) invention"). The cyclic peptide II of this invention has formed therein a cyclic structure in which the amino acids Zₐₐ₁ to Zₐₐ₁₂, or if Zₐₐ₁ is absent, the amino acids Zₐₐ₂ to Zₐₐ₁₂, are joined together by amide bonds to form an oligopeptide chain, and the N-terminal amino acid (Zₐₐ₁ or Zₐₐ₂) and the C-terminal amino acid (Zₐₐ₁₂) of the oligopeptide chain are joined together via a ring-forming group B.

In formula (X), B is selected from the following ring-forming groups B₁ to B₆ having an amino acid structure:

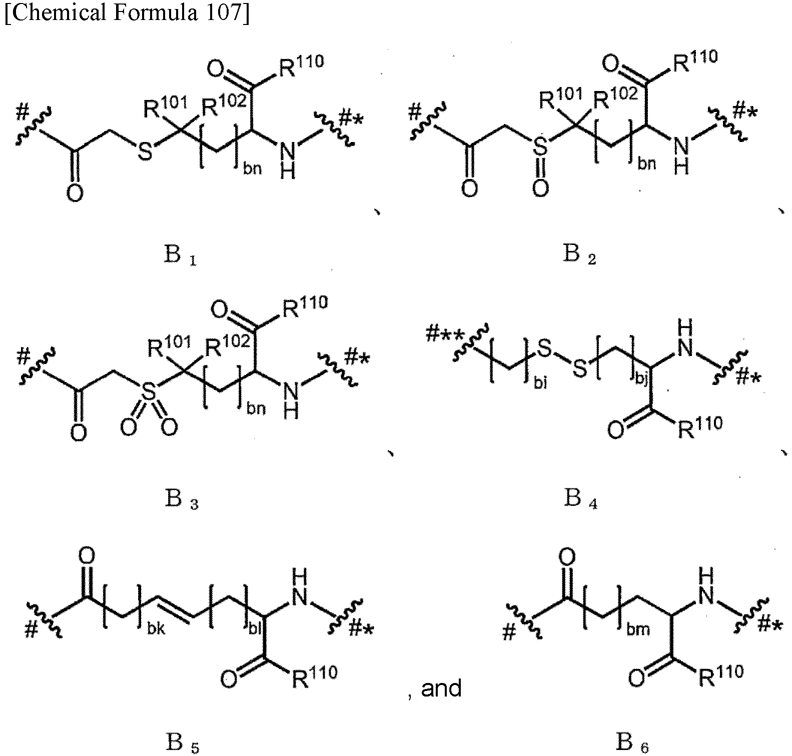

As found in either terminus of B, 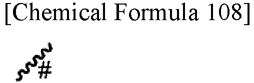 represents a point of attachment to the N-terminal amino group of Zₐₐ₁, or in the absence of Zₐₐ₁, represents a point of attachment to the N-terminal amino group of Zₐₐ₂, 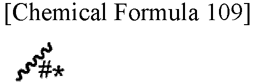 represents a point of attachment to the C-terminal carbonyl group of Zₐₐ₁₂, and 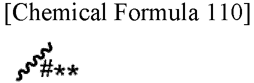 represents a point of attachment to α carbon of Zₐₐ₁, or in the absence of Zₐₐ₁, represents a point of attachment to α carbon of Zₐₐ₂. The asymmetric centers in the amino acid structures contained in the aforementioned ring-forming groups may have the R- or S-configuration.

In one mode, B is 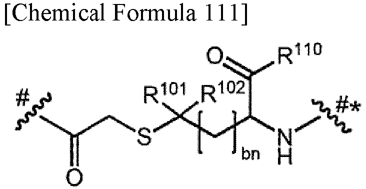 (hereinafter referred to as "B₁"). In the ring-forming group B₁, bn is an integer of 0 to 3 *(e.g.,* 0, 1, 2, or 3), R¹⁰¹ and R¹⁰² are each independently a hydrogen atom or C₁₋₃ alkyl, and R¹¹⁰ is amino or hydroxy. C₁₋₃ alkyl is preferably methyl.

A preferred mode of B₁ is a ring-forming group 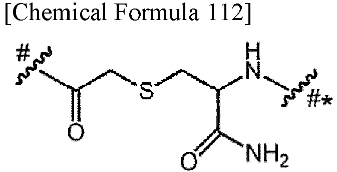 wherein R¹⁰¹ and R¹⁰² are each a hydrogen atom, R¹¹⁰ is amino, and bn is 0 (hereinafter referred to as "B₁ₐ"). B₁ₐ can be obtained by, for example, allowing the -SH group of Cys (provided that the carboxy group is converted to amide) which is amide-bonded to the C-terminus of Zₐₐ₁₂ to react with -COCH₂X (wherein X is a leaving group such as Cl) which is bonded to the N-terminal amino group of Zₐₐ₁ or Zₐₐ₂. In such a ring-forming group, the Cys may be an L-amino acid or a D-amino acid. In particular, that version of the ring-forming group B₁ₐ, where the Cys (provided that the carboxy group is converted to amide) which is amide-bonded to the C-terminus of Zₐₐ₁₂ is an L-amino acid, is referred to as "B₁ₐ₁".

Another preferred mode of B₁ is a ring-forming group 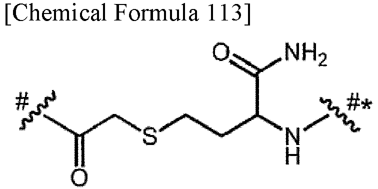 wherein R¹⁰¹ and R¹⁰² are each a hydrogen atom, R¹¹⁰ is amino, and bn is 1 (hereinafter referred to as "B_{1b}"). B_{1b} can be obtained by, for example, allowing the -SH group of HCY (provided that the carboxy group is converted to amide) which is amide-bonded to the C-terminus of Zₐₐ₁₂ to react with -COCH₂X (wherein X is a leaving group such as Cl) which is bonded to the N-terminal amino group of Zₐₐ₁ or Zₐₐ₂. In such a ring-forming group, the HCY may be an L-amino acid or a D-amino acid. In particular, that version of the ring-forming group B_{1b}, where the HCY (provided that the carboxy group is converted to amide) which is amide-bonded to the C-terminus of Zₐₐ₁₂ is an L-amino acid, is referred to as "B_{1b1}".

Another preferred mode of B₁ is a ring-forming group:

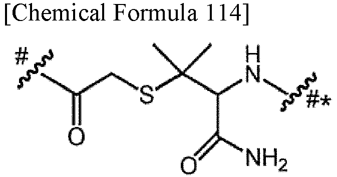

wherein R¹⁰¹ and R¹⁰² are each methyl, R¹¹⁰ is amino, and bn is 0 (hereinafter referred to as "B_{1c}"). B_{1c} can be obtained by, for example, allowing the -SH group of Pen (provided that the carboxy group is converted to amide) which is amide-bonded to the C-terminus of Zₐₐ₁₂ to react with -COCH₂X (wherein X is a leaving group such as Cl) which is bonded to the N-terminal amino group of Zₐₐ₁ or Zₐₐ₂. In such a ring-forming group, the Pen may be an L-amino acid or a D-amino acid. In particular, that version of the ring-forming group B_{1c}, where the Pen (provided that the carboxy group is converted to amide) which is amide-bonded to the C-terminus of Zₐₐ₁₂ is an L-amino acid, is referred to as "B_{1c1}".

In another mode, the ring-forming group B is 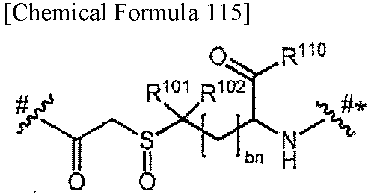 (hereinafter referred to as "B₂"). In the ring-forming group B₂, bn is an integer of 0 to 3 (*e.g.,* 0, 1, 2, or 3), R¹⁰¹ and R¹⁰² are each independently a hydrogen atom or C₁₋₃ alkyl, and R¹¹⁰ is amino or hydroxy.

A preferred mode of B₂ is that version of said ring-forming group, where R¹⁰¹ and R¹⁰² are each a hydrogen atom, R¹¹⁰ is amino, and bn is 0 (hereinafter referred to as "B₂ₐ"). B₂ₐ can be obtained by, for example, oxidizing the sulfide group of the ring-forming group B₁ₐ as shown above to sulfoxide. In particular, the ring-forming group obtained by oxidizing the sulfide group of B₁ₐ₁ to sulfoxide is referred to as "B₂ₐ₁".

In another mode, B is

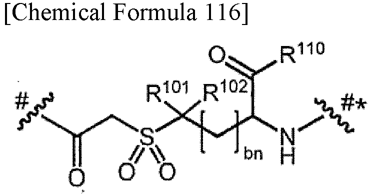

(hereinafter referred to as "B₃"). In the ring-forming group B₃, bn is an integer of 0 to 3 (*e.g.,* 0, 1, 2, or 3), R¹⁰¹ and R¹⁰² are each independently a hydrogen atom or C₁₋₃ alkyl, and R¹¹⁰ is amino or hydroxy.

A preferred mode of B₃ is that version of said ring-forming group, where R¹⁰¹ and R¹⁰² are each a hydrogen atom, R¹¹⁰ is amino, and bn is 0 (hereinafter referred to as "B₃ₐ"). B₃ₐ can be obtained by, for example, oxidizing the sulfide group of the ring-forming group B₁ₐ as shown above to sulfone. In particular, the ring-forming group obtained by oxidizing the sulfide group of B₁ₐ₁ to sulfone is referred to as "B₃ₐ₁".

In another mode, B is 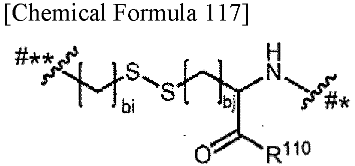 (hereinafter referred to as "B₄"). In the ring-forming group B₄, bi and bj are each independently an integer of 1 to 3 (*e.g.*, 1, 2, or 3), and R¹¹⁰ is amino or hydroxy.

When B is B₄, Zₐₐ₁ or Zₐₐ₂ is a residue of an amino acid represented by 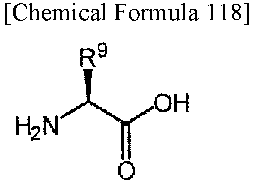 [wherein R⁹ is a group represented by the formula -(CH₂)ₜSH (wherein t is an integer of 1 to 3 (*e.g.*, 1, 2, or 3))].

In a preferred mode of B₄, bi and bj are each 1, and R¹¹⁰ is amino (hereinafter, referred to as "B₄ₐ"). B₄ₐ can be obtained by, for example, allowing the -SH group of Cys (provided that the carboxy group is converted to amide) which is amide-bonded to the C-terminus of Zₐₐ₁₂ to react with the -SH group of Zₐₐ₁ or Zₐₐ₂. In such a ring-forming group, the Cys may be an L-amino acid or a D-amino acid. In particular, that version of the ring-forming group B₄ₐ, where the Cys (provided that the carboxy group is converted to amide) which is amide-bonded to the C-terminus of Zₐₐ₁₂ is an L-amino acid, is referred to as "B₄ₐ₁".

In another mode, B is 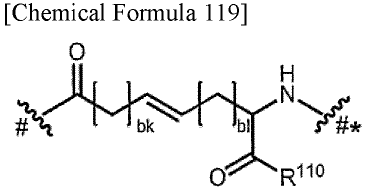 (hereinafter referred to as "B₅"). In the ring-forming group B₅, bk and bl are each independently an integer of 0 to 3 (*e.g*., 0, 1, 2, or 3), and R¹¹⁰ is amino or hydroxy. The ring-forming group B₅ can be obtained by, for example, olefin metathesis of the amino acid (provided that the carboxy group may be converted to amide) which has the group - (CH₂)_{bl}CH=CH₂ on its side chain and is amide-bonded to the C-terminus of Zₐₐ₁₂, and the group -CO(CH₂)_{bk}CH=CH₂ which is bonded to the N-terminal amino group of Zₐₐ₁ or Zₐₐ₂. In such a ring-forming group, the amino acid which is amide-bonded to the C-terminus of Zₐₐ₁₂ may be an L-amino acid or a D-amino acid.

In another mode, B is 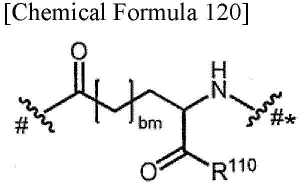 (hereinafter referred to as "B₆"). In the ring-forming group b₆, bm is an integer of 1 to 7 (*e.g.,* 1, 2, 3, 4, 5, 6, or 7), and R¹¹⁰ is amino or hydroxy. B₆ can be obtained by, for example, reducing the carbon-carbon double bond of B₅.

In formula (X), Zₐₐ₁ is a residue of an aliphatic amino acid, an aromatic amino acid, a basic amino acid, a neutral amino acid, or an acidic amino acid, or is absent. In a preferred mode, Zₐₐ₁ is a residue of Arg, Lys, His, Gly, Ala, Asn, Thr, Ser, Met, Leu, Ile, Val, Gln, Phe, Tyr, Trp, or Cys, or is absent, and more preferably is absent.

In formula (X), Zₐₐ₂ is a residue of an aromatic amino acid or a neutral amino acid. In a preferred mode, Zₐₐ₂ is a residue of an aromatic amino acid, more preferably a residue of Phe, Tyr, Trp, 2Nal, 4CF, or DCF, even more preferably a 2Nal residue.

In formula (X), Zₐₐ₃ is a residue of an aliphatic amino acid, an aromatic amino acid, or a basic amino acid. In a preferred mode, Zₐₐ₃ is a residue of Ile, Leu, Nle, Tle, Trp, 2Nal, 4CF, or Arg, more preferably a residue of Arg or Tle.

In formula(X), Zₐₐ₄ is a residue of Ser, Thr, Ala, or ^{m}S. In a preferred mode, Zₐₐ₄ is a Ser residue.

In formula (X), Zₐₐ₅ is a residue of Gly or Ser. In a preferred mode, Zₐₐ₅ is a Gly residue.

In formula (X), Zₐₐ₆ is a residue of a basic amino acid or a neutral amino acid. In a preferred mode, Zₐₐ₆ is a residue of Arg, Lys, His, Ser, Cit, or AGB, more preferably a residue of Arg or AGB.

In formula (X), Zₐₐ₇ is a residue of a neutral amino acid or an acidic amino acid. In a preferred mode, Zₐₐ₇ is a residue of Asn or Asp.

In formula (X), Zₐₐ₈ is a residue of an aromatic amino acid. In a preferred mode, Zₐₐ₈ is a residue of Trp or 2Nal, more preferably a Trp residue.

In formula (X), Zₐₐ₉ is a residue of an aliphatic amino acid, a neutral amino acid, or an aromatic amino acid. In a preferred mode, Zₐₐ₉ is a residue of Val, Nle, Ahp, or Met, more preferably a residue of Val or Nle.

In formula (X), Zₐₐ₁₀ is a residue of a basic amino acid, an aliphatic amino acid, an aromatic amino acid, or a neutral amino acid. In a preferred mode, Zₐₐ₁₀ is a residue of Arg, AGB, Lys, His, AMF, Phg, or Val, more preferably a residue of Arg, AGB, or Val.

In formula (X), Zₐₐ₁₁ is a residue of an aromatic amino acid. In a preferred mode, Zₐₐ₁₁ is a residue of Trp or 2Nal, more preferably a Trp residue.

In formula (X), Zₐₐ₁₂ is a residue of an aliphatic amino acid, an aromatic amino acid, or a basic amino acid. In a preferred mode, Zₐₐ₁₂ is a residue of Val, Tle, CPTG, CHXG, or Phe, more preferably a residue of Val, CPTG, or CHXG.

A preferred mode of the cyclic peptide II of the present invention is a cyclic peptide represented by formula (X), wherein
B is the ring-forming group B₁ₐ, B_{1b}, or B_{1c};
Zₐₐ₁ is a residue of Arg, Lys, His, Gly, Ala, Asn, Thr, Ser, Met, Leu, Ile, Val, Gln, Phe, Tyr, Trp, or Cys, or is absent;
Zₐₐ₂ is a residue of Phe, Tyr, Trp, 2Nal, 4CF, or DCF;
Zₐₐ₃ is a residue of Ile, Leu, Nle, Tle, Trp, 2Nal, 4CF, or Arg;
Zₐₐ₄ is a Ser residue;
Zₐₐ₅ is a Gly residue;
Zₐₐ₆ is a residue of Arg, Lys, His, Ser, Cit, or AGB;
Zₐₐ₇ is a residue of Asn or Asp;
Zₐₐ₈ is a residue of Trp or 2Nal;
Zₐₐ₉ is a residue of Val, Nle, Ahp, or Met;
Zₐₐ₁₀ is a residue of Arg, AGB, Lys, His, AMF, Phg, or Val;
Zₐₐ₁₁ is a residue of Trp or 2Nal; and
Zₐₐ₁₂ is a residue of Val, Tle, CPTG, CHXG, or Phe.

A more preferred mode of the cyclic peptide II of the present invention is a cyclic peptide represented by formula (X), wherein
B is the ring-forming group B₁ₐ;
Zₐₐ₁ is absent;
Zₐₐ₂ is a 2Nal residue;
Zₐₐ₃ is a residue of Arg or Tle;
Zₐₐ₄ is a Ser residue;
Zₐₐ₅ is a Gly residue;
Zₐₐ₆ is a residue of Arg or AGB;
Zₐₐ₇ is a residue of Asn or Asp;
Zₐₐ₈ is a Trp residue;
Zₐₐ₉ is a residue of Val or Nle;
Zₐₐ₁₀ is a residue of Arg, AGB, or Val;
Zₐₐ₁₁ is a Trp residue; and
Zₐₐ₁₂ is a residue of Val, CPTG, or CHXG.

A yet more preferred mode of the cyclic peptide II of the present invention is a cyclic peptide selected from the group consisting of compounds represented by the formulas: 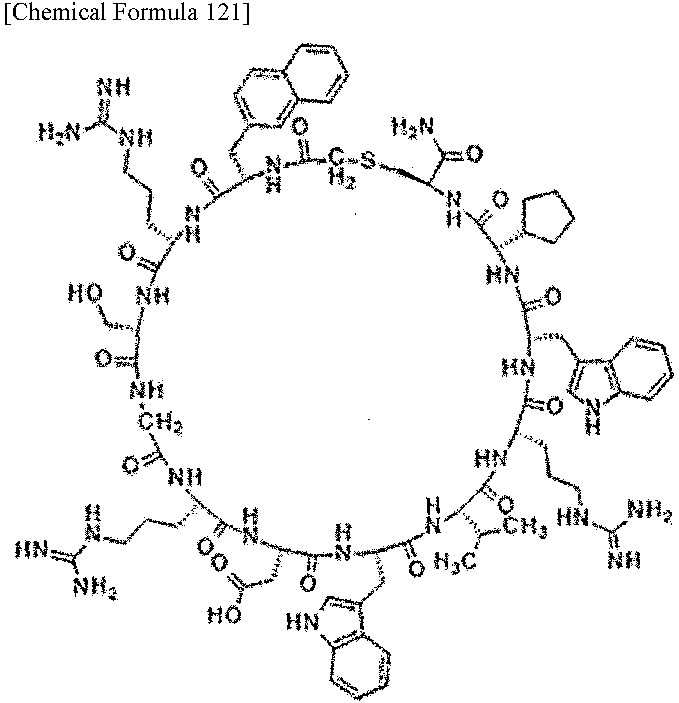 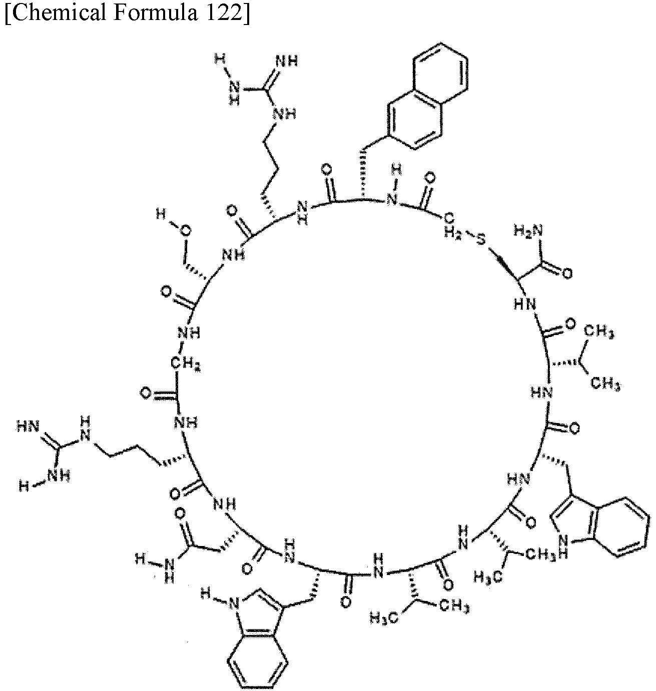 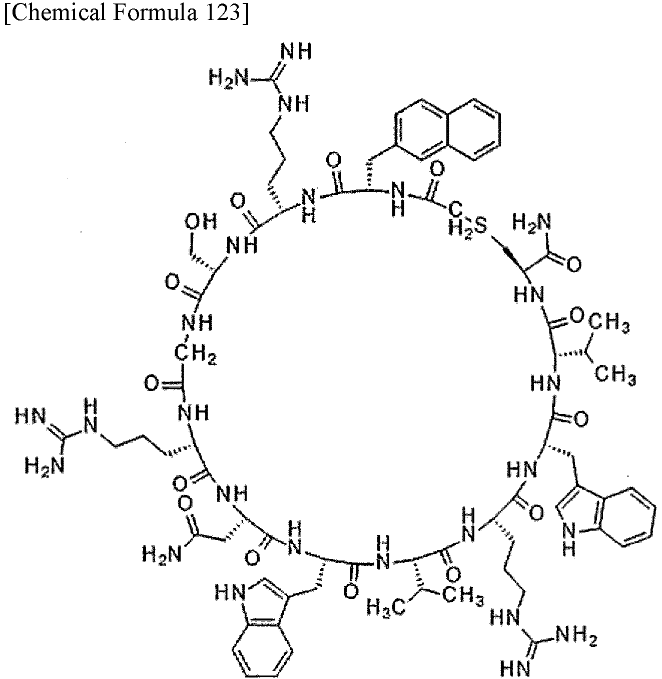 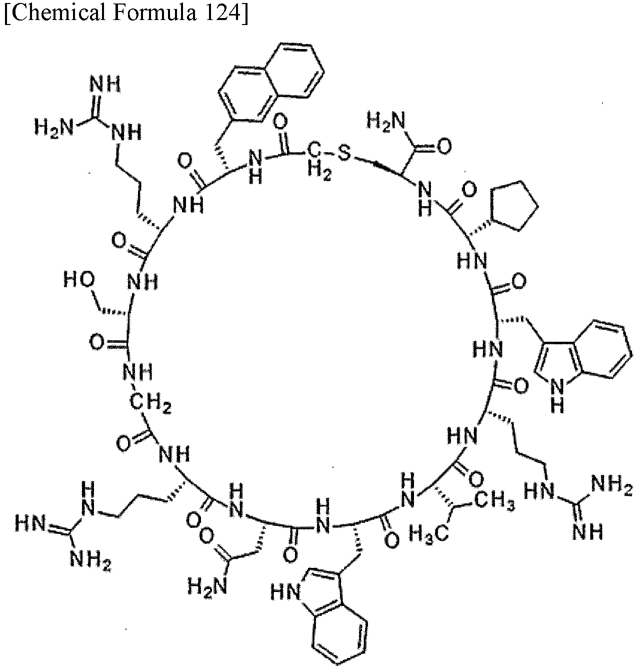 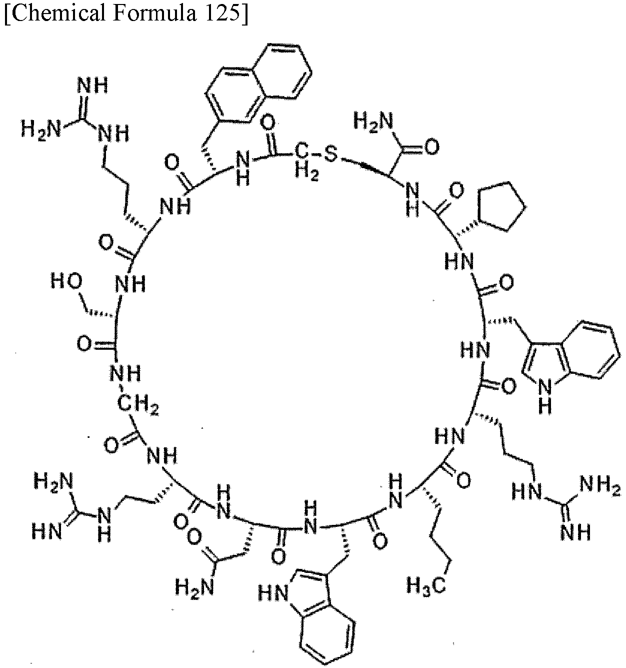 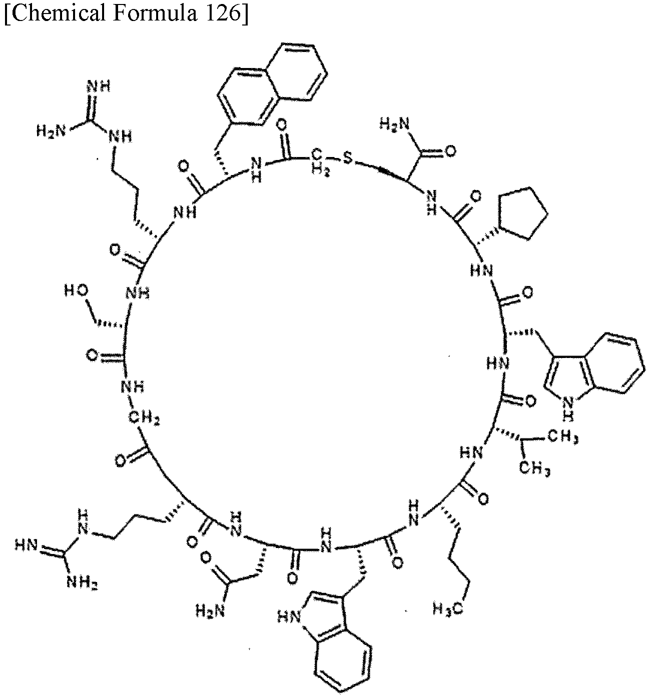 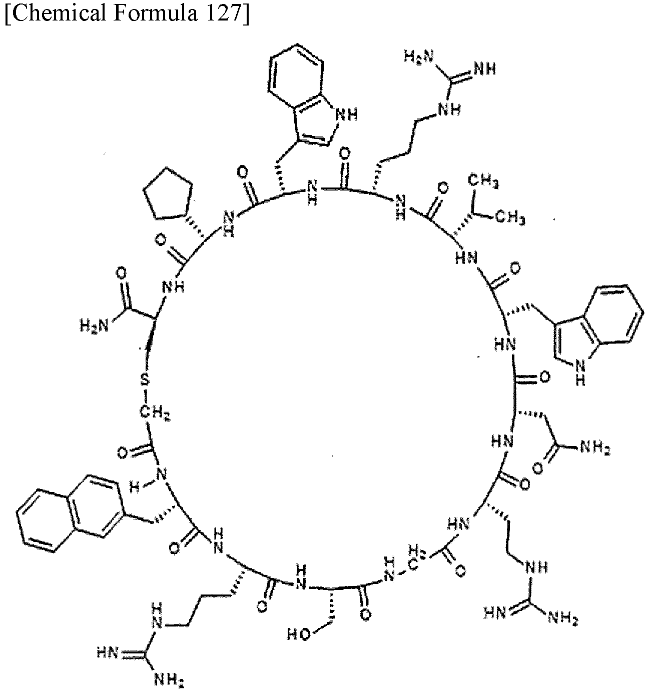 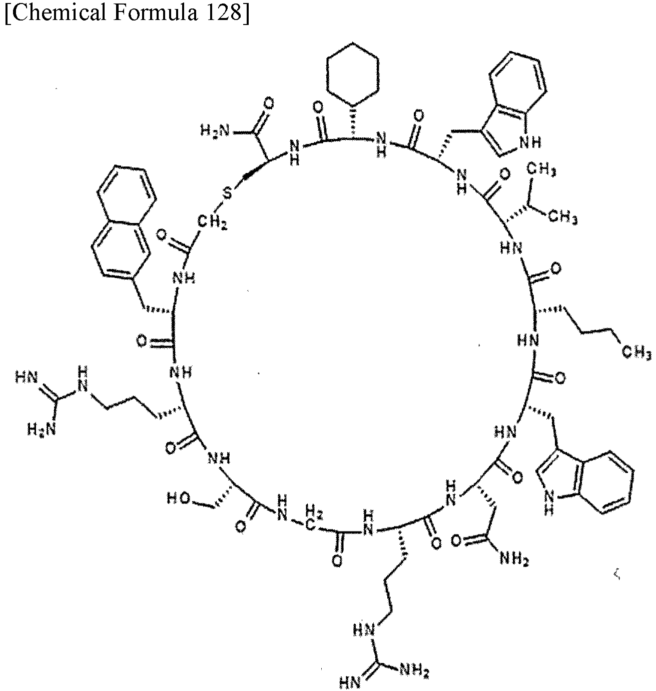 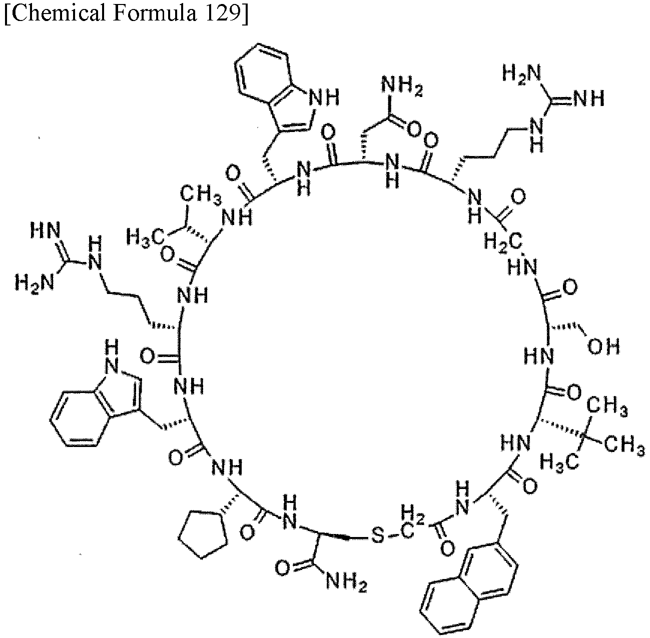 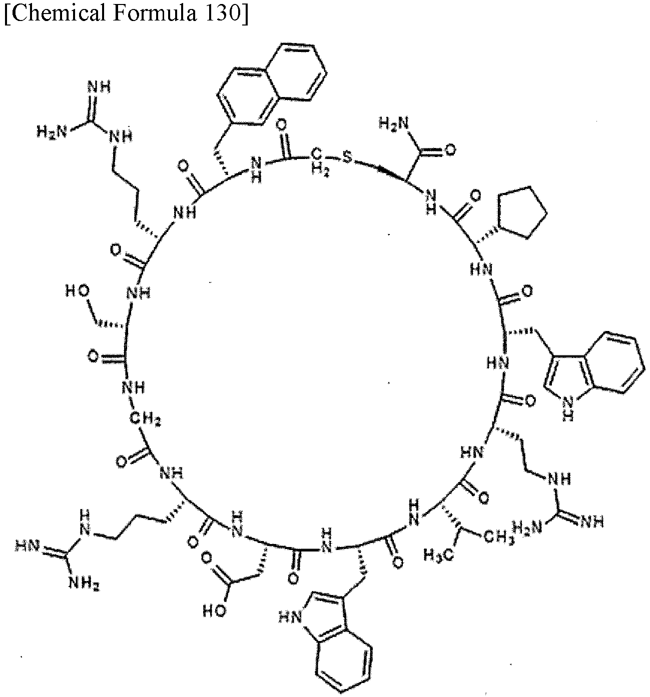 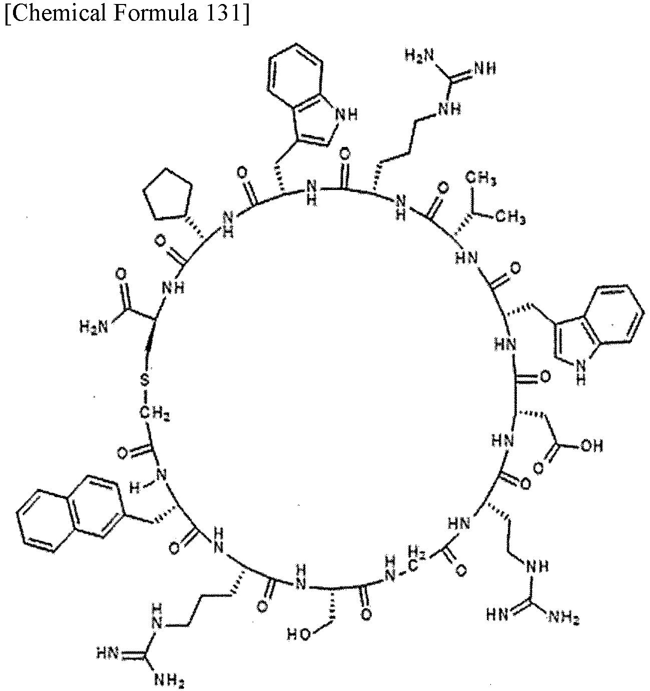 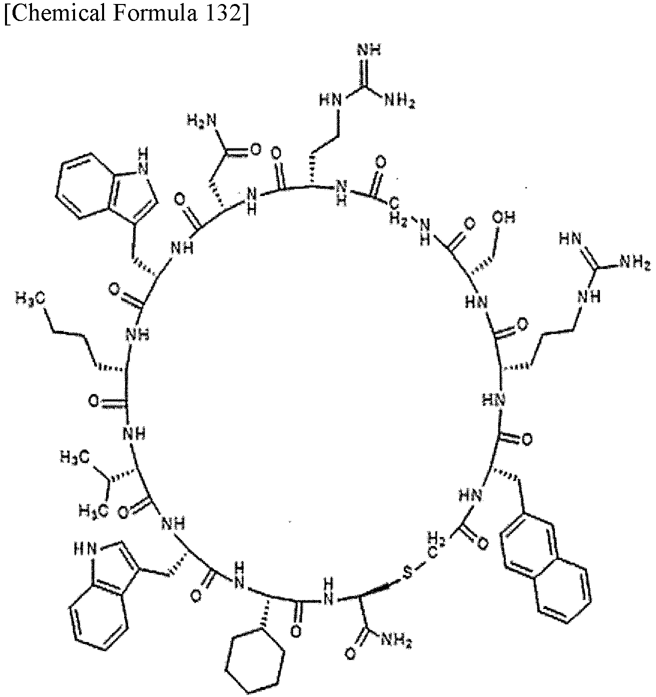 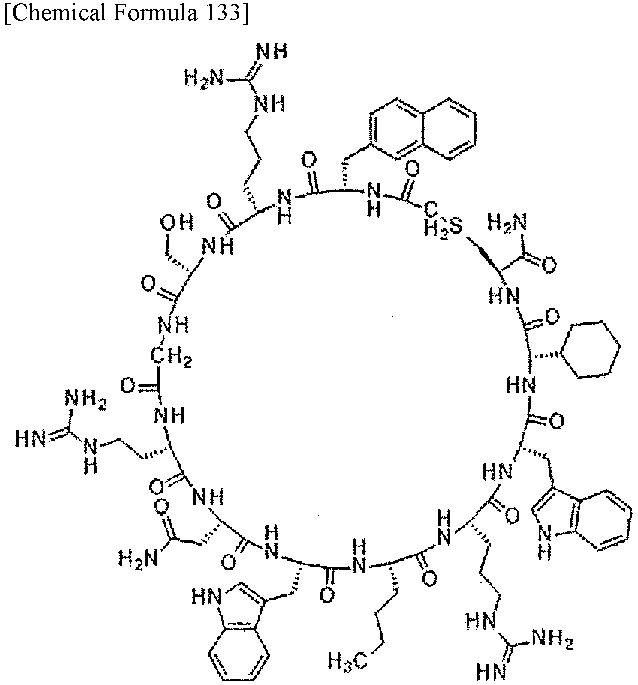 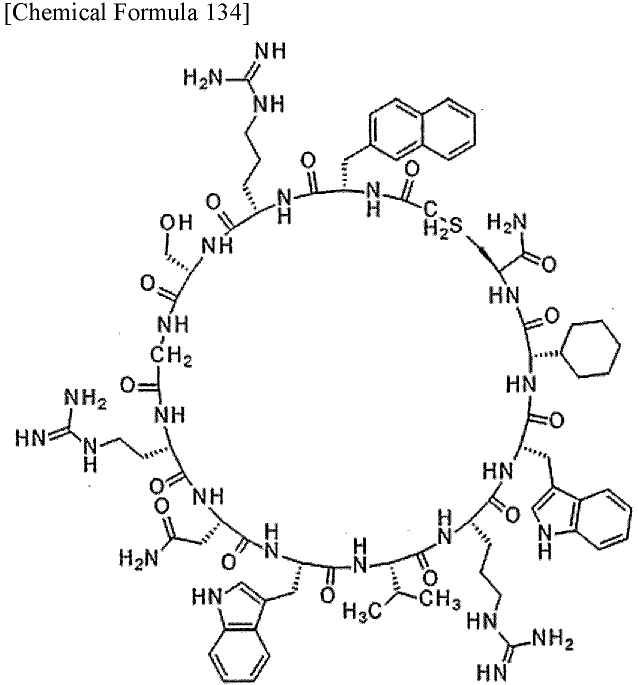 and 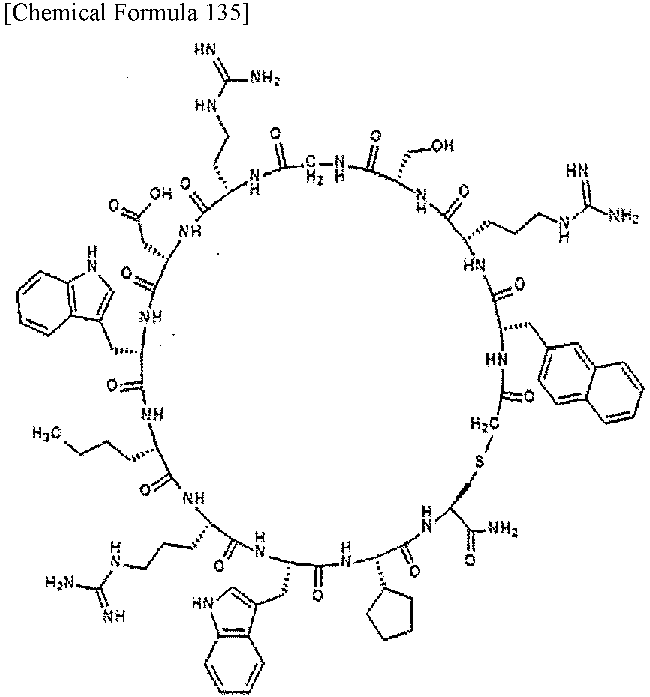 The oligopeptides shown above are cyclic peptides in Conjugate-13 to Conjugate-63, Conjugate-67 to Conjugate-71, Conjugate-73, Conjugate-75 to Conjugate-79, Conjugate-83 to Conjugate-86, Conjugate-88 to Conjugate-95, and Conjugate-97 of Examples as shown hereinbelow. In all of these oligopeptides, the ring-forming group B is B₁ₐ₁.

The cyclic peptide II of this invention can be prepared by a method known in the art, such as chemical synthesis and cell-free translation system in the same way as cyclic peptide I of this invention (refer to WO 2018/052002).

In a preferred mode, the cyclic peptide in the conjugate of the present invention may be the cyclic peptide I of this invention.

### 3. Linker structure

In the conjugate of the present invention, the cyclic peptide is coupled to a carrier molecule via a side chain of an amino acid residue constituting the cyclic peptide or a carbonyl group to which R¹⁰ of the ring-forming group A or R¹¹⁰ of the ring-forming group B is bonded. In one mode, the cyclic peptide is coupled to a carrier molecule via a carbonyl group to which R¹⁰ of the ring-forming group A or R¹¹⁰ of the ring-forming group B is bonded. In one mode, the conjugate of this invention may be a compound in which the cyclic peptide and the carrier molecule are coupled via a linker structure. The binding of the cyclic peptide to the linker structure or carrier molecule can be carried out according to a conventional procedure.

The linker structure can be any structure as long as the conjugate of the present invention can exert a TSP-1 inhibitory effect. In a preferred mode, the linker structure may include at least one structure selected from the group consisting of a polyoxyalkylene chain, an amino acid residue, and an oligopeptide chain consisting of two or more amino acid residues.

As referred to herein, a molecule from which a linker structure is derived may be referred to as a "linker molecule".

The term "backbone" in the linker structure refers to a linear portion that is essential for joining the cyclic peptide and the carrier molecule, while the term "side chain" in the linker structure refers to a portion branched from backbone. Even for individual amino acid residues that constitute the linker structure, the portion of the bond that is essential for joining the cyclic peptide and the carrier molecule is referred to as "backbone", while the portion branched from backbone is referred to as a side chain. For example, a side chain in an amino acid residue generally refers to a group bonded to an α carbon other than an amino group, a carboxyl group, and a hydrogen directly bonded to the α carbon (although hydrogen is the side chain in a glycine residue), but the portion of such a side chain may be "backbone" in the linker structure of the present invention (*e.g.,* if KPEG is included in the linker structure, the ε amino group of KPEG is "backbone" and the α amino group and the PEG chain bonded thereto are the "side chains").

The polyoxyalkylene chain contained in the linker structure is not particularly limited as long as it is pharmaceutically acceptable, and examples thereof include a polymethylene glycol chain, a polyethylene glycol chain, a polypropylene glycol chain, a polybutylene glycol chain, a polypentylene glycol chain, a polyhexylene glycol chain, and a polyoxyalkylene chain in which an ethylene glycol group and a propylene glycol group are coupled in a block or random manner. In a preferred mode, the polyoxyalkylene chain may be a polyethylene glycol (PEG) chain, wherein
the polyoxyalkylene chain contained in backbone (*i.e*., the linear portion that is essential for joining the cyclic peptide and the carrier molecule) in the linker structure can be used to regulate the distance between the cyclic peptide and the carrier molecule. The polyoxyalkylene chain contained in backbone of such a linker structure may be contained in the linker structure as a part of non-natural amino acids containing a polyoxyalkylene chain such as P6P, P12P, P24P, and P36P.

The degree of polymerization of each polyoxyalkylene chain contained in backbone of the linker structure may be, but is not particularly limited to, 2 to 50, 6 to 50, 6 to 30, or 6 to 12, for example, 2, 3, 4, 5, 6, 7, 8, 9, 10, 11, 12, 24, or 36, preferably 6, 12, 24, or 36, more preferably 6, 12, or 24. Also, the total degree of polymerization of polyoxyalkylene chains contained in backbone of the linker structure (*i.e*., the degree of polymerization of only one polyoxyalkylene chain contained in backbone of one linker structure, or the total degree of polymerization of all polyoxyalkylene chains contained in backbone of one linker structure) may be 6 to 100, more preferably 6 to 50, for example, 6, 12, 18, 24, 30, 36, 42, 48, or 50. In a preferred mode, the polyoxyalkylene chain contained in backbone of the linker structure is a PEG chain, and the total degree of polymerization can be 6 to 100, more preferably 6 to 50, for example, 6, 12, 18, 24, 30, 36, 42, 48, or 50.

The amino acid residue contained in the linker structure may be either a residue of a natural amino acid or a residue of a non-natural amino acid and may be either D form or L form. In a preferred mode, the amino acid residue contained in the linker structure may be a residue of Gly, N-methylglycine (NMeG), Asp, D-Asp (d), Glu, D-Glu, Lys, D-Lys, β-alanine (bAla), Ser, D-Ser, a non-natural amino acid containing a polyoxyalkylene chain in a side chain (*i.e.,* non-natural amino acid containing a polyoxyalkylene chain in a portion serving as a side chain in the linker structure; *e.g*., KPEG), or a non-natural amino acid containing a glycan such as an SG chain in a side chain (i.e., non-natural amino acid containing a glycan in a portion serving as a side chain in the linker structure; e.g., KSG).

The amino acid residue constituting the oligopeptide chain contained in the linker structure may be either a residue of a natural amino acid or a residue of a non-natural amino acid and may be either D form or L form. The oligopeptide chain may consist of 2 to 20 amino acid residues, for example, 1, 2, 3, 4, 5, 6, 7, 8, 9, 10, 11, 12, 13, 14, 15, 16, 17, 18, 19, or 20 amino acid residues, without particular limitation. In a preferred mode, the oligopeptide chain contained in the linker structure may contain one or more amino acid residues selected from the group consisting of Gly, N-methylglycine (NMeG), Asp, D-Asp, Glu, D-Glu, Lys, D-Lys, β-alanine (bAla), Ser, D-Ser, a non-natural amino acid containing a polyoxyalkylene chain in a side chain (*i.e*., non-natural amino acid containing a polyoxyalkylene chain in a portion serving as a side chain in the linker structure; *e.g*., KPEG), and a non-natural amino acid containing a glycan such as an SG chain in a side chain (*i.e*., non-natural amino acid containing a glycan in a portion serving as a side chain in the linker structure; *e.g.*, KSG).

The linker structure may be coupled to the cyclic peptide at the carbonyl group to which R¹⁰ of the ring-forming group A or R¹¹⁰ of the ring-forming group B is bonded. Various methods known in the field of organic synthetic chemistry can be applied to the binding between the linker structure and the cyclic peptide, without particular limitations. When the linker structure includes an amino acid residue or an oligopeptide chain, the N-terminal amino group of the amino acid residue or oligopeptide chain preferably forms an amide bond with the carbonyl group to which R¹⁰ of the ring-forming group A or R¹¹⁰ of the ring-forming group B is bonded, where R¹⁰ or R¹¹⁰ is substituted with NH of the N-terminal amino group.

Various methods known in the field of organic synthetic chemistry can be applied to the binding between the linker structure and the carrier molecule, without particular limitations. In general, this binding can be carried out with reference to a conjugation method applicable to antibody-drug conjugate synthesis (Bioconjugate Chem. 2015, 26, 2198-2215). Examples of ring-forming methods through the Huisgen reaction of an azide group with an acetylene group include a cycloaddition reaction to form a 1,2,3-triazole ring [Cu(I)-catalyzed azide-alkyne cycloaddition (CuAAC)] and a cycloaddition reaction to form a 1,2,3-triazole ring through a reaction of an azide group with dibenzylcyclooctene (DBCO) [strain-promoted azide-alkyne cycloaddition (SPAAC)]. In this context, other compounds such as a compound having a twisted cyclooctyne structure, bicyclo[6.1.0]non-4-ene (BCN) (Angew. Chem. Int. Ed. 2010, 49, 9422-9425), and a cycloalkyne containing a heteroatom on a medium ring structure (Angew. Chem. Int. Ed. 2015, 54, 1190-1194) can also be utilized in the same manner. Other examples of the ring-forming reaction include a Diels-Alder cycloaddition reaction of a 1,2,4,5-tetrazine ring with twisted alkene (Curr. Opin. Chem. Biol., 2014, 21, 89-95). Examples of cyclocondensation based on a Pictet-Spengler reaction with an aldehyde group include Hydrazino-Pictet-Spengler ligation (Bioconjugate Chem. 2013, 24,846-851.) and Oxyamine-based Pictet-Spengler ligation (Proc. Natl. Acad. Sci. U.S.A. 110, 46-51). Additional examples thereof include an amide bond between an amino group and a carboxyl group, Michael addition of a thiol group to a maleimide group, reaction of a thiol group with a methylsulfonylphenyloxadiazole group (Angew. Chem., Int. Ed. 2013, 52, 12592-6), a bond between a thiol group and an iodoacetyl group, cross-disulfide formation of a thiol group with activated disulfide, hydrazone condensation of an aldehyde group with hydrazide, and oxime condensation of an aldehyde group with an aminooxy group. The linker molecule adopted in the present invention can be appropriately selected as a molecule having a functional group conforming to any of these binding patterns and used for the formation of the linker structure.

In such a binding reaction, stereoisomers, optical isomers, geometric isomers, or the like may be formed depending on the binding pattern. These isomers may be used separately by a known method or as a mixture. Since the final conjugate is a macromolecule, the isomeric structural differences in these substructures are likely to have almost no influence on the conjugate.

In a preferred mode, the linker structure includes a linking group C that binds to the carrier molecule. More preferably, the linking group C may include a maleimide group-derived moiety in a thioether bond formed by reaction of a maleimide group with a thiol group or 1,2,3-triazole ring.

When the linking group C contains a maleimide group-derived moiety, the maleimide group is preferably bonded to a thiol group of a cysteine residue of the carrier molecule through maleimide condensation to form a thioether bond. In this case, the thiol group is preferably a thiol group produced by reduction of the disulfide bond at a hinge part in the Fc-containing molecule.

When the linking group C includes a 1,2,3-triazole ring, the 1,2,3-triazole ring preferably binds to a glycan which is bonded to an asparagine residue corresponding to position 297 according to the Eu index of the Fc-containing molecule (N297-linked glycan). Such a linker structure can be, for example, a structure with a 1,2,3-triazole ring formed by a Click reaction between an azide group introduced into the N297-linked glycan of the Fc-containing molecule and a DBCO group bonded to the linker molecule. In this structure, a geometric isomer can be formed in which the Fc-containing molecule is bonded to the 1- or 3-position of the triazole ring. When the reaction occurs with two to four azide groups per Fc-containing molecule, geometric isomeric structures may be mixed in one molecule of the conjugate.

In a preferred mode, the linking group C has a structure represented by any of the following formulas: 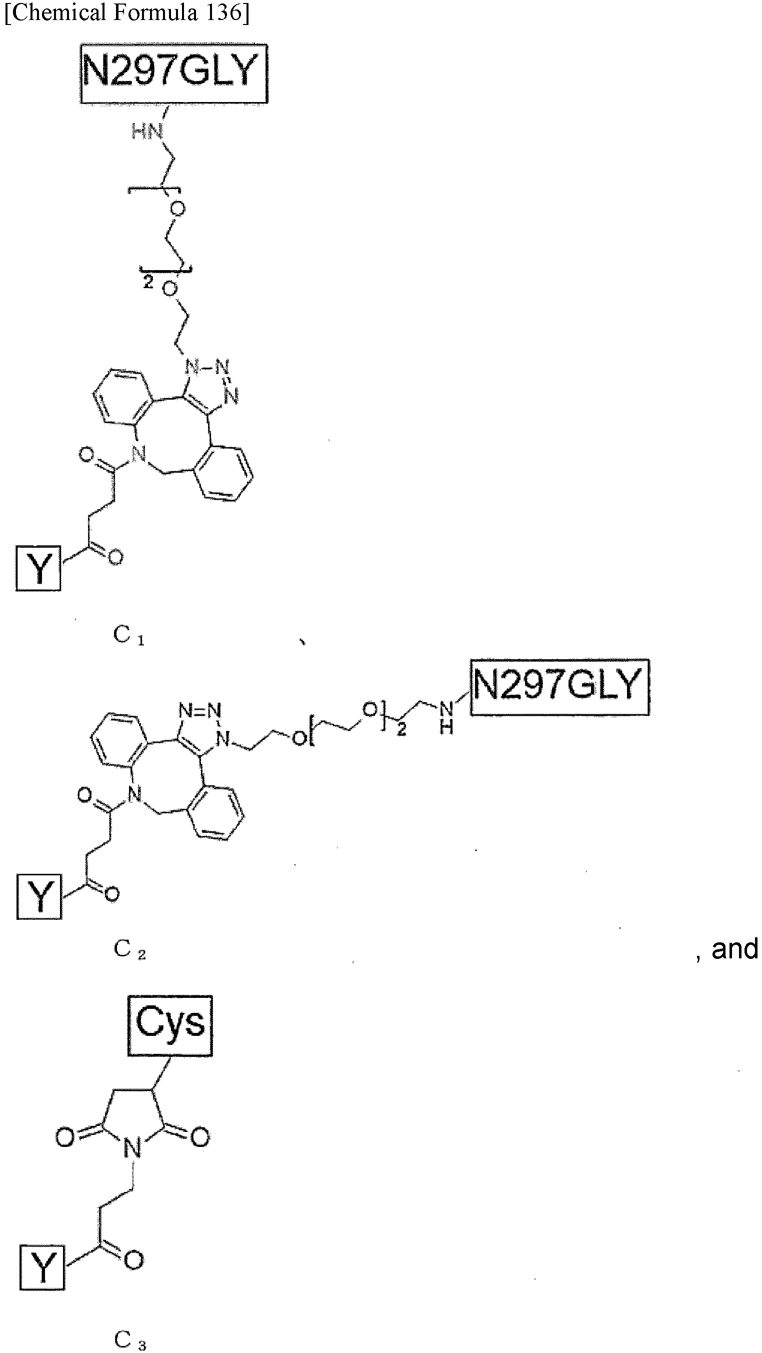 (wherein
N297GLY represents binding to the non-reducing terminus of N297-linked glycan of the Fc-containing molecule;
Cys represents binding to the thiol group of a cysteine residue of the carrier molecule; and
Y represents a point of attachment to the adjacent amino acid residue).

In one mode, the linker structure can be represented by the formula (XX):

-L1-L2-L3-C-

[wherein
L1 is an oligopeptide containing 3 to 6 neutral amino acid residues bonded in a linear manner;
L2 is L2a or L2b, wherein
L2a is a non-natural amino acid residue comprising a polyoxyalkylene chain in backbone, or an oligopeptide comprising a non-natural amino acid residue comprising a polyoxyalkylene chain in backbone, wherein the total degree of polymerization of the polyoxyalkylene chain contained in backbone of L2a is 6 to 100;
L2b is an oligopeptide containing 10 to 20 (*e.g.,* 10, 11, 12, 13, 14, 15, 16, 17, 18, 19, or 20) natural or non-natural α-amino acid residues bonded in a linear manner;
L3 is an amino acid residue represented by the formula 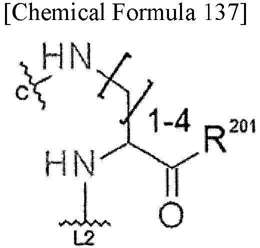
(wherein R²⁰¹ is a hydroxy group or an amino group, 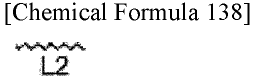
represents a point of attachment to an adjacent amino acid residue in L2, and 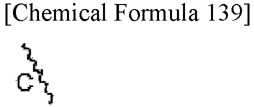
represents a point of attachment to the linking group C);
C is the linking group C;
the N-terminal amino group of L1 forms an amide bond with a carbonyl group to which R¹⁰ of the ring-forming group A or R¹¹⁰ of the ring-forming group B is bonded, where R¹⁰ or R¹¹⁰ is substituted with NH of the N-terminal amino group;
L1 and L2 are joined together by an amide bond; and
L2 and L3 are joined together by an amide bond.

Amino acid residues present between L1 and L2 may be determined to be present in either the adjacent L1 or L2, but if the linker structure as a whole matches the linker structure of formula (XX) above, then the linker structure is included in formula (XX) above.

Regarding the linker structure, amino acid residues "bonded in a linear manner" means that the amino acid residues are joined together so as to constitute an essential portion that is essential for joining the cyclic peptide and the carrier molecule. The neutral amino acid residues need not to be contiguous.

In a preferred mode, the neutral amino acid residue contained in L1 may be a residue of one or more amino acids selected from the group consisting of Gly, Ser, D-Ser, N-methylglycine (NMeG), and β-alanine (bAla). L1 can be an oligopeptide consisting of 3 to 6 (*e.g*., 3, 4, 5, or 6) amino acid residues, preferably an oligopeptide consisting of 4 or 5 amino acid residues, and more preferably an oligopeptide consisting of 4 amino acid residues.

In a preferred mode, the polyoxyalkylene chain contained in backbone of L2a may be a PEG chain. In this case, the non-natural amino acid residue containing a PEG chain in backbone may be a residue of an amino acid represented by the following formula: 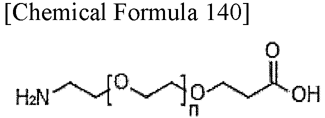 [wherein n represents an integer of 5 to 40 (*e.g*., 5, 11, 23, or 35)]. The total degree of polymerization of the PEG chain contained in backbone of L2a can be, for example, 5 to 100, preferably 6 to 50, more preferably 10 to 50, even more preferably 10 to 40 or 20 to 40. Alternatively, the total degree of polymerization of the PEG chain contained in backbone of L2a can be, for example, 5 or more, preferably 6 or more, more preferably 10 or more or 20 or more, and also, for example, 100 or less, preferably 50 or less, more preferably 40 or less (*e.g.,* 6, 12, 18, 24, 30, or 36). The non-natural amino acid residue contained in L2a, which contains a polyoxyalkylene chain in backbone, can be a residue of P6P, P12P, P24P, or P36P, or a combination of such non-natural amino acid residues. In a preferred mode, the non-natural amino acid residue contained in L2a, which contains a polyoxyalkylene chain in backbone, can be a residue of P6P, P12P, or P24P, or a combination of such non-natural amino acid residues.

In a preferred mode, the a-amino acid of L2b may be glycine or N-methylglycine, more preferably N-methylglycine. The oligopeptide of L2b can be oligo-N-methylglycine (also referred to herein as "NMeG13") with 10 to 50, preferably 10 to 20, more preferably 10 to 15, and even more preferably 13 consecutive N-methylglycines.

L2 may further contain a residue of a non-natural amino acid containing a polyoxyalkylene chain or glycan in a side chain. Examples of the non-natural amino acid containing a polyoxyalkylene chain in a side chain include, but are not limited to, non-natural amino acids containing PEG in a portion serving as a side chain in the linker structure, such as KPEG, and examples of the non-natural amino acid containing a glycan in a side chain include, but are not limited to, non-natural amino acids containing a sialyl glycan in a portion serving as a side chain in the linker structure, such as KSG. L2 may contain two or more residues of non-natural amino acids containing a polyoxyalkylene chain or glycan in a side chain, and preferably contains one residue of a non-natural amino acid containing a polyoxyalkylene chain or glycan in a side chain. In a preferred mode, L2 may contain one KPEG residue or KSG residue.

In a preferred mode, backbones of the oligopeptides of L1 and L2 can further contain 1 to 3 (*e.g*., 1, 2, or 3), more preferably 2 acidic amino acid residues in total at one or more positions not adjacent to the ring-forming group A or ring-forming group B. The term "backbone" herein means a linear portion that is essential for joining the cyclic peptide and the carrier molecule. The acidic amino acid residues need not to be contiguous. The position of the acidic amino acid residue is not particularly limited as long as it is not a position adjacent to the ring forming group A or the ring forming group B, but the acidic amino acid residue preferably exists between L1 and L2. If an acidic amino acid residue is present between L1 and L2, the acidic amino acid residue may be determined to be present in either L1 or L2. The acidic amino acid residue may be a residue of one or more amino acids selected from the group consisting of Asp, D-Asp, Glu, and D-Glu, preferably a residue of one or more amino acids selected from the group consisting of Asp, D-Asp, and Glu, more preferably an Asp residue. Most preferably, two Asp residues are present at positions adjacent to L1 and L2.

The number of acidic amino acid residues contained in L1 and L2 may be determined in consideration of the balance of charges of the amino acid residues contained in the cyclic peptide and linker structure. In this case, a charge of an acidic amino acid residue is defined as + 1 if the amino acid residue is monovalent and + 2 if the amino acid residue is divalent. The charge of a basic amino acid residue is defined as -1 if the amino acid residue is monovalent and -2 if the amino acid residue is divalent. The number of acidic amino acid residues contained in L1 and L2 can be determined such that the charges of the amino acid residues contained in the cyclic peptide and linker structure are -4 to + 1, more preferably -2 to 0. When the charges of the amino acid residues contained in the cyclic peptide and linker structure are in the above numerical range, the conjugate tends to have an excellent TSP-1 inhibitory effect.

In a preferred mode, L1 may be an oligopeptide containing or consisting of the sequence Gly-Gly-Gly-Gly, Gly-Ser-Gly-Ser, NMeG-NMeG-NMeG-NMeG, bAla-bAla-bAla-bAla, or Gly-Gly-Glu-Gly-Gly. L1 is more preferably an oligopeptide containing or consisting of the sequence Gly-Gly-Gly-Gly or Gly-Ser-Gly-Ser, even more preferably an oligopeptide consisting of the sequence Gly-Gly-Gly-Gly.

If L1 contains an acidic amino acid residue, L1 may be an oligopeptide consisting of a sequence of two acidic amino acid residues, preferably an Asp residue, joined to the C-terminus of Gly-Gly-Gly-Gly, Gly-Ser-Gly-Ser, NMeG-NMeG-NMeG-NMeG, or bAla-bAla-bAla-bAla.

In a preferred mode, L2 may be an oligopeptide consisting of the sequence Asp-Asp-KPEG-P12P-P12P, KSG-P12P-P12P, Asp-Asp-P12P, Asp-Asp-P12P-P12P, or Asp-Asp-P12P-KPEG-P12P. L2 is more preferably an oligopeptide consisting of the sequence Asp-Asp-KPEG-P12P-P12P.

In a more preferred mode, L3 may be a Lys residue. The carboxyl group of the Lys residue in L3 may be an aminocarbonyl group.

In a more preferred mode, the linker structure may be a structure in which L 1 is an oligopeptide consisting of the sequence Gly-Gly-Gly-Gly or Gly-Ser-Gly-Ser, L2a is an oligopeptide consisting of the sequence Asp-Asp-KPEG-P12P-P12P, and L3 is a Lys residue;
a structure in which L1 is an oligopeptide consisting of the sequence Gly-Gly-Gly-Gly, L2a is an oligopeptide consisting of the sequence KSG-P12P-P12P, and L3 is a Lys residue;
a structure in which L1 is an oligopeptide consisting of the sequence Gly-Gly-Gly-Gly, L2a is an oligopeptide consisting of the sequence Asp-Asp-P12P, and L3 is a Lys residue K;
a structure in which L1 is an oligopeptide consisting of the sequence or Gly-Ser-Gly-Ser, L2a is an oligopeptide consisting of the sequence Asp-Asp-P12P-P12P, and L3 is a Lys residue; or
a structure in which L1 is an oligopeptide consisting of the sequence Gly-Gly-Gly-Gly, L2a is an oligopeptide consisting of the sequence Asp-Asp-P12P-KPEG-P12P, and L3 is a Lys residue.

In the above structure, the carboxyl group of the Lys residue in L3 may be an aminocarbonyl group.

A even more preferred mode of the cyclic peptide and linker structure is represented by any of the following formulas: 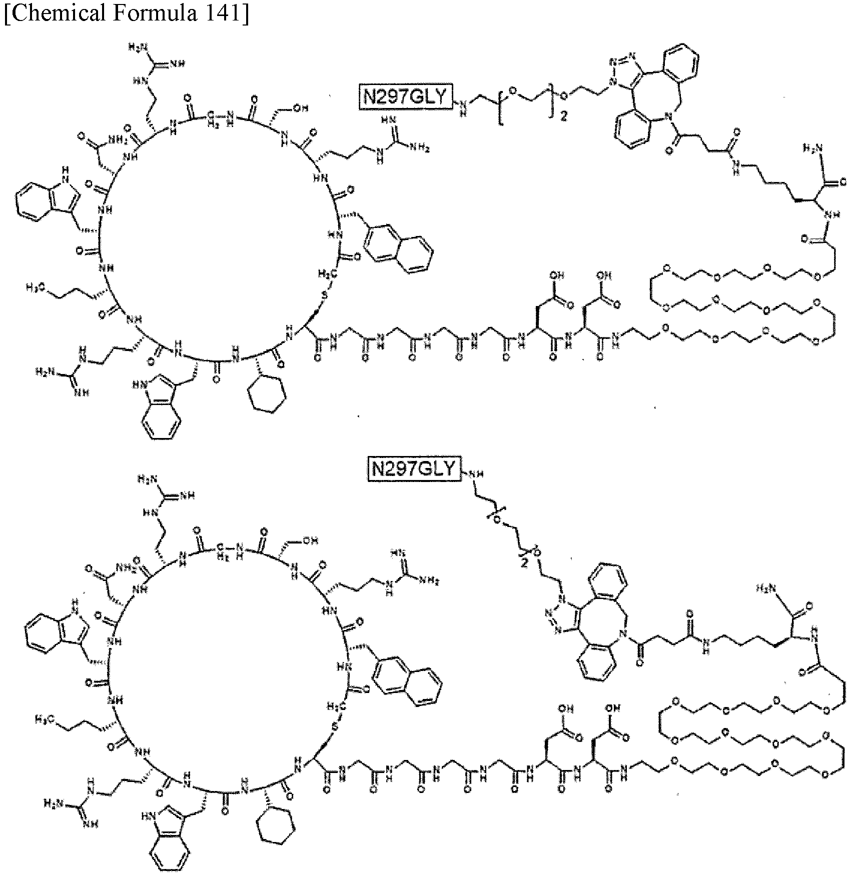 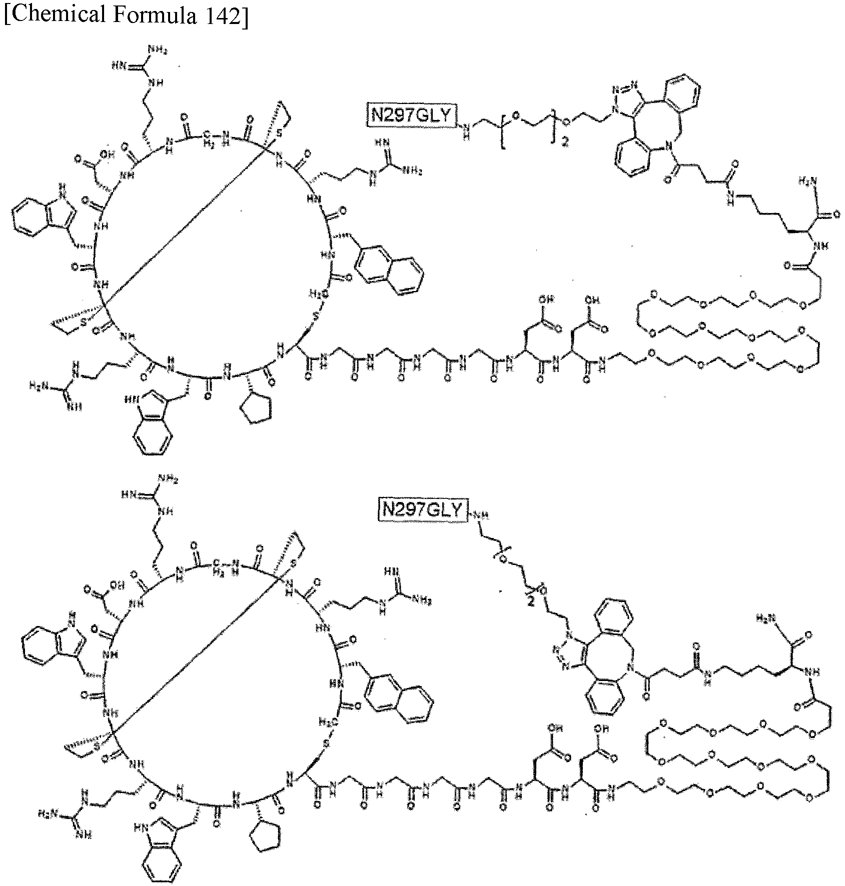 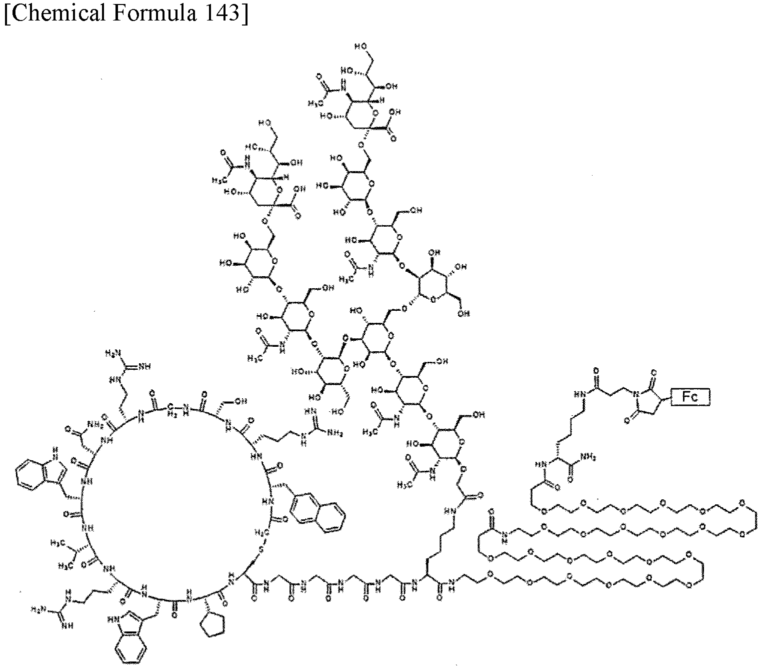 and 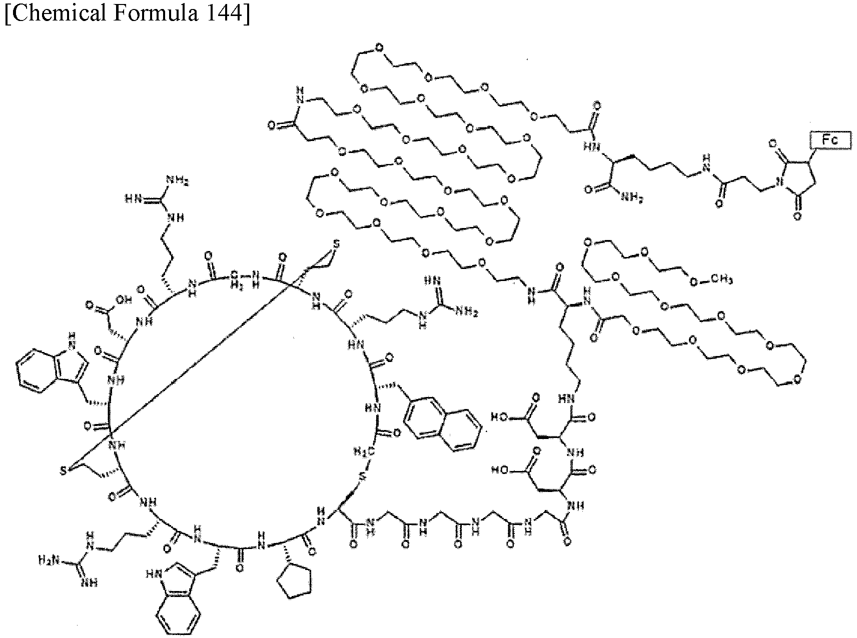 wherein
N297GLY represents binding to the non-reducing terminus of N297-linked glycan of the Fc-containing molecule; and
Fc represents binding to the thiol group of a cysteine residue of the Fc-containing molecule). The structures shown above are cyclic peptide and linker structures in Conjugate-67, Conjugate-64, Conjugate-56, and Conjugate-4, each of which is described in Examples hereinbelow.

The conjugate of the present invention can be of any combination of the cyclic peptide, carrier molecule, and linker structure described above. In a preferred mode, the cyclic peptide and linker structure may be selected from any combination of the cyclic peptide and linker structures described above, and the carrier molecule may be selected from an Fc fragment.

In a more preferred mode, the cyclic peptide is Peptide-11, Peptide-115, or Peptide-121 in Examples, and the linker structure may be
Gly-Gly-Gly-Gly-Asp-Asp-KPEG-P12P-P12P,
Gly-Gly-Gly-Gly-KSG-P12P-P12P,
Gl y -Gl y -Gl y -Gl y - Asp - Asp- P 12P,
Gly- Ser-Gly- Ser-Asp-Asp-KPEG-P 12P-P12P,
Gly-Ser-Gly-Ser-Asp-Asp-P12P-P12P, or
Gly-Gly-Gly-Gly-Asp-Asp-P12P-KPEG-P12P.

In a more preferred mode, the cyclic peptide and linker structure is any one selected from Peptide-13 to Peptide-109 in Examples, and the carrier molecule can be Fc-A or Fc-C.

In a more preferred mode, the cyclic peptide and linker structure is any one selected from Peptide-39, Peptide-75, Peptide-76, and Peptide-79 in Examples, and the carrier molecule can be Fc-A.

In yet more preferred mode, the cyclic peptide and linker structure is any one selected from Peptide-16, Peptide-22, Peptide-46, Peptide-55, and Peptide-68 in Examples, and the carrier molecule can be Fc-C.

When the conjugate of the present invention contains a basic group, the conjugate can combine with an acid to form a salt thereof, and such a salt is included in this invention. Examples of such a salt include inorganic acid salt, organic acid salt, amino acid salt, and sulfonate. Examples of the inorganic acid salt include hydrochloride, hydrobromate, sulfate, nitrate, and phosphate. Example of the organic acid salt include acetate, oxalate, malonate, fumarate, maleate, phthalate, and trifluoroacetate. Examples of the amino acid salt include glutamate, and aspartate. Examples of the sulfonate include methanesulfonate, benzenesulfonate, p-toluenesulfonate, 2,4-dimethylbenzenesulfonate, 2,4,6-trimethylbenzenesulfonate, 4-ethylbenzenesulfonate, and naphthalenesulfonate. A preferred mode of the pharmaceutically acceptable salt of the conjugate of this invention is an acetate, a hydrochloride, or a trifluoroacetate, more preferably an acetate.

When the conjugate of the present invention contains an acidic group, the conjugate can combine with a base to form a salt thereof, and such a salt is also included in this invention. Examples of such a salt include metal salt, inorganic amine salt, organic amine salt, and amino acid salt. Examples of the metal salt include: alkali metal salts such as sodium salt, potassium salt, and lithium salt; alkaline-earth metal salts such as calcium salt and magnesium salt; as well as aluminum salt, iron salt, zinc salt, copper salt, nickel salt, and cobalt salt. Examples of the inorganic amine salt include ammonium salt. Examples of the organic amine salt include morpholine salt, glucosamine salt, ethylenediamine salt, guanidine salt, diethylamine salt, triethylamine salt, dicyclohexylamine salt, diethanolamine salt, piperazine salt, and tetramethylammonium salt. Examples of the amino acid salt include lysine salt, and arginine salt.

The conjugate or pharmaceutically acceptable salt thereof of the present invention may be used in a crystalline form. Such a crystal may be formed exclusively of the conjugate or pharmaceutically acceptable salt thereof of this invention, or the conjugate or pharmaceutically acceptable salt thereof may be formed as a cocrystal or a solvate (*e.g*., hydrate). One type of the conjugate or the pharmaceutically acceptable salt thereof of this invention may be used alone, or two or more types thereof may be used in combination as appropriate.

The conjugate or pharmaceutically acceptable salt thereof of the present invention can form an isotopic compound in which one or more constitutional atom is substituted with an isotopic atom in a non-natural proportion. The isotopic atom may be radioactive or non-radioactive, and examples thereof include deuterium (²H; D), tritium (³H; T), carbon-14 (¹⁴C), and iodine-125 (¹²⁵I). Compounds labeled with a radioactive isotopic atom can be used as therapeutic or prophylactic agents for various diseases, research reagents (*e.g*., assay reagent), diagnostic agents (*e.g*., image diagnostic agent), or the like. This invention also includes such a radioactive or non-radioactive isotopic compound.

### 4. Method for Producing Conjugate

The conjugate of the present invention can be produced by appropriately binding the intermediates such as the cyclic peptide, linker molecule, and carrier molecule described above through the use of reactions known in the field of organic synthetic chemistry. The order of production thereof is not particularly limited, and various methods can be adopted by a conventional procedure according to the structures of the intermediates and the compound of interest. A functional group carried by each intermediate may be appropriately activated, inactivated, protected with a protective group, and deprotected, for example, by a conventional procedure according to the production steps.

The intermediates or final product in each reaction step is appropriately separated and purified and subjected to the next reaction or utilized as a bulk pharmaceutical or a reagent.

The conjugate of this invention can be prepared by, for example, a method including steps (1) to (6) as follows:
(1) synthesizing an oligopeptide X-A-L or Z-B-L represented by the following formula from the C-terminal side by a solid-phase synthesis method:

   Xₐₐ₁-Xₐₐ₂-Xₐₐ₃-Xₐₐ₄-Xₐₐ₅-Xₐa₆-Xₐₐ₇-Xₐₐ₈-Xₐₐ₉-Xₐₐ₁₀-Xₐₐ₁₁-A₀-L1-L2-L3 or

   Zₐₐ₁-Zₐₐ₂-Zₐₐ₃-Zₐₐ₄-Zₐₐ₅-Zₐₐ₆-Zₐₐ₇-Zₐₐ₈-Zₐₐ₉-Zₐₐ₁₀-Zₐₐ₁₁-Zₐₐ₁₂-B₀-L1-L2-L3

   wherein
   Xₐₐ₁ to Xₐₐ₁₁ are as defined in formula (I) (provided that in the structure of Xₐₐ₃ and Xₐₐ₈, thiol groups remain as they are without forming a bond);
   L1 to L3 are as defined in formula (XX) (provided that when L2 comprises a residue of a non-natural amino acid comprising a glycan in a side chain, the residue is replaced with a Lys residue);
   Zₐₐ₁ to Zₐₐ₁₂ are as defined in formula (X);
   A₀ is a residue represented by the formula 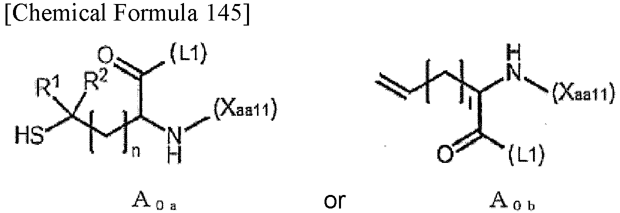
   (wherein (Xₐₐ₁₁) represents a point of attachment to Xₐₐ₁₁, (L1) represents a point of attachment to the N-terminal amino acid residue in L1, and l and n are as defined with respect to formula (I);
      B₀ is a residue represented by the formula 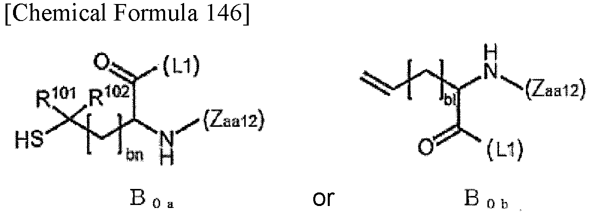
   (wherein (Zₐₐ₁₂) represents a point of attachment to Zₐₐ₁₂, (L1) represents a point of attachment to the N-terminal amino acid residue in L1, and bl and bn are as defined with respect to formula (X),
(2) cyclizing the oligopeptide synthesized in step (1) to afford a cyclic peptide by
   (a) joining the N-terminal amino group of Xₐₐ₁ to A₀ₐ to form any of the ring-forming group A₁ to A₃ (which are coupled to L1 at the position of R¹⁰) in the oligopeptide X-A-L;
   (b) joining the N-terminal amino group of Xₐₐ₁ to A_{0b} to form the ring-forming group A₄ or A₅ (which are coupled to L1 at the position of R¹⁰) in the oligopeptide X-A-L;
   (c) joining the N-terminal amino group of Zₐₐ₁ or Zₐₐ₂ to B₀ₐ to form any of the ring-forming groups B₁ to B₄ (which are coupled to L1 at the position of R¹¹⁰) in the oligopeptide Z-B-L; or
   (d) joining the N-terminal amino group of Zₐₐ₁ or Zₐₐ₂ to B_{0b} to form the ring-forming group B₅ or B₆ (which is coupled to L1 at the position of R¹¹⁰) in the oligopeptide Z-B-L,
(3) when the oligopeptide X-A-L is synthesized in step (1), allowing the thiol groups of Xₐₐ₃ and Xₐₐ₈ of the oligopeptide X-A-L cyclized in step (2) to form a bond represented by -S-S-, - S-CH₂-S-, or -S-X-S- (wherein X is as defined with respect to formula (I)) to afford a bicyclic peptide,
(4) when L2 comprises a residue of a non-natural amino acid comprising a glycan in a side chain, which is replaced with a Lys residue in the oligopeptide X-A-L or Z-B-L, introducing a glycan into the Lys residue to thereby form a residue of a non-natural amino acid comprising a glycan in a side chain,
(5) binding a maleimide group or a reactive group containing a 1,2,3-triazole ring to L3, and
(6) binding the cyclic peptide to the carrier molecule via the reactive group of step (5).

In step (1), an oligopeptide serving as an origin of the cyclic peptide and an oligopeptide serving as an origin of the linker structure are synthesized from the C-terminal side as one oligopeptide. Synthesis of the oligopeptide can be carried out according to a common solid-phase synthesis method using, for example, a 9-fluorenylmethoxycarbonyl group (Fmoc group) as an amino group-protecting group. Solid-phase synthesis can be performed using a commercially available automatic synthesizer (*e.g*., Syro II (produced by Biotage Japan), Liberty Blue (produced by CEM)). With the use of an automatic peptide synthesizer, for example, amino acids protected with an amino group-protecting group (*e.g*., Fmoc group) are coupled to a solid support (*e.g*., Rink Amide Resin AM (produced by Novaviochem), 2-chlorotrityl chloride resin (produced by Novaviochem)) in a stepwise manner starting with the C-terminal amino acid (the C-terminal amino acid is Yₐₐ₁₀). Deprotection of amino groups can be performed by a method known to skilled artisans (*e.g.*, using 20% piperidine/1-methyl-2-pyrrolidinone to remove Fmoc groups). The amino acids up to the N-terminus are coupled to yield the oligopeptide X-A-L or Z-B-L.

In the oligopeptide X-A-L or Z-B-L, where L2 contains a residue of a non-natural amino acid containing a polyoxyalkylene chain in a side chain, the residue can be coupled by a conventional procedure. For example, a Lys residue is first coupled to a synthesized portion, and a compound containing a polyoxyalkylene chain (e.g., PEG 011A as described in the Synthesis Procedure 3 in Examples) is bonded to an amino group of a portion of the Lys residue that is a side chain in the linker structure (e.g., α amino group of the Lys residue in the Synthesis Procedure 3 in Examples), whereby a residue of a non-natural amino acid containing a polyoxyalkylene chain in a side chain can be coupled.

In step (2), the oligopeptide synthesized in step (1) is cyclized by forming ring-forming groups A₁ to A₅ or B₁ to B₆ to yield a cyclic peptide. To form a ring-forming group, the amino group of the N-terminal amino acid residue of the oligopeptide is first deprotected, and if necessary, a group for forming a ring-forming group can be introduced into the amino group to form a cyclic peptide.

In step (2) (a) or (c), when any of ring-forming groups A₁ to A₃ and B₁ to B₃ are formed, for example, -COCH₂X (where X is a leaving group such as Cl) is introduced into the N-terminal amino group. Specifically, chloroacetic acid can be reacted with the N-terminal amino group to introduce a chloromethyl carbonyl group or the like into the amino group. -COCH₂X is reacted with the thiol group of A₀ₐ or B₀ₐ to yield a cyclic peptide having the ring-forming group A₁ or B₁. Further, oxidation of a sulfide group of the ring-forming group A₁ or B₁ yields a cyclic peptide having the ring-forming group A₂, A₃, B₂, or B₃.

In step (2) (b), when the ring-forming group A₄ or A₅ is formed, for example, - CO(CH₂)ₖCH=CH₂ is introduced into the N-terminal amino group. Olefin metathesis of - CO(CH₂)ₖCH=CH₂ and the side chain -(CH₂)₁CH=CH₂ of A_{0b} yields a cyclic peptide having the ring-forming group A₄. Further, reduction of the carbon-carbon double bond at the ring forming group A₄ yields a cyclic peptide having the ring forming group A₅.

In step (2) (c), when the ring-forming group B₄ is formed, a thiol group of Zₐₐ₁ or Zₐₐ₂ is reacted with a thiol group of B₀ₐ, thereby obtaining a cyclic peptide having the ring-forming group B₄. A disulfide bond can be formed by a conventional method (*e.g*., iodine oxidation method).

In step (2) (d), when the ring-forming group B₅ or B₆ is formed, for example, - CO(CH₂)_{bk}CH=CH₂ is introduced into the N-terminal amino group. Olefin metathesis of - CO(CH₂)_{bk}CH=CH₂ and the side chain -(CH₂)_{bl}CH=CH₂ of B_{0b} yields a cyclic peptide having the ring-forming group B₅. Further, reduction of the carbon-carbon double bond at the ring forming group B₅ yields a cyclic peptide having the ring forming group B₆.

Then, the resulting peptide is cleaved from the peptide resin by a conventional procedure (*e.g*., conditions using trifluoroacetic acid/ethanedithiol/triisopropylsilane/water to cleave from Rink Amide Resin AM). After a crude peptide is recovered by ether precipitation, the peptide of interest can be purified by a conventional procedure (*e.g*., reverse-phase high-performance liquid chromatography).

Step (3) is to form a bicyclic peptide when the oligopeptide X-A-L is synthesized in step (1). The cyclic peptides obtained in step (2) can be subjected to a conventional method (*e.g.,* iodine oxidation method) to form a bond such as a disulfide bond between Xₐₐ₃ and Xₐₐ₈, thereby obtaining a bicyclic peptide. After a crude peptide is recovered by ether precipitation, the peptide of interest can be purified by a conventional procedure (*e.g.,* reverse-phase high-performance liquid chromatography).

Step (4) is to form a residue of a non-natural amino acid containing a glycan in a side chain of the oligopeptide X-A-L or Z-B-L when the residue of the non-natural amino acid containing the glycan in a side chain of L2 is replaced with a Lys residue. The conversion of the Lys residue can be carried out by binding a compound containing a glycan (e.g., SGA as described in Synthesis Procedure 5 in Examples) to an ε amino group of the Lys residue by a conventional procedure. After the reaction, the peptide of interest can be purified by a conventional procedure (*e.g*., reverse-phase high-performance liquid chromatography).

In step (5), a reactive group capable of forming a maleimide group or a 1,2,3-triazole ring for binding the oligopeptide X-A-L or Z-B-L to the carrier molecule is bonded to L3. Attachment of the maleimide group to the oligopeptide can be carried out by a conventional procedure using a reagent such as N-succinimidyl 3-maleimidopropionate. To form the 1,2,3-triazole ring, a cycloaddition reaction such as CuAAC and SPAAC can be used. Depending on the reaction to be used, a reactive group including an acetylene group or DBCO is selected as a reactive group capable of forming a 1,2,3-triazole ring. In a preferred mode, SPAAC is utilized to form a 1,2,3-triazole ring, and in this case, the reactive group including DBCO may be selected as a reactive group capable of forming a 1,2,3-triazole ring. Attachment of the reaction group including DBCO to the oligopeptide can be carried out by a conventional procedure, for example, using a reagent such as dibenzocyclooctin-N-hydroxysuccinimidyl ester.

The carrier molecules in step (6) are prepared according to the method described in III above under " 1. Carrier Molecule". Depending on a reactive group attached to the oligopeptide in step (5), the carrier molecule can be prepared to have an appropriate reactive group. For example, in the case of coupling with an oligopeptide having a maleimide group, a carrier molecule having a thiol group may be prepared. In the case of coupling with an oligopeptide having a reactive group capable of forming a 1,2,3-triazole ring, for example, a reactive group containing an azide group may be introduced at the 2-position of the sialic acid at the non-reducing terminus of the glycan-remodeled Fc-containing molecule. Molecules having a reactive group such as an azide group at the non-reducing terminus of N297-linked glycans may be synthesized in the glycan remodeling step of Fc-containing molecules. For example, glycan remodeling can be performed using a glycan donor molecule in which an azide group is bonded to the 2-position of sialic acid at the non-reducing terminus.

A known method such as the method described in III above under "3. Linker Structure" can be applied to a bond of the cyclic peptide to the carrier molecule, which can be carried out, for example, according to Synthesis Procedures 8 to 10 in Examples.

The conjugate or pharmaceutically acceptable salt thereof of the present invention has TSP1 inhibitory activity and has an IC₅₀ of 500 nM or less, preferably 200 nM or less, as measured by a cell adhesion inhibition assay using human TSP1 and vascular endothelial cells as described hereinbelow in the Examples section. Therefore, the conjugate or pharmaceutically acceptable salt thereof of the present invention has an ability to block the adhesion of vascular endothelial cells to TSP1, and are thus useful as blood flow improving agents. When the carrier molecule is an Fc-containing molecule, and two Fc-containing molecules are crosslinked to form one conjugated molecule (*i.e*., dimer), the molar concentration in the calculation of IC₅₀ is determined using the molecular weight of the dimer. When the carrier molecule is an Fc-containing molecule, the Fc-containing molecule being not crosslinked, and the conjugate remains monomeric (*e.g*., bonded to a cyclic peptide via a thiol group generated by reduction of a disulfide bond at the hinge part of the Fc-containing molecule), the molar concentration in the calculation of IC₅₀ is determined using twice the molecular weight of the monomer. In addition, when the carrier molecule is an Fc-containing molecule that is bonded to a cyclic peptide via a thiol group generated by reducing the disulfide bond at the hinge part, various glycans can bind to the Fc-containing molecule. In this case, the molar concentration in the calculation of IC₅₀ is calculated using the molecular weight of the deglycosylated product.

### 5. Pharmaceutical Composition

The present invention further provides a pharmaceutical composition comprising, as an active component, the conjugate or pharmaceutically acceptable salt thereof of the present invention (hereinafter referred to as "the pharmaceutical composition II of the present(this) invention"). The pharmaceutical composition II of this invention has excellent TSP 1 inhibitory activity and an IC₅₀ of 500 nM or less, preferably 200 nM or less, as measured by a cell adhesion inhibition assay using human TSP 1 and vascular endothelial cells as described hereinbelow in the Examples section. Therefore, the pharmaceutical composition II of this invention is useful for the treatment or prophylaxis of diseases or symptoms that can be treated or prevented by inhibiting a function of TSP1 (*e.g*., various diseases, including diseases that may be caused by angiogenesis inhibition, diseases that may be caused by increased thrombogenesis, inflammatory diseases, diseases that may be caused by deterioration of renal cellular function, diseases that may be caused by suppression of vasorelaxation, ischemic diseases, and cancerous diseases; and various symptoms, including vaso-occlusive crisis associated with sickle cell disease, angiogenesis inhibition, tissue necrosis, insulin resistance, and common wounds). Specific examples of the diseases or symptoms are as described for the pharmaceutical composition I of the present invention.

The pharmaceutical composition II of the present invention has excellent TSP1 inhibitory activity and may have a blood flow improving effect, and is thus useful for the treatment or prophylaxis of diseases that can be treated or prevented by improving blood flow. Examples of such diseases include chronic limb threatening ischemia, critical limb ischemia, peripheral vascular disorder, myocardial infarction, angina, kidney injury, and ischemia-reperfusion injury. Examples of the chronic limb threatening ischemia include diabetic foot, and examples of the diabetic foot include diabetic foot ulcer.

In a preferred mode, the pharmaceutical composition II of this invention is useful for the treatment or prophylaxis of critical limb ischemia, peripheral arterial disease, or chronic limb threatening ischemia, and is, for example, useful for the acceleration of wound healing in critical limb ischemia patients and for the improvement of prognosis after endovascular treatment for critical limb ischemia.

It is known that the expression of TSP1 increases in the lower-limb skeletal muscles of critical limb ischemia patients. Although not wishing to be bound by any theory, it is believed that the pharmaceutical composition II of the present invention blocks the adhesion of vascular endothelial cells to TSP1, and thereby can promote angiogenesis or vasodilation inhibited by TSP1. By virtue of this function, the pharmaceutical composition II of this invention can accelerate wound healing in critical limb ischemia patients and improve prognosis (*e.g*., prevent restenosis) when combined with endovascular treatment with a catheter or the like.

The pharmaceutical composition II of the present invention can be formulated into a pharmaceutical preparation by mixing the cyclic peptide or pharmaceutically acceptable salt thereof of this invention with any appropriate pharmaceutically acceptable additives. For example, the pharmaceutical composition II of this invention can be administered as oral preparations such as tablets, capsules and granules, or as parenteral preparations such as injectables and percutaneous absorption agents.

Such pharmaceutical preparations are prepared by a well-known method using different additives, including excipient, binder, disintegrant, lubricant, emulsifier, stabilizer, diluent, injectable solvent, solubilizer, suspending agent, isotonizing agent, buffer, analgesic, antiseptic, and antioxidant. Specific examples of the additives are as described for the pharmaceutical composition I of the present invention.

The subject to be administered the pharmaceutical composition II of the present invention is, for example, a mammalian animal, preferably a human.

The administration route of the pharmaceutical composition II of the present invention can be either of oral and parenteral administrations, and a suitable administration route can be selected depending on the disease to be treated. Further, the administration route of said inventive pharmaceutical composition can be either of systemic and topical administrations. Examples of parenteral administrations include intravenous administration, intraarterial administration, intramuscular administration, intracutaneous administration, subcutaneous administration, intraperitoneal administration, percutaneous administration, intraosseous administration, and intraarticular administration. A preferred mode of the administration route of said inventive pharmaceutical composition II is intravenous administration, subcutaneous administration, or percutaneous administration.

The conjugate or pharmaceutically acceptable salt thereof of the present invention is administered to a subject in a therapeutically or prophylactically effective amount. The term "therapeutically or prophylactically effective amount" means an amount required for an active agent to exhibit a therapeutic or prophylactic effect in consideration of particular disease, dosage form, and administration route, and is determined, as appropriate, depending on the species of a subject, type of a disease, symptom, sex, age, pre-existing condition, and other elements.

The dose of the cyclic peptide I or II or pharmaceutically acceptable salt thereof of the present invention is determined, as appropriate, depending on the species of a subject, type of a disease, symptom, sex, age, pre-existing condition, and other elements. Human adults can take medication with said inventive cyclic peptide I or II or pharmaceutically acceptable salt thereof in a dose of generally 0.1 to 1000 mg/kg, preferably 0.1 to 10 mg/kg, once every 1 to 7 days, or two or three or more times daily.

The pharmaceutical composition II of the present invention may be combined with at least one known therapeutic agent or therapy. For example, during critical limb ischemia treatment, the pharmaceutical composition II of this invention can be administered before, after or simultaneously with endovascular treatment with a catheter or the like.

The conjugate or pharmaceutically acceptable salt thereof of this invention can be delivered by means of a drug-eluting stent (DES). The drug-eluting stent refers to a stent that has an active drug carried on its surface so that it can gradually elute the active drug after being placed into a blood vessel.

Furthermore, the present invention is directed to a method for the treatment or prophylaxis of a disease or symptom, the method comprising administering a therapeutically or prophylactically effective amount of the conjugate or pharmacologically acceptable salt thereof of this invention to a subject in need thereof.

As referred to in the present invention, the term "therapeutically or prophylactically effective amount" means an amount required for an active agent to exhibit a therapeutic or prophylactic effect in consideration of particular disease or symptom, dosage form, and administration route, and is determined, as appropriate, depending on the species of a subject, type of a disease or symptom, symptom, sex, age, pre-existing condition, and other elements.

As referred to in the present invention, the "subject" is, for example, a mammalian animal, preferably a human.

As referred to in the present invention, the term "administration" includes oral and parenteral administrations, and can be either of systemic and topical administrations. Examples of parenteral administrations include intravenous administration, intraarterial administration, intramuscular administration, intracutaneous administration, subcutaneous administration, intraperitoneal administration, percutaneous administration, intraosseous administration, and intraarticular administration. A preferred mode of the "administration" as referred to in this invention is intravenous administration, subcutaneous administration, or percutaneous administration.

Examples of the "disease(s) or symptom(s)" as referred to in the present invention include diseases or symptoms that can be treated or prevented by inhibiting a function of TSP1. Specific examples of the diseases or symptoms are as described for the pharmaceutical composition I of the present invention. In a preferred mode, the method for the treatment or prophylaxis of this invention is useful for the treatment or prophylaxis of critical limb ischemia, peripheral arterial disease, or chronic limb threatening ischemia, and is, for example, useful for the acceleration of wound healing in critical limb ischemia patients and for the improvement of prognosis after endovascular treatment for critical limb ischemia.

The conjugate of the present invention may have an improved PK-PD profile compared to the cyclic peptide I or II of the present invention. Therefore, according to the method for the treatment or prophylaxis with the pharmaceutical composition II of this invention and the conjugate or pharmaceutically acceptable salt thereof of this invention allows treatment or prophylaxis of diseases or symptoms that can be treated or prevented by inhibiting a function of TSP1 at a lower frequency of administration.

Hereunder, the present invention will be described in more detail with reference to working and test examples, but the scope of this invention is not limited to these examples.

### EXAMPLES

### [Synthesis Example]

### 1. Synthesis of Peptide-1 to Peptide-12 and Synthesis of Peptide-115, Peptide-119, and Peptide-125 to Peptide-139

As shown in the following table, Peptide-1 was synthesized according to any of Synthesis Procedure 1 (yield: 19%) and Synthesis Procedure 4 (yield: 16%) as shown hereinbelow. The yield indicates result for a representative compound of each synthesis procedure.

Peptide-2 to Peptide-12 were also synthesized using the same procedure.

As shown in the following table, Peptide-115 and Peptide-119 were synthesized according to Synthesis Procedure 1 as shown hereinbelow.

As shown in the following table, Peptide-125 was synthesized according to any of Synthesis Procedure 1, Synthesis Procedure 11, and Synthesis Procedure 14 as shown hereinbelow.

As shown in the following table, Peptide-126, Peptide-128, Peptide-129, Peptide-134, Peptide-135, and Peptide-138 were synthesized according to any of Synthesis Procedure 1, Synthesis Procedure 11, and Synthesis Procedure 12 as shown hereinbelow.

As shown in the following table, Peptide-127, Peptide-130 to Peptide-133, Peptide-136, Peptide-137, and Peptide-139 were synthesized according to any of Synthesis Procedure 1, Synthesis Procedure 11, and Synthesis Procedure 13 as shown hereinbelow.

**[Table 1]**

| **Peptide-** | MW | **procedure 1** | **procedure 2** | **procedure 3** | **LC-MS condition** | MS measured value | MS valence | **Rt (min)** |
|---|---|---|---|---|---|---|---|---|
| **1** | **1713.02** | Synthesis Procedure 1 | Synthesis Procedure 4 | | Condition 1 | **856.88** | **2** | **1.51** |
| **2** | **1699.00** | Synthesis Procedure 1 | Synthesis Procedure 4 | | Condition 1 | **849.87** | **2** | **1.52** |
| **3** | **1689.98** | Synthesis Procedure 1 | Synthesis Procedure 4 | | Condition 1 | **845.35** | **2** | **2.09** |
| **4** | **1689.98** | Synthesis Procedure 1 | Synthesis Procedure 4 | | Condition 1 | **845.35** | **2** | **1.72** |
| **5** | **1705.00** | Synthesis Procedure 1 | Synthesis Procedure 4 | | Condition 1 | **852.86** | **2** | **1.50** |
| **6** | **1643.96** | Synthesis Procedure 1 | Synthesis Procedure 4 | | Condition 1 | **822.36** | **2** | **1.55** |
| **7** | **1669.99** | Synthesis Procedure 1 | Synthesis Procedure 4 | | Condition 1 | **835.37** | **2** | **1.66** |
| **8** | **1684.02** | Synthesis Procedure 1 | Synthesis Procedure 4 | | Condition 1 | **842:38** | **2** | **1.57** |
| **9** | **1714.01** | Synthesis Procedure 1 | Synthesis Procedure 4 | | Condition 1 | **857.37** | **2** | **1.53** |
| **10** | **1727.05** | Synthesis Procedure 1 | Synthesis Procedure 4 | | Condition 1 | **863.87** | **2** | **1.52** |
| **11** | **1728.03** | Synthesis Procedure 1 | Synthesis Procedure 4 | | Condition 1 | **864.38** | **2** | **1.53** |
| **12** | **1701.01** | Synthesis Procedure 1 | Synthesis Procedure 4 | | Condition 1 | **850.88** | **2** | **2.07** |
| **125** | **1784.10** | Synthesis Procedure 1 | Synthesis Procedure 11 | Synthesis Procedure 14 | Condition 1 | **892.39** | **2** | **1.78** |
| **126** | **1714.01** | Synthesis Procedure 1 | Synthesis Procedure 11 | Synthesis Procedure 12 | Condition 1 | **857.37** | **2** | **1.77** |
| **127** | **1742.06** | Synthesis Procedure 1 | Synthesis Procedure 11 | Synthesis Procedure 13 | Condition 1 | **871.39** | **2** | **1.78** |
| **128** | **1699.98** | Synthesis Procedure 1 | Synthesis Procedure 11 | Synthesis Procedure 12 | Condition 1 | **850.36** | **2** | **1.70** |
| **129** | **1714.01** | Synthesis Procedure 1 | Synthesis Procedure 11 | Synthesis Procedure 12 | Condition 1 | **857.37** | **2** | **1.80** |
| **130** | **1714.01** | Synthesis Procedure 1 | Synthesis Procedure 11 | Synthesis Procedure 13 | Condition 1 | **857.37** | **2** | **1.74** |
| **131** | **1728.03** | Synthesis Procedure 1 | Synthesis Procedure 11 | Synthesis Procedure 13 | Condition 1 | **864.38** | **2** | **1.75** |
| **132** | **1728.03** | Synthesis Procedure 1 | Synthesis Procedure 11 | Synthesis Procedure 13 | Condition 1 | **864.38** | **2** | **1.72** |
| **133** | **1756.09** | Synthesis Procedure 1 | Synthesis Procedure 11 | Synthesis Procedure 13 | Condition 1 | **878.39** | **2** | **1.81** |
| **134** | **1742.06** | Synthesis Procedure 1 | Synthesis Procedure 11 | Synthesis Procedure 12 | Condition 1 | **871.39** | **2** | **1.85** |
| **135** | **1742.06** | Synthesis Procedure 1 | Synthesis Procedure 11 | Synthesis Procedure 12 | Condition 1 | **871.39** | **2** | **1.82** |
| **136** | **1756.09** | Synthesis Procedure 1 | Synthesis Procedure 11 | Synthesis Procedure 13 | Condition 1 | **878.39** | **2** | **1.83** |
| **137** | **1742.06** | Synthesis Procedure 1 | Synthesis Procedure 11 | Synthesis Procedure 13 | Condition 1 | **871.39** | **2** | **1.80** |
| **138** | **1728.03** | Synthesis Procedure 1 | Synthesis Procedure 11 | Synthesis Procedure 12 | Condition 1 | **864.38** | **2** | **1.77** |
| **139** | **1742.06** | Synthesis Procedure 1 | Synthesis Procedure 11 | Synthesis Procedure 13 | Condition 1 | **871.39** | **2** | **1.81** |
| **115** | **1666.91** | Synthesis Procedure 1 | | | Condition 1 | **1666.91** | **1** | **1.47** |
| **119** | **1667.89** | Synthesis Procedure 1 | | | Condition 1 | **834.40** | **2** | **1.48** |

The residues constituting the bicyclic peptides Peptide-1 to Peptide-12 are shown in the following table. It should be noted that the cysteine residue at position 12 has been converted to a ring-forming group in the cyclic peptide. The HCY residues at positions 3 and 8 have disulfide bonds formed therein.

The residues constituting Peptide-115 and Peptide-119 are shown in the following table. It should be noted that the cysteine residue at position 12 has been converted to a ring-forming group in the cyclic peptide.

The residues constituting the bicyclic peptide Peptide-125 are shown in the following table. It should be noted that the cysteine residue at position 12 has been converted to a ring-forming group in the cyclic peptide. The HCY residues at positions 3 and 8 also have thiol groups forming a bond represented by -S-CH₂-CO-CH₂-S- (also referred to as "acetone bridge").

The residues constituting the bicyclic peptides Peptide-126, Peptide-128, Peptide-129, Peptide-134, Peptide-135, and Peptide-138 are shown in the following table. It should be noted that the cysteine residue at position 12 has been converted to a ring-forming group in the cyclic peptide. The cysteine residues, HCY residues, or Pen residues at positions 3 and 8 also have disulfide bonds formed therein.

The residues constituting the bicyclic peptides Peptide-127, Peptide-130 to Peptide-133, Peptide-136, Peptide-137, and Peptide-139 are shown in the following table. It should be noted that the cysteine residue at position 12 has been converted to a ring-forming group in the cyclic peptide. The cysteine residues, HCY residues, or Pen residues at positions 3 and 8 also have thiol groups forming a bond represented by -S-CH₂-S- (also referred to as "methylene bridge").

**[Table 2-1]**

| Peptide- | Ring-forming group | 1 | 2 | 3 | 4 | 5 | 6 | 7 | 8 | 9 | 10 | 11 | 12 | IC50 (nM) |
|---|---|---|---|---|---|---|---|---|---|---|---|---|---|---|
| 1 | A₁ₐ₁ | 2NAL | R | HCY | G | AGB | N | W | HCY | R | W | CPTG | C | 12.6 |
| 2 | A₁ₐ₁ | 2NAL | AGB | HCY | G | AGB | N | W | HCY | R | W | CPTG | C | 20.0 |
| 3 | A₁ₐ₁ | 2NAL | PHG | HCY | G | AGB | N | W | HCY | R | W | CPTG | C | 29.9 |
| 4 | A₁ₐ₁ | 2NAL | R | HCY | 6 | AGB | N | W | HCY | PHG | W | CPTG | C | 33.8 |
| 5 | A₁ₐ₁ | 2NAL | R | HCY | G | AGB | N | W | HCY | 4PAL | W | CPTG | C | 23.6 |
| 6 | A₁ₐ₁ | 2NAL | R | HCY | G | R | N | W | HCY | V | W | V | C | 32.1 |
| 7 | A₁ₐ₁ | 2NAL | R | HCY | G | R | N | W | HCY | V | W | CPTG | C | 31.1 |
| 8 | A₁ₐ₁ | 2NAL | TLE | HCY | G | R | N | W | HCY | R | W | CPTG | C | 12.6 |
| 9 | A₁ₐ₁ | 2NAL | R | HCY | G | AGB | D | W | HCY | R | W | CPTG | C | 22.4 |
| 10 | A₁ₐ₁ | 2NAL | R | HCY | G | R | N | W | HCY | R | W | CPTG | C | 14.3 |
| 11 | A₁ₐ₁ | 2NAL | R | HCY | G | R | D | W | HCY | R | W | CPTG | C | 26.0 |
| 12 | A₁ₐ₁ | 2NAL | R | HCY | G | R | N | W | HCY | R | W | V | C | 18.6 |
| 125 | A₁ₐ₁ | 2NAL | R | HCY | G | R | D | W | HCY | R | W | CPTG | C | 305.2 |
| 126 | A₁ₐ₁ | 2NAL | R | C | G | R | D | W | HCY | R | W | CPTG | C | 10.8 |
| 127 | A₁ₐ₁ | 2NAL | R | HCY | G | R | D | W | HCY | R | W | CPTG | C | 11.6 |
| 128 | A₁ₐ₁ | 2NAL | R | C | G | R | D | W | C | R | W | CPTG | C | 8.4 |
| 129 | A₁ₐ₁ | 2NAL | R | HCY | G | R | D | W | C | R | W | CPTG | C | 43.7 |
| 130 | A₁ₐ₁ | 2NAL | R | C | G | R | D | W | C | R | W | CPTG | C | 7.7 |
| 131 | A₁ₐ₁ | 2NAL | R | C | G | R | D | W | HCY | R | W | CPTG | C | 14.2 |
| 132 | A₁ₐ₁ | 2NAL | R | HCY | G | R | D | W | C | R | W | CPTG | C | 7.0 |
| 133 | A₁ₐ₁ | 2NAL | R | Pen | G | R | D | W | HCY | R | W | CPTG | C | 125.3 |
| 134 | A₁ₐ₁ | 2NAL | R | HCY | 6 | R | D | W | Pen | R | W | CPTG | C | 102.0 |
| 135 | A₁ₐ₁ | 2 NAL | R | Pen | G | R | D | W | HCY | R | W | CPTG | C | 207.7 |
| 136 | A₁ₐ₁ | 2NAL | R | HCY | G | R | D | W | Pen | R | W | CPTG | C | 161.6 |
| 137 | A₁ₐ₁ | 2NAL | R | Pen | G | R | D | W | C | R | W | CPTG | C | 80.6 |
| 138 | A₁ₐ₁ | 2NAL | R | C | G | R | D | W | Pen | R | W | CPTG | C | 92.5 |
| 139 | A₁ₐ₁ | 2NAL | R | C | G | R | D | W | Pen | R | W | CPTG | C | 30.9 |
| 115 | B₁ₐ₁ | 2NAL | R | S | G | AGB | N | W | V | R | W | CPTG | C | 17.5 |
| 119 | B₁ₐ₁ | 2NAL | R | S | G | AGB | D | W | V | R | W | CPTG | C | 22.0 |

### 2. Synthesis of Peptide-13 to Peptide-24

As shown in the following table, Peptide-13 was synthesized according to any of Synthesis Procedure 1 (yield: 6%), Synthesis Procedure 4 (yield: 44%), and Synthesis Procedure 6 (yield: 50%) as shown hereinbelow.

Peptide-14 was also synthesized using the same procedure.

As shown in the following table, Peptide-15 was synthesized according to any of Synthesis Procedure 2 (yield: 3%), Synthesis Procedure 4 (yield: 84%), and Synthesis Procedure 6 (yield: 60%) as shown hereinbelow.

As shown in the following table, Peptide-16 was synthesized according to any of Synthesis Procedure 3 (yield: 13%), Synthesis Procedure 4 (yield: 28%), and Synthesis Procedure 6 (yield: 53%) as shown hereinbelow.

Peptide-17 to Peptide-24 were also synthesized using the same procedure.

**[Table 3]**

| **Peptide-** | MW | **procedure 1** | **procedure 2** | **procedure 3** | **LC-MS condition** | MS measured value | MS valence | **Rt (min)** |
|---|---|---|---|---|---|---|---|---|
| **13** | **3165.49** | Synthesis Procedure 1 | Synthesis Procedure 4 | Synthesis Procedure 6 | Condition 1 | **1055.46** | **3** | **1.45** |
| **14** | **3051.39** | Synthesis Procedure 1 | Synthesis Procedure 4 | Synthesis Procedure 6 | Condition 1 | **1017.45** | **3** | **1.51** |
| **15** | **2937.29** | Synthesis Procedure 2 | Synthesis Procedure 4 | Synthesis Procedure 6 | Condition 1 | **1468.65** | **3** | **1.49** |
| **16** | **4305.88** | Synthesis Procedure 3 | Synthesis Procedure 4 | Synthesis Procedure 6 | Condition 1 | **1435.37** | **3** | **2.10** |
| **17** | **4365.94** | Synthesis Procedure 3 | Synthesis Procedure 4 | Synthesis Procedure 6 | Condition 1 | **1455.38** | **3** | **1.57** |
| **18** | **4305.88** | Synthesis Procedure 3 | Synthesis Procedure 4 | Synthesis Procedure 6 | Condition 1 | **1435.37** | **3** | **1.59** |
| **19** | **4305.88** | Synthesis Procedure 3 | Synthesis Procedure 4 | Synthesis Procedure 6 | Condition 1 | **1435.37** | **3** | **2.10** |
| **20** | **4361.99** | Synthesis Procedure 3 | Synthesis Procedure 4 | Synthesis Procedure 6 | Condition 1 | **1454.06** | **3** | **1.92** |
| **21** | **4361.99** | Synthesis Procedure 3 | Synthesis Procedure 4 | Synthesis Procedure 6 | Condition 1 | **1454.06** | **3** | **1.65** |
| **22** | **4365.94** | Synthesis Procedure 3 | Synthesis Procedure 4 | Synthesis Procedure 6 | Condition 1 | **1455.37** | **3** | **1.58** |
| **23** | **4365.94** | Synthesis Procedure 3 | Synthesis Procedure 4 | Synthesis Procedure 6 | Condition 1 | **1455.37** | **3** | **1.57** |
| **24** | **4361.99** | Synthesis Procedure 3 | Synthesis Procedure 4 | Synthesis Procedure 6 | Condition 1 | **1454.05** | **3** | **1.63** |

The residues constituting the bicyclic peptides Peptide-13 to Peptide-24, which have a linker structure, are shown in the following table. It should be noted that the cysteine residue at position 12 has been converted to a ring-forming group in the cyclic peptide. The ring-forming group forms an amide bond with the linker structure at an aminocarbonyl group, and NH₂ in the aminocarbonyl group has been substituted with NH of the N-terminal amino group of the N-terminal amino acid residue in the linker structure. The HCY residues at positions 3 and 8 have disulfide bonds formed therein. The C-terminal lysine residue has been maleimidated by Synthesis Procedure 6.

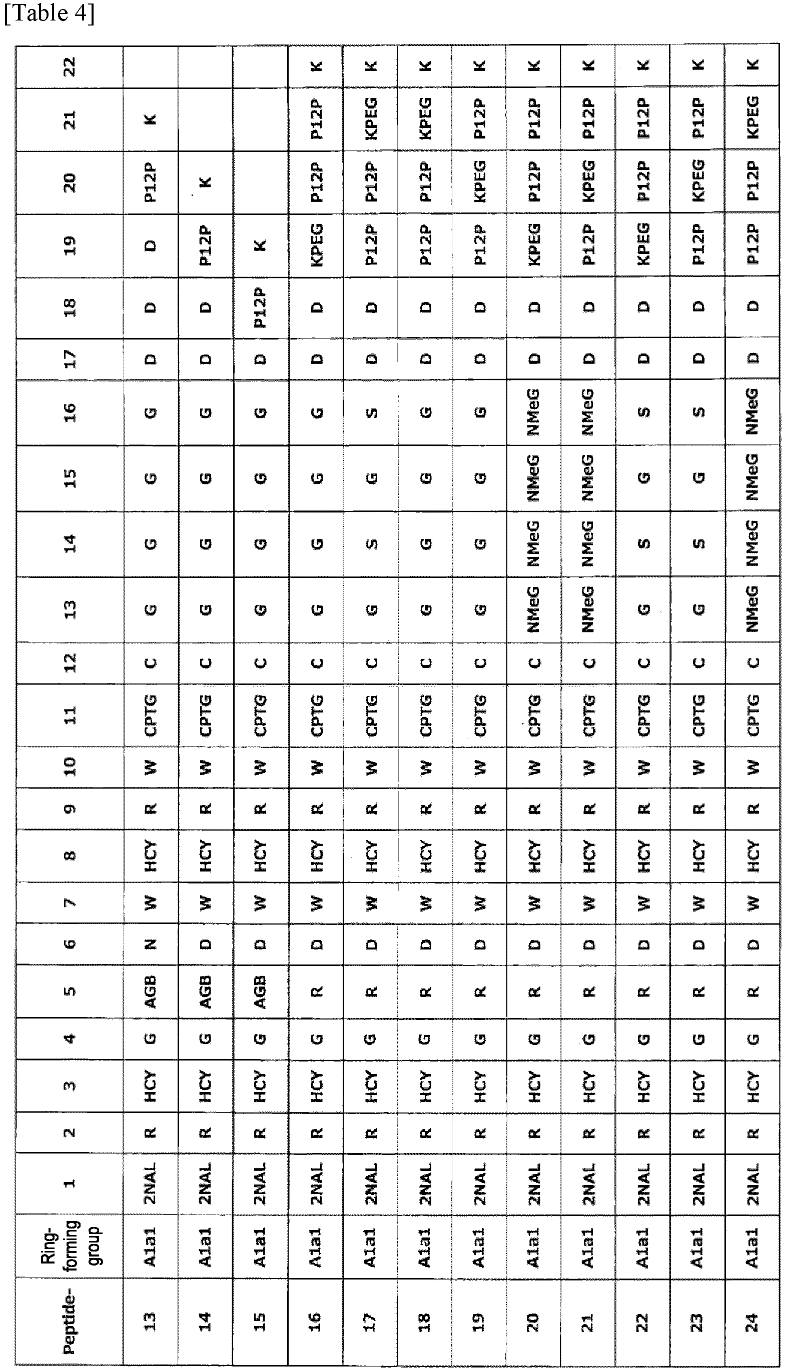

### 3. Synthesis of Peptide-25 to Peptide-72

As shown in the following table, Peptide-25 was synthesized according to any of Synthesis Procedure 1 (yield: 13%) and Synthesis Procedure 6 (yield: 71%) as shown hereinbelow.

Peptide-26 to Peptide-35, Peptide-37 to Peptide-50, and Peptide-57 to Peptide-59 were also synthesized using the same procedure.

As shown in the following table, Peptide-36 was synthesized according to any of Synthesis Procedure 2 (yield: 6%) and Synthesis Procedure 6 (yield: 71%) as shown hereinbelow.

As shown in the following table, Peptide-51 was synthesized according to any of Synthesis Procedure 3 (yield: 9%) and Synthesis Procedure 6 (yield: 32%) as shown hereinbelow. Fmoc-Lys(Boc)-OH was used as the C-terminal Lys, and Fmoc-Lys(ivDde)-OH was used as the other Lys (converted to KPEG).

Peptide-52 to Peptide-56 and Peptide-70 to Peptide-72 were also synthesized using the same procedure.

As shown in the following table, Peptide-60 was synthesized according to any of Synthesis Procedure 1 (yield: 15%), Synthesis Procedure 5 (yield: 23%), and Synthesis Procedure 6 (yield: 50%) as shown hereinbelow. Fmoc-Lys(ivDde)-OH was used as the C-terminal Lys, and Fmoc-Lys(Boc)-OH was used as the other Lys (converted to KSG).

Peptide-61 to Peptide-69 were also synthesized using the same procedure.

**[Table 5-1]**

| **Peptide-** | MW | **procedure 1** | **procedure 2** | **procedure 3** | **LC-MS condition** | MS measured value | MS valence | **Rt (min)** |
|---|---|---|---|---|---|---|---|---|
| **25** | **2705.05** | Synthesis Procedure 1 | Synthesis Procedure 6 | | Condition 1 | **1353.18** | **2** | **1.48** |
| **26** | **2762.11** | Synthesis Procedure 1 | Synthesis Procedure 6 | | Condition 1 | **1381.69** | **2** | **1.42** |
| **27** | **3004.29** | Synthesis Procedure 1 | Synthesis Procedure 6 | | Condition 1 | **1001.81** | **3** | **1.49** |
| **28** | **2774.11** | Synthesis Procedure 1 | Synthesis Procedure 6 | | Condition 1 | **925.13** | **3** | **1.47** |
| **29** | **3004.29** | Synthesis Procedure 1 | Synthesis Procedure 6 | | Condition 1 | **1001.81** | **3** | **1.47** |
| **30** | **3060.39** | Synthesis Procedure 1 | Synthesis Procedure 6 | | Condition 1 | **1020.49** | **3** | **1.46** |
| **31** | **3004.29** | Synthesis Procedure 1 | Synthesis Procedure 6 | | Condition 1 | **1001.81** | **3** | **1.46** |
| **32** | **3060.39** | Synthesis Procedure 1 | Synthesis Procedure 6 | | Condition 1 | **1020.48** | **3** | **1.45** |
| **33** | **2759.14** | Synthesis Procedure 1 | Synthesis Procedure 6 | | Condition 1 | **1379.68** | **2** | **1.69** |
| **34** | **2989.31** | Synthesis Procedure 1 | Synthesis Procedure 6 | | Condition 1 | **1494.72** | **2** | **1.65** |
| **35** | **2890.19** | Synthesis Procedure 1 | Synthesis Procedure 6 | | Condition 1 | **963.80** | **3** | **1.48** |
| **36** | **2776.08** | Synthesis Procedure 2 | Synthesis Procedure 6 | | Condition 1 | **925.79** | **3** | **1.49** |
| **37** | **2745.11182** | Synthesis Procedure 1 | Synthesis Procedure 6 | | Condition 1 | **915.4669** | **3** | **1.703** |
| **38** | **2975.28662** | Synthesis Procedure 1 | Synthesis Procedure 6 | | Condition 1 | **992.1473** | **3** | **1.661** |
| **39** | **3032.34131** | Synthesis Procedure 1 | Synthesis Procedure 6 | | Condition 1 | **1011.1617** | **3** | **1.565** |
| **40** | **2904.21216** | Synthesis Procedure 1 | Synthesis Procedure 6 | | Condition 1 | **968.4718** | **3** | **1.533** |
| **41** | **3018.3147** | Synthesis Procedure 1 | Synthesis Procedure 6 | | Condition 1 | **1006.474** | **3** | **2.081** |
| **42** | **2904.21216** | Synthesis Procedure 1 | Synthesis Procedure 6 | | Condition 1 | **968.4725** | **3** | **1.532** |
| **43** | **3560.972** | Synthesis Procedure 1 | Synthesis Procedure 6 | | Condition 1 | **1187.2642** | **3** | **2.097** |
| **44** | **3632.0499** | Synthesis Procedure 1 | Synthesis Procedure 6 | | Condition 1 | **1210.9423** | **3** | **2.1** |
| **45** | **3688.1563** | Synthesis Procedure 1 | Synthesis Procedure 6 | | Condition 1 | **1229.6301** | **3** | **1.823** |
| **46** | **3692.1019** | Synthesis Procedure 1 | Synthesis Procedure 6 | | Condition 1 | **1230.9509** | **3** | **1.596** |
| **47** | **3660.1031** | Synthesis Procedure 1 | Synthesis Procedure 6 | | Condition 1 | **1220.2886** | **3** | **1.598** |
| **48** | **3632.0499** | Synthesis Procedure 1 | Synthesis Procedure 6 | | Condition 1 | **1210.942** | **3** | **1.591** |
| **49** | **3632.0499** | Synthesis Procedure 1 | Synthesis Procedure 6 | | Condition 1 | **1210.9407** | **3** | **1.599** |
| **50** | **3632.0499** | Synthesis Procedure 1 | Synthesis Procedure 6 | | Condition 1 | **1210.9404** | **3** | **1.59** |
| **51** | **4272.8105** | Synthesis Procedure 3 | Synthesis Procedure 6 | | Condition 1 | **1424.4027** | **3** | **1.684** |
| **52** | **4272.8105** | Synthesis Procedure 3 | Synthesis Procedure 6 | | Condition 1 | **1424.4024** | **3** | **1.68** |
| **53** | **4272.8105** | Synthesis Procedure 3 | Synthesis Procedure 6 | | Condition 1 | **1424.396** | **3** | **1,685** |
| **54** | **4244.7573** | Synthesis Procedure 3 | Synthesis Procedure 6 | | Condition 1 | **1415.0S77** | **3** | **1.568** |
| **55** | **4244.7573** | Synthesis Procedure 3 | Synthesis Procedure 6 | | Condition 1 | **1415.056** | **3** | **1.533** |
| **56** | **4244.7573** | Synthesis Procedure 3 | Synthesis Procedure 6 | | Condition 1 | **1415.0559** | **3** | **1.534** |

**[Table 5-2]**

| | | | | | | | | |
|---|---|---|---|---|---|---|---|---|
| **57** | **3075.3661** | Synthesis Procedure 1 | Synthesis Procedure 6 | | Condition 1 | **1025.4912** | **3** | **1.444** |
| **58** | **3075.3661** | Synthesis Procedure 1 | Synthesis Procedure 6 | | Condition 1 | **1025.4925** | **3** | **1.445** |
| **59** | **3075.3661** | Synthesis Procedure 1 | Synthesis Procedure 6 | | Condition 1 | **1025.4922** | **3** | **1.443** |
| **60** | **6024.2405** | Synthesis Procedure 1 | Synthesis Procedure 5 | Synthesis Procedure 6 | Condition 1 | **1506.1701** | **4** | **1.476** |
| **61** | **6024.2405** | Synthesis Procedure 1 | Synthesis Procedure 5 | Synthesis Procedure 6 | Condition 1 | **1506.1697** | **4** | **1.468** |
| **62** | **5794.0657** | Synthesis Procedure 1 | Synthesis Procedure 5 | Synthesis Procedure 6 | Condition 1 | **1931.2051** | **3** | **1.486** |
| **63** | **5794.0657** | Synthesis Procedure 1 | Synthesis Procedure 5 | Synthesis Procedure 6 | Condition 1 | **1448.6553** | **4** | **1.489** |
| **64** | **5794.0657** | Synthesis Procedure 1 | Synthesis Procedure 5 | Synthesis Procedure 6 | Condition 1 | **1931.2045** | **3** | **1.495** |
| **65** | **5996.1874** | Synthesis Procedure 1 | Synthesis Procedure 5 | Synthesis Procedure 6 | Condition 1 | **1499.1625** | **4** | **2.071** |
| **66** | **5996.1874** | Synthesis Procedure 1 | Synthesis Procedure 5 | Synthesis Procedure 6 | Condition 1 | **1499.1659** | **4** | **2.07** |
| **67** | **5996.1874** | Synthesis Procedure 1 | Synthesis Procedure 5 | Synthesis Procedure 6 | Condition 1 | **1998.5501** | **3** | **1.408** |
| **68** | **5766.0126** | Synthesis Procedure 1 | Synthesis Procedure 5 | Synthesis Procedure 6 | Condition 1 | **1441.6496** | **4** | **1.421** |
| **69** | **5766.0126** | Synthesis Procedure 1 | Synthesis Procedure 5 | Synthesis Procedure 6 | Condition 1 | **1441.6473** | **4** | **1.412** |
| **70** | **4328.9168** | Synthesis Procedure 3 | Synthesis Procedure 6 | | Condition 1 | **1443.0842** | **3** | **1.666** |
| **71** | **4332.8624** | Synthesis Procedure 3 | Synthesis Procedure 6 | | Condition 1 | **1.444.4025** | **3** | **1.647** |
| **72** | **4332.8624** | Synthesis Procedure 3 | Synthesis Procedure 6 | | Condition 1 | **1444.4083** | **3** | **1.984** |

The residues constituting the cyclic peptides Peptide-25 to Peptide-72, which have a linker structure, are shown in the following table. It should be noted that in the following table, the 1st to 11th amino acid residues of the cyclic peptides Peptide-25 to Peptide-72 correspond to Zₐₐ₂ to Zₐₐ₁₂ in the cyclic peptide II of the present invention, and Zₐₐ₁ is absent. The 12th cysteine residue has been converted to a ring-forming group in the cyclic peptide. The ring-forming group forms an amide bond with the linker structure at an aminocarbonyl group, and NH₂ in the aminocarbonyl group has been substituted with NH of the N-terminal amino group of the N-terminal amino acid residue in the linker structure. The C-terminal lysine residue has been maleimidated by Synthesis Procedure 6.

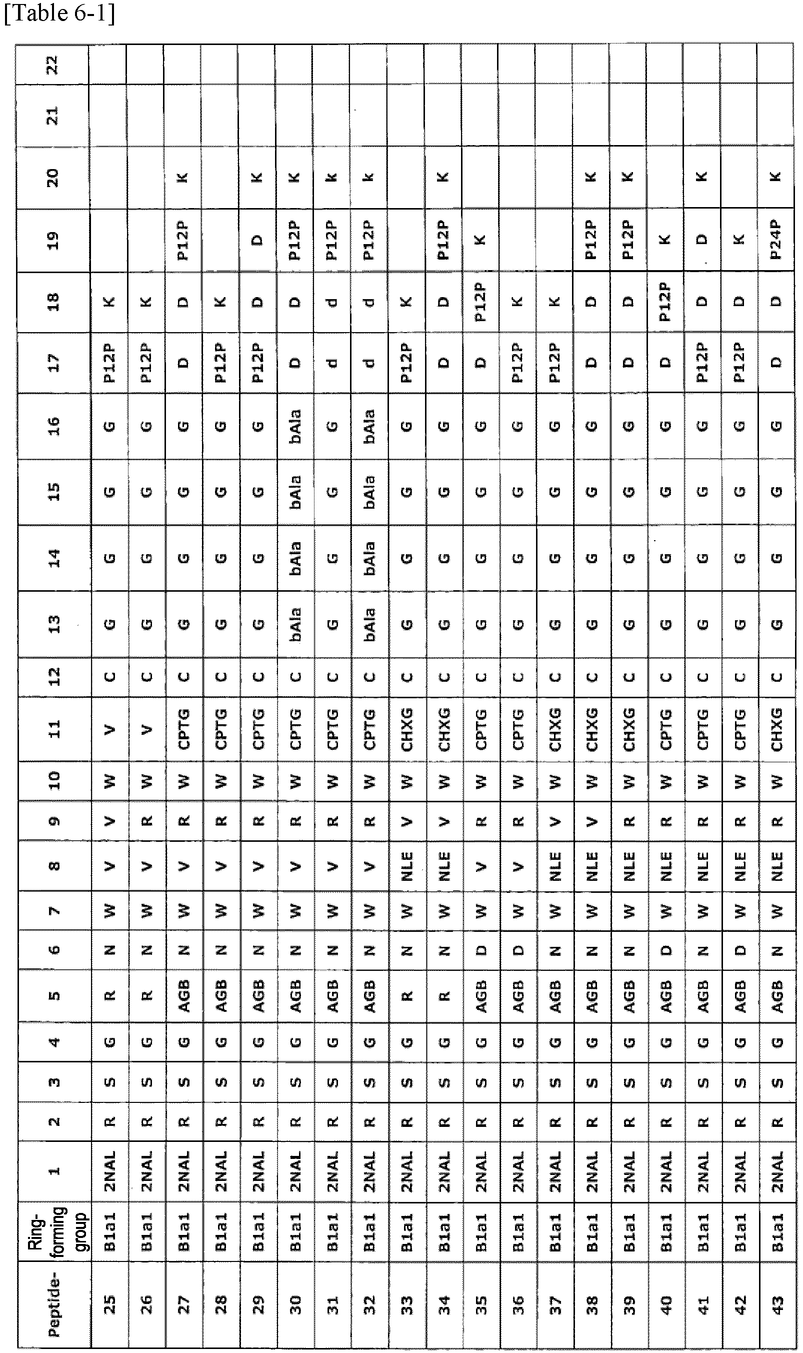

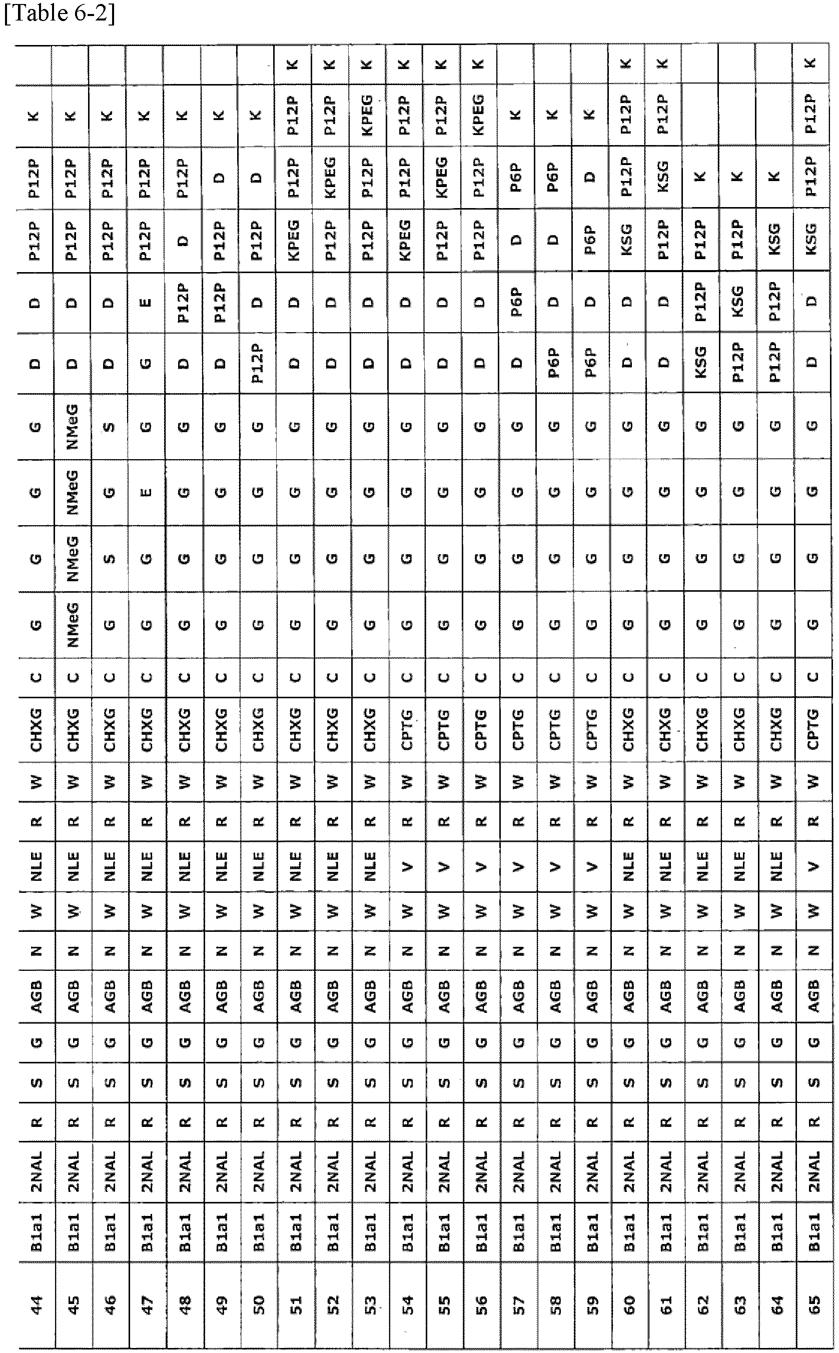

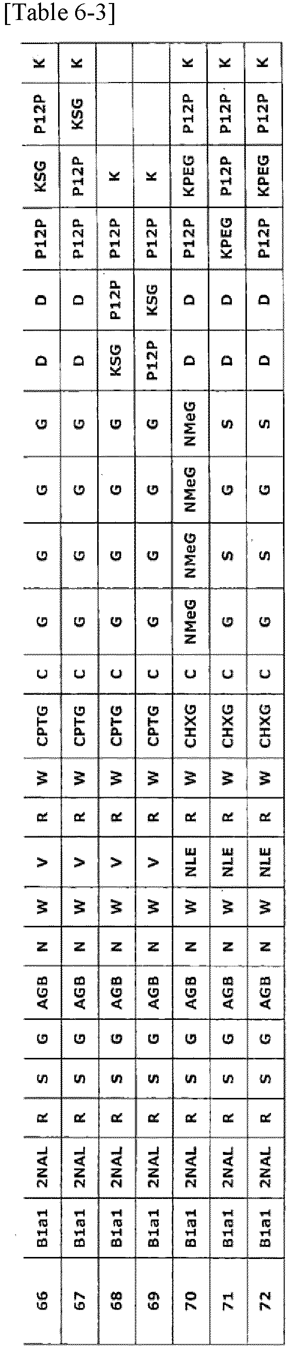

### 4. Synthesis of Peptide-73 to Peptide-109

As shown in the following table, Peptide-73 was synthesized according to any of Synthesis Procedure 1 (yield: 13%) and Synthesis Procedure 7 (yield: 76%) as shown hereinbelow.

Peptide-74, Peptide-75, Peptide-79 to Peptide-83, Peptide-85, Peptide-87 to Peptide-91, Peptide-95, Peptide-96, Peptide-98, Peptide-100 to Peptide-106, and Peptide-109 were also synthesized using the same procedure.

As shown in the following table, Peptide-76 was synthesized according to any of Synthesis Procedure 1 (yield: 20%), Synthesis Procedure 4 (yield: 14%), and Synthesis Procedure 7 (yield: 73%) as shown hereinbelow.

Peptide-77, Peptide-78, Peptide-84, Peptide-86, and Peptide-92 to Peptide-94 were also synthesized using the same procedure.

As shown in the following table, Peptide-97 was synthesized according to any of Synthesis Procedure 2 (yield: 6%) and Synthesis Procedure 7 (yield: 60%) as shown hereinbelow.

Peptide-107 was also synthesized using the same procedure.

As shown in the following table, Peptide-99 was synthesized according to any of Synthesis Procedure 2 (yield: 20%), Synthesis Procedure 4 (yield: 14%), and Synthesis Procedure 7 (yield: 73%) as shown hereinbelow.

Peptide-108 was also synthesized using the same procedure.

**[Table 7]**

| **Peptide-** | MW | **procedure 1** | **procedure 2** | **procedure 3** | **LC-MS condition** | MS measured value | MS valence | **Rt (min)** |
|---|---|---|---|---|---|---|---|---|
| **73** | **2841.24** | Synthesis Procedure 1 | Synthesis Procedure 7 | | Condition 1 | **1421.21** | **2** | **1.90** |
| **74** | **2910.31** | Synthesis Procedure 1 | Synthesis Procedure 7 | | Condition 1 | **1455.23** | **2** | **1.78** |
| **75** | **3140.48** | Synthesis Procedure 1 | Synthesis Procedure 7 | | Condition 1 | **1047.17** | **3** | **1.74** |
| **76** | **3201.61** | Synthesis Procedure 1 | Synthesis Procedure 4 | Synthesis Procedure 7 | Condition 1 | **1067.48** | **3** | **1.77** |
| **77** | **3301.68** | Synthesis Procedure 1 | Synthesis Procedure 4 | Synthesis Procedure 7 | Condition 1 | **1100.83** | **3** | **1.75** |
| **78** | **3187.58** | Synthesis Procedure 1 | Synthesis Procedure 4 | Synthesis Procedure 7 | Condition 1 | **1062.81** | **3** | **1.76** |
| **79** | **3168.53** | Synthesis Procedure 1 | Synthesis Procedure 7 | | Condition 1 | **1056.51** | **3** | **1.79** |
| **80** | **3186.50** | Synthesis Procedure 1 | Synthesis Procedure 7 | | Condition 1 | **1593.25** | **2** | **1.72** |

**[Table 8]**

| **Peptide-** | MW | **procedure 1** | **procedure 2** | **procedure 3** | **LC-MS condition** | MS measured value | MS valence | **Rt (min)** |
|---|---|---|---|---|---|---|---|---|
| **81** | **2924.33** | Synthesis Procedure 1 | Synthesis Procedure 7 | | Condition 1 | **975.49** | **3** | **1.74** |
| **82** | **3165.54** | Synthesis Procedure 1 | Synthesis Procedure 7 | | Condition 1 | **1582.77** | **2** | **1.61** |
| **83** | **2911.29** | Synthesis Procedure 1 | Synthesis Procedure 7 | | Condition 1 | **1455.72** | **2** | **1.74** |
| **84** | **2887.36** | Synthesis Procedure 1 | Synthesis Procedure 4 | Synthesis Procedure 7 | Condition 1 | **1443.68** | **2** | **1.76** |
| **85** | **2881.30** | Synthesis Procedure 1 | Synthesis Procedure 7 | | Condition 1 | **960.82** | **3** | **1.85** |
| **86** | **2956.42** | Synthesis Procedure 1 | Synthesis Procedure 4 | Synthesis Procedure 7 | Condition 1 | **985.80** | **3** | **1.78** |
| **87** | **2881.30** | Synthesis Procedure 1 | Synthesis Procedure 7 | | Condition 1 | **960.82** | **3** | **1.78** |
| **88** | **3370.66** | Synthesis Procedure 1 | Synthesis Procedure 7 | | Condition 1 | **1123.86** | **3** | **1.72** |
| **89** | **3255.5681** | Synthesis Procedure 1 | Synthesis Procedure 7 | | Condition 1 | **1085.5172** | **3** | **1.727** |
| **90** | **3025.3933** | Synthesis Procedure 1 | Synthesis Procedure 7 | | Condition 1 | **1008.8277** | **3** | **1.746** |
| **91** | **3154.5073** | Synthesis Procedure 1 | Synthesis Procedure 7 | | Condition 1 | **1051.8421** | **3** | **1.749** |
| **92** | **3186.5954** | Synthesis Procedure 1 | Synthesis Procedure 4 | Synthesis Procedure 7 | Condition 1 | **1062.481** | **3** | **1.759** |
| **93** | **3071.506** | Synthesis Procedure 1 | Synthesis Procedure 4 | Synthesis Procedure 7 | Condition 1 | **1024.1387** | **3** | **1.765** |
| **94** | **3186.5954** | Synthesis Procedure 1 | Synthesis Procedure 4 | Synthesis Procedure 7 | Condition 1 | **1062.48** | **3** | **1.747** |
| **95** | **3154.5073** | Synthesis Procedure 1 | Synthesis Procedure 7 | | Condition 1 | **1051.8465** | **3** | **1.726** |
| **96** | **3040.4046** | Synthesis Procedure 1 | Synthesis Procedure 7 | | Condition 1 | **1013.8938** | **3** | **1.75** |
| **97** | **3040.4046** | Synthesis Procedure 2 | Synthesis Procedure 7 | | Condition 1 | **1013.8308** | **3** | **1.755** |
| **98** | **3182.5604** | Synthesis Procedure 1 | Synthesis Procedure 7 | | Condition 1 | **1061.1877** | **3** | **1.732** |
| **99** | **3201.6067** | Synthesis Procedure 2 | Synthesis Procedure 4 | Synthesis Procedure 7 | Condition 1 | **1067.4832** | **3** | **1.767** |
| **100** | **3140.4807** | Synthesis Procedure 1 | Synthesis Procedure 7 | | Condition 1 | **1047.173** | **3** | **1.731** |
| **101** | **3196.587** | Synthesis Procedure 1 | Synthesis Procedure 7 | I | Condition 1 | **1065.8611** | **3** | **1.731** |
| **102** | **3140.4807** | Synthesis Procedure 1 | Synthesis Procedure 7 | | Condition 1 | **1047.172** | **3** | **1.74** |
| **103** | **3196.587** | Synthesis Procedure 1 | Synthesis Procedure 7 | | Condition 1 | **1065.8563** | **3** | **1.729** |
| **104** | **2895.331** | Synthesis Procedure 1 | Synthesis Procedure 7 | | Condition 1 | **965.491** | **3** | **1.885** |
| **105** | **3125.5058** | Synthesis Procedure 1 | Synthesis Procedure 7 | | Condition 1 | **1042.1788** | **3** | **1.852** |
| **106** | **3026.378** | Synthesis Procedure 1 | Synthesis Procedure 7 | | Condition 1 | **1009.1514** | **3** | **1.744** |
| **107** | **2912.2754** | Synthesis Procedure 2 | Synthesis Procedure 7 | | Condition 1 | **971.142** | **3** | **1.764** |
| **108** | **3073.4775** | Synthesis Procedure 2 | Synthesis Procedure 4 | Synthesis Procedure 7 | Condition 1 | **1024.7938** | **3** | **1.778** |
| **109** | **3154.5073** | Synthesis Procedure 1 | Synthesis Procedure 7 | | Condition 1 | **1051.8419** | **3** | **1.772** |

The residues constituting the cyclic peptides Peptide-73 to Peptide-109, which have a linker structure, are shown in the following table. It should be noted that in the following table, the 1st to 11th amino acid residues of the cyclic peptides Peptide-73 to Peptide-75, Peptide-79 to Peptide-83, Peptide-85, Peptide-87 to Peptide-91, Peptide-95 to Peptide-98, Peptide-100 to Peptide-107, and Peptide-109 correspond to Zₐₐ₂ to Zₐₐ₁₂ in the cyclic peptide II of the present invention, and Zₐₐ₁ is absent. The cysteine residue at position 12 has been converted to a ring-forming group in the cyclic peptide. The ring-forming group forms an amide bond with the linker structure at an aminocarbonyl group, and NH₂ in the aminocarbonyl group has been substituted with NH of the N-terminal amino group of the N-terminal amino acid residue in the linker structure. The HCY residues at positions 3 and 8 in Peptide-76 to Peptide-78, Peptide-84, Peptide-86, Peptide-92 to Peptide-94, Peptide-99, and Peptide-108 also have disulfide bonds formed therein. The C-terminal lysine residue has been subjected to DBCO modification by Synthesis Procedure 7.

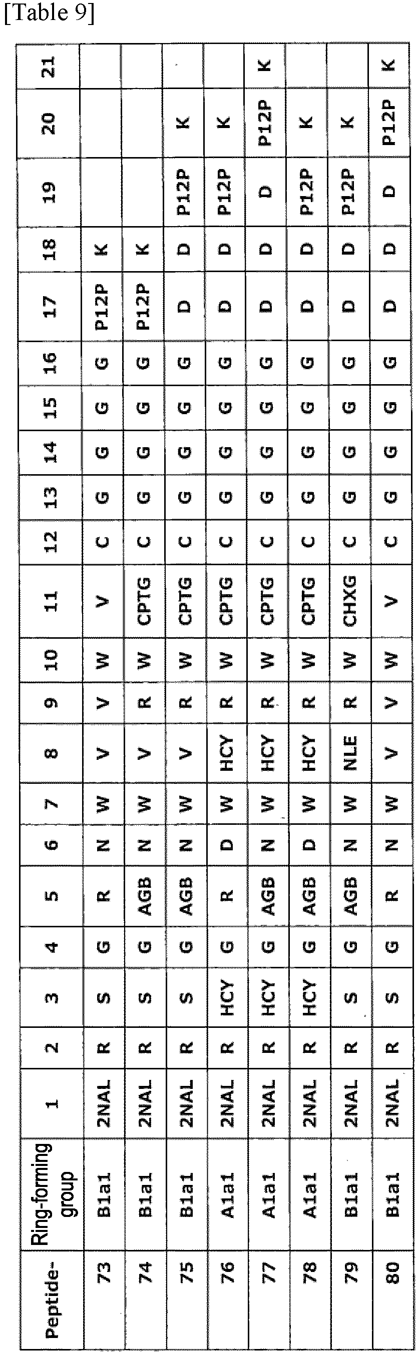

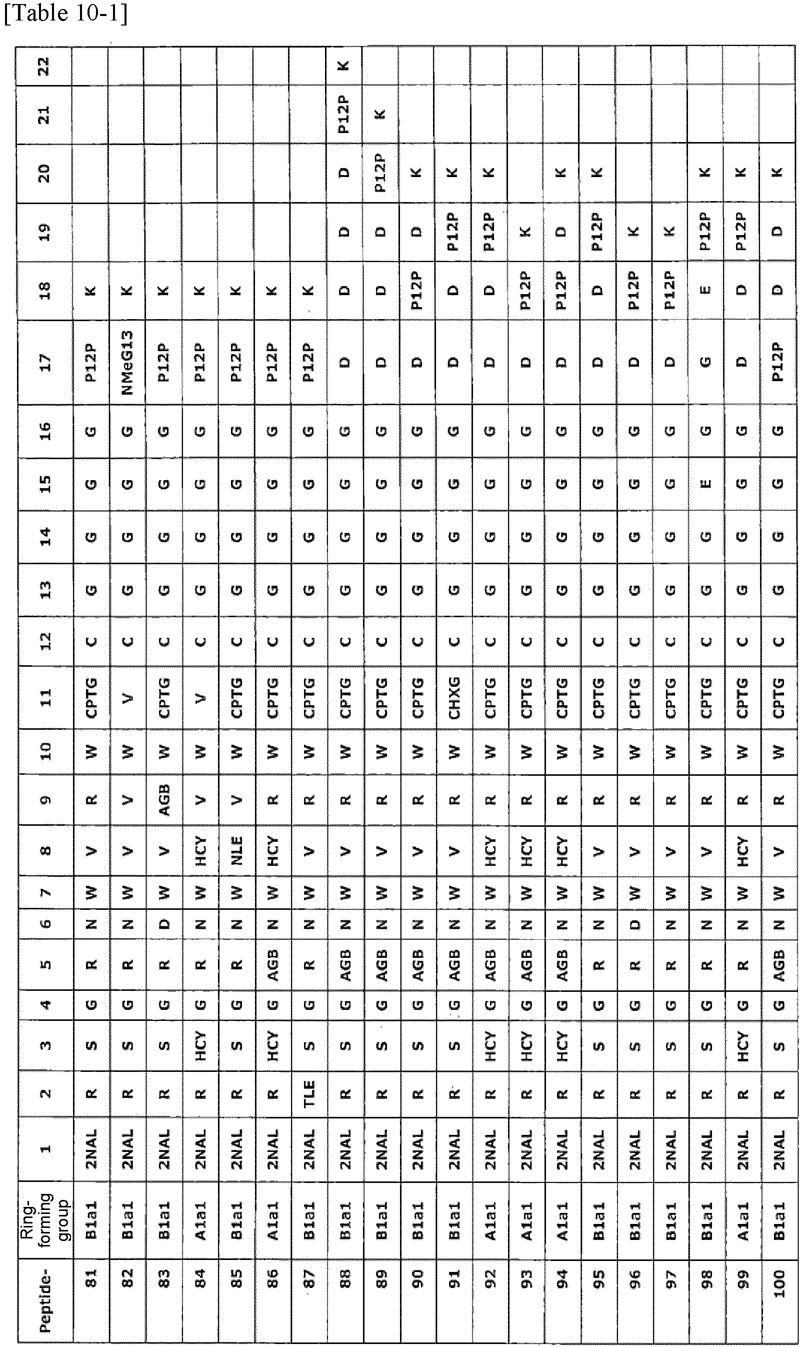

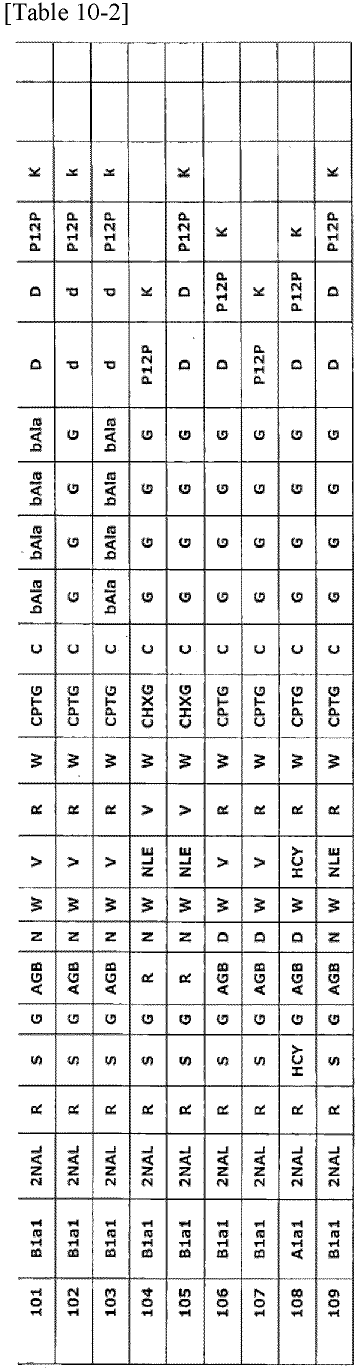

### 5. Preparation of Fc-Containing Molecule

### (1) Preparation of Fc-A (LALA form)

Fc-A was prepared according to the description in Example 2-5 of WO 2018/003983. Fc-A is a LALA form of Fc-B (SEQ ID NO: 135), which is a partial antibody molecule consisting of the Fc fragment of IgG1, with an N-terminal extension (SEQ ID NO: 137).

### (2) Preparation of Fc-C (LALA-PA form)

### (i) Construction of Fc-C H chain expression vector

A DNA fragment (SEQ ID NO: 138) containing the nucleotide sequence encoding the amino acid sequence of Fc-C, which was a partial antibody consisting of the Fc fragment of human IgG1, was synthesized as a carrier molecule (Thermo Fisher Scientific). Using the synthesized DNA fragment, the vector pCMA-LK described in WO 2015/046505 was digested with XbaI and PmeI and then bonded using an In-Fusion HD PCR cloning kit (produced by Takara Bio USA, Inc.) to construct an Fc-C expression vector. The resulting expression vector was designated as "pCMA/Fc-C". The nucleotide sequence encoding the amino acid sequence of Fc-C is at nucleotide positions 61 to 741 of SEQ ID NO: 165 (nucleotide positions 1 to 60 encode the signal sequence). The amino acid sequence of Fc-C is the sequence at amino acid positions 21 to 247 of SEQ ID NO: 139 (amino acid positions 1 to 20 are signal sequences).

### (ii) Production of Fc-C

FreeStyle 293F cells (produced by Thermo Fisher Scientific) were passaged and cultured in a spinner flask at 37°C and 8% CO₂ using a BCP-type animal cell culture device (produced by Biott Corporation). Subsequent culturing was performed using WAVE BIOREACTOR (produced by GE Healthcare) according to the instruction. FreeStyle 293F cells in a logarithmic growth phase were diluted with a FreeStyle 293 expression medium (produced by Thermo Fisher Scientific), adjusted to 2.0 to 2.4 × 10⁶ cells/mL, and transferred at a volume of 6 L into a 50 L culture WAVE bag (produced by Cytiva). Subsequently, 6L of FreeStyle 293 expression medium was also transferred and cultured overnight with shaking at 37°C and 8% CO₂. 37.5 mg of Polyethyleneimine (produced by Polysciences, Inc.) was added to 400 mL of Opti-Pro SFM medium (produced by Thermo Fisher Scientific). Then, 12.5 mg of the expression vector pCMA/Fc-C was added to 400 mL of the Opti-Pro SFM medium. The expression vector/Opti-Pro SFM mixture was added to the Polyethyleneimine/Opti-Pro SFM mixture, gently stirred, and left to stand for another 5 minutes before being added entirely to the 50 L culture WAVE bag in which FreeStyle 293F cells were cultured. After 4 hours of culture with shaking at 37°C and 8% CO₂, 12 L of EX-CELL VPRO medium (produced by SAFC Biosciences) and 500 mL of 43.4 g/L BD Recharge CD (produced by BD Biosciences) were added thereto and cultured with shaking at 37°C and 8% CO₂ for 6 days. The resulting culture supernatant was passed through a depth filter (pore size: 5 µm, produced by GE Healthcare), followed by filtration through a capsule cartridge filter (pore size: 0.45 µm, produced by ADVANTEC TOYO KAISHA, LTD.).

### (iii) Purification of Fc-C

Purification of Fc-C was carried out in the same manner as the purification method of Fc-B described in Example 2-4 (2-4C) of WO 2018/003983 through a two-step process of Protein A affinity chromatography and ceramic hydroxyapatite.

### 6. Synthesis of Conjugate-1 to Conjugate-12

As shown in the following table, Conjugate-1 to Conjugate-12 were synthesized using Peptide-13 to Peptide-24 according to Synthesis Procedure 8 as shown hereinbelow. In Conjugate-1 to Conjugate-12, Peptide-13 to Peptide-24 were each bonded to Cys226 and Cys229 (Cys at positions 226 and 229 of the Eu index, respectively) of the Fc-containing molecule. In Tables 11 to 14, two cyclic peptide-conjugated Fc-containing molecules were cross-linked by a disulfide bond to form one molecule in the glycan conjugates Conjugate-61 to Conjugate-97, whereas the Fc-containing molecules (monomers) were not cross-linked to each other in the maleimide conjugates Conjugate-1 to Conjugate-60. Although each maleimide conjugates exists as a mixture in which various glycans are heterogeneously bonded to Fc-containing molecules, the glycans of the Fc-containing molecules are excluded in the calculation of the molecular weight of the maleimide conjugate and measurement by LC-MS in Tables 11 to 14. In other words, the molecular weight of the glycan is not considered in the calculation of the molecular weight of the maleimide conjugate, and in the MS measurement, deglycosylation is performed before the LC-MS measurement.

**[Table 11]**

| **Conjugate-** | Precursor | Carrier | Linking group C | MW | **procedure** | **LC-MS condition** | **MS (deconvolution)** | **Rt(min)** | **IC₅₀ (nM)** |
|---|---|---|---|---|---|---|---|---|---|
| **1** | **Peptide-13** | **Fc-A** | **C3** | **31663.4** | Synthesis Procedure 8 | Condition 4 | **31662.30** | **2.21** | **205.5** |
| **2** | **Peptide-14** | **Fc-A** | **C3** | **31435.2** | Synthesis Procedure 8 | Condition 4 | **31434.13** | **2.32** | **207** |
| **3** | **Peptide-15** | **Fc-A** | **C3** | **31207** | Synthesis Procedure 8 | Condition 4 | **31205.80** | **2.36** | **116** |
| **4** | **Peptide-16** | **Fc-C** | **C3** | **33949.7** | Synthesis Procedure 8 | Condition 4 | **33949.09** | **2.39** | **20.1** |
| **5** | **Peptide-17** | **Fc-C** | **C3** | **34069.7** | Synthesis Procedure 8 | Condition 4 | **34069.23** | **2.37** | **28.75** |
| **6** | **Peptide-18** | **Fc-C** | **C3** | **33949.7** | Synthesis Procedure 8 | Condition 4 | **33948.96** | **2.39** | **38.15** |
| **7** | **Peptide-19** | **Fc-C** | **C3** | **33949.7** | Synthesis Procedure 8 | Condition 4 | **33949.19** | **2.38** | **21.95** |
| **8** | **Peptide-20** | **Fc-C** | **C3** | **34061.9** | Synthesis Procedure 8 | Condition 4 | **34061.42** | **2.43** | **38.2** |
| **9** | **Peptide-21** | **Fc-C** | **C3** | **34061.9** | Synthesis Procedure 8 | Condition 4 | **34061.50** | **2.42** | **43.55** |
| **10** | **Peptide-22** | **Fc-C** | **C3** | **34069.7** | Synthesis Procedure 8 | Condition 4 | **34069.45** | **2.36** | **12.75** |
| **11** | **Peptide-23** | **Fc-C** | **C3** | **34069.7** | Synthesis Procedure 8 | Condition 4 | **34069.38** | **2.36** | **17.0** |
| **12** | **Peptide-24** | **Fc-C** | **C3** | **34061.9** | Synthesis Procedure 8 | Condition 4 | **34061.25** | **2.42** | **44.65** |

### 7. Synthesis of Conjugate-13 to Conjugate-97

As shown in the following table, Conjugate-13 to Conjugate-97 were synthesized using Peptide-25 to Peptide-109 (compounds composed of a cyclic peptide and a linker structure) according to Synthesis Procedure 9 or Synthesis Procedure 10 as shown hereinbelow. In Conjugate-13 to Conjugate-60, Peptide-25 to Peptide-72 were each bonded to Cys226 and Cys229 (Cys at positions 226 and 229 of the Eu index, respectively) of the Fc-containing molecule. Further, N297-linked glycans of the Fc-containing molecules in Conjugate-61 to Conjugate-97 are each subjected to glycan remodeling into N297-(Fuc)SG. It should be noted that the cyclic peptide corresponding to cyclic peptide moieties of Conjugate-65, 74, and 80 to 82 is Peptide-1. The cyclic peptide corresponding to a cyclic peptide moiety of Conjugate-64 is Peptide-11. The cyclic peptide corresponding to cyclic peptide moieties of Conjugates 66 and 96 is Peptide-9. The cyclic peptide corresponding to a cyclic peptide moiety of Conjugate-72 is Peptide-6. The cyclic peptide corresponding to a cyclic peptide moiety of Conjugate-87 is Peptide-10. Cyclic peptides corresponding to cyclic peptide moieties of other conjugates are referred to as Peptides-110 to 124, as shown below.
Conjugate-13, 61, 68, 70: Peptide-110
Conjugate-14: Peptide-111
Conjugate-69, 83, 85, 86: Peptide-112
Conjugate-29, 97: Peptide-113
Conjugate-73: Peptide-114
Conjugate-15 to 20, 42 to 47, 53 to 57, 62, 63, 76 to 78, 88 to 91: Peptide-115
Conjugate-21, 22, 92, 93: Peptide-116
Conjugate-75: Peptide-117
Conjugate-71: Peptide-118
Conjugate-23, 24, 94, 95: Peptide-119
Conjugate-25, 26: Peptide-120
Conjugate-27, 31 to 41, 48 to 52, 58 to 60, 67: Peptide-121
Conjugate-79: Peptide-122
Conjugate-28, 30: Peptide-123
Conjugate-84: Peptide-124

**[Table 12-1]**

| **Conjugate-** | Precursor | Carrier | Linking group C | MW | **procedure** | **LC-MS condition** | **MS (deconvolution)** | **Rt(min)** | **IC₅₀ (nM)** |
|---|---|---|---|---|---|---|---|---|---|
| **13** | **Peptlde-25** | **Fc-A** | **C3** | **30742.5** | Synthesis Procedure 9 | Condition 4 | **30741.70** | **2.19** | **36.5** |
| **14** | **Peptide-26** | **Fc-A** | **C3** | **30856.6** | Synthesis Procedure 9 | Condition 4 | **30855.70** | **2.06** | **12.8** |
| **15** | **Peptide-27** | **Fc-A** | **C3** | **31341** | Synthesis Procedure 9 | Condition 4 | **31340.20** | **2.09** | **23.7** |
| **16** | **Peptide-28** | **Fc-A** | **C3** | **30880.6** | Synthesis Procedure 9 | Condition 4 | **30880.00** | **2.11** | **11.7** |
| **17** | **Peptide-29** | **Fc-A** | **C3** | **31341** | Synthesis Procedure 9 | Condition 4 | **31340.00** | **2.21** | **24.9** |
| **18** | **Peptide-30** | **Fc-A** | **C3** | **3145.3** | Synthesis Procedure 9 | Condition 4 | **31452.00** | **2.2** | **23.5** |
| **19** | **Peptide-31** | **Fc-A** | **C3** | **31341** | Synthesis Procedure 9 | Condition 4 | **31340.00** | **2.21** | **52.6** |
| **20** | **Peptlde-32** | **Fc-A** | **C3** | **31453.2** | Synthesis Procedure 9 | Condition 4 | **31452.60** | **2.18** | **31.9** |
| **21** | **Peptide-33** | **Fc-A** | **C3** | **30860.6** | Synthesis Procedure 9 | Condition 4 | **30849.70** | **2.53** | **41.0** |
| **22** | **Peptlde-34** | **Fc-A** | **C3** | **31311** | Synthesis Procedure 9 | Condition 4 | **31310.20** | **2.5** | **43.8** |
| **23** | **Peptide-35** | **Fc-A** | **C3** | **31112.8** | Synthesis Procedure 9 | Condition 4 | **31111.80** | **2.23** | **34.9** |
| **24** | **Peptide-36** | **Fc-A** | **C3** | **30884.6** | Synthesis Procedure 9 | Condition 4 | **30883.70** | **2.27** | **56.5** |
| **25** | **Peptide-37** | **Fc-A** | **C3** | **30822.6** | Synthesis Procedure 9 | Condition 4 | **30821.5** | **2.56** | **40.6** |
| **26** | **Peptide-38** | **Fc-A** | **C3** | **31283** | Synthesis Procedure 9 | Condition 4 | **31282.6** | **2.53** | **115.0** |
| **27** | **Peptide-39** | **Fc-A** | **C3** | **31397** | Synthesis Procedure 9 | Condition 4 | **31396** | **2.38** | **79.8** |
| **28** | **Peptide-40** | **Fc-A** | **C3** | **31141** | Synthesis Procedure 9 | Condition 4 | **31140.1** | **2.32** | **97.3** |
| **29** | **Peptlde-41** | **Fc-A** | **C3** | **31371** | Synthesis Procedure 9 | Condition 4 | **31368** | **2.3** | **30.3** |
| **30** | **Peptide-42** | **Fc-A** | **C3** | **31140.8** | Synthesis Procedure 9 | Condition 4 | **31140** | **2.34** | **36.5** |
| **31** | **Peptlde-43** | **Fc-C** | **C3** | **32459.9** | Synthesis Procedure 9 | Condition 4 | **32459.4** | **2.34** | **44.2** |
| **32** | **Peptide-44** | **Fc-C** | **C3** | **32601.9** | Synthesis Procedure 9 | Condition 4 | **32601.4** | **2.31** | **35.2** |
| **33** | **Peptide-45** | **Fc-C** | **C3** | **32713.9** | Synthesis Procedure 9 | Condition 4 | **32713.8** | **2.31** | **27.3** |
| **34** | **Peptide-46** | **Fc-C** | **C3** | **32721.9** | Synthesis Procedure 9 | Condition 4 | **32721.6** | **2.27** | **25.2** |
| **35** | **Peptide-47** | **Fc-C** | **C3** | **32657.9** | Synthesis Procedure 9 | Condition 4 | **32657.5** | **2.28** | **31.2** |
| **36** | **Peptide-48** | **Fc-C** | **C3** | **32601.9** | Synthesis Procedure 9 | Condition 4 | **32601.6** | **2.29** | **24.7** |
| **37** | **Peptide-49** | **Fc-C** | **C3** | **32601.9** | Synthesis Procedure 9 | Condition 4 | **32601.5** | **2.3** | **22.0** |
| **38** | **Peptide-50** | **Fc-C** | **C3** | **32601.9** | Synthesis Procedure 9 | Condition 4 | **32601.6** | **2.28** | **16.5** |
| **39** | **Peptide-51** | **Fc-C** | **C3** | **33833.5** | Synthesis Procedure 9 | Condition 4 | **33883** | **2.41** | **31.6** |
| **40** | **Peptide-52** | **Fc-C** | **C3** | **33833.5** | Synthesis Procedure 9 | Condition 4 | **33883** | **2.4** | **30.1** |
| **41** | **Peptide-53** | **Fc-C** | **C3** | **33833.5** | Synthesis Procedure 9 | Condition 4 | **33883** | **2.41** | **28.6** |
| **42** | **Peptide-54** | **Fc-C** | **C3** | **33827.5** | Synthesis Procedure 9 | Condition 4 | **33827** | **2.26** | **16.9** |
| **43** | **Peptide-55** | **Fc-C** | **C3** | **33827.5** | Synthesis Procedure 9 | Condition 4 | **33827** | **2.25** | **15.9** |
| **44** | **Peptide-56** | **Fc-C** | **C3** | **33827.5** | Synthesis Procedure 9 | Condition 4 | **33826.9** | **2.26** | **20.1** |
| **45** | **Peptide-57** | **Fc-C** | **C3** | **31488.7** | Synthesis Procedure 9 | Condition 4 | **31488.1** | **2.05** | **16.2** |
| **46** | **Peptide-58** | **Fc-C** | **C3** | **31488.7** | Synthesis Procedure 9 | Condition 4 | **31288.2** | **2.05** | **14.5** |
| **47** | **Peptide-59** | **Fc-C** | **C3** | **31488.7** | Synthesis Procedure 9 | Condition 4 | **31488.2** | **2.05** | **11.8** |
| **48** | **Peptide-60** | **Fc-C** | **C3** | **37386.3** | Synthesis Procedure 9 | Condition 4 | **37385.7** | **2.21** | **27.0** |
| **49** | **Peptide-61** | **Fc-C** | **C3** | **37386.3** | Synthesis Procedure 9 | Condition 4 | **37385.7** | **2.21** | **26.9** |
| **50** | **Peptide-62** | **Fc-C** | **C3** | **36926.1** | Synthesis Procedure 9 | Condition 4 | **36925.2** | **2.25** | **21.2** |
| **51** | **Peptide-63** | **Fc-C** | **C3** | **36926.1** | Synthesis Procedure 9 | Condition 4 | **36925.2** | **2.28** | **15.7** |
| **52** | **Peptide-64** | **Fc-C** | **C3** | **36926.1** | Synthesis Procedure 9 | Condition 4 | **36925.1** | **2.28** | **26.7** |
| **53** | **Peptlde-65** | **Fc-C** | **C3** | **37330.3** | Synthesis Procedure 9 | Condition 4 | **37329.5** | **2.09** | **12.2** |
| **54** | **Peptide-66** | **Fc-C** | **C3** | **37330.3** | Synthesis Procedure 9 | Condition 4 | **37329.6** | **2.08** | **11.3** |
| **55** | **Peptide-67** | **Fc-C** | **C3** | **37330.3** | Synthesis Procedure 9 | Condition 4 | **37329.5** | **2.1** | **14.0** |
| **56** | **Peptide-68** | **Fc-C** | **C3** | **36869.9** | Synthesis Procedure 9 | Condition 4 | **36869.2** | **2.12** | **6.4** |
| **57** | **Peptide-69** | **Fc-C** | **C3** | **36869.9** | Synthesis Procedure 9 | Condition 4 | **36869.4** | **2.1** | **6.9** |
| **58** | **Peptide-70** | **Fc-C** | **C3** | **33995.7** | Synthesis Procedure 9 | Condition 4 | **33995.4** | **2.42** | **23.7** |
| **59** | **Peptide-71** | **Fc-C** | **C3** | **34003.7** | Synthesis Procedure 9 | Condition 4 | **34003.2** | **2.39** | **22.1** |
| **60** | **Peptide-72** | **Fc-C** | **C3** | **34003.7** | Synthesis Procedure 9 | Condition 4 | **34003.2** | **2.38** | **21.9** |

**[Table 13]**

| **Conjugate-** | Precursor | Carrier | Linking group C | MW | **procedure** | **LC-MS condition** | **MS (deconvolution)** | **Rt(min)** | **IC₅₀ (nM)** |
|---|---|---|---|---|---|---|---|---|---|
| **61** | **Peptide-73** | **Fc-A** | **C1,C2** | **67770.8** | Synthesis Procedure 10 | Condition 3 | **33767.81** | **2.17** | **25.1** |
| **62** | **Peptide-74** | **Fc-A** | **C1,C2** | **67804.2** | Synthesis Procedure 10 | Condition 2 | **67804.21** | **2.17** | **7.1** |
| **63** | **Peptide-75** | **Fc-A** | **C1,C2** | **68724.9** | Synthesis Procedure 10 | Condition 2 | **68724.88** | **2.17** | **14.0** |
| **64** | **Peptide-76** | **Fc-A** | **C1,C2** | **68970.4** | Synthesis Procedure 10 | Condition 2 | **68968.15** | **2.26** | **15.8** |
| **65** | **Peptide-77** | **Fc-A** | **C1,C2** | **69369.8** | Synthesis Procedure 10 | Condition 2 | **69370.16** | **2.25** | **26.3** |
| **66** | **Peptide-78** | **Fc-A** | **C1,C2** | **6891.4.3** | Synthesis Procedure 10 | Condition 2 | **68912.06** | **2.48** | **36.5** |
| **67** | **Peptide-79** | **Fc-A** | **C1,C2** | **68838** | Synthesis Procedure 10 | Condition 2 | **68837.88** | **2.53** | **44.6** |
| **68** | **Peptide-80** | **Fc-A** | **C1,C2** | **68909** | Synthesis Procedure 10 | Condition 3 | **34459.22** | **2.11** | **451.1** |

**[Table 14-1]**

| **Conjugate-** | Precursor | Carrier | Linking group C | MW | **procedure** | **LC-MS condition** | **MS (deconvolution)** | **Rt (min)** | **IC₅₀ (nM)** |
|---|---|---|---|---|---|---|---|---|---|
| **69** | **Peptide-81** | **Fc-A** | **C1,C2** | **67860.2** | Synthesis Procedure 10 | Condition 2 | **67860.70** | **2.17** | **5.1** |
| **70** | **Peptide-82** | **Fc-A** | **C1,C2** | **68825** | Synthesis Procedure 10 | Condition 2 | **68824.60** | **2.19** | **32.1** |
| **71** | **Peptide-83** | **Fc-A** | **C1,C2** | **67809.2** | Synthesis Procedure 10 | Condition 2 | **67807.50** | **2.22** | **9.3** |
| **72** | **Peptide-84** | **Fc-A** | **C1,C2** | **67712.6** | Synthesis Procedure 10 | Condition 2 | **67712.80** | **2.36** | **14.3** |
| **73** | **Peptide-85** | **Fc-A** | **C1,C2** | **67688.2** | Synthesis Procedure 10 | Condition 2 | **67688.13** | **2.56** | **13.3** |
| **74** | **Peptide-86** | **Fc-A** | **C1,C2** | **67988.6** | Synthesis Procedure 10 | Condition 2 | **67989.11** | **2.35** | **15.8** |
| **75** | **Peptide-87** | **Fc-A** | **C1,C2** | **67688.2** | Synthesis Procedure 10 | Condition 2 | **67689.11** | **2.34** | **11.4** |
| **76** | **Peptide-S8** | **Fc-A** | **C1,C2** | **69645** | Synthesis Procedure 10 | Condition 2 | **69646.95** | **2.16** | **14.5** |
| **77** | **Peptide-89** | **Fc-A** | **C1,C2** | **69185.4** | Synthesis Procedure 10 | Condition 2 | **69185.945** | **2.18** | **26.9** |
| **78** | **Peptide-90** | **Fc-A** | **C1,C2** | **68725** | Synthesis Procedure 10 | Condition 2 | **68725.5** | **2.17** | **9.2** |
| **79** | **Peptide-91** | **Fc-A** | **C1,C2** | **68781** | Synthesis Procedure 10 | Condition 2 | **68780.859** | **2.2** | **17.1** |
| **80** | **Peptide-92** | **Fc-A** | **C1,C2** | **68909.4** | Synthesis Procedure 10 | Condition 2 | **68908.984** | **2.25** | **11.2** |
| **81** | **Peptide-93** | **Fc-A** | **C1,C2** | **68449** | Synthesis Procedure 10 | Condition 2 | **68449.875** | **2.27** | **10.2** |
| **82** | **Peptide-94** | **Fc-A** | **C1,C2** | **68909.4** | Synthesis Procedure 10 | Condition 2 | **68910.289** | **2.25** | **10.2** |
| **83** | **Peptide-95** | **Fc-A** | **C1,C2** | **68782** | Synthesis Procedure 10 | Condition 2 | **68780.68** | **2.08** | **9.5** |
| **84** | **Peptide-96** | **Fc-A** | **C1,C2** | **68326** | Synthesis Procedure 10 | Condition 2 | **68325.148** | **2.15** | **8.1** |
| **85** | **Peptide-97** | **Fc-A** | **C1,C2** | **68326** | Synthesis Procedure 10 | Condition 2 | **68323.922** | **2.16** | **7.1** |
| **86** | **Peptide-98** | **Fc-A** | **C1,C2** | **68894** | Synthesis Procedure 10 | Condition 2 | **68893.117** | **2.1** | **8.5** |
| **87** | **Peptide-99** | **Fc-A** | **C1,C2** | **68970** | Synthesis Procedure 10 | Condition 2 | **68969.18** | **2.26** | **15.7** |
| **88** | **Peptide-100** | **Fc-A** | **C1,C2** | **68726** | Synthesis Procedure 10 | Condition 2 | **68724.961** | **2.27** | **17.3** |
| **89** | **Peptide-101** | **Fc-A** | **C1,C2** | **68950.4** | Synthesis Procedure 10 | Condition 2 | **68948.516** | **2.26** | **18.3** |
| **90** | **Peptide-102** | **Fc-A** | **C1,C2** | **68726** | Synthesis Procedure 10 | Condition 2 | **68724.57** | **2.26** | **41.5** |
| **91** | **Peptide-103** | **Fc-A** | **C1,C2** | **68950.4** | Synthesis Procedure 10 | Condition 2 | **68948.234** | **2.24** | **22.9** |
| **92** | **Peptide-104** | **Fc-A** | **C1,C2** | **67745.2** | Synthesis Procedure 10 | Condition 2 | **67743.961** | **2.67** | **35.6** |
| **93** | **Peptide-105** | **Fc-A** | **C1,C2** | **68666** | Synthesis Procedure 10 | Condition 2 | **68664.492** | **2.63** | **73.4** |
| **94** | **Peptide-106** | **Fc-A** | **C1,C2** | **68269.6** | Synthesis Procedure 10 | Condition 2 | **68269.688** | **2.38** | **31.4** |
| **95** | **Peptide-107** | **Fc-A** | **C1,C2** | **67813.2** | Synthesis Procedure 10 | Condition 2 | **67812.203** | **2.42** | **28.1** |
| **96** | **Peptide-108** | **Fc-A** | **C1,C2** | **68458** | Synthesis Procedure 10 | Condition 2 | **68457.031** | **2.53** | **44.8** |
| **97** | **Peptide-109** | **Fc-A** | **C1,C2** | **68782** | Synthesis Procedure 10 | Condition 2 | **68781.586** | **2.45** | **47.2** |

### Synthesis Procedure 1

This procedure was carried out using the automatic synthesizer Syro II (produced by Biotage Japan) according to a common solid-phase synthesis method using a 9-fluorenylmethyloxycarbonyl group (Fmoc group) as an α-amino group-protecting group.

The equivalent of 88 µmol of Rink Amide Resin AM (produced by Novaviochem) was added to a reaction vessel, and 3 equivalents each of O-(7-azabenzotriazol-1-yl)-N,N,N',N'-tetramethyluronium hexafluorophosphate (HATU), N,N-diisopropylamine, and an Fmoc group-protected amino acid were added and reacted together in 1-methyl-2-pyrrolidinone.

Removal of Fmoc groups was carried out using 20% piperidine/1-methyl-2-pyrrolidinone.

After the N-terminal amino acid was coupled and the Fmoc group of this amino acid was removed, 3 equivalents each of N,N-diisopropylcarbodiimide, 1-hydroxy-7-azabenzotriazole (HOAt), and chloroacetic acid were added and reacted together in 1-methyl-2-pyrrolidinone.

A produced peptide resin was washed three times with 1-methyl-2-pyrrolidinone and three times with dichloromethane, followed by drying. 2 mL of trifluoroacetic acid/ethanedithiol/triisopropylsilane/water (92.5:2.5:2.5:2.5 by volume) was added, and the mixture was stirred at room temperature for 3 hours.

A crude peptide cleaved was recovered by ether precipitation, washed twice with tert-butyl methyl ether, dried, and then dissolved in dimethylsulfoxide (8 mL). After addition of n-propylamine (80 µL), the mixture was left to stand overnight. Further, after addition of acetic acid (80 µL), the reaction solution was concentrated using V10 (produced by Biotage Japan) and purified by reverse-phase high performance liquid chromatography using Kinetex 5u XB-C18 (150 × 21.2 mm, produced by Phenomenex) to yield the product of interest. The mobile phase used was water/acetonitrile containing 0.1% trifluoroacetic acid. A fraction of the product of interest was lyophilized to yield the product of interest as a trifluoroacetic acid salt.

### Synthesis Procedure 2

2-Chlorotrityl chloride resin (100 - 200 mesh) and the equivalent of 88 µmol of 1% DVB (produced by MERCK) were added to a reaction vessel, and the mixture was suspended in 2 mL of dichloromethane to allow swelling of the resin. After suction filtration, 1.5 mL of 1.5 equivalents of amino acids protected with an Fmoc group supported on the resin in dichloromethane was added, followed by addition of 5 equivalents of N,N-diisopropylamine, and the mixture was stirred. After filtration, the resin was washed with a mixed solution of dichloromethane/methanol/N,N-diisopropylamine = 17/2/1. The subsequent amino acid elongation was carried out using the automatic synthesizer Syro II (produced by Biotage Japan) according to a common solid-phase synthesis method using a 9-fluorenylmethoxycarbonyl group (Fmoc group) as an α-amino group-protecting group. In 1-methyl-2-pyrrolidinone, 3 equivalents each of O-(7-azabenzotriazol-1-yl)-N,N,N',N'-tetramethyluronium hexafluorophosphate (HATU), N,N-diisopropylamine, and an Fmoc group-protected amino acid were added and reacted together.

Removal of Fmoc groups was carried out using 20% piperidine/1-methyl-2-pyrrolidinone.

After the N-terminal amino acid was coupled and the Fmoc group of this amino acid was removed, 3 equivalents each of N,N-diisopropylcarbodiimide, 1-hydroxy-7-azabenzotriazole (HOAt), and chloroacetic acid were added and reacted together in 1-methyl-2-pyrrolidinone.

A produced peptide resin was washed three times with 1-methyl-2-pyrrolidinone and three times with dichloromethane, followed by drying. 2 mL of trifluoroacetic acid/ethanedithiol/triisopropylsilane/water (92.5:2.5:2.5:2.5 by volume) was added, and the mixture was stirred at room temperature for 3 hours.

A crude peptide cleaved was recovered by ether precipitation, washed twice with tert-butyl methyl ether, dried, and then dissolved in dimethylsulfoxide (8 mL). After addition of n-propylamine (80 µL), the mixture was left to stand overnight. Further, after addition of acetic acid (80 µL), the reaction solution was concentrated using V10 (produced by Biotage Japan) and purified by reverse-phase high performance liquid chromatography using Kinetex 5u XB-C18 (150 × 21.2 mm, produced by Phenomenex) to yield the product of interest. The mobile phase used was water/acetonitrile containing 0.1% trifluoroacetic acid. A fraction of the product of interest was lyophilized to yield the product of interest as a trifluoroacetic acid salt.

### Synthesis Procedure 3

This procedure was carried out using the automatic synthesizer Syro II (produced by Biotage Japan) according to a common solid-phase synthesis method using a 9-fluorenylmethoxycarbonyl group (Fmoc group) as an α-amino group-protecting group.

The equivalent of 88 µmol of Rink Amide Resin AM (produced by Novaviochem) was added to a reaction vessel, and 3 equivalents each of O-(7-azabenzotriazol-1-yl)-N,N,N',N'-tetramethyluronium hexafluorophosphate (HATU), N,N-diisopropylamine, and an Fmoc group-protected amino acid or PEG O11A were added and reacted together in 1-methyl-2-pyrrolidinone.

Removal of Fmoc groups was carried out using 20% piperidine/1-methyl-2-pyrrolidinone.

After O11A was introduced to the α-amino group of Lys having a ε-amino group protected with a (4,4-dimethyl-2,6-dioxocyclohex-1-ylidene)-3-methylbutyl group (ivDde group), the ivDde group was deprotected according to the following method.

Cleavage of the ivDde group on the solid phase was carried out using 3% hydrazine/dimethylformamide. 1 mL of 3% hydrazine/dimethylformamide was added to 100 mg of peptide resin, and the mixture was stirred. This procedure was repeated three times to remove the ivDde group. Subsequently, the peptide chain was extended in the same manner as above.

After the N-terminal amino acid was coupled and the Fmoc group of this amino acid was removed, 3 equivalents each of N,N-diisopropylcarbodiimide, 1-hydroxy-7-azabenzotriazole (HOAt), and chloroacetic acid were added and reacted together in 1-methyl-2-pyrrolidinone.

A produced peptide resin was washed three times with 1-methyl-2-pyrrolidinone and three times with dichloromethane, followed by drying. 2 mL of trifluoroacetic acid/ethanedithiol/triisopropylsilane/water (92.5:2.5:2.5:2.5 by volume) was added, and the mixture was stirred at room temperature for 3 hours.

A crude peptide cleaved was recovered by ether precipitation, washed twice with tert-butyl methyl ether, dried, and then dissolved in dimethylsulfoxide (8 mL). After addition of n-propylamine (80 µL), the mixture was left to stand overnight. Further, after addition of acetic acid (80 µL), the reaction solution was concentrated using V10 (produced by Biotage Japan) and purified by reverse-phase high performance liquid chromatography using Kinetex 5u XB-C18 (150 × 21.2 mm, produced by Phenomenex) to yield the product of interest. The mobile phase used was water/acetonitrile containing 0.1% trifluoroacetic acid. A fraction of the product of interest was lyophilized to yield the product of interest as a trifluoroacetic acid salt.

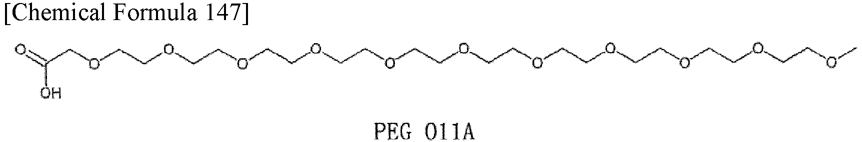

### Synthesis Procedure 4

First, 5 µmol of the peptide was dissolved in 1.4 mL of acetic acid. 300 µL of 25 mg/mL iodine/acetic acid solution was added thereto, and the mixture was stirred. After the disappearance of the raw materials was confirmed by HPLC, 300 µL of an aqueous 200 mg/mL ascorbic acid solution was added to stop the reaction. The reaction solution was concentrated using V10 (produced by Biotage Japan) and purified by reverse-phase high performance liquid chromatography using Kinetex 5u XB-C18 (150 × 21.2 mm, produced by Phenomenex) to yield the product of interest. The mobile phase used was water/acetonitrile containing 0.1% trifluoroacetic acid. A fraction of the product of interest was lyophilized to yield the product of interest as a trifluoroacetic acid salt.

### Synthesis Procedure 5

First, 1.15 equivalents of SGA was dissolved in 300 µL of dimethylformamide, and 1.15 equivalents of O-(N-succinimidyl)-N,N,N',N' -tetramethyluronium tetrafluoroborate and 4.6 equivalents of N,N-diisopropylethylamine were added thereto, followed by stirring for 1 hour. 5 µmol of the peptide was dissolved in 200 µL of dimethylformamide, and 4.6 equivalents of N,N-diisopropylethylamine were added. The reaction solution of SGA was added thereto, followed by stirring. After the disappearance of the raw materials was confirmed by HPLC, 18.5 µL of hydrazine monohydrate was added, and the mixture was stirred. After disappearance of the raw material was confirmed by HPLC, the reaction solution was purified by reverse-phase high performance liquid chromatography using Kinetex 5u XB-C18 (150 × 21.2 mm, produced by Phenomenex) to yield the product of interest. The mobile phase used was water/acetonitrile containing 0.1% trifluoroacetic acid. A fraction of the product of interest was lyophilized to yield the product of interest as a trifluoroacetic acid salt.

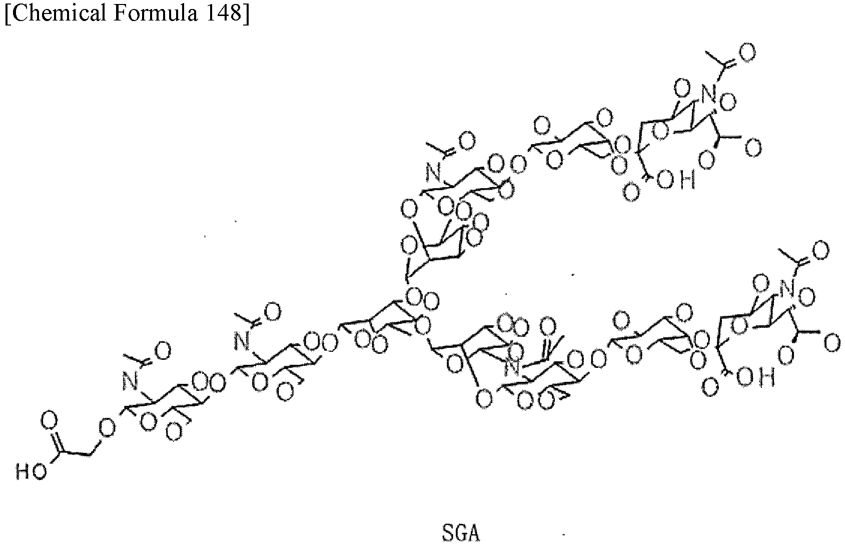

### Synthesis Procedure 6

First, 5 µmol of the peptide was dissolved in 250 µL of dimethylformamide. Next, 1.5 equivalents of N-succinimidyl 3-maleimidopropionate was dissolved in 400 µL of dimethylformamide, and the mixture was added to the peptide solution. Finally, 2 µL of N, N-diisopropylethylamine was added and reacted together. After disappearance of the raw material was confirmed by HPLC, the reaction solution was purified by reverse-phase high performance liquid chromatography using Kinetex 5u XB-C18 (150 × 21.2 mm, produced by Phenomenex) to yield the product of interest. The mobile phase used was water/acetonitrile containing 0.1% trifluoroacetic acid. A fraction of the product of interest was lyophilized to yield the product of interest as a trifluoroacetic acid salt.

### Synthesis Procedure 7

First, 5 µmol of the peptide was dissolved in 250 µL of dimethylformamide. Next, 1.5 equivalents of dibenzocyclooctyne-N-hydroxysuccinimidyl ester was dissolved in 800 µL of dimethylformamide, and the mixture was added to the peptide solution. Finally, 2 µL of N, N-diisopropylethylamine was added and reacted together. After disappearance of the raw material was confirmed by HPLC, the reaction solution was purified by reverse-phase high performance liquid chromatography using Kinetex 5u XB-C18 (150 × 21.2 mm, produced by Phenomenex) to yield the product of interest. The mobile phase used was water/acetonitrile containing 0.1% trifluoroacetic acid. A fraction of the product of interest was lyophilized to yield the product of interest as a trifluoroacetic acid salt.

### Synthesis Procedure 8

First, 10 mg/mL tris(2-carboxyethyl)phosphine (TCEP) hydrochloride solution (5% sorbitol/10 mM acetate buffer solution (pH 5.5)) (28 µL) was added to 10.2 mg/ml Fc solution (5% sorbitol/10 mM acetate buffer solution (pH 5.5) (1.2 ml) to reduce the disulfide bond at the hinge region. After completion of the reaction, unreacted TCEP was removed with NAP25 (produced by GE Healthcare) and 5% sorbitol/10 mM acetate buffer solution (pH 5.5). Then, a dimethyl sulfoxide solution of the maleimidated peptide (8 equivalents) was added and incubated at 30°C for 1 hour. The reaction solution was subjected to crude purification with NAP25 (produced by GE Healthcare) and 5% sorbitol/10 mM acetate buffer solution (pH 5.5). After the progress of the reaction was confirmed by hydrophobic interaction chromatography under the following conditions, buffer exchange was performed with Amicon Ultra-15 (30K) (produced by MERCK) to remove the unreacted peptide.

### Synthesis Procedure 9

First, 10 mg/mL tris(2-carboxyethyl)phosphine hydrochloride solution (5% sorbitol / 10 mM acetate buffer solution (pH 5.5)) (28 µL) was added to 10.2 mg/ml Fc solution (5% sorbitol/10 mM acetate buffer solution (pH 5.5)) (1.2 ml), to reduce the disulfide bond at the hinge region. After completion of the reaction, a dimethyl sulfoxide solution of the maleimidated peptide (8 equivalents) was added and incubated at 30°C for 1 hour. The reaction solution was subjected to crude purification with NAP25 (produced by GE Healthcare) and 5% sorbitol/10 mM acetate buffer solution (pH 5.5). After the progress of the reaction was confirmed by hydrophobic interaction chromatography under the following conditions, buffer exchange was performed with Amicon Ultra-15 (30K) to remove the unreacted peptide.

### Synthesis Procedure 10

A dimethyl sulfoxide solution (1.93 ml) of a DBCO-modified peptide (7 equivalents) was added to a solution (5.00 mg/mL) (5% sorbitol/10 mM acetate buffer solution (pH 5.5)) (7.74 ml) containing Fc with an added SG-type N297-linked glycan (where an azide group was introduced to sialic acid at the non-reducing terminus), which had been subjected to glycan remodeling according to WO 2018/003983, and incubated at 30°C for 16 hours. The reaction solution was subjected to crude purification with NAP25 (produced by GE Healthcare) and 5% sorbitol/10 mM acetate buffer solution (pH 5.5). After the progress of the reaction was confirmed by hydrophobic interaction chromatography under the following conditions, buffer exchange was performed with Amicon Ultra-15 (30K) to remove the unreacted peptide. It should be noted that he N297-linked glycan of the Fc-containing molecule in the conjugate obtained by Synthesis Procedure 10 is N297-(Fuc)SG.

### Synthesis Procedure 11

First, 44 µmol of the peptide was dissolved in 29.17 mL of water. 292 mg of silver acetate was added thereto, and the mixture was stirred at room temperature for 3 hours. After the disappearance of the raw materials was confirmed by LC/MS, 40.44 mL of a water/acetonitrile (1:1) mixed solution containing 10 mg/mL (±)-dithiothreitol and 0.1% trifluoroacetic acid was added to stop the reaction. The resulting suspension was transferred to a Falcon tube and centrifuged (2500 rpm, 5 min). After recovery of the supernatant, the precipitate was resuspended in 10 mL of a water/acetonitrile (1:1) mixed solution containing 0.1% trifluoroacetic acid and centrifuged (2500 rpm, 5 min) to recovery the supernatant. This washing and recovery operation was performed once again. The recovered supernatants were collectively lyophilized, and the resulting crude product was subjected to medium-pressure reversed-phase flash chromatography to purify the product of interest. The column used was Universal Column ODS-SM (50 µm, 3.0 × 16.5 cm, 37 g, Yamazen Corporation), and the mobile phase used was water/acetonitrile containing 0.1% trifluoroacetic acid. A fraction of the product of interest was lyophilized to yield the product of interest as a trifluoroacetic acid salt.

### Synthesis Procedure 12

First, 5 µmol of the peptide was dissolved in 5.24 mL of a water/acetonitrile (1:1) mixed solution. 14.5 µL of triethylamine was added thereto, and the mixture was stirred at room temperature for 2 days. After the disappearance of the raw materials was confirmed by LC/MS and HPLC, the reaction solution was directly purified by medium-pressure reversed-phase flash chromatography. The column used was Universal Column ODS-SM (50 µm, 3.0 × 16.5 cm, 37 g, Yamazen Corporation), and the mobile phase used was water/acetonitrile containing 0.1% trifluoroacetic acid. A fraction of the product of interest was lyophilized to yield the product of interest as a trifluoroacetic acid salt.

### Synthesis Procedure 13

First, 6 µmol of the peptide was dissolved in a mixed solution of 5.95 mL of water and 1.19 mL of tetrahydrofuran. 16.5 µL of triethylamine and 3.8 µL of diiodomethane were added thereto, and the mixture was stirred at room temperature for 7 hours. After the disappearance of the raw materials was confirmed by LC/MS and HPLC, the reaction solution was lyophilized. The resulting crude product was subjected to medium-pressure reversed-phase flash chromatography to purify the product of interest. The column used was Universal Column ODS-SM (50 µm, 3.0 × 16.5 cm, 37 g, Yamazen Corporation), and the mobile phase used was water/acetonitrile containing 0.1% trifluoroacetic acid. A fraction of the product of interest was lyophilized to yield the product of interest as a trifluoroacetic acid salt.

### Synthesis Procedure 14

First, 9 µmol of the peptide was dissolved in 8.96 mL of an aqueous 50 mM ammonium bicarbonate solution. 3.4 mg of 1,3-dichloroacetone was dissolved in 1 mL of DMF and added to the peptide solution, and the mixture was stirred at room temperature for 3 hours. After the disappearance of the raw materials was confirmed by LC/MS, the reaction solution was lyophilized. The resulting crude product was subjected to medium-pressure reversed-phase flash chromatography to purify the product of interest. The column used was Universal Column ODS-SM (50 µm, 3.0 × 16.5 cm, 37 g, Yamazen Corporation), and the mobile phase used was water/acetonitrile containing 0.1% trifluoroacetic acid. A fraction of the product of interest was lyophilized to yield the product of interest as a trifluoroacetic acid salt.

### LC-MS condition 1

The LC-MS analysis was performed using Agilent 6530 Accurate-Mass Q-TOF LC/MS (produced by Agilent Technology).
Column: Kinetex 1.7u XB-C18 (50 × 2.1 mm)
Mobile phase: A: 0.1% TFA/water, B: 0.1% TFA/acetonitrile
Temperature: 40°C
Flow rate: 0.6 mL/min.
Gradient: 0.01 min. (B 10%), 0.33 min. (B 10%), 0.34 min. (B 20%), 0.66 min. (B 20%), 0.67 min. (B 30%), 0.99 min. (B 30%), 1.00 min. (B 40%), 1.33 min. (B 40%), 1.34 min. (B 50%), 1.66 min. (B 50%), 1.67 min. (B 80%), 2.50 min. (B 80%)

### LC-MS condition 2

The LC-MS analysis was performed using LC: Ultimate 3000 (produced by Thermo Fisher) and MS: Q-Exactive (produced by Thermo Fisher). Before the analysis, 8M-Guanidine-HCl was added in the same amount as the sample solution, reacted at 37°C for 30 minutes, and then measured.
Column: PLRP-S 1000A 8 µm 2.1 × 50 mm (Agilent)
Mobile phase: A: H2O (0.02% TFA, 0.1% HCO2H), B: Acetonitrile
Temperature: 60°C
Flow rate: 0.6 mL/min.
Gradient: 0 min. (B 20%), 4 min. (B 50%), 4.2 min. (B 50%)

### LC-MS condition 3

The LC-MS analysis was performed using LC: Ultimate 3000 (produced by Thermo Fisher) and MS: Q-Exactive (produced by Thermo Fisher). Before the analysis, 8M-Guanidine-HCl and TCEP (final concentration: 10 mM) were added in the same amount as the sample solution, reacted at 37°C for 30 minutes, and then measured.
Column: PLRP-S 1000A 8 µm 2.1 × 50 mm (Agilent)
Mobile phase: A: H2O (0.02% TFA, 0.1% HCO2H), B: Acetonitrile
Temperature: 60°C
Flow rate: 0.6 mL/min.
Gradient: 0 min. (B 20%), 4 min. (B 50%), 4.2 min. (B 50%)

### LC-MS condition 4

The LC-MS analysis was performed using LC: Ultimate 3000 (produced by Thermo Fisher) and MS: Q-Exactive (produced by Thermo Fisher). Before the analysis, 4 uL of Rapid PNGaseF buffer (non-reducing format) was added to 16 ul of the sample solution, and the mixture was reacted at 90°C for 3 minutes. Subsequently, 1 uL of Rapid PNGaseF was added, reacted at 50°C for 10 minutes, and then measured.
Column: PLRP-S 1000A 8 µm 2.1 × 50 mm (Agilent)
Mobile phase: A: H2O (0.02% TFA, 0.1% HCO2H), B: Acetonitrile
Temperature: 60°C
Flow rate: 0.6 mL/min.
Gradient: 0 min. (B 20%), 4 min. (B 50%), 4.2 min. (B 50%)

### [Test Example 1]

### Cell adhesion inhibition assay

### 1. Materials and reagents

- 384-well Low Flange Black Flat Bottom Polystyrene Not Treated Microplate (Corning, 3573)
- Proteosave(registered trademark) SS 15 mL (Sumitomo Bakelite, MS-52150)
- Thrombospondin human platelet (Calbiochem, 605225)
- HUVEC (KURABO, KE-4109P10)
- EGM-2 MV (Lonza, CC-3202): for HUVEC passaging
- Collagen coated dish (IWAKI, 4020-010): for HUVEC passaging
- Lipidure(registered trademark)-BL802 (NOF Corporation), 5% solution
- DMEM low glucose (GIBCO, 11054-020)
- Bovine serum albumin (BSA) solution 30% (Sigma A9205)
- CellTiter-Glo 2.0 (Promega G9242)

### 2. Assay

### <Plate coat>

Thrombospondin 1 vial (25 µg) was dissolved in Tris-buffered saline (TBS) (+2 mM CaCl₂; the same shall apply hereunder) to prepare a 200 µg/mL solution, which was diluted with TBS to a concentration of 10 µg/mL in Proteosave(registered trademark) 15 mL tube. The 10 µg/mL solution was dispensed into a 384 well plate at a volume of 12 µL/well, and the plate was left to stand at 4°C overnight.

### <Plate blocking>

Lipidure(registered trademark) was diluted 10-fold with TBS to prepare a 0.5% solution. After a buffer was discarded from the plate that had been coated at the step of <Plate coat> as described above, the 0.5% solution was dispensed into all wells to be used at a volume of 25 µL/well, and the plate was left to stand at room temperature for 1 hour.

### <Sample dilution>

A 0.2 µM solution of each sample was prepared using a dilution buffer (DMEM + 0.5% BSA). 2 µL of the 0.2 µM solution was diluted with 500 µL of dilution buffer to prepare a diluted sample. As a vehicle control, DMSO was diluted in the same way as the samples.

### <Sample dispensation>

A blocking solution was discarded from the plate that had been treated at the step of <Plate blocking> as described above, and the plate was washed three times with 50 µL/well of TBS. The washing solution used for the third washing was removed, and the diluted sample was dispensed into the plate at a volume of 10 µL/well. Then, the plate was left to stand at room temperature for 15 to 30 minutes.

### <HUVEC seeding>

To maintain human umbilical vein endothelial cells, HUVECs, EGM2-MV and a collagen coated dish were used (the cells were ceased to be used after 10th or 11th passage since the cell proliferation rate declined at this stage). The cells were dispersed with 0.05% Trypsin/EDTA, harvested in DMEM/0.5% BSA, and centrifuged at 1000 rpm for 3 minutes. After removal of supernatant, the cells were resuspended in a DMEM buffer, counted for cell number, and diluted to 6000 cells/10 µL. The cell suspension was dispensed at a volume of 10 µL/well into the plate into which the sample had been dispensed, mixed gently, and incubated at 37°C for 2.5 hours.

### <Washing and detection>

The culture medium was removed, and the plate was washed twice with 30 µL/well of HBSS/Hepes 0.5% BSA. 20 µL/well of CellTiter-Glo 2.0(registered trademark) diluted 2-fold with HBSS/Hepes 0.5% BSA was dispensed at a volume of 20 µL/well, and the solution was stirred and left to stand at room temperature for 15 minutes. Fluorescence emission was detected using EnVision Xcite Multilabel Reader (produced by Perkin Elmer).

### 3. Results

On the basis of the measured fluorescence values, the IC50 values (nM) of the different cyclic peptides and conjugates for inhibition of cell adhesion were calculated. It should be noted that two cyclic peptide-conjugated Fc-containing molecules were cross-linked by a disulfide bond to form one molecule in the glycan conjugates Conjugate-61 to Conjugate-97, whereas the Fc-containing molecules (monomers) were not cross-linked to each other in the maleimide conjugates Conjugate-1 to Conjugate-60. Therefore, the molar concentrations of the maleimide conjugates in the calculation of IC50 values (nM) were determined by doubling molecular weights of the monomers for comparison with the glycan conjugates. Since each maleimide conjugate exists as a mixture in which various glycans are heterogeneously bonded to Fc-containing molecules, calculations were performed using the molecular weights of the deglycosylated products.

The obtained results are shown in Tables 2 and 11 to 14. The test results given above demonstrated that Peptide-1 to Peptide-12, Peptide-115, Peptide-119, Peptide-125 to Peptide-139, and Conjugate-1 to Conjugate-97 have the ability to block the adhesion of TSP1 to vascular endothelial cells and are useful for the treatment or prophylaxis of critical limb ischemia, peripheral arterial disease, or chronic limb threatening ischemia.

### [Test Example 2]

### Blood flow improvement measurement tests

Six to seven-week-old C57BL/6 mice were purchased from Charles River Laboratories Japan Inc., and acclimated over six days by feeding with a FR-2 solid diet (produced by Funabashi Farm Co., Ltd.). The rearing facility switched between light and dark modes in a 12-hour cycle with light turning on at 7 a.m. and turning off at 7 p.m. Under isoflurane inhalation anaesthesia, the left thighs of the mice were dissected, and the superficial femoral arteries and veins (from just below the deep femoral arteries to the popliteal artery and vein) were ligated and excised. After animal models were made, the test substances (Conjugate-4, Conjugate-10, Conjugate-27, Conjugate-34, Conjugate-43, Conjugate-56, Conjugate-63, Conjugate-64, and Conjugate-67) were each dissolved in a solution of 10 mM acetate buffer 5% sorbitol (pH 5.5), and administered intravenously at a single dose of 5 mL/kg. Five mice were used for each treatment group.

### (Test 1)

Vehicle-treated group
Conjugate-63 (0.3, 1, 3 mg/kg)-treated group
Duration of test: 20 days

### (Test 2)

Vehicle-treated group
Conjugate-64 (0.3, 1, 3 mg/kg)-treated group
Duration of test: 16 days

### (Test 3)

Vehicle-treated group
Conjugate-67 (0.3, 1 mg/kg)-treated group
Duration of test: 13 days

### (Test 4)

Vehicle-treated group
Conjugate-27 (1, 3 mg/kg)-treated group
Duration of test: 13 days

### (Test 5)

Vehicle-treated group
Conjugate-34 (0.3, 1 mg/kg)-treated group
Conjugate-43 (0.3, 1 mg/kg)-treated group
Duration of test: 15 days

### (Test 6)

Vehicle-treated group
Conjugate-56 (0.3, 1 mg/kg)-treated group
Conjugate-4 (0.3, 1 mg/kg)-treated group
Conjugate-10 (0.3, 1 mg/kg)-treated group
Duration of test: 14 days

Blood flows were measured in ischemic (left) and non-ischemic (right) limbs using a laser Doppler perfusion imager (PeriScan PIM III, Moor LD12-HR). The improvement of blood flow was evaluated by the ratio of ischemic limb flow to non-ischemic limb flow. Values are expressed as mean ± standard error of 5 measurements. In addition, blood flow AUC was calculated to determine the ratio (%) of the mean value of the test substance-treated group to the mean value of the vehicle-treated group.

Blood flow values from the mice were the lowest immediately after surgery and then recovered over time. As compared to vehicle treatment, treatment with the test substances investigated in the animal models of this study improved blood flow recovery after surgery. The results of blood flow (%) on the last day of measurement and blood flow AUC from the beginning to the end of measurement (vs. vehicle-treated group) are shown in Table 15.

**[Table 15]**

| | | %, ischemia / nonischemia | | | % change AUC (vs. vehicle) |
|---|---|---|---|---|---|
| Test 1 | Vehicle-treated group | 19.8 | ± | 3.2 | |
| | Conjugate-63 treated group (0.3 mg/kg) | 38.3 | ± | 9.9 | 201.4 |
| | Conjugate-63 treated group (1 mg/kg) | 54.6 | ± | 15.9 | 304.4 |
| | Conjugate-63 treated group (3 mg/kg) | 89.8 | ± | 13.9 | 371.4 |
| Test 2 | Vehicle-treated group | 22.2 | ± | 5.7 | |
| | Conjugate-64 treated group (0.3 mg/kg) | 43.2 | ± | 4.6 | 217.2 |
| | Conjugate-64 treated group (1 mg/kg) | 70.5 | ± | 3.7 | 319.2 |
| | Conjugate-64 treated group (3 mg/kg) | 67.6 | ± | 10.9 | 338.6 |
| Test 3 | Vehicle-treated group | 19.1 | ± | 4.6 | |
| | Conjugate-67 treated group (0.3 mg/kg) | 86.4 | ± | 21.3 | 244.4 |
| | Conjugate-67 treated group (1 mg/kg) | 75.5 | ± | 5.2 | 304.3 |
| Test 4 | Vehicle-treated group | 20.6 | ± | 2.5 | |
| | Conjugate-27 treated group (1 mg/kg) | 23.4 | ± | 4.4 | 110.2 |
| | Conjugate-27 treated group (3 mg/kg) | 41.8 | ± | 4.7 | 174.8 |
| Test 5 | Vehicle-treated group | 20.0 | ± | 2.5 | |
| | Conjugate-34 treated group (0.3 mg/kg) | 46.3 | ± | 4.9 | 199.0 |
| | Conjugate-34 treated group (1 mg/kg) | 71.9 | ± | 6.2 | 349.0 |
| | Conjugate-43 treated group (0.3 mg/kg) | 50.5 | ± | 4.2 | 227.0 |
| | Conjugate-43 treated group (1 mg/kg) | 70.7 | ± | 10.1 | 347.7 |
| Test 6 | Vehicle-treated group | 15.2 | ± | 3.0 | |
| | Conjugate-56 treated group (0.3 mg/kg) | 40.2 | ± | 5.6 | 187.6 |
| | Conjugate-56 treated group (1 mg/kg) | 74.8 | ± | 11.0 | 318.4 |
| | Conjugate-4 treated group (0.3 mg/kg) | 54.5 | ± | 10.2 | 263.9 |
| | Conjugate-4 treated group (1 mg/kg) | 76.1 | ± | 11.2 | 346.8 |
| | Conjugate-10 treated group (0.3 mg/kg) | 42.3 | ± | 7.2 | 241.8 |
| | Conjugate-10 treated group (1 mg/kg) | 51.6 | ± | 2.5 | 324.1 |

### [Test Example 3]

### Serum stability tests

### I. Materials and reagents

- Mouse serum (BALB/c CrSlc, male, Japan SLC, Inc.)
- Monkey serum (cynomolgus monkey, male, Japan SLC, Inc.)
- Human serum (Caucasian, pool of 5 males and 5 females, KAC Co., Ltd.)
- Phenacetin (Nacalai Tesque, Inc.)
- Niflumic acid (Nacalai Tesque, Inc.)
- Furosemide (Nacalai Tesque, Inc.)
- Acetonitrile (FUJIFILM Wako Pure Chemical Corporation)
- Methanol (FUJIFILM Wako Pure Chemical Corporation)
- Formic acid (FUJIFILM Wako Pure Chemical Corporation)
- Dimethyl sulfoxide (DMSO) (FUJIFILM Wako Pure Chemical Corporation)

### II. Evaluation

### <Internal standard (IS) solution>

Solutions of Phenacetin, niflumic acid, and furosemide were prepared in DMSO at 10 µg/ml, 150 µg/ml, and 200µg/ml, respectively. 10 µg/ml of phenacetin in DMSO, 150 µg/ml of niflumic acid in DMSO, and 200 µg/ml of furosemide in DMSO were dissolved in a mixture of acetonyl/methanol (75/25, v/v) (final concentrations: phenacetin; 1 ng/mL, niflumic acid: 15 ng/mL, and furosemide 20 ng/mL), and formic acid was added to the prepared test solution (final concentration: 1% (v/v)) to prepare a formic acid-containing IS solution.

### <Test substance solution>

The test substances were prepared in DMSO to a concentration of 10 mM each. Further, the solution was diluted with DMSO to a concentration of 2 mM to make a test substance solution.

### <Test>

Each serum was preincubated at 37°C for 5 minutes, and then the test substance solution was added to a final concentration of 20 µM to initiate the reaction. The test substance-added serum was incubated at 37°C. At 0 and 4 hours after the start of the reaction, a portion of the serum was taken out and added to the formic acid-containing IS solution to stop the reaction, which was used as a measurement sample.

### <Measurement>

The test substance and IS (phenacetin, niflumic acid, and furosemide) in the measurement sample were measured by LC-MS/MS, and the peak area ratio (IS ratio) of the test substance to IS (any of phenacetin, niflumic acid, and furosemide) was calculated. It should be noted that the IS with the most stable measured value was selected for each test.

### III. Results

The percentage of the IS ratio at 4 hours after the start of the reaction relative to that at 0 hours after the start of the reaction was used as a residual ratio to evaluate serum stability. The obtained results are shown in Table 16. The bicyclic peptides Peptide-1, Peptide-9, Peptide-11, Peptide-126 to Peptide-128, Peptide-130, and Peptide-131 all exhibited high serum stability.

Peptide-115 and Peptide-119 are monocyclic peptides having amino acid sequences that differ from Peptide-1 and Peptide-9, respectively, only in the residues at positions 3 and 8. Peptide-1 and Peptide-9 exhibited higher serum stability than Peptide-115 and Peptide-119, respectively. These results indicate that metabolic stability of the bicyclic peptides is enhanced compared to that of the monocyclic peptides, and also suggest that the bicyclic peptides have higher blood retention compared to the monocyclic peptides and are thus suitable for the production of more sustained medicaments.

**[Table 16]**

| **Peptide-** | Stability in mouse serum (At 4 hours) | Stability in monkey serum (At 4 hours) | Stability in human serum (At 4 hours) |
|---|---|---|---|
| **1** | **100.8** | **90.6** | **93.9** |
| **9** | **95.8** | **93** | **117.5** |
| **11** | **88** | **89.9** | **105.1** |
| **126** | **98.3** | **100.4** | **99.6** |
| **127** | **90** | **95.1** | **97.5** |
| **128** | **98.1** | **98.6** | **99** |
| **130** | **98** | **93.5** | **88** |
| **131** | **89.2** | **89.2** | **100.9** |
| **115** | **64.5** | **14.9** | **78.2** |
| **119** | **70.9** | **21.1** | **88.5** |

### INDUSTRIAL APPLICABILITY

The cyclic peptide I of the present invention can bind to TSP1 to block the adhesion of cells such as vascular endothelial cells to TSP1. Therefore, the cyclic peptide I or pharmaceutically acceptable salt thereof of this invention is useful for the treatment or prophylaxis of diseases or symptoms that can be treated or prevented by inhibiting a function of TSP1. Also, the cyclic peptide or pharmaceutically acceptable salt thereof of this invention is useful as a blood flow improving agent. Furthermore, the conjugate or pharmaceutically acceptable salt thereof of the present invention is useful in providing a means of treatment or prophylaxis of diseases or symptoms that can be treated or prevented by inhibiting a function of TSP1 at lower frequency of administration. The conjugate or pharmaceutically acceptable salts thereof of this invention is also useful as a blood flow improving agent.

### SEQUENCE LISTING FREE TEXT

SEQ ID Nos: 1 to 123: Peptide-1 to Peptide-123
SEQ ID No: 124: Nucleotide sequence encoding heavy chain of anti-LPS mAb-A
SEQ ID No: 126: Nucleotide sequence encoding light chain of anti-LPS mAb-A
SEQ ID No: 128: Nucleotide sequence encoding heavy chain of CLCH-A
SEQ ID No: 130: Nucleotide sequence encoding light chain of CLCH-A
SEQ ID No: 132: Nucleotide sequence encoding heavy chain of CLCH-B (LALA form)
SEQ ID No: 134: Nucleotide sequence encoding Fc fragment (wild type)
SEQ ID No: 136: Nucleotide sequence encoding Fc-A (LALA form)
SEQ ID No: 138: DNA fragment containing nucleotide sequence encoding Fc-C (LALA-PA form)
SEQ ID NOs: 140 to 144: Linker L1
SEQ ID NOs: 145 to 147: Linker L2a
SEQ ID No: 148: Polypeptide consisting of hinge, CH2 domain, and CH3 domain of human IgG heavy chain
SEQ ID No: 149: Peptide-124
SEQ ID Nos: 150 to 164: Peptide-125 to Peptide-139
SEQ ID No: 165: Nucleotide sequence encoding Fc-C (LALA-PA form)
SEQ ID No: 166: Amino acid sequence of Fc-C (LALA-PA form)

## Claims

1. A cyclic peptide represented by formula (I) 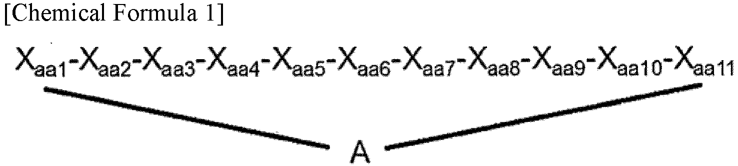 [wherein A is selected from ring-forming groups of the formulas: 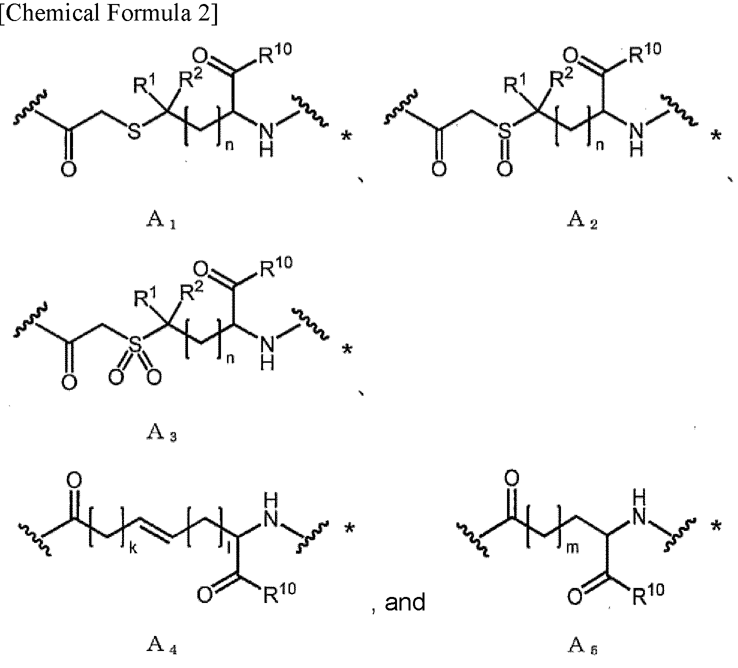 wherein 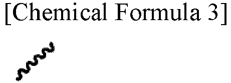
represents a point of attachment to the N-terminal amino group of Xₐₐ₁, 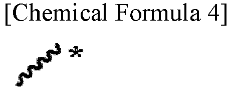
represents a point of attachment to the C-terminal carbonyl group of Xₐₐ₁₁,
R¹ and R² are each independently a hydrogen atom or C₁₋₃ alkyl,
R¹⁰ is an amino group or a hydroxy group,
n is an integer of 0 to 3,
k and l are each independently an integer of 0 to 3,
m is an integer of 1 to 7;
Xₐₐ₁ is a residue of an aromatic amino acid;
Xₐₐ₂ is a residue of an aromatic amino acid, a basic amino acid, or an aliphatic amino acid;
Xₐₐ₃ and Xₐₐ₈ each have a structure independently selected from a residue of an amino acid represented by formula (III) 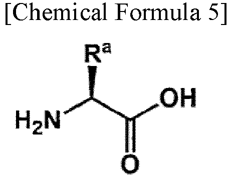
[wherein R^{a} is a group represented by the formula -(CH₂)ₜSH (wherein t is an integer of 1 to 3)] or formula (III') 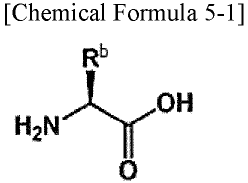
[wherein R^{b} is a group represented by the formula -CHᵥ(CH₃)₂₋ᵥCH_{w}(CH₃)_{2-w}SH (wherein v is an integer of 0 to 2; when v is 0 or 1, w is 2; and when v is 2, w is 0 or 1) or - CHₓ(CH₃)₂₋ₓSH (wherein x is 0 or 1)], in which thiol groups of Xₐₐ₃ and Xₐₐ₈ form a bond represented by -S-S-, -S-CH₂-S-, or -S-X-S- (wherein X is selected from 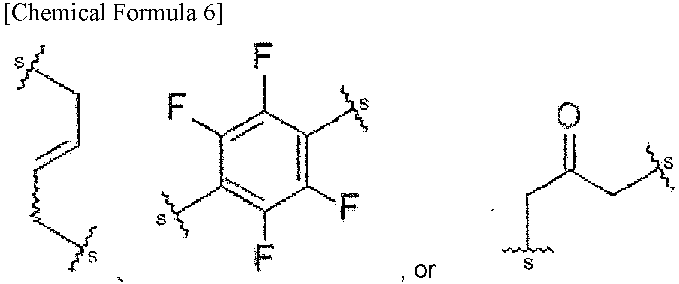
(wherein 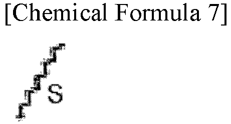
represents a point of attachment to S)), and at least one of Xₐₐ₃ and Xₐₐ₈ is a residue of the amino acid represented by formula (III);
Xₐₐ₄ is a residue of a neutral amino acid;
Xₐₐ₅ is a residue of a basic amino acid;
Xₐₐ₆ is a residue of a neutral amino acid or an acidic amino acid;
Xₐₐ₇ is a residue of an aromatic amino acid;
Xₐₐ₉ is a residue of an aromatic amino acid, an aliphatic amino acid, or a basic amino acid;
Xₐₐ₁₀ is a residue of an aromatic amino acid;
Xₐₐ₁₁ is a residue of an aliphatic amino acid;
wherein the aliphatic amino acid is an amino acid represented by formula (IIa) 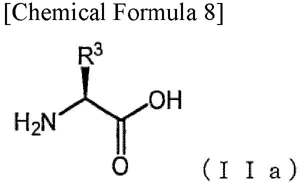
(wherein R³ is C₁₋₆ alkyl, C₂₋₆ alkenyl or C₃₋₆ cycloalkyl);
the aromatic amino acid is an amino acid represented by formula (IIb) 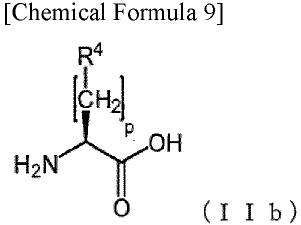
(wherein R⁴ is an aromatic group selected from phenyl, thienyl, pyridyl, naphthyl, indolyl, benzofuranyl, and benzothienyl, wherein the aromatic group may be substituted with one or more substituents independently selected from the group consisting of C₁₋₃ alkyl, halogen atoms, hydroxy, and C₁₋₃ alkoxy; and p is an integer of 0 to 3);
the basic amino acid is an amino acid represented by formula (IIc) 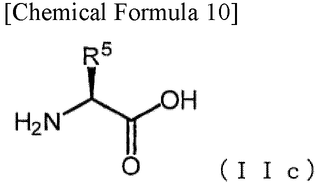
[wherein R⁵ is a group represented by
the formula -(CH₂)_{qa}NH₂ (wherein qa is an integer of 1 to 6),
the formula 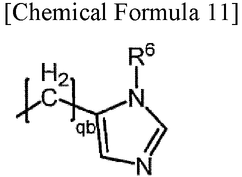
(wherein R⁶ is a hydrogen atom or C₁₋₃ alkyl, and qb is an integer of 1 to 6), the formula -(CH₂)_{qc}NHC(=NH)NH₂ (wherein qc is an integer of 1 to 6), or the formula 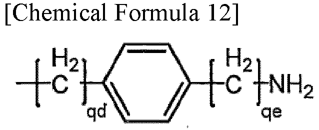
(wherein qd and qe are each independently an integer of 1 to 3)];
the neutral amino acid is an amino acid represented by formula (IId) 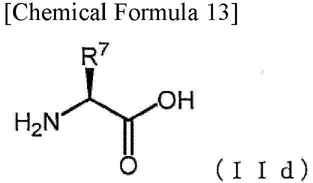
[wherein R⁷ is a group represented by the formula -(CH₂)ᵣₐNHCONH₂ (wherein ra is an integer of 1 to 6) or the formula -(CH₂)_{rb}SH (wherein rb is an integer of 1 to 3)], Gly, Met, Pro, 3HyP, Asn, Gln, Ser, ^{m}S, MS, or Thr;
the acidic amino acid is an amino acid represented by formula (IIe) 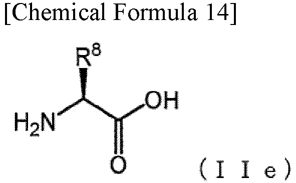
[wherein R⁸ is a group represented by the formula -(CH₂)ₛCOOH (wherein s is an integer of 1 to 6)]], or
a pharmaceutically acceptable salt thereof.

2. The cyclic peptide or pharmaceutically acceptable salt thereof according to claim 1, wherein
Xₐₐ₁ is a 2Nal residue;
Xₐₐ₂ is a residue of Arg, AGB, PHG, or Tle;
in the structure of Xₐₐ₃ and Xₐₐ₈, the residue of an amino acid represented by formula (III) or (III') is a residue of HCY or Cys;
Xₐₐ₄ is a Gly residue;
Xₐₐ₅ is a residue of Arg or AGB;
Xₐₐ₆ is a residue of Asn or Asp;
Xₐₐ₇ is a Trp residue;
Xₐₐ₉ is a residue of Val, Arg, PHG, 4PAL, or AGB;
Xₐₐ₁₀ is a Trp residue; and
Xₐₐ₁₁ is a residue of Val, CPTG, or CHXG.

3. The cyclic peptide or pharmaceutically acceptable salt thereof according to claim 1 or 2, wherein A is a ring-forming group

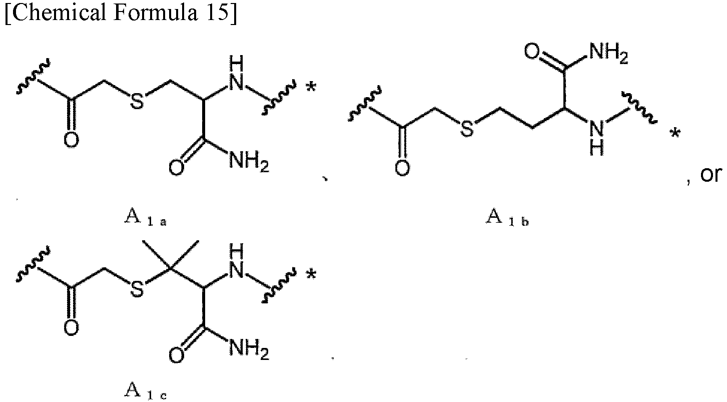

4. The cyclic peptide or pharmaceutically acceptable salt thereof according to claim 3, wherein A is a ring-forming group

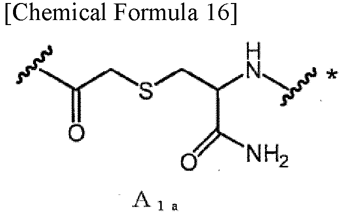

5. The cyclic peptide or pharmaceutically acceptable salt thereof according to any one of claims 1 to 4, wherein in the structure of Xₐₐ₃ and Xₐₐ₈, the thiol groups form the bond represented by -S-S- or -S-CH₂-S-.

6. The cyclic peptide or pharmaceutically acceptable salt thereof according to any one of claims 1 to 5, wherein the cyclic peptide or pharmaceutically acceptable salt thereof is selected from the group consisting of compounds represented by the formulas:

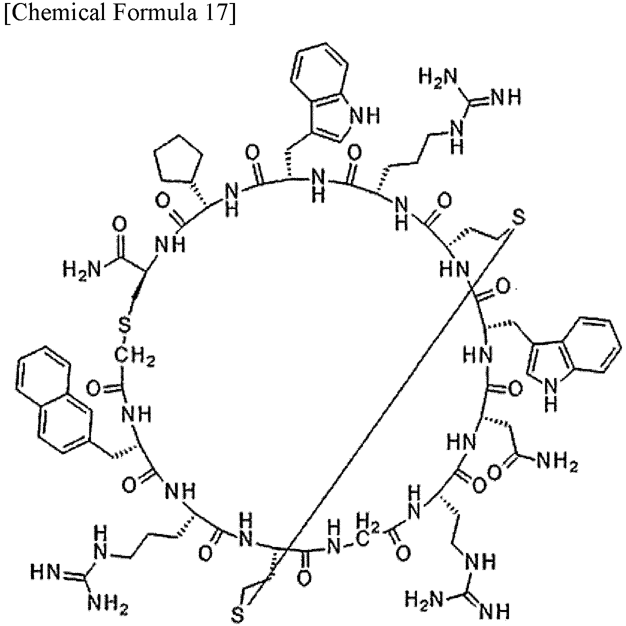

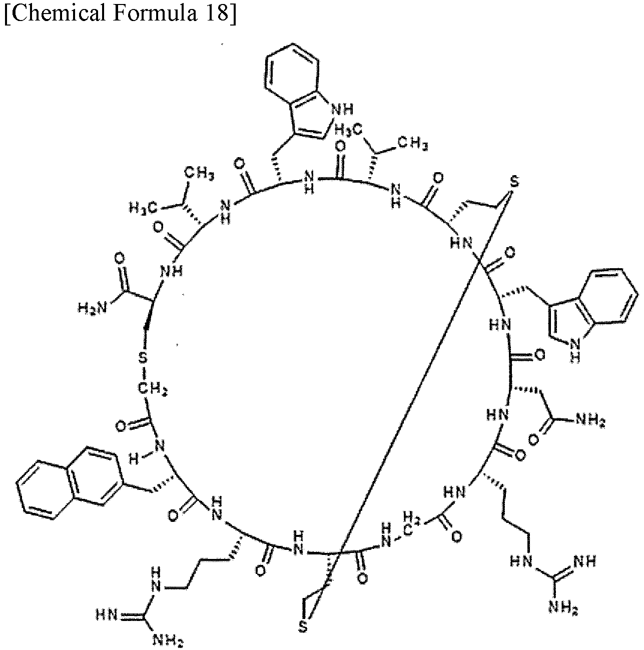

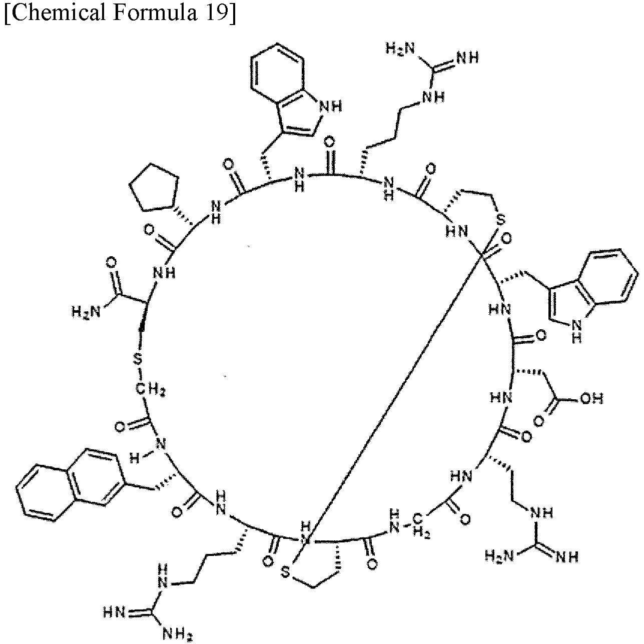

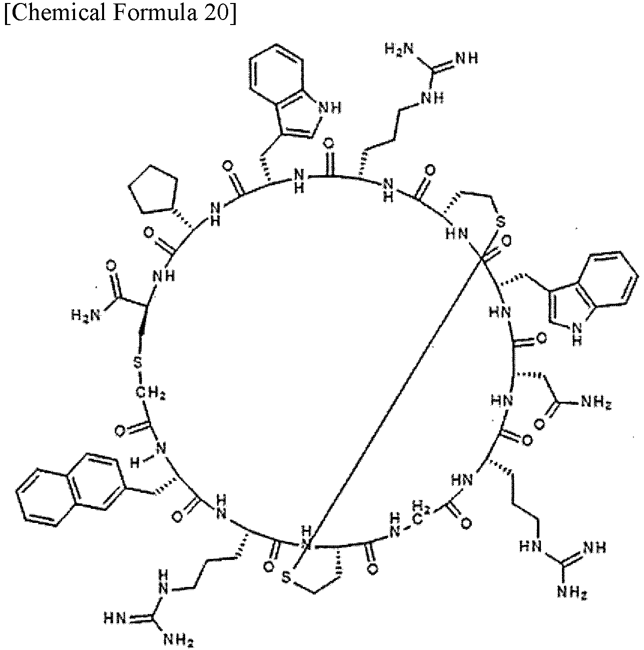

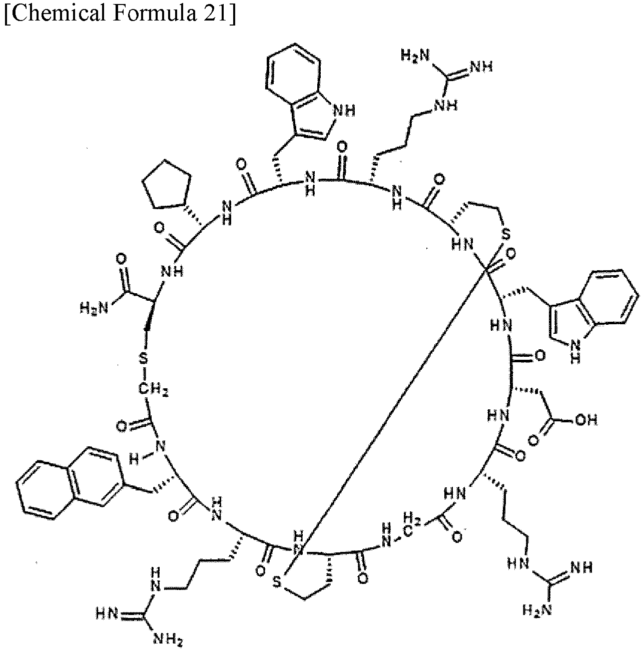

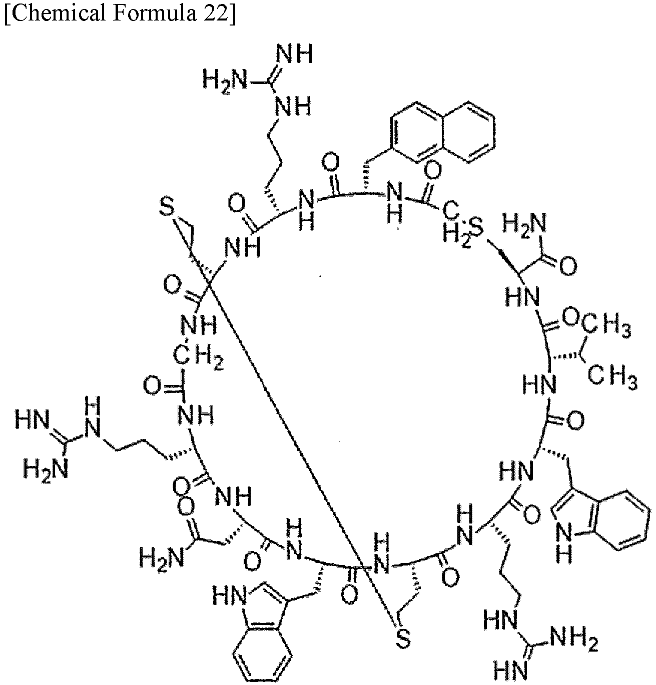

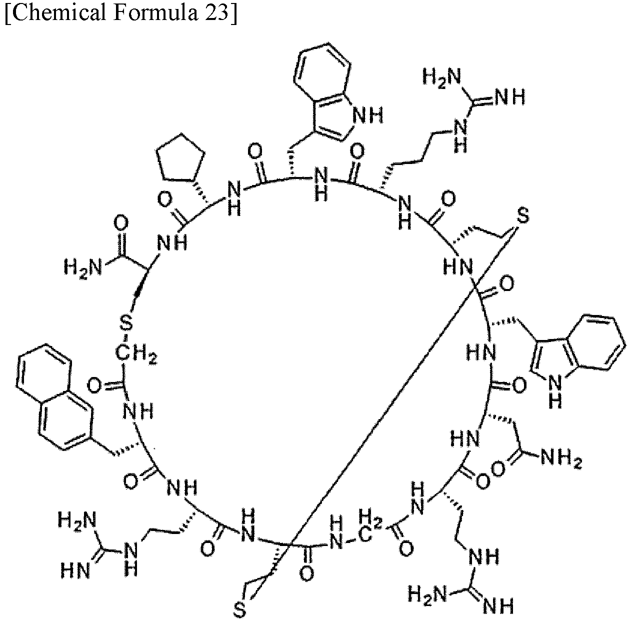

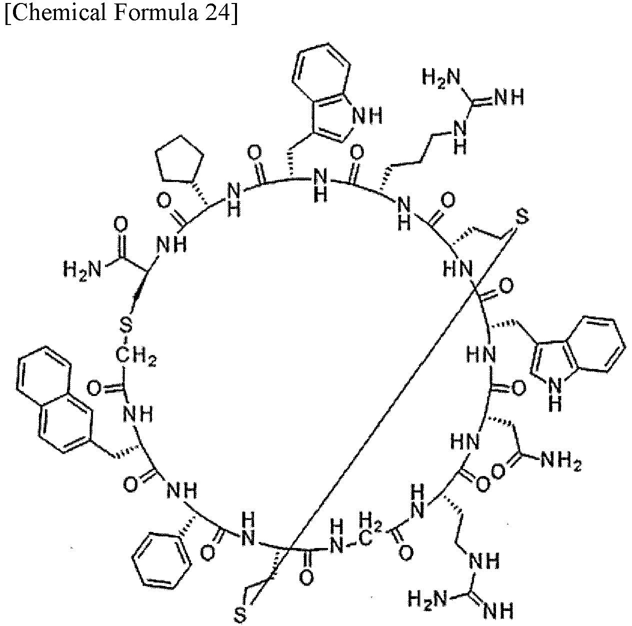

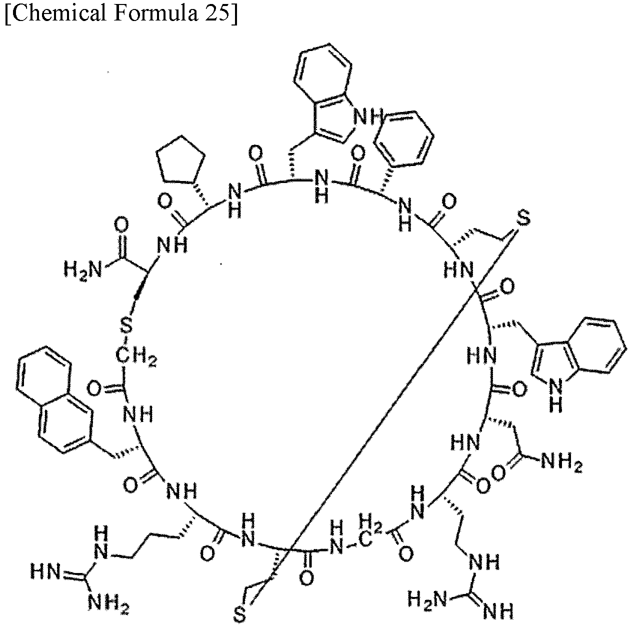

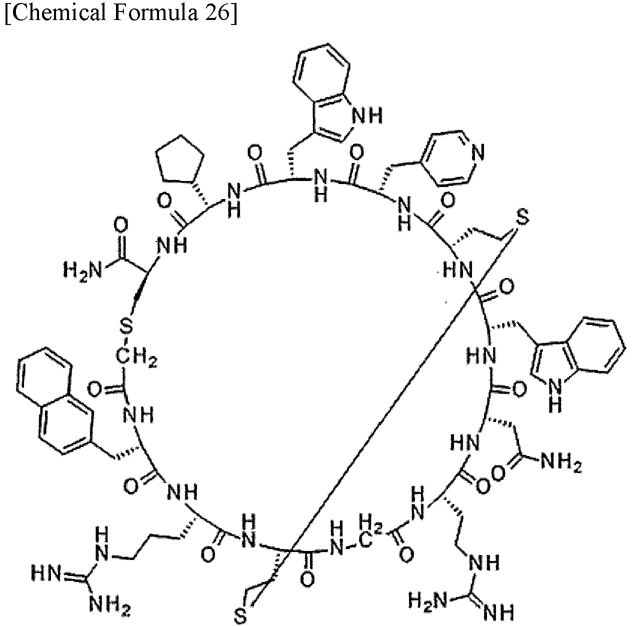

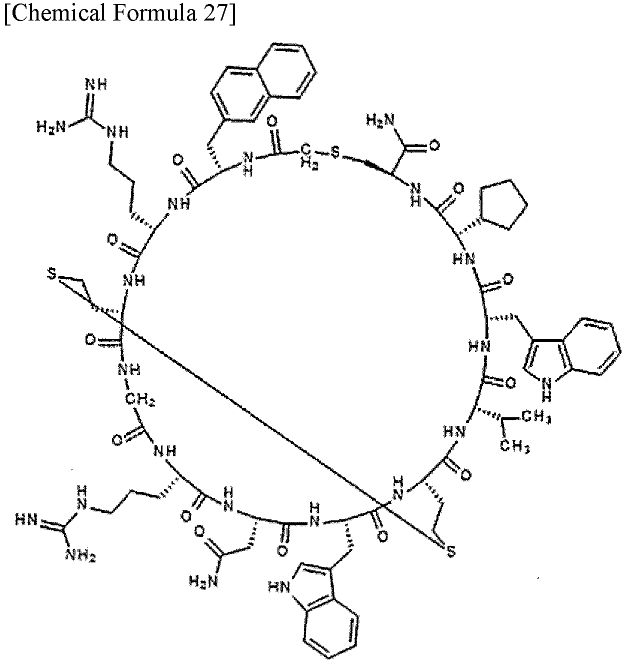

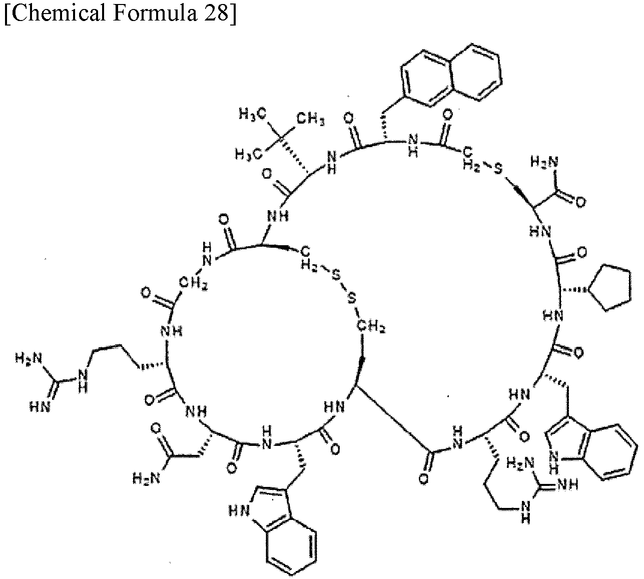

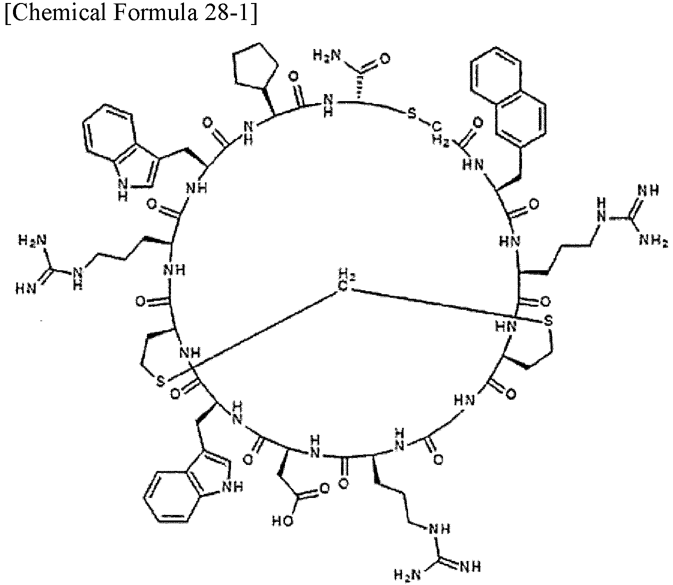

7. A pharmaceutical composition comprising, as an active component, the cyclic peptide or pharmaceutically acceptable salt thereof according to any one of claims 1 to 6.

8. The pharmaceutical composition according to claim 7, for use in the treatment or prophylaxis of a disease that can be treated or prevented by inhibiting a function of TSP1.

9. The pharmaceutical composition according to claim 8, for use in the treatment or prophylaxis of critical limb ischemia, peripheral arterial disease, or chronic limb threatening ischemia.

10. A blood flow improving agent comprising, as an active component, the cyclic peptide or pharmaceutically acceptable salt thereof according to any one of claims 1 to 6.

11. A conjugate comprising:
the cyclic peptide according to any one of claims 1 to 6, or
a cyclic peptide represented by formula (X) 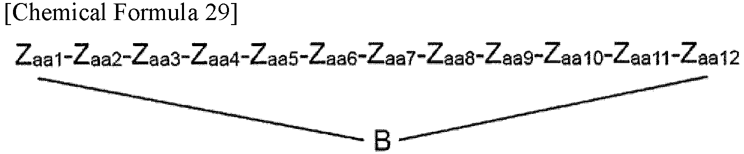
[wherein B is selected from the ring-forming groups 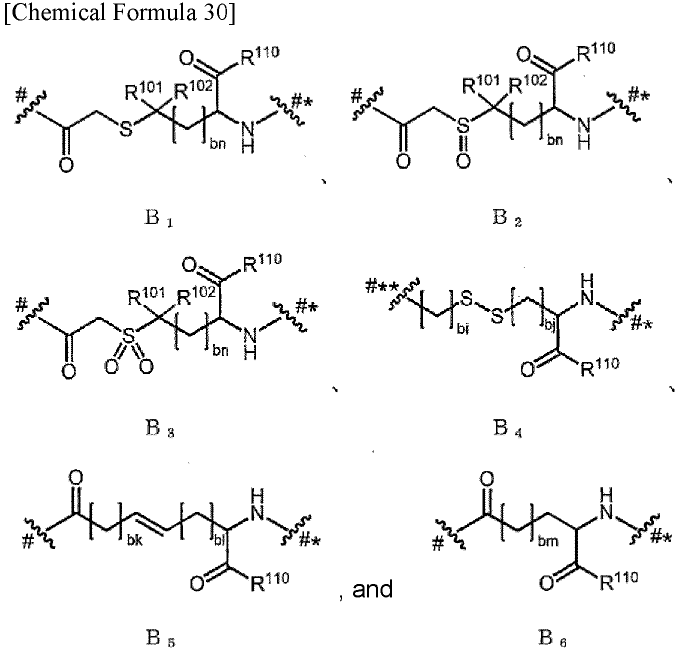
wherein 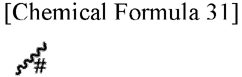
represents a point of attachment to the N-terminal amino group of Zₐₐ₁, or in the absence of Zₐₐ₁, represents a point of attachment to the N-terminal amino group of Zₐₐ₂, 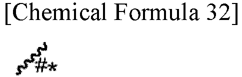
represents a point of attachment to the C-terminal carbonyl group of Zₐₐ₁₂, 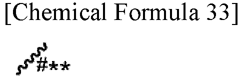
represents a point of attachment to α carbon of Zₐₐ₁, or in the absence of Zₐₐ₁, represents a point of attachment to α carbon of Zₐₐ₂,
R¹⁰¹ and R¹⁰² are each independently a hydrogen atom or C₁₋₃ alkyl,
R¹¹⁰ is amino or hydroxy,
bn is an integer of 0 to 3,
bi and bj are each independently an integer of 1 to 3,
bk and bl are each independently an integer of 0 to 3,
bm is an integer of 1 to 7;
Zₐₐ₁ is a residue of an aliphatic amino acid, an aromatic amino acid, a basic amino acid, a neutral amino acid, or an acidic amino acid, or is absent;
Zₐₐ₂ is a residue of an aromatic amino acid or a neutral amino acid;
Zₐₐ₃ is a residue of an aliphatic amino acid, an aromatic amino acid, or a basic amino acid;
Zₐₐ₄ is Ser, Thr, Ala, or ^{m}S;
Zₐₐ₅ is Gly or Ser;
Zₐₐ₆ is a residue of a basic amino acid or a neutral amino acid;
Zₐₐ₇ is a residue of a neutral amino acid or an acidic amino acid;
Zₐₐ₈ is a residue of an aromatic amino acid;
Zₐₐ₉ is a residue of an aliphatic amino acid, a neutral amino acid, or an aromatic amino acid;
Zₐₐ₁₀ is a residue of a basic amino acid, an aliphatic amino acid, an aromatic amino acid, or a neutral amino acid;
Zₐₐ₁₁ is a residue of an aromatic amino acid; and
Zₐₐ₁₂ is a residue of an aliphatic amino acid, an aromatic amino acid, or a basic amino acid,
wherein the aliphatic amino acid, the aromatic amino acid, the basic amino acid, the neutral amino acid, and the acidic amino acid are as defined in claim 1]; and
a carrier molecule bonded to the cyclic peptide, or
a pharmaceutically acceptable salt thereof.

12. The conjugate or pharmaceutically acceptable salt thereof according to claim 11, wherein
Zₐₐ₁ is a residue of Arg, Lys, His, Gly, Ala, Asn, Thr, Ser, Met, Leu, Ile, Val, Gln, Phe, Tyr, Trp, or Cys, or is absent;
Zₐₐ₂ is a residue of Phe, Tyr, Trp, 2Nal, 4CF, or DCF;
Zₐₐ₃ is a residue of Ile, Leu, Nle, Tle, Trp, 2Nal, 4CF, or Arg;
Zₐₐ₄ is a Ser residue;
Zₐₐ₅ is a Gly residue;
Zₐₐ₆ is a residue of Arg, Lys, His, Ser, Cit, or AGB;
Zₐₐ₇ is a residue of Asn or Asp;
Zₐₐ₈ is a residue of Trp or 2Nal;
Zₐₐ₉ is a residue of Val, Nle, Ahp, or Met;
Zₐₐ₁₀ is a residue of Arg, AGB, Lys, His, AMF, Phg, or Val;
Zₐₐ₁₁ is a residue of Trp or 2Nal;
Zₐₐ₁₂ is a residue of Val, Tle, CPTG, CHXG, or Phe; and
B is a ring-forming group

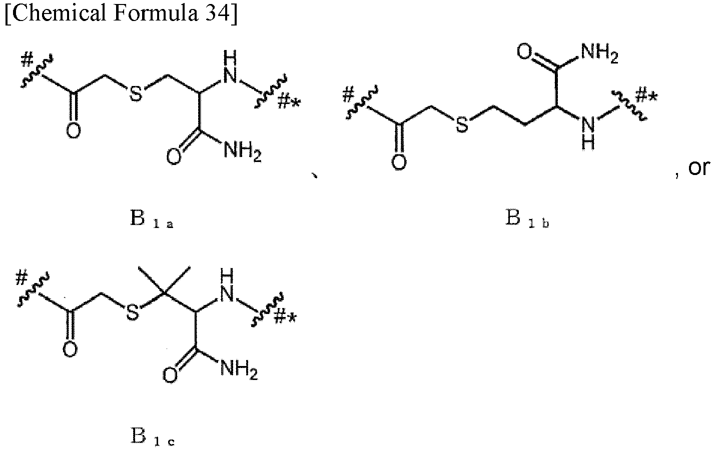

13. The conjugate or pharmaceutically acceptable salt thereof according to claim 12, wherein
Zₐₐ₁ is absent;
Zₐₐ₂ is a 2Nal residue;
Zₐₐ₃ is a residue of Arg or Tle;
Zₐₐ₄ is a Ser residue;
Zₐₐ₅ is a Gly residue;
Zₐₐ₆ is a residue of Arg or AGB;
Zₐₐ₇ is a residue of Asn or Asp;
Zₐₐ₈ is a Trp residue;
Zₐₐ₉ is a residue of Val or Nle;
Zₐₐ₁₀ is a residue of Arg, AGB, or Val; Zₐₐ₁₁ is a Trp residue;
Zₐₐ₁₂ is a residue of Val, CPTG, or CHXG; and
B is

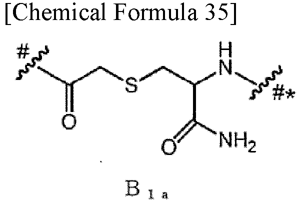

14. The conjugate or pharmaceutically acceptable salt thereof according to claim 13, wherein the cyclic peptide represented by formula (X) is selected from the group consisting of compounds represented by the formulas: 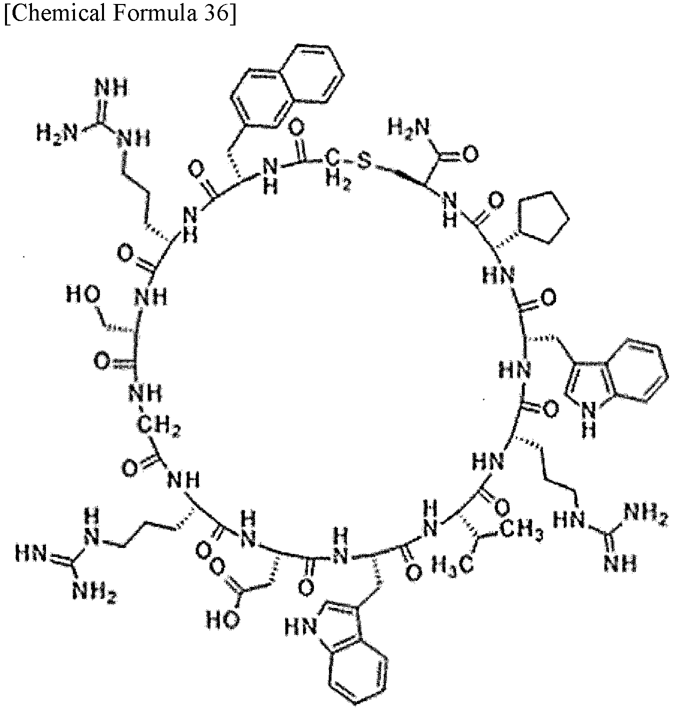 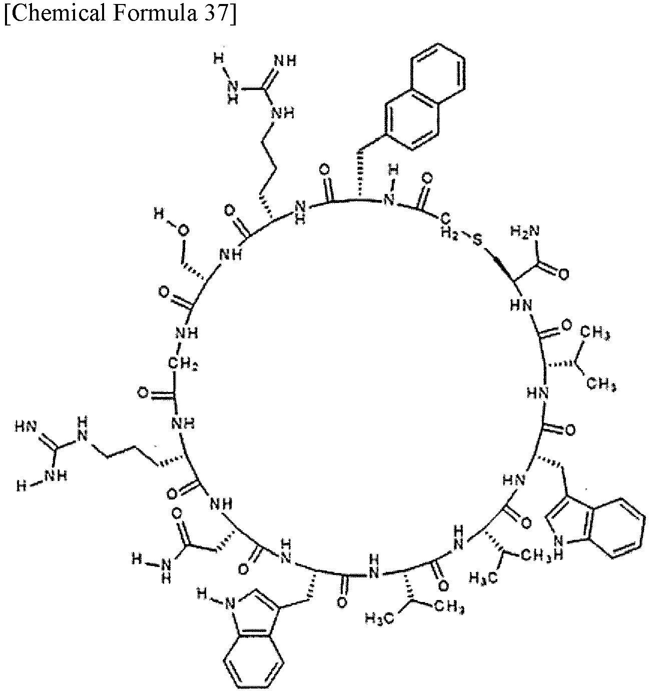 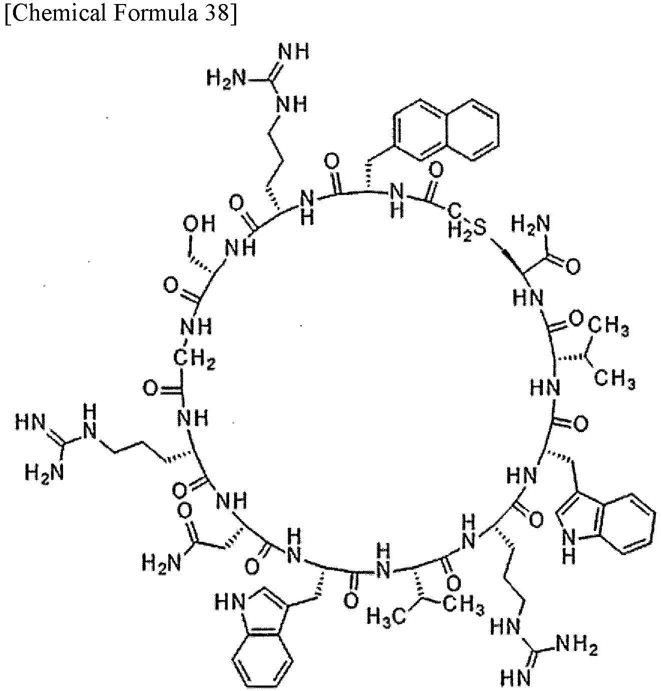 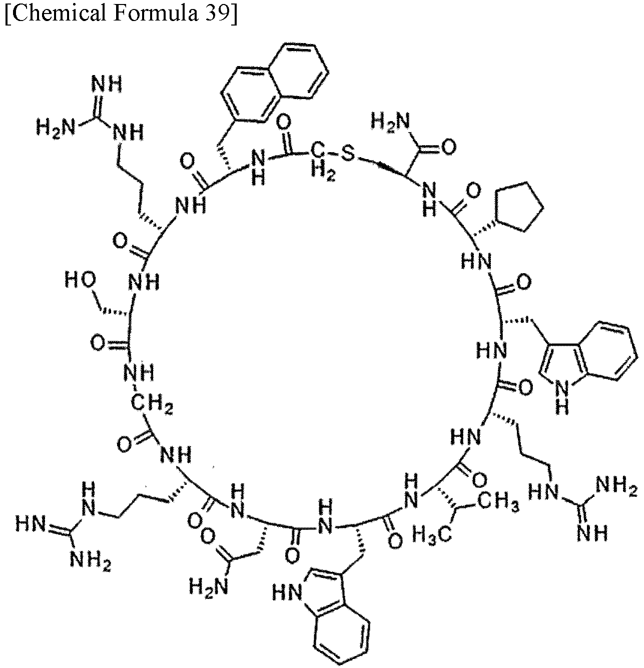 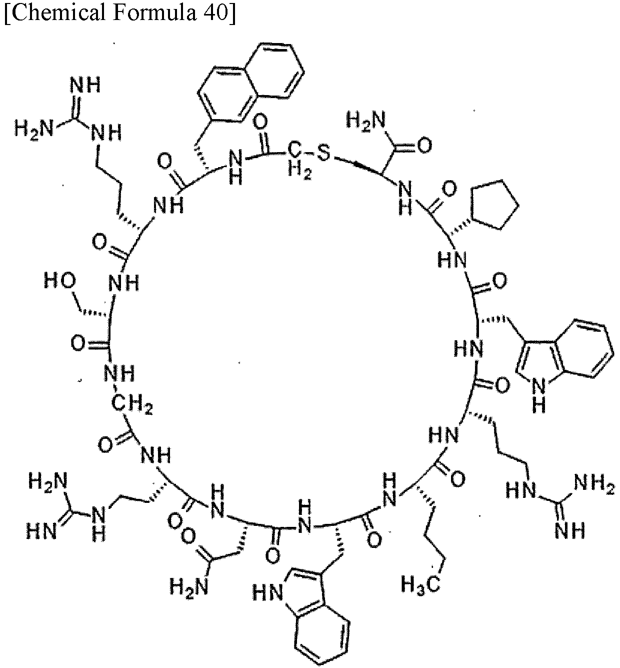 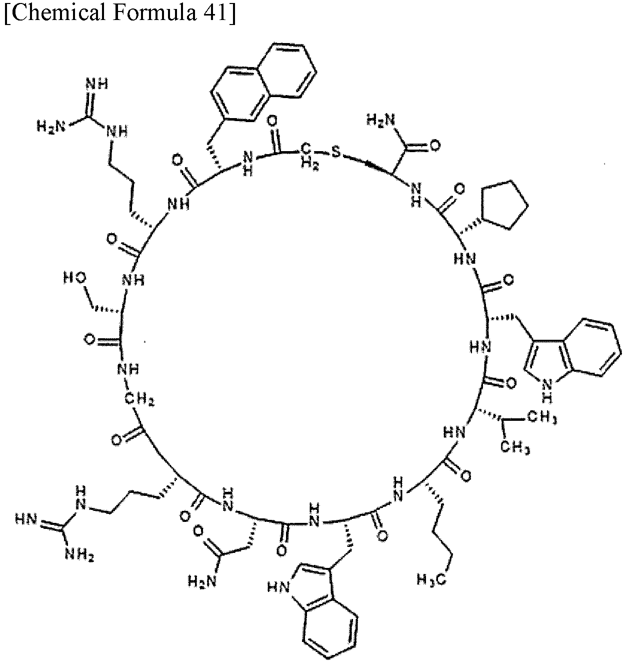 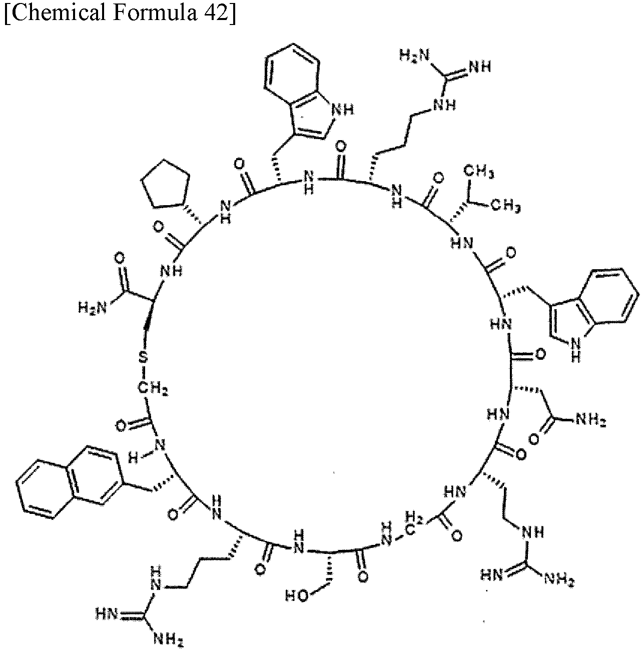 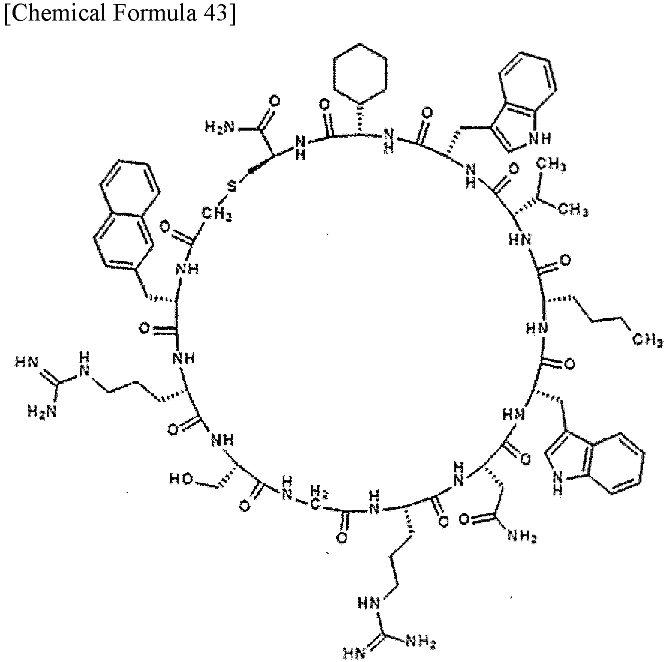 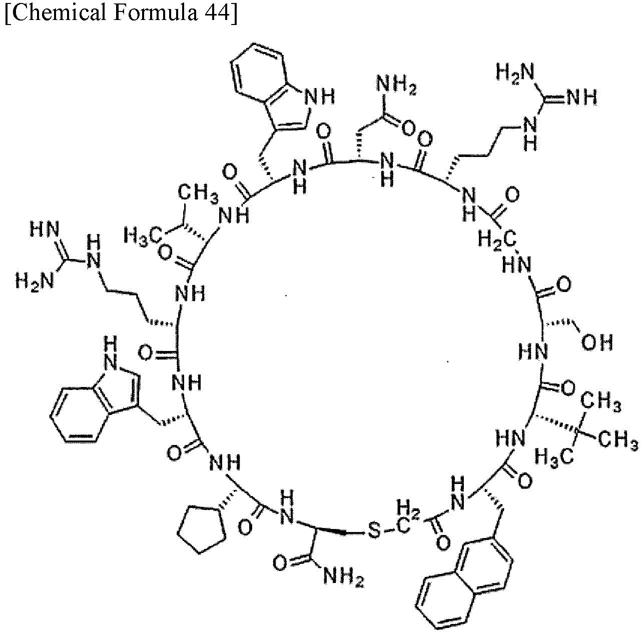 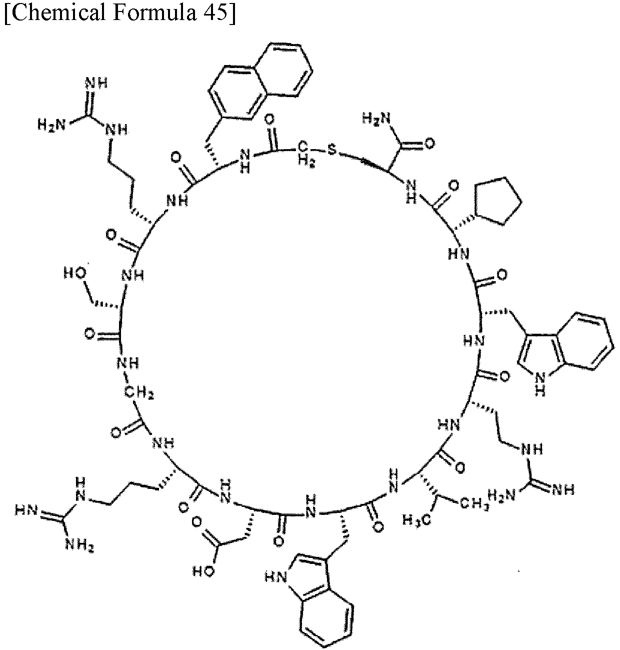 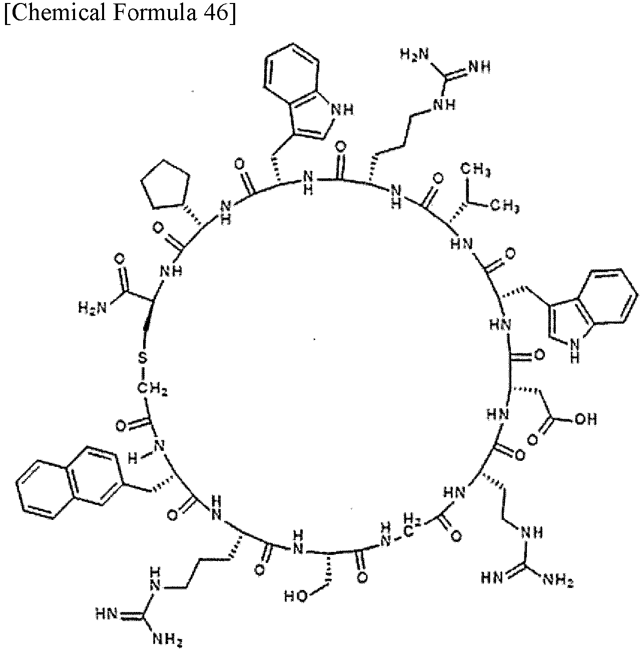 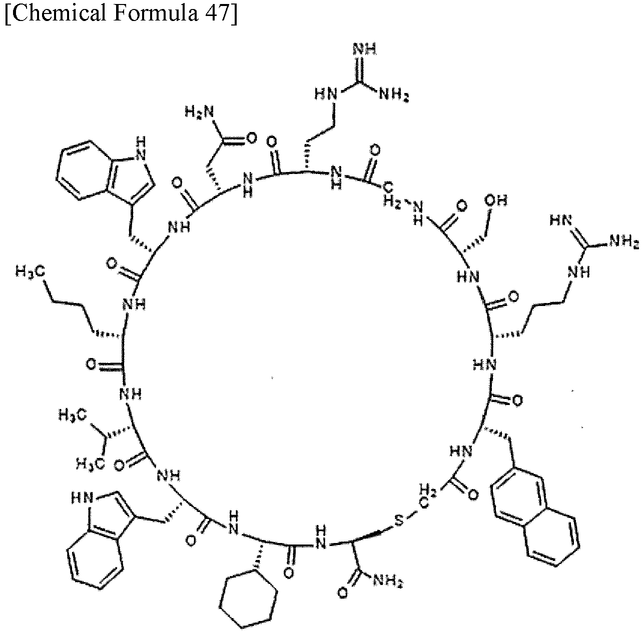 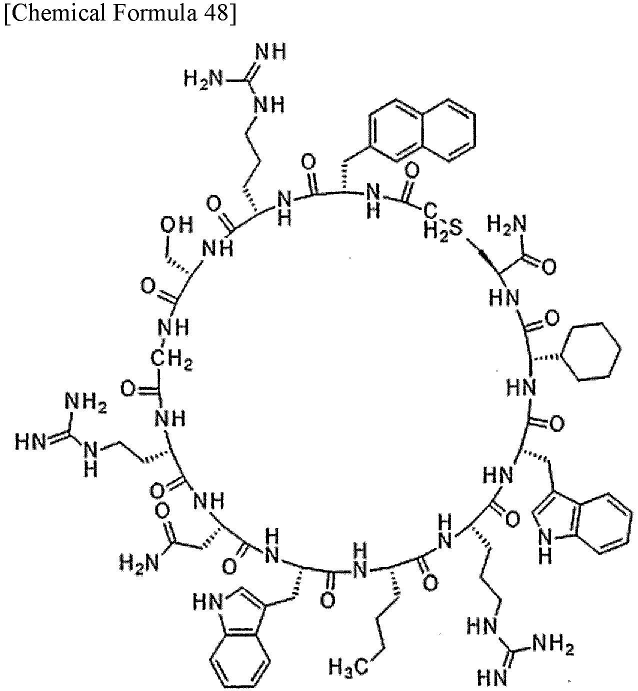 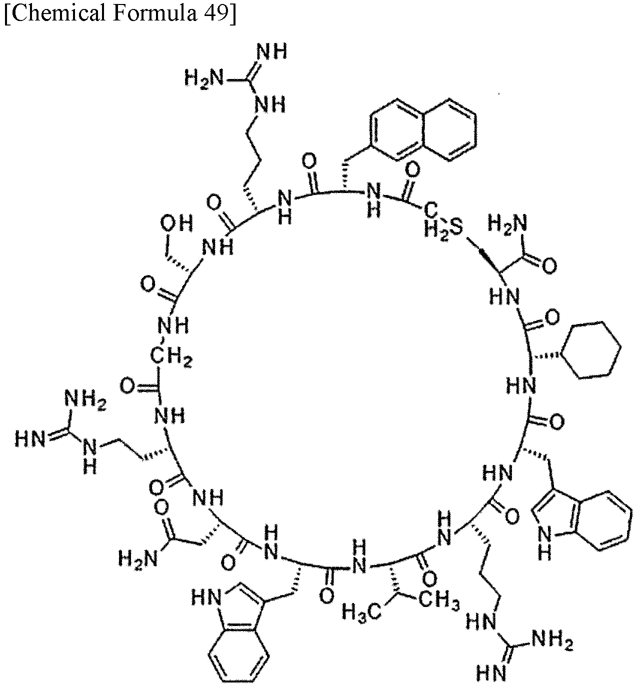 and

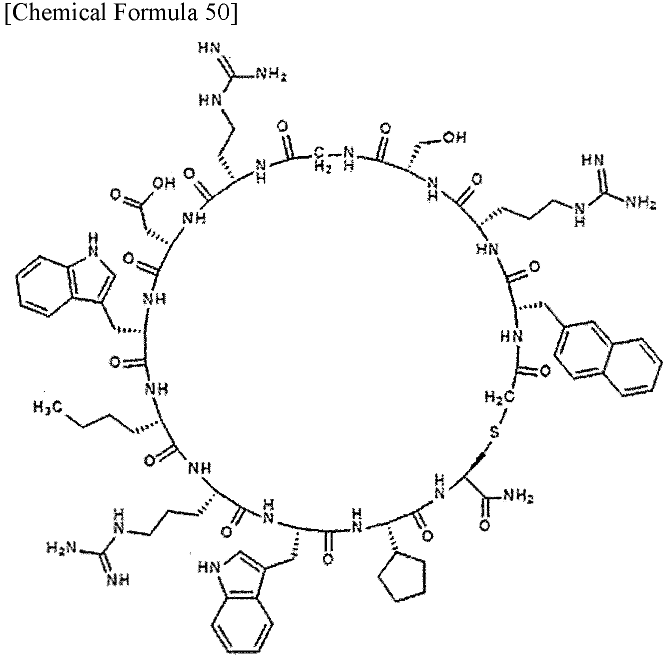

15. The conjugate or pharmaceutically acceptable salt thereof according to any one of claims 11 to 14, wherein the carrier molecule is human serum albumin or a fragment or variant thereof.

16. The conjugate or pharmaceutically acceptable salt thereof according to any one of claims 11 to 14, wherein the carrier molecule is an Fc-containing molecule.

17. The conjugate or pharmaceutically acceptable salt thereof according to claim 16, wherein the Fc-containing molecule is a full-length IgG, a full-length IgG heavy chain, a fragment of IgG comprising all or part of an Fc region, or a variant thereof.

18. The conjugate or pharmaceutically acceptable salt thereof according to claim 16 or 17, wherein the Fc-containing molecule comprises CH2 and CH3 domains of a constant region of an IgG heavy chain.

19. The conjugate or pharmaceutically acceptable salt thereof according to any one of claims 16 to 18, wherein the Fc-containing molecule comprises at least one amino acid residue of a cysteine residue corresponding to position 226, a cysteine residue corresponding to position 229, and an asparagine residue corresponding to position 297 according to the Eu index in a human IgG heavy chain.

20. The conjugate or pharmaceutically acceptable salt thereof according to any one of claims 16 to 19, wherein the Fc-containing molecule comprises an amino acid sequence corresponding to positions 221 to 230, 222 to 230, 223 to 230, 224 to 230, 225 to 230, or 226 to 230 according to the EU index in a human IgG heavy chain.

21. The conjugate or pharmaceutically acceptable salt thereof according to any one of claims 16 to 20, wherein the Fc-containing molecule comprises substitutions of a leucine residue corresponding to position 234 and a leucine residue corresponding to position 235 according to the Eu index in a human IgG heavy chain with alanine residues, or substitutions of a leucine residue corresponding to position 234, a leucine residue corresponding to position 235, and a proline residue corresponding to position 329 according to the Eu index in a human IgG heavy chain with alanine residues.

22. The conjugate or pharmaceutically acceptable salt thereof according to any one of claims 16 to 21, wherein the Fc-containing molecule is CH consisting of a constant region of a human IgG heavy chain, CLCH consisting of a constant region of a human IgG, or a molecule consisting of an Fc region of a human IgG or a fragment thereof, or a variant thereof.

23. The conjugate or pharmaceutically acceptable salt thereof according to claim 22, wherein the Fc-containing molecule is
an antibody (mAb-A) consisting of a combination of a heavy chain consisting of an amino acid sequence at amino acid positions 20 to 474 of SEQ ID NO: 125 and a light chain consisting of an amino acid sequence at amino acid positions 21 to 234 of SEQ ID NO: 127;
CH (CH-A) consisting of an amino acid sequence at amino acid positions 20 to 349 of SEQ ID NO: 129;
CH (CH-B) consisting of an amino acid sequence at amino acid positions 20 to 349 of SEQ ID NO: 133;
CLCH (CLCH-A) consisting of a combination of a heavy chain consisting of an amino acid sequence at amino acid positions 20 to 349 of SEQ ID NO: 129 and a light chain consisting of an amino acid sequence at amino acid positions 21 to 125 of SEQ ID NO: 131;
CLCH (CLCH-B) consisting of a combination of a heavy chain consisting of an amino acid sequence at amino acid positions 20 to 349 of SEQ ID NO: 133 and a light chain consisting of an amino acid sequence at amino acid positions 21 to 125 of SEQ ID NO: 131;
an Fc fragment (Fc-B) consisting of an amino acid sequence at amino acid positions 21 to 243 of SEQ ID NO: 135;
an Fc fragment (Fc-A) consisting of an amino acid sequence at amino acid positions 21 to 247 of SEQ ID NO: 137; or
an Fc fragment (Fc-C) consisting of an amino acid sequence at amino acid positions 21 to 247 of SEQ ID NO: 139;
or
a variant thereof.

24. The conjugate or pharmaceutically acceptable salt thereof according to any one of claims 16 to 23, wherein the Fc-containing molecule comprises one or two or more modifications selected from the group consisting of N-linked glycosylation, O-linked glycosylation, N-terminal processing, C-terminal processing, deamidation, isomerization of aspartic acid, oxidation of methionine, addition of a methionine residue to the N-terminus, amidation of a proline residue, and a deletion of one or two amino acid residues at the carboxyl terminus of a heavy chain.

25. The conjugate or pharmaceutically acceptable salt thereof according to claim 24, wherein one or two amino acid residues are deleted at the carboxyl terminus of a heavy chain of the Fc-containing molecule.

26. The conjugate or pharmaceutically acceptable salt thereof according to claim 25, wherein one amino acid residue is deleted at the carboxyl terminus of each of two heavy chains of the Fc-containing molecule.

27. The conjugate or a pharmaceutically acceptable salt thereof according to any one of claims 24 to 26, wherein the proline residue at the carboxyl terminus of a heavy chain of the Fc-containing molecule is further amidated.

28. The conjugate or pharmaceutically acceptable salt thereof according to any one of claims 11 to 27, wherein the cyclic peptide and the carrier molecule are bonded to each other via a linker structure.

29. The conjugate or pharmaceutically acceptable salt thereof according to claim 28, wherein the linker structure comprises at least one structure selected from the group consisting of a polyoxyalkylene chain, an amino acid residue, and an oligopeptide chain consisting of two or more amino acid residues.

30. The conjugate or pharmaceutically acceptable salt thereof according to claim 29, wherein the polyoxyalkylene chain contained in the linker structure is a polyethylene glycol (PEG) chain.

31. The conjugate or pharmaceutically acceptable salt thereof according to claim 30, wherein the total degree of polymerization of the PEG chain contained in backbone of the linker structure is 6 to 100.

32. The conjugate or pharmaceutically acceptable salt thereof according to claim 29, wherein the amino acid residue contained in the linker structure is a residue of Gly, N-methylglycine, Asp, D-Asp, Glu, D-Glu, Lys, D-Lys, β-alanine, Ser, D-Ser, a non-natural amino acid comprising a polyoxyalkylene chain in a side chain, or a non-natural amino acid comprising an SG chain in a side chain.

33. The conjugate or pharmaceutically acceptable salt thereof according to claim 29, wherein the oligopeptide chain contained in the linker structure consists of 2 to 20 amino acid residues.

34. The conjugate or pharmaceutically acceptable salt thereof according to claim 29, wherein the oligopeptide chain contained in the linker structure comprises one or more amino acid residues selected from the group consisting of Gly, N-methylglycine, Asp, D-Asp, Glu, D-Glu, Lys, D-Lys, β-alanine, Ser, D-Ser, a non-natural amino acid comprising a polyoxyalkylene chain in a side chain, and a non-natural amino acid comprising an SG chain in a side chain.

35. The conjugate or pharmaceutically acceptable salt thereof according to claim 29, wherein an N-terminal amino group of the amino acid residue or oligopeptide chain contained in the linker structure forms an amide bond with a carbonyl group to which R¹⁰ of the ring-forming group A or R¹¹⁰ of the ring-forming group B is bonded, where R¹⁰ or R¹¹⁰ is substituted with NH of the N-terminal amino group.

36. The conjugate or pharmaceutically acceptable salt thereof according to any one of claims 28 to 35, wherein the linker structure comprises a linking group C that binds to the carrier molecule.

37. The conjugate or pharmaceutically acceptable salt thereof according to claim 36, wherein the linking group C comprises a maleimide group-derived moiety in a thioether bond formed by reaction of a maleimide group with a thiol group or 1,2,3-triazole ring.

38. The conjugate or pharmaceutically acceptable salt thereof according to claim 37, wherein a maleimide group is bonded to a thiol group of a cysteine residue of the carrier molecule to form a thioether bond.

39. The conjugate or pharmaceutically acceptable salt thereof according to claim 37, wherein the carrier molecule is an Fc-containing molecule, wherein the 1,2,3-triazole ring contained in the linking group C is bonded to a glycan which is bonded to an asparagine residue corresponding to position 297 according to the Eu index of the Fc-containing molecule (N297-linked glycan).

40. The conjugate or pharmaceutically acceptable salt thereof according to any one of claims 36 to 39, wherein
the linking group C is represented by any one of the following formulas: 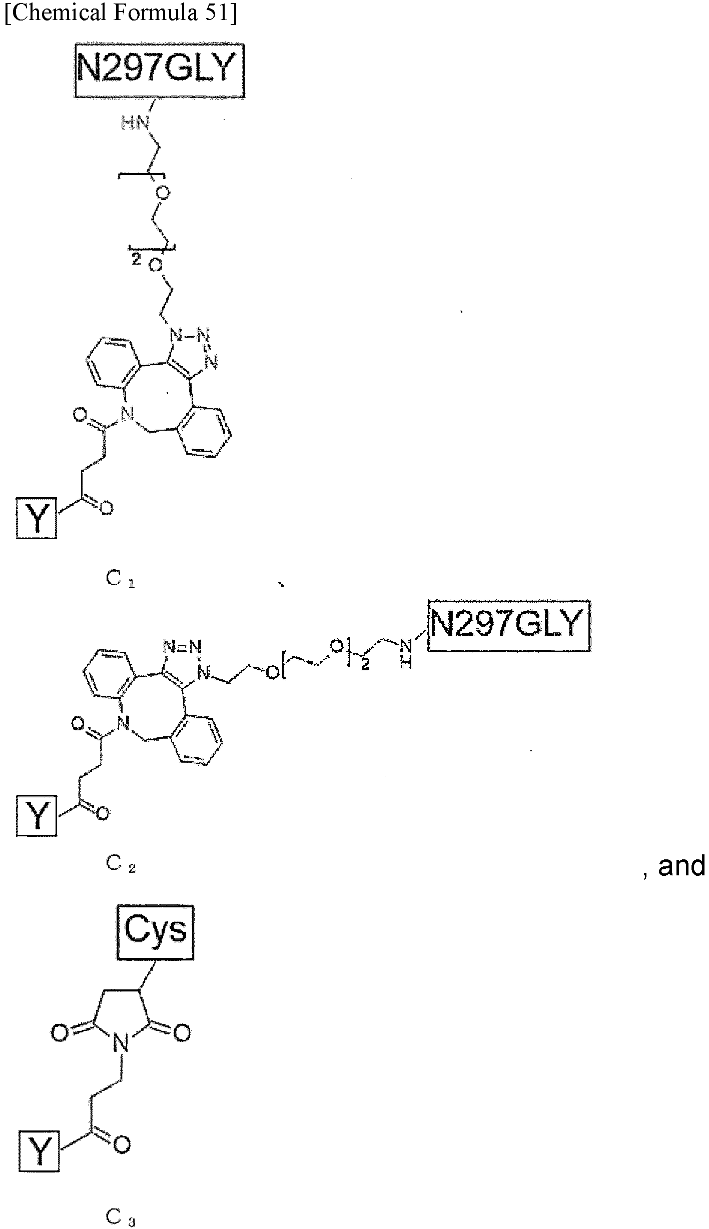
(wherein
N297GLY represents binding to the non-reducing terminus of N297-linked glycan of the Fc-containing molecule;
Cys represents binding to the thiol group of a cysteine residue of the carrier molecule; and
Y represents a point of attachment to an adjacent amino acid residue in the linker structure).

41. The conjugate or pharmaceutically acceptable salt thereof according to claim 39 or 40, wherein the N297-linked glycan of the Fc-containing molecule is at least one selected from glycans N297-(Fuc) SG, N297-(Fuc)MSG1, and N297-(Fuc)MSG2 having structures represented by the following formulas: 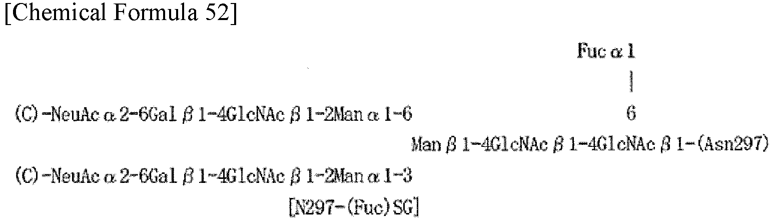 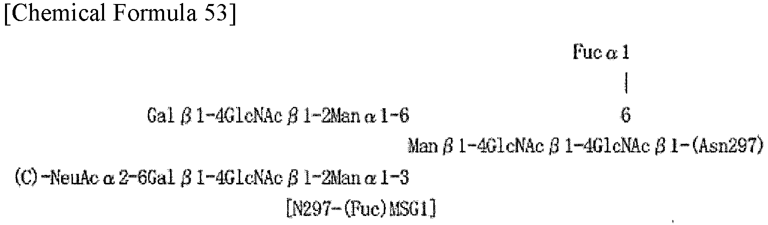 and 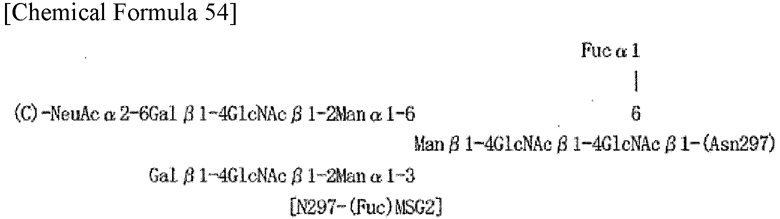 [wherein (C) represents a point of attachment to the linking group C and (Asn297) represents a point of attachment to the asparagine residue corresponding to position 297 according to the Eu index of the Fc-containing molecule].

42. The conjugate or pharmaceutically acceptable salt thereof according to claim 41, wherein the N297-linked glycan of the Fc-containing molecule is N297-(Fuc)SG.

43. The conjugate or pharmaceutically acceptable salt thereof according to any one of claims 36 to 42, wherein the linker structure is represented by formula (XX)
-L1-L2-L3-C-
[wherein
L1 is an oligopeptide containing 3 to 6 neutral amino acid residues bonded in a linear manner;
L2 is L2a or L2b, wherein
L2a is a non-natural amino acid residue comprising a polyoxyalkylene chain in backbone, or an oligopeptide comprising a non-natural amino acid residue comprising a polyoxyalkylene chain in backbone, wherein the total degree of polymerization of the polyoxyalkylene chain contained in backbone of L2a is 6 to 100, and
L2b is an oligopeptide comprising 10 to 20 natural or non-natural α-amino acid residues bonded in a linear manner;
L3 is an amino acid residue represented by the formula 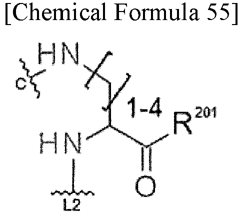
(wherein R²⁰¹ is a hydroxy group or an amino group, 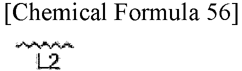
represents a point of attachment to an adjacent amino acid residue in L2, and 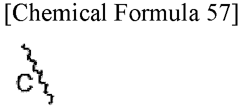
represents a point of attachment to the linking group C);
C is the linking group C;
the N-terminal amino group of L1 forms an amide bond with a carbonyl group to which R¹⁰ of the ring-forming group A or R¹¹⁰ of the ring-forming group B is bonded, where R¹⁰ or R¹¹⁰ is substituted with NH of the N-terminal amino group;
L1 and L2 are joined together by an amide bond; and
L2 and L3 are joined together by an amide bond].

44. The conjugate or pharmaceutically acceptable salt thereof according to claim 43, wherein the neutral amino acid residue contained in L1 is a residue of one or more amino acids selected from the group consisting of Gly, Ser, D-Ser, N-methylglycine (NMeG), and β-alanine (bAla).

45. The conjugate or pharmaceutically acceptable salt thereof according to claim 39 or 44, wherein the polyoxyalkylene chain in backbone, which is contained in L2a, is a PEG chain.

46. The conjugate or pharmaceutically acceptable salt thereof according to claim 45, wherein the non-natural amino acid residue comprising a PEG chain in backbone, which is contained in L2a, is a residue of an amino acid represented by the formula 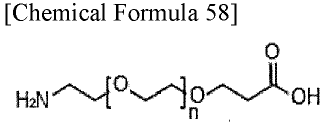 [wherein n represents an integer of 5 to 40].

47. The conjugate or pharmaceutically acceptable salt thereof according to claim 46, wherein the total degree of polymerization of the PEG chain is 10 to 40.

48. The conjugate or pharmaceutically acceptable salt thereof according to claim 43 or 44, wherein an α-amino acid of L2b is glycine or N-methylglycine.

49. The conjugate or pharmaceutically acceptable salt thereof according to claim 48, wherein the oligopeptide of L2b is oligo-N-methylglycine with 13 consecutive N-methylglycines.

50. The conjugate or pharmaceutically acceptable salt thereof according to any one of claims 43 to 49, wherein L2 comprises a non-natural amino acid residue comprising a PEG chain in a side chain or a non-natural amino acid residue comprising an SG chain in a side chain.

51. The conjugate or pharmaceutically acceptable salt thereof according to claim 50, wherein the non-natural amino acid comprising a PEG chain in a side chain is KPEG.

52. The conjugate or pharmaceutically acceptable salt thereof according to claim 50, wherein the non-natural amino acid comprising an SG chain in a side chain is KSG.

53. The conjugate or pharmaceutically acceptable salt thereof according to any one of claims 43 to 52, wherein a total of 1 to 3 acidic amino acid residues is contained at one or more positions not adjacent to the ring-forming group A or the ring-forming group B in the main chain of the oligopeptide of L1 and L2.

54. The conjugate or pharmaceutically acceptable salt thereof according to claim 53, wherein the acidic amino acid residue is an amino acid selected from the group consisting of Asp, D-Asp, Glu, and D-Glu.

55. The conjugate or pharmaceutically acceptable salt thereof according to any one of claims 43 to 54, wherein L1 is an oligopeptide comprising or consisting of the sequence
Gly-Gly-Gly-Gly,
Gly-Ser-Gly-Ser,
NMeG-NMeG-NMeG-NMeG,
bAla-bAla-bAla-bAla, or
Gly-Gly-Glu-Gly-Gly.

56. The conjugate or pharmaceutically acceptable salt thereof according to claim 55, wherein L1 is an oligopeptide consisting of a sequence of two acidic amino acid residues coupled to the C-terminus of
Gly-Gly-Gly-Gly,
Gly-Ser-Gly-Ser,
NMeG-NMeG-NMeG-NMeG, or
bAla-bAla-bAla-bAla.

57. The conjugate or pharmaceutically acceptable salt thereof according to claim 56, wherein the acidic amino acid residue is an Asp residue.

58. The conjugate or pharmaceutically acceptable salt thereof according to any one of claims 43 to 55, wherein L2 is an oligopeptide consisting of the sequence
Asp-Asp-KPEG-P12P-P12P,
KSG-P12P-P12P,
Asp-Asp-P12P,
Asp-Asp-P12P-P12P, or
Asp-Asp-P12P-KPEG-P12P.

59. The conjugate or pharmaceutically acceptable salt thereof according to any one of claims 43 to 58, wherein L3 is a Lys residue.

60. The conjugate or pharmaceutically acceptable salt thereof according to claim 43, wherein the linker structure is
a structure in which L1 is an oligopeptide consisting of the sequence Gly-Gly-Gly-Gly or Gly-Ser-Gly-Ser, L2a is an oligopeptide consisting of the sequence Asp-Asp-KPEG-P12P-P12P, and L3 is a Lys residue;
a structure in which L1 is an oligopeptide consisting of the sequence Gly-Gly-Gly-Gly, L2a is an oligopeptide consisting of the sequence KSG-P12P-P12P, and L3 is a Lys residue;
a structure in which L1 is an oligopeptide consisting of the sequence Gly-Gly-Gly-Gly, L2a is an oligopeptide consisting of the sequence Asp-Asp-P12P, and L3 is a Lys residue;
a structure in which L1 is an oligopeptide consisting of the sequence Gly-Ser-Gly-Ser, L2a is an oligopeptide consisting of the sequence Asp-Asp-P12P-P12P, and L3 is a Lys residue; or
a structure in which L1 is an oligopeptide consisting of the sequence Gly-Gly-Gly-Gly, L2a is an oligopeptide consisting of the sequence Asp-Asp-P12P-KPEG-P12P, and L3 is a Lys residue.

61. The conjugate or pharmaceutically acceptable salt thereof according to claim 11, wherein the cyclic peptide and the linker structure are represented by any of the formulas: 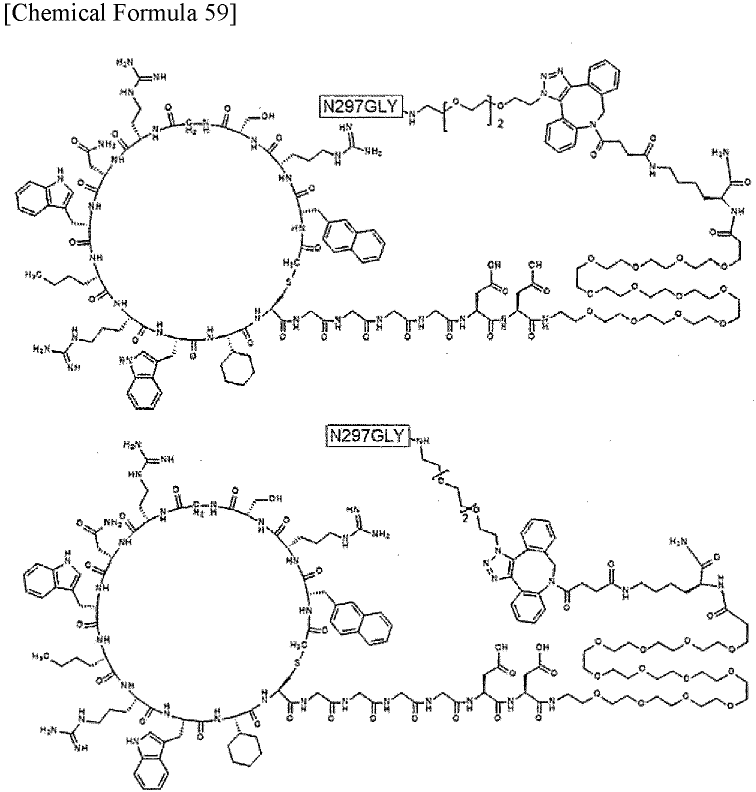 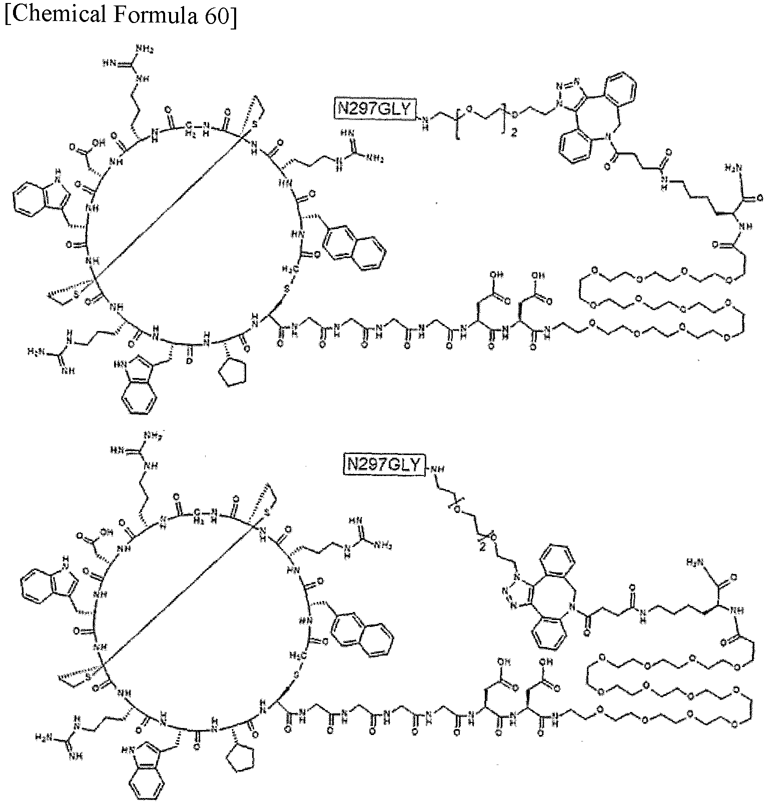 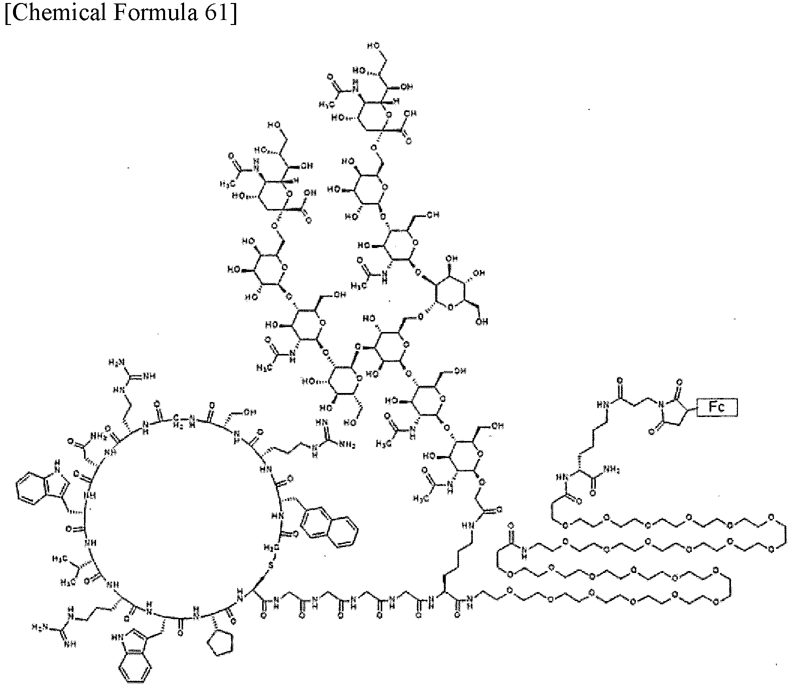 and 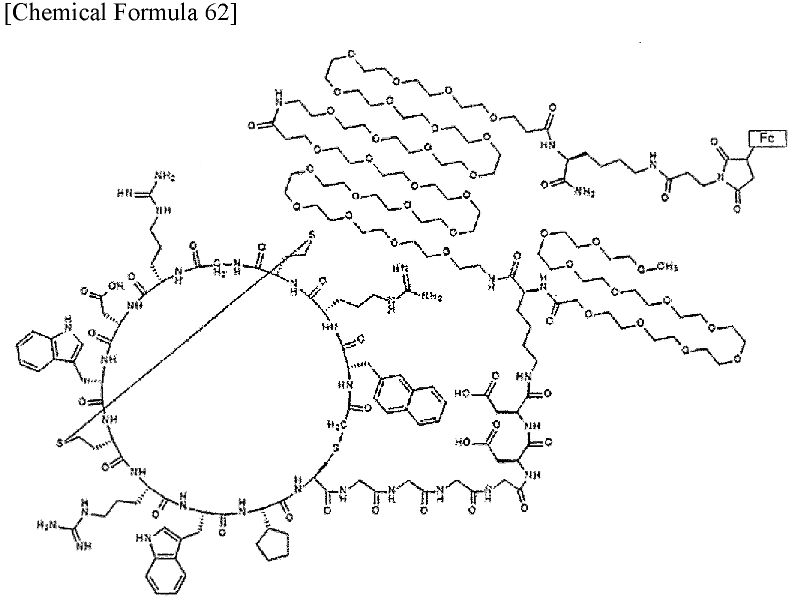 (wherein
N297GLY represents binding of 1,2,3-triazole ring contained in the linking group C to the non-reducing terminus of N297-linked glycan of the Fc-containing molecule; and
Fc represents binding of the maleimide group contained in the linking group C to the thiol group of a cysteine residue of the Fc-containing molecule).

62. A pharmaceutical composition comprising, as an active component, the conjugate or pharmaceutically acceptable salt thereof according to any one of claims 11 to 61.

63. The pharmaceutical composition according to claim 62, for use in the treatment or prophylaxis of a disease that can be treated or prevented by inhibiting a function of TSP1.

64. The pharmaceutical composition according to claim 63, for use in the treatment or prophylaxis of critical limb ischemia, peripheral arterial disease, or chronic limb threatening ischemia.

65. A blood flow improving agent comprising, as an active component, the conjugate or pharmaceutically acceptable salt thereof according to any one of claims 11 to 61.

66. A method for producing the conjugate or pharmaceutically acceptable salt thereof according to any one of claims 11 to 61, the method comprising the steps of:
(1) synthesizing an oligopeptide X-A-L or Z-B-L represented by the following formula from the C-terminal side by a solid-phase synthesis method:
Xₐₐ₁-Xₐₐ₂-Xₐₐ₃-Xₐₐ₄-Xₐₐ₅-Xₐₐ₆-Xₐₐ₇-Xₐₐ₈-Xₐₐ₉-Xₐₐ₁₀-Xₐₐ₁₁-A₀-L1-L2-L3 or
Zₐₐ₁-Zₐₐ₂-Zₐₐ₃-Zₐₐ₄-Zₐₐ₅-Zₐₐ₆-Zₐₐ₇-Zₐₐ₈-Zₐₐ₉-Zₐₐ₁₀-Zₐₐ₁₁-Zₐₐ₁₂-B₀-L1-L2-L3
wherein
Xₐₐ₁ to Xₐₐ₁₁ are as defined in claim 1 (provided that in the structure of Xₐₐ₃ and Xₐₐ₈, thiol groups remain as they are without forming a bond);
L1 to L3 are as defined in claim 43 (provided that when L2 comprises a residue of a non-natural amino acid comprising a glycan in a side chain, the residue is replaced with a Lys residue);
Zₐₐ₁ to Zₐₐ₁₂ are as defined in claim 11;
A₀ is a residue represented by the formula 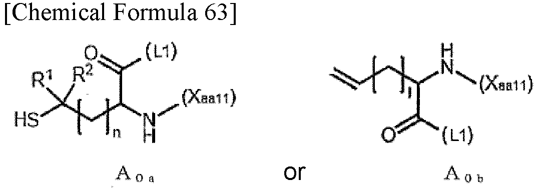
(wherein (Xₐₐ₁₁) represents a point of attachment to Xₐₐ₁₁ (L1) represents a point of attachment to the N-terminal amino acid residue in L1, and 1 and n are as defined in claim 1; B₀ is a residue represented by the formula 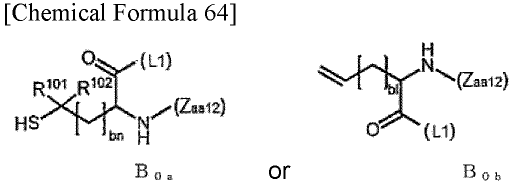
(wherein (Zₐₐ₁₂) represents a point of attachment to Zₐₐ₁₂, (L1) represents a point of attachment to the N-terminal amino acid residue in L1, and bl and bn are as defined in claim 11),
(2) cyclizing the oligopeptide synthesized in step (1) to afford a cyclic peptide by
(a) joining the N-terminal amino group of Xₐₐ₁ to A₀ₐ to form any of the ring-forming groups A₁ to A₃ (which are coupled to L1 at the position of R¹⁰) in the oligopeptide X-A-L;
(b) joining the N-terminal amino group of Xₐₐ₁ to A_{0b} to form the ring-forming group A₄ or A₅ (which are coupled to L1 at the position of R¹⁰) in the oligopeptide X-A-L;
(c) joining the N-terminal amino group of Zₐₐ₁ or Zₐₐ₂ to B₀ₐ to form any of the ring-forming groups B₁ to B₄ (which are coupled to L1 at the position of R¹¹⁰) in the oligopeptide Z-B-L; or
(d) joining the N-terminal amino group of Zₐₐ₁ or Zₐₐ₂ to B_{0b} to form the ring-forming group B₅ or B₆ (which is coupled to L1 at the position of R¹¹⁰) in the oligopeptide Z-B-L,
(3) when the oligopeptide X-A-L is synthesized in step (1), allowing the thiol groups of Xₐₐ₃ and Xₐₐ₈ of the oligopeptide X-A-L cyclized in step (2) to form a bond represented by -S-S-, - S-CH₂-S-, or -S-X-S- (wherein X is as defined in claim 1) to afford a bicyclic peptide;
(4) when L2 comprises a residue of a non-natural amino acid comprising a glycan in a side chain, which is replaced with a Lys residue in the oligopeptide X-A-L or Z-B-L, introducing a glycan into the Lys residue to thereby form a residue of a non-natural amino acid comprising a glycan in a side chain;
(5) binding a reactive group capable of forming a maleimide group or a 1,2,3-triazole ring to L3; and
(6) binding the cyclic peptide to the carrier molecule via the reactive group of step (5).
